# EUROPEAN PATENT APPLICATION

(11) **EP 0 994 104 A1**
(43) Date of publication of application: **19.04.2000**
(21) Application number: 97928467.6
(22) Date of filing: 25.06.1997
(51) Int. Cl.: C07C 317/44, C07C 323/62, C07D 209/18, C07D 409/12, C07D 405/12, C07D 413/12, C07D 263/57, C07D 307/79, C07D 333/24, A61K 31/405, A61K 31/42

(54) **ARYL (SULFIDE, SULFOXIDE AND SULFONE) DERIVATIVES AND DRUGS CONTAINING THE SAME AS THE ACTIVE INGREDIENT**

(30) Priority: 27.06.1996 JP 18537096
(71) Applicant: ONO PHARMACEUTICAL CO., LTD., Osaka-shi, Osaka 541-8526 (JP)
(72) Inventor: TAKAHASHI, Kanji, ONO Pharmaceutical Co., Ltd., Shimamoto-cho, Mishima-gun, Osaka 618 (JP); SUGIURA, Tsuneyuki, ONO Pharmaceutical Co., Ltd., Shimamoto-cho, Mishima-gun, Osaka 618 (JP)
(74) Representative: Bentham, Stephen
(86) International application number: JP9702200
(87) International publication number: WO9749679

(57) **Abstract**

Pharmaceutical composition containing aryl (sulfide, sulfoxide, sulfone) derivatives of the formula (I) and the salts thereof as active ingredient (wherein R¹ is H, alkyl ; R² is COOR⁷, CONHOR⁸ ; E is -CONR⁹-, -NR⁹CO-, -OCO-, -COO-, -CH₂-O-, -(CH₂)₂-, vinylene, ethynylene ; J is bond, alkylene ; A is H, alkyl, Ar, alkyl-OH ; R³, R⁴ is H, alkyl, COOR¹⁹, hydroxy, -NR²⁰R²¹, 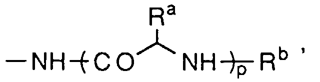 Ar₁ etc.) ; R⁵, R⁶ is H, methyl) and the novel aryl (sulfide, sulfoxide, sulfone) derivatives of the formula (I).

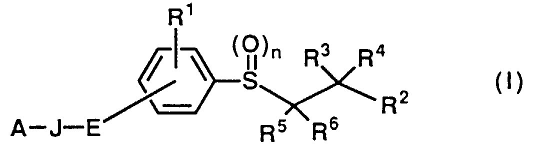

The compounds of the formula (I) have inhibitory activity against matrix metalloproteinases, therefore, the compounds of the formula (I) are useful for prevention and / or treatment of rheumatoid diseases, arthrosteitis, unusual bone resorption, osteoporosis, periodontitis, interstital nephritis, arteriosclerosis, pulmonary emphysema, cirrhosis, cornea injury, autoimmune diseases, diseases caused by vascular emigration or infiltration of leukocytes, arterialization etc.

## Description

### Field of the Invention

This invention relates to aryl (sulfide, sulfoxide, sulfone) derivatives, processes for the preparation thereof, and matrix metalloproteinase inhibitors containing them as active ingredient.

More particularly, this invention relates to matrix metalloproteinase inhibitors containing aryl (sulfide, sulfoxide, sulfone) derivatives of the formula (I) 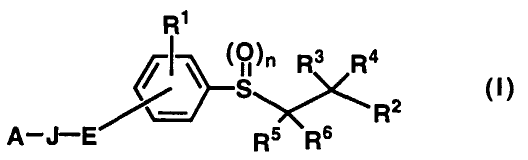 (wherein all the symbols have the same meanings as hereinafter described.), non-toxic salts thereof, as active ingredient, and the above-mentioned novel aryl (sulfide, sulfoxide, sulfone) derivatives of the formula (I), non-toxic salts thereof, and processes for the preparation thereof.

### Background of the Invention

The matrix metalloproteinases (hereinafter abbreviated as MMP) are neutral metalloproteinases and zinc (hereinafter abbreviated as Zn²⁺) is essential in the active site for their activation. They degrade collagen, laminin, proteoglycans, fibronectin, elastin, gelatin etc. under physiological conditions and, therefore, are effective on growth and tissue remodeling of articulation tissue, bone tissue and connective tissue. At least 10 classes of MMP which differ in primary structure are identified.

As common characteristics of these enzymes, MMP
(1) have Zn²⁺ in the active site and the activity depends on calcium (Ca²⁺),
(2) are secreted as an inactive proenzyme and activated outside of cells,
(3) have high homology on amino acid sequence,
(4) have an ability to degrade various extracellular matrix components in vivo,
(5) are regulated by tissue inhibitors of metalloproteinases (TIMP) which are specific to MMP.

MMP inhibitors are useful for prevention and / or treatment of various diseases induced by overexpression or excess activation of MMP. Such diseases are, for example, rheumatoid diseases, arthrosteitis, unusual bone resorption, osteoporosis, periodontitis, interstitial nephritis, arteriosclerosis, pulmonary emphysema, cirrhosis, cornea injury, metastasis of, invasion of or growth of tumor cells, autoimmune diseases (e.g. Crohn's disease, Sjogren's syndrome), diseases caused by vascular emigration or infiltration of leukocytes, arterialization etc.

Some compounds possessing inhibitory activity against matrix metalloproteinase are known. A sequence in the vicinity of the cleavage site of collagen (Gly-Ile-Ala-Gly or Gly-Leu-Ala-Gly) has high affinity for collagenase. Much research and development on substrate analogous matrix metalloproteinase inhibitors, which are chemically modified so as to have zinc affinity groups on a cleavage site of the substrate, has energetically been carried out [Inhibitors of matrix metalloproteinases (MMP's), Nigel RA Beeley, Phillip RJ Ansell, Andrew JP Docherty et al., Curr. Opin. Ther. Patents, 4, 7-16 (1994), Current Drugs Ltd ISSN 0962-2594]. However, these substrate-analogous inhibitors might have various problems. Therefore, it is desired to obtain a non-peptide inhibitor and some compounds are reported.

For example, (1) in the specification of EP 606046, arylsulfonamide derivatives of the formula (X) 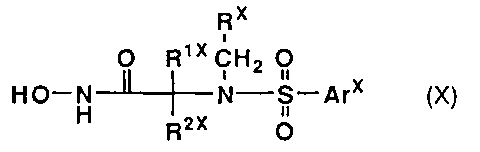 (wherein (a) Ar^{X} is carbocyclic or heterocyclic aryl; R^{X} is hydrogen, lower alkyl, carbocyclic aryl-lower alkyl etc.; R^{1X} is hydrogen, lower alkyl, carbocyclic aryl-lower alkyl etc.; R^{2X} is hydrogen, lower alkyl; or (b) R^{X} and R^{1X} taken together with the chain to which they are attached form 1, 2, 3, 4-tetrahydroisoquinoline, piperidine etc.; Ar^{X} and R^{2X} are as defined in (a); or (c) R^{1X} and R^{2X} taken together with the carbon to which they are attached form C3-7 cycloalkane, oxa-cyclohexane, thia-cyclohexane etc. which is unsubstituted or substituted by lower alkyl; and Ar^{X} and R^{2X} are as defined in (a).) are disclosed to have inhibitory activity against matrix metalloproteinase.
(2) In the specification of WO 9535276, the compounds of the formula (Y) 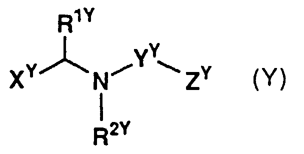 (wherein X^{Y} is COOH, CONHOH; R^{1Y} is α-amino acid; R^{2Y} is Z^{1Y}Q^{Y}W^{Y}; Z^{1Y} is hydrogen, aryl etc.; (i) Q^{Y}W^{Y} together form bond, (ii) Q^{Y} is O, S, W^{Y} is C1-20 alkyl etc., (iii) Q^{Y} is bond, W^{Y} is C9-20 alkyl etc., (iv) Q^{Y} is bond, W^{Y} is C1-8 alkyl; Y^{Y} is SO₂; Z^{Y} is aryl, heteroaryl.) are disclosed to have inhibitory activity against matrix metalloproteinase.
(3) In the specification of WO 9615096, the compounds of the formula (Z)

   (T^{Z})x^{Z}A^{Z}-B^{Z}-D^{Z}-E^{Z}-G^{Z} (Z)

   (wherein (T^{Z})x^{Z}A^{Z} is unsubstituted or substituted various aromatic ring or aromatic hetero ring; B^{Z} is various aromatic ring or aromatic hetero ring; D^{Z} is -CO-, -CH(OH)-, -CH₂- etc.; E^{Z} is Cn carbon chain optionally having R^{6Z} (in which R^{6Z} is -(CH₂)v^{Z}Z^{Z}R^{8Z} (in which v^{Z} is 0, integer of 1 ∼ 4; Z^{Z} is -S-, -SO-, -SO₂- etc.; R^{8Z} is optionally substituted C6-10 aryl etc.)); G^{Z} is carboxyl, alkoxycarbonyl.)
   are disclosed to have inhibitory activity against matrix metalloproteinase.
(4) In the specification of WO 9509841, the compounds of the formula (E) 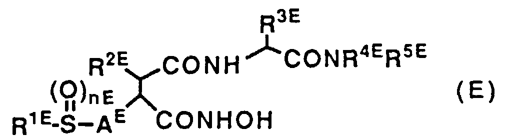 (wherein R^{1E} is phenyl optionally having substituent etc.; R^{2E} is hydrogen, C1-6 alkyl etc.; R^{3E} is amino acid residue optionally having substituent; R^{4E} is hydrogen, C1-6 alkyl etc.; R^{5E} is hydrogen, methyl; n^{E} is 0, 1, 2; A^{E} is C1-6 hydrocarbon chain.)
   are disclosed to have inhibitory activity against the liberation of TNF, and inhibitory activity against matrix metalloproteinase.
(5) In the specification of WO 9324449, the compounds of the formula (F) 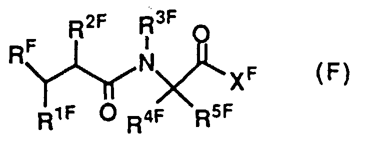 (wherein R^{F} is -CONHOH, ,carboxyl, ,esterified carboxyl etc.; R^{1F} is hydrogen, optionally substituted alkyl, alkenyl, aryl, aralky, heteroaralkyl, heteroarylthioalkyl; R^{2F} is optionally substituted arylthio, arylthioalkyl etc.; R^{3F} is hydrogen, alkyl; R^{4F} is hydrogen, alkyl; R^{5F} is optionally substituted alkyl etc.) are disclosed to have inhibitory activity against matrix metalloproteinase.
(6) In the specification of WO 9616027, the compounds of the formula (G) 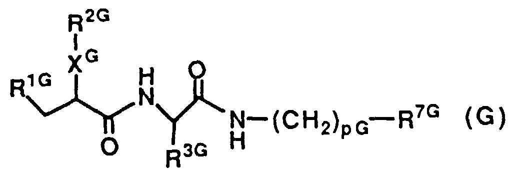 (wherein R^{1G} is -CONHOH, carboxyl, alkoxycarbonyl, aryloxycarbonyl, benzyloxycarbonyl etc.; R^{2G} is aryl etc.; R^{3G} is alkyl etc.; R^{7G} is aryl etc.; X^{G} is -(CH₂)m^{G}Y^{G}(CH₂)n^{G} (in which Y^{G} is S etc.; m^{G}, n^{G}, p^{G} is 0 ∼ 4.) are disclosed to have inhibitory activity against matrix metalloproteinase.
   Also, (7) in the specification of Japanese Patent Kokai No. 4-226939 and (8) Japanese Patent Kokai No. 4-293576, each of the compound of the formula (W-1) 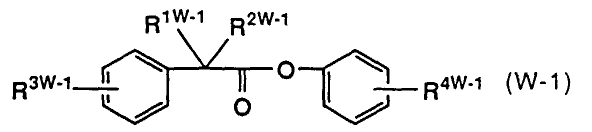 (wherein R^{1W-1}, R^{2W-1} is hydrogen, C1-6 alkyl, C3-6 cycloalkyl, or together form methylene, ethylene, polymethylene; R^{3W-1} is hydrogen, halogen, haloalkyl, C1-12 alkyl, C1-12 alkoxy etc.; R^{4W-1} is hydrogen, halogen, nitro, -C(O)CH₃, S(O)ₚR^{9W-1} (in which p is 0, 1, 2, R^{9w-1} is hydroxy, -ONa, optionally substituted C1-12 alkyl, cycloalkyl)),
   and the compounds of the formula (W-2) 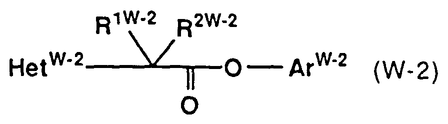 (wherein R^{1W-2}, R^{2W-2} is hydrogen, C1-4 alkyl, C3-6 cycloalkyl, or together form methylene, ethylene, polymethylene; Ar^{W-2} is optionally substituted phenyl; HET^{W-2} is hetero ring containing nitrogen, sulfur or oxygen atom over 1 atom.) are disclosed to have inhibitory activity against elastase.
(9) In the specification of EP 0173516, the compounds of the formula (J) 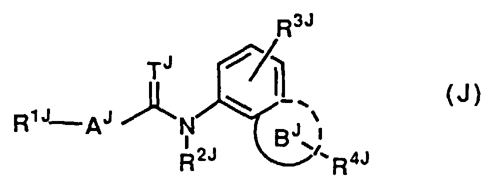 (wherein B^{J} is -SCH₂- etc.; T^{J} is oxygen etc.; R^{1J} is optionally substituted phenyl, naphthyl by R^{5J}, R^{6J}, or C1-20 alkyl, alkenyl, alkynyl; R^{2J} is hydrogen, C1-6 alkyl; R^{3J} is hydrogen, alkyl etc.; R^{4J} is -(CH₂)p^{J}-COOR^{8J} (in which p^{J} is 0 ∼ 10; R^{8J} is hydrogen, C1-6 alkyl.)
   are disclosed to have SRS antagonistic activity or 5α-reductase inhibitory activity.
(10) In the specification of British Patent 2031408, the compounds of the formula (K) 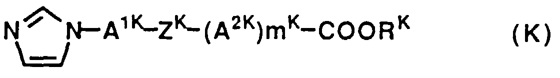 (wherein R^{K} is hydrogen, alkyl; A^{1K}, A^{2K} is alkylene, alkenylene; m^{K} is 0, 1; Z^{K} is 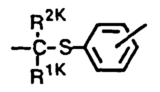 etc.; R^{1K}, R^{2K} is hydrogen, alkyl.)
   are disclosed to have inhibitory activity against TXA₂ synthetase.
(11) In the specification of British Patent 2039903, the compounds of the formula (L) 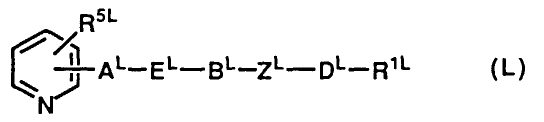 (wherein A^{L} is C1-5 alkylene optionally substituted by hydroxy; E^{L} is 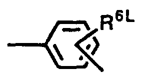 etc.; B^{L} is sulfur etc.; Z^{L} is bond, C≡C, 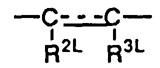 ;D^{L} is bond, C1-5 alkylene; R^{1L} is COOR^{4L} etc.; R^{4L} is hydrogen, C1-12 alkyl etc.)
   are disclosed to have inhibitory activity against TXA₂ synthetase.
(12) In the specification of US Patent 4461905, the compounds of the formula (M) 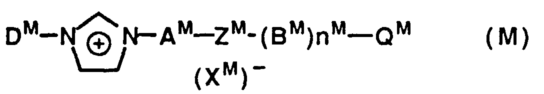 (wherein A^{M}, B^{M} is C1-8 alkylene, alkenylene; D^{M} is C2-10 acyl, C2-7 alkoxycarbonyl etc.; Q^{M} is C2-7 alkoxycarbonyl etc.; X^{M} is halogen; n^{M} is 0, 1; Z^{M} is 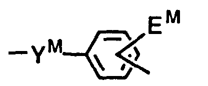 ; E^{M} is hydrogen, C1-6 alkyl etc.; Y^{M} is sulfur etc..)
   are disclosed to have inhibitory activity against TXA₂ synthetase.
(13) In the specification of WO 865779, the compounds of the formula (N) 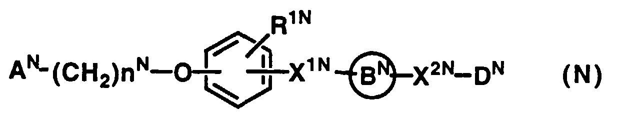 (wherein X^{1N} is -CH₂CH₂-, -CH=CH-, -CH₂-Y^{1N}-, -Y^{1N}-CH₂-, -COY^{2N}-, -Y^{2N}-CO- (in which Y^{1N} is oxygen etc.; Y^{2N} is -NH-, -CH₂Y^{1N}-, -Y^{1N}CH₂-); 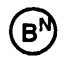 is phenylene etc.; X^{2N} is -Y^{3N}-Y^{4N}- (in which Y^{3N} is sulfur etc.; Y^{4N} is C1-6 alkylene); D^{N} is -COOH, lower alkoxycarbonyl etc.; R^{1N} is hydrogen, lower alkyl; n^{N} is 3 ∼ 10; A^{N} is hydrogen, phenyl, phenoxy.)
   are disclosed to have SRS antagonistic activity.
(14) In the specification of EP 181568, the compounds of the formula (P) 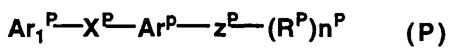 (wherein Ar^{P} is phenyl etc.; Z^{P} is C1-10 alkylene optionally containing 0 ∼ 2 double bonds, and it may be attached to Ar^{P} through sulfur etc.; R^{P} is carboxy, alkoxycarbonyl etc.; n^{P} is 0, 1; X^{P} is -CH=CH-, ethynylene, -COO-, -CONR^{1P}-etc.; Ar₁^{P} is phenyl, hetero ring containing N, S, O atom.)
   are disclosed to have 5-lipoxygenase inhibitory activity.

Also, the following compounds are already known. However, it is not disclosed that these compounds have inhibitory activity against matrix metalloproteinases, and there is no disclosure to suggest that these compounds have the activity thereof (the figure in the parentheses represents Chemical Abstract number.).
(1) 3-(4-methylphenylsulfonyl)propionic acid isopropyl ester (122-323393),
(2) 3-(4-methylphenylsulfonyl)propionic acid phenyl ester (095-006058),
(3) 3-(4-methylphenylsulfonyl)propionic acid sodium salt (094-174529),
(4) 3-(4-methylphenylsulfonyl)propionic acid methyl ester (122-323393),
(5) 3-(4-methylphenylsulfonyl)propionic acid ethyl ester (122-323393),
(6) 3-(4-methylphenylsulfonyl)propionic acid (121-009456),
(7) 3-(4-ethylphenylsulfonyl)propionic acid (100-200853),
(8) 3-(4-methoxyphenylsulfonyl)propionic acid phenyl ester (095-006058),
(9) 3-(4-methoxyphenylsulfonyl)propionic acid,
(10) 3-(4-nitrophenylsulfonyl)propionic acid methyl ester (122-323393),
(11) 3-(4-nitrophenylsulfonyl)propionic acid isopropyl ester (122-323393),
(12) 3-(4-nitrophenylsulfonyl)propionic acid,
(13) 3-(4-aminophenylsulfonyl)propionic acid ethyl ester (115-072840),
(14) 3-(4-aminophenylsulfonyl)propionic acid (085-048254),
(15) 3-(4-hydroxyphenylsulfonyl)propionic acid,
(16) 3-(4-hydroxyphenylsulfonyl)propionic acid phenyl ester (111-164337),
(17) 3-(4-bromophenylsulfonyl)propionic acid methyl ester (066-104778),
(18) 3-(4-bromophenylsulfonyl)propionic acid ethyl ester (066-104778),
(19) 3-(4-bromophenylsulfonyl)propionic acid phenyl ester (095-006058),
(20) 3-(4-chlorophenylsulfonyl)propionic acid methyl ester (066-104778),
(21) 3-(4-chlorophenylsulfonyl)propionic acid ethyl ester (066-104778),
(22) 3-(4-chlorophenylsulfonyl)propionic acid t-butyl ester (122-323393),
(23) 3-(4-chlorophenylsulfonyl)propionic acid isopropyl ester (122-323393),
(24) 3-(4-chlorophenylsulfonyl)propionic acid (101-006755),
(25) 3-(4-chlorophenylsulfonyl)propionic acid phenyl ester (095-006058),
(26) 3-(4-iodophenylsulfonyl)propionic acid ethyl ester (066-104778),
(27) 3-(4-iodophenylsulfonyl)propionic acid methyl ester (066-104778),
(28) 3-(4-acetylaminophenylsulfonyl)propionic acid methyl ester (114-014686),
(29) 3-(4-acetylaminophenylsulfonyl)propionic acid ethyl ester (115-072840),
(30) 3-(4-vinylphenylsulfonyl)propionic acid sodium salt (094-174529),
(31) 3-(4-carboxyphenylsulfonyl)propionic acid,
(32) 3-(4-cyanophenylsulfonyl)propionic acid ethyl ester,
(33) 3-(4-formylphenylsulfonyl)propionic acid ethyl ester,
(34) 3-(4-biphenylsulfonyl)propionic acid methyl ester (093-061046),
(35) 2-amino-3-(2-methylphenylsulfonyl)propionic acid (53-14959g),
(36) 2-amino-3-(3-methylphenylsulfonyl)propionic acid (53-14959g),
(37) 2-amino-3-(4-methylphenylsulfonyl)propionic acid (53-14959g),
(38) 2-amino-3-(4-fluorophenylsulfonyl)propionic acid (53-14959g),
(39) 2-t-butoxycarbonylamino-3-(4-fluorophenylsulfonyl)propionic acid (124-289512),
(40) 2-amino-3-(4-chlorophenylsulfonyl)propionic acid (53-14959g),
(41) 2-t-butoxycarbonylamino-3-(4-chlorophenylsulfonyl)propionic acid (124-117961),
(42) 2-amino-3-(3-trifluoromethylphenylsulfonyl)propionic acid (53-14959h),
(43) 2-amino-3-(4-nitrophenylsulfonyl)propionic acid (119-95106),
(44) 2-amino-3-(2-nitrophenylsulfonyl)propionic acid (119-95106),
(45) 2-amino-3-(4-aminophenylsulfonyl)propionic acid (119-95106),
(46) 2-amino-3-(2-aminophenylsulfonyl)propionic acid (119-95106),
(47) 2,2-dimethyl-3-(4-hydroxyphenylthio)propionic acid,
(48) 4-(2-carboxy-2-methylpropylmercapto)phenyl 2-phenylbutyrate,
(49) 4-(2-carboxy-2-methylpropylsulfinyl)phenyl 2-phenylbutyrate,
(50) 4-(2-carboxy-2-methylpropylsulfonyl)phenyl 2-phenylbutyrate,
(51) 4-(2-carboxy-2-methylpropylmercapto)phenyl 2-(4-methoxyphenyl)isobutyrate,
(52) 4-(2-carboxy-2-methylpropylmercapto)phenyl 2-(3,4-diethylphenyl)isobutyric acid,
(53) 4-(2-carboxy-2-methylpropylmercapto)phenyl 2-(1,2,3,4-tetrahydro-6-naphthyl)butyrate,
(54) 4-(2-carboxy-2-methylpropylmercapto)phenyl 2-(1-methyl-2-pyrrole)butyrate,
(55) 4-(2-carboxy-2-methylpropylsulfinyl)phenyl 2-(1-methyl-2-pyrrole)butyrate,
(56) 3-(4-bromophenylthio)propionic acid,
(57) N-t-butoxy-3-(4-bromophenylthio)propionamide,
(58) N-t-butoxy-3-(4-biphenylthio)propionamide

Compounds (47) ∼ (49) and Compounds (50) ∼ (55), respectively, are described in the above-mentioned (7) Japanese Patent Kokai 4-226939 and (8) Japanese Patent Kokai 4-283576.

### Disclosure of the Invention

Energetic investigations have been carried out in order to make a matrix metalloproteinase inhibitor. As a result, the present inventors have found that the purpose may be achieved with aryl (sulfide, sulfoxide, sulfone) derivatives of the formula (I).

Most of aryl (sulfide, sulfoxide, sulfone) derivatives of the formula (I) are not known and are novel compounds.

Further, the present inventors have also found that the compounds of the present invention may have a particularly inhibitory activity against matrix metalloproteinases, especially, class of gelatinases.

The present invention relates to
1) matrix metalloproteinase inhibitors containing aryl (sulfide, sulfoxide, sulfone) of the formula (I) 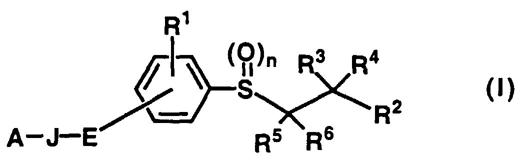 (wherein
   R¹ is hydrogen, or C1-4 alkyl,
   R² is -COOR⁷ or -CONHOR⁸,
   R⁷ is hydrogen, C1-8 alkyl, phenyl, or
   C1-4 alkyl substituted by phenyl, -OCOR²³ (in which R²³ is C1-4 alkyl.), or -CONR²⁴R²⁵ (in which R²⁴ and R²⁵, each independently, is hydrogen or C1-4 alkyl.),
   R⁸ is hydrogen, C1-8 alkyl, phenyl, or C1-4 alkyl substituted by phenyl,
   E is -CONR⁹-, -NR⁹CO-, -OCO-, -COO-, -CH₂-O-, -CO-OH₂-, -(CH₂)₂-, -CH=CH-or -C≡C- (in which R⁹ is hydrogen, C1-4 alkyl, phenyl, or C1-4 alkyl substituted by phenyl, and the left side of each group is attached to J group.),
   J is bond or C1-8 alkylene,
   A is
      1) hydrogen,
      2) C1-8 alkyl,
      3) Ar group (Ar group is carbocyclic ring or heterocyclic ring optionally substituted by 1 ∼ 3 of
         i) C1-15 alkyl,
         ii) C1-15 alkoxy,
         iii) halogen,
         iv) nitro,
         v) cyano,
         vi) guanidino,
         vii) amidino,
         viii) hydroxy,
         ix) benzyloxy,
         x) NR¹²R¹³ (in which R¹² and R¹³, each independently, is hydrogen, C1-4 alkyl or -COOR¹⁴ (in which R¹⁴ is C1-4 alkyl or benzyloxy.).),
         xi) -COOR¹⁵ (in which R¹⁵ is hydrogen, C1-4 alkyl, phenyl, or C1-4 alkyl substituted by phenyl.),
         xii) trifluoromethyl,
         xiii) carbocyclic ring,
         xiv) heterocyclic ring or
         xv) C1-4 alkyl substituted by hydroxy, C1-4 alkoxy, NR¹²R¹³ (in which R¹² and R¹³ are as hereinbefore defined.), -COOR¹⁵ (in which R¹⁵ is as hereinbefore defined.), carbocyctic ring or heterocyclic ring.)
         or
      4) C1-4 alkyl substituted by hydroxy or C1-4 alkoxy, or
         A, J and E taken together, represent methyl, halogen, trifluoromethyl, nitro, cyano, formyl, phenyl, hydroxy, NR¹⁶R¹⁷ (in which R¹⁶ and R¹⁷, each independently, is hydrogen, C1-4 alkyl, -COOR¹⁸ (in which R¹⁸ is C1-4 alkyl or benzyl.).), or heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are as hereinbefore defined.) or CONR²⁴R²⁵ (in which R²⁴ and R²⁵ are as hereinbefore defined.).),
         R³ and R⁴, each independently, is
         (1) hydrogen,
         (2) C1-8 alkyl (with the proviso that one of the carbon atoms in C1-8 alkyl may be replaced by a sulfur atom.),
         (3) -COOR¹⁹ (in which R¹⁹ is hydrogen, C1-8 alkyl, phenyl, or C1-4 alkyl substituted by phenyl.),
         (4) Ar₁ group (Ar₁ group is carbocyclic ring or heterocyclic ring optionally substituted by 1 ∼ 3 of C1-4 alkyl, C1-4 alkoxy, halogen, hydroxy or trifluoromethyl.),
         (5) hydroxy,
         (6) -NR²⁰R²¹ (in which R²⁰ and R²¹, each independently, is hydrogen, C1-4 alkyl, -COOR²² or -COR²² (in which R²² is C1-4 alkyl or benzyl.),
         (7) 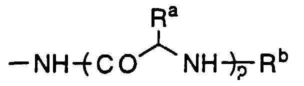 (in which R^{a} is hydrogen or phenyl, R^{b} is hydrogen, -COOR²² or -COR²² (in which P²² is as hereinbefore defined.), p is 1 or 2.), or
         (8) C1-8 alkyl substituted by substituent selected from the following (a) ∼ (f) (with the proviso that one of the carbon atoms in C1-8 alkyl may be replaced by a sulfur atom.);
            (a) -COOR¹⁹ (in which R¹⁹ is as hereinbefore defined.)
            (b) C1-4 alkoxy,
            (c) hydroxy,
            (d) benzyloxy,
            (e) -NR²⁰R²¹ (in which R²⁰ and R²¹ are as hereinbefore defined.), or
            (f) Ar₁ group (in which Ar₁ is as hereinbefore defined.)
   ,or R³ and R⁴ taken together with the carbon to which they are attached, form C3-7 cycloalkyl,
   R⁵ and R⁶ are hydrogen or methyl, or R³ and R⁵ taken together, form a bond and R⁴ and R⁶ are as hereinbefore defined, and
   n is 0, 1 or 2;
   With the proviso that :
   when A, J and E taken together, form phenyl, and R² is CONHOH, then n is 1 or 2.)
   ,or non-toxic salts thereof, as active ingredient,
2) aryl (sulfide, sulfoxide, sulfone) derivatives of the formula (I) 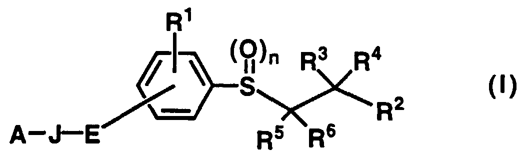 (wherein
   R¹ is hydrogen, or C1-4 alkyl,
   R² is -COOR⁷ or -CONHOR⁸,
   R⁷ is hydrogen, C1-8 alkyl, phenyl, or
   C1-4 alkyl substituted by phenyl, -OCOR²³ (in which R²³ is C1-4 alkyl.), or -CONR²⁴R²⁵ (in which R²⁴ and R²⁵, each independently, is hydrogen or C1-4 alkyl.),
   R⁸ is hydrogen, C1-8 alkyl, phenyl, or C1-4 alkyl substituted by phenyl,
   E is -CONR⁹-, -NR⁹CO-, -OCO-, -COO-, -CH₂-O-, -CO-CH₂-, -(CH₂)₂-, -CH=CH-or -C≡C- (in which R⁹ is hydrogen, C1-4 alkyl, phenyl, or C1-4 alkyl substituted by phenyl, and the left side of each group is attached to J group.),
   J is bond or C1-8 alkylene,
   A is
      1) hydrogen,
      2) C1-8 alkyl,
      3) Ar group (Ar group is carbocyclic ring or heterocyclic ring optionally substituted by 1 ∼ 3 of
         i) C1-15 alkyl,
         ii) C1-15 alkoxy,
         iii) halogen,
         iv) nitro,
         v) cyano,
         vi) guanidino,
         vii) amidino,
         viii) hydroxy,
         ix) benzyloxy,
         x) NR¹²R¹³ (in which R¹² and R¹³, each independently, is hydrogen, C1-4 alkyl or -COOR¹⁴ (in which R¹⁴ is C1-4 alkyl or benzyloxy.).),
         xi) -COOR¹⁵ (in which R¹⁵ is hydrogen, C1-4 alkyl, phenyl, or C1-4 alkyl substituted by phenyl.),
         xii) trifluoromethyl,
         xiii) carbocyclic ring,
         xiv) heterocyclic ring or
         xv) C1-4 alkyl substituted by hydroxy, C1-4 alkoxy, NR¹²R¹³ (in which R¹² and R¹³ are as hereinbefore defined.), -COOR¹⁵ (in which R¹⁵ is as hereinbefore defined.), carbocyclic ring or heterocyclic ring.)
         or
      4) C1-4 alkyl substituted by hydroxy or C1-4 alkoxy, or
         A, J and E taken together, represent methyl, halogen, trifluoromethyl, nitro, cyano, formyl, phenyl, hydroxy, NR¹⁶R¹⁷ (in which R¹⁶ and R¹⁷, each independently, is hydrogen, C1-4 alkyl, -COOR¹⁸ (in which R¹⁸ is C1-4 alkyl or benzyl.).), or heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are as hereinbefore defined.) or CONR²⁴R²⁵ (in which R²⁴ and R²⁵ are as hereinbefore defined.).),
         R³ and R⁴, each independently, is
         (1) hydrogen,
         (2) C1-8 alkyl (with the proviso that one of the carbon atoms in C1-8 alkyl may be replaced by a sulfur atom.),
         (3) -COOR¹⁹ (in which R¹⁹ is hydrogen, C1-8 alkyl, phenyl, or C1-4 alkyl substituted by phenyl.),
         (4) Ar₁ group (Ar₁ group is carbocyclic ring or heterocyclic ring optionally substituted by 1 ∼ 3 of C1-4 alkyl, C1-4 alkoxy, halogen, hydroxy or trifluoromethyl.),
         (5) hydroxy,
         (6) -NR²⁰R²¹ (in which R²⁰ and R²¹, each independently, is hydrogen, C1-4 alkyl, -COOR²² or -COR²² (in which R²² is C1-4 alkyl or benzyl.),
         (7) 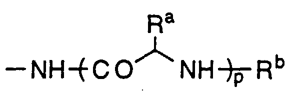 (in which R^{a} is hydrogen or phenyl, R^{b} is hydrogen, -COOR²² or -COR²² (in which R²² is as hereinbefore defined.), p is 1 or 2.), or
         (8) C1-8 alkyl substituted by substituent selected from the following (a) ∼ (f) (with the proviso that one of the carbon atoms in C1-8 alkyl may be replaced by a sulfur atom.);
            (a) -COOR¹⁹ (in which R¹⁹ is as hereinbefore defined.)
            (b) C1-4 alkoxy,
            (c) hydroxy,
            (d) benzyloxy,
            (e) -NR²⁰R²¹ (in which R²⁰ and R²¹ are as hereinbefore defined.), or
            (f) Ar₁ group (in which Ar₁ is as hereinbefore defined.)
   ,or R³ and R⁴ taken together with the carbon to which they are attached, form C3-7 cycloalkyl,
   R⁵ and R⁶ are hydrogen or methyl, or R³ and R⁵ taken together, form a bond and R⁴ and R⁶ are as hereinbefore defined, and
   n is 0, 1 or 2;
   With the proviso that :
   (a) when A, J and E taken together, form phenyl, and R² is CONHOH, then n is 1 or 2.
   (b) the following compounds (1) ∼ (58) are excluded:
      (1) 3-(4-methylphenylsulfonyl)propionic acid isopropyl ester,
      (2) 3-(4-methylphenylsulfonyl)propionic acid phenyl ester,
      (3) 3-(4-methylphenylsulfonyl)propionic acid sodium salt,
      (4) 3-(4-methylphenylsulfonyl)propionic acid methyl ester,
      (5) 3-(4-methylphenylsulfonyl)propionic acid ethyl ester,
      (6) 3-(4-methylphenylsulfonyl)propionic acid,
      (7) 3-(4-ethylphenylsulfonyl)propionic acid,
      (8) 3-(4-methoxyphenylsulfonyl)propionic acid phenyl ester,
      (9) 3-(4-methoxyphenylsulfonyl)propionic acid,
      (10) 3-(4-nitrophenylsulfonyl)propionic acid methyl ester,
      (11) 3-(4-nitrophenylsulfonyl)propionic acid isopropyl ester,
      (12) 3-(4-nitrophenylsulfonyl)propionic acid,
      (13) 3-(4-aminophenylsulfonyl)propionic acid ethyl ester,
      (14) 3-(4-aminophenylsulfonyl)propionic acid,
      (15) 3-(4-hydroxyphenylsulfonyl)propionic acid,
      (16) 3-(4-hydroxyphenylsulfonyl)propionic acid phenyl ester,
      (17) 3-(4-bromophenylsulfonyl)propionic acid methyl ester,
      (18) 3-(4-bromophenylsulfonyl)propionic acid ethyl ester,
      (19) 3-(4-bromophenylsulfonyl)propionic acid phenyl ester,
      (20) 3-(4-chlorophenylsulfonyl)propionic acid methyl ester,
      (21) 3-(4-chlorophenylsulfonyl)propionic acid ethyl ester,
      (22) 3-(4-chlorophenylsulfonyl)propionic acid t-butyl ester,
      (23) 3-(4-chlorophenylsulfonyl)propionic acid isopropyl ester,
      (24) 3-(4-chlorophenylsulfonyl)propionic acid,
      (25) 3-(4-chlorophenylsulfonyl)propionic acid phenyl ester,
      (26) 3-(4-iodophenylsulfonyl)propionic acid ethyl ester,
      (27) 3-(4-iodophenylsulfonyl)propionic acid methyl ester,
      (28) 3-(4-acetylaminophenylsulfonyl)propionic acid methyl ester,
      (29) 3-(4-acetylaminophenylsulfonyl)propionic acid ethyl ester,
      (30) 3-(4-vinylphenylsulfonyl)propionic acid sodium salt,
      (31) 3-(4-carboxyphenylsulfonyl)propionic acid,
      (32) 3-(4-cyanophenylsulfonyl)propionic acid ethyl ester,
      (33) 3-(4-formylphenylsulfonyl)propionic acid ethyl ester,
      (34) 3-(4-biphenylsulfonyl)propionic acid methyl ester,
      (35) 2-amino-3-(2-methylphenylsulfonyl)propionic acid,
      (36) 2-amino-3-(3-methylphenylsulfonyl)propionic acid,
      (37) 2-amino-3-(4-methylphenylsulfonyl)propionic acid,
      (38) 2-amino-3-(4-fluorophenylsulfonyl)propionic acid,
      (39) 2-t-butoxycarbonylamino-3-(4-fluorophenylsulfonyl)propionic acid,
      (40) 2-amino-3-(4-chlorophenylsulfonyl)propionic acid,
      (41) 2-t-butoxycarbonylamino-3-(4-chlorophenylsulfonyl)propionic acid,
      (42) 2-amino-3-(3-trifluoromethylphenylsulfonyl)propionic acid,
      (43) 2-amino-3-(4-nitrophenylsulfonyl)propionic acid,
      (44) 2-amino-3-(2-nitrophenylsulfonyl)propionic acid,
      (45) 2-amino-3-(4-aminophenylsulfonyl)propionic acid,
      (46) 2-amino-3-(2-aminophenylsulfonyl)propionic acid,
      (47) 2,2-dimethyl-3-(4-hydroxyphenylthio)propionic acid,
      (48) 4-(2-carboxy-2-methylpropylmercapto)phenyl **2-phenylbutyrate,**
      (49) 4-(2-carboxy-2-methylpropylsulfinyl)phenyl **2-phenylbutyrate,**
      (50) 4-(2-carboxy-2-methylpropylsulfonyl)phenyl **2-phenylbutyrate,**
      (51) 4-(2-carboxy-2-methylpropylmercapto)phenyl 2-(4-**methoxyphenyl)isobutyrate,**
      (52) 4-(2-carboxy-2-methylpropylmercapto)phenyl 2-(3,4-diethylphenyl)isobutyric acid,
      (53) 4-(2-carboxy-2-methylpropylmercapto)phenyl 2-(1,2,3,4-tetrahydro-6-naphthyl)butyrate,
      (54) 4-(2-carboxy-2-methylpropylmercapto)phenyl 2-(1-methyl-2-pyrrole)butyrate,
      (55) 4-(2-carboxy-2-methylpropylsulfinyl)phenyl 2-(1-methyl-2-pyrrole)butyrate,
      (56) 3-(4-bromophenylthio)propionic acid,
      (57) N-t-butoxy-3-(4-bromophenylthio)propionamide,
      (58) N-t-butoxy-3-(4-biphenylthio)propionamide.)
      ,or non-toxic salts thereof, and
3) processes for the preparation of aryl (sulfide, sulfoxide, sulfone) derivatives and non-toxic salts thereof.

### Detailed Description of the Invention

Unless otherwise specified, all isomers are included in the present invention. For example, alkyl, alkoxy and alkylene include straight and branched isomers. The double bonds in the alkenylene group include E, Z and EZ mixture. Isomers produced by the existence of asymmetric carbon atoms are included in the present invention when branched-chain alkyl, alkoxy and alkylene etc. exist.

In the formula (I), C1-4 alkyl represented by R¹, R⁹, R¹⁰, R¹¹, R¹², R¹³, R¹⁴, R¹⁵, R¹⁶, R¹⁷, R¹⁸, R²⁰, R²¹ R²² R²³, R²⁴, R²⁵, or C1-4 alkyl as a substituent of Ar₁ group and heterocyclic ring represented by A, J and E taken together, means methyl, ethyl, propyl, butyl and the isomers thereof.

In the formula (I), C1-8 alkyl represented by R², R³, R⁴, R⁷, R⁸, R¹⁹, A, means methyl, ethyl, propyl, butyl, pentyl, hexyl, heptyl, octyl and the isomers thereof.

In the formula (I), C1-15 alkyl as a substituent of Ar group, means methyl, ethyl, propyl, butyl, pentyl, hexyl, heptyl, octyl, nonyl, decyl, undecyl, dodecyl, tridecyl, tetradecyl, pentadecyl and the isomers thereof.

In the formula (I), C1-4 alkyl substituted by phenyl represented by R⁷, R⁸, R⁹, R¹⁵, R¹⁹, means methyl, ethyl, propyl, butyl and the isomers substituted by one phenyl.

In the formula (I), C1-4 alkoxy in R³ or R⁴, or C1-4 alkoxy as a substituent of heterocyclic ring represented by A, J and E taken together, means methoxy, ethoxy, propoxy, butoxy and the isomers thereof.

In the formula (I), C1-15 alkyl as a substituent of Ar group, means methoxy, ethoxy, propoxy, butoxy, pentyloxy, hexyloxy, heptyloxy, octyloxy, nonyloxy, decyloxy, undecyloxy, dodecyloxy, tridecyloxy, tetradecyloxy, pentadecyloxy and the isomers thereof.

In the formula (I), halogen as a substituent of Ar group or Ar₁ group, or halogen represented by A, J and E taken together, or halogen as a substituent of heterocyclic ring represented by A, J and E taken together, means fluoro, chloro, bromo and jodo.

In the formula (I), C3-7 cycloalkyl formed by R³ and R⁴ taken together with the carbon to which they are attached, means cyclopropyl, cyclobutyl, cyclopentyl, cyclohexyl and cycloheptyl.

In the formula (I), carbocyclic ring represented by Ar group, Ar₁ group, means C5-10 carbocyclic aryl or the above-mentioned C3-7 cycloalkyl. For example, C5-10 carbocyclic aryl includes benzene, pentalene, indene, naphthalene, azulene etc.

In the formula (I), heterocyclic ring represented by Ar group, Ar₁ group, R³ or R⁴, heterocyclic ring represented by A, J and E taken together and heterocyclic ring as a substituent of Ar group, means C5-15 membered mono- or bi-heterocyclic ring containing 1-2 of nitrogen, 1 of oxygen, or 1 of sulfur. The heterocyclic ring includes partially or fully saturated analogues of the above C5-15 membered mono- or bi-heterocyclic ring containing 1-2 of nitrogen, 1 of oxygen, or 1 of sulfur. For example, C5-15 membered mono- or bi-heterocyclic ring containing 1-2 of nitrogen, 1 of oxygen, or 1 of sulfur, includes pyrrole, imidazole, pyrazole, pyridine, pyrazine, pyrimidine, pyridazine, azepine, diazepine, furan, pyran, oxepine, oxazepine, thiophene, thiaine (thiopyran), thiepine, oxazole, isooxazole, thiazole, isothiazole, oxadiazole, oxadiazine, oxazepine, oxadiazepine, thiadiazole, thiadiazine, thiadiazepine, indole, isoindole, benzofuran, isobenzofuran, benzothiophene, isobenzothiophene, indazole, quinoline, isoquinoline, phthalazine, naphthyridine, quinoxaline, quinazoline, cinnoline, benzoxazole, benzothiazole, benzoimidazole etc.

Also, partially or fully saturated C5-15 membered mono- or bi-heterocyclic ring containing 1-2 of nitrogen, 1 of oxygen, or 1 of sulfur, includes pyrroline, pyrrolidine, imidazoline, imidazolidine, pyrazoline, pyrazolidine, piperidine, piperazine, tetrahydropyrimidine, tetrahydropyridazine, dihydrofuran, tetrahydrofuran, dihydropyran, tetrahydropyran, dihydrothiophene, tetrahydrothiophene, dihydrothiaine (dihydrothiopyran), tetrahydrothiaine (tetrahydrothiopyran), dihydrooxazole, tetrahydrooxazole, dihydroisooxazole, tetrahydroisooxazole, dihydrothiazole, tetrahydrothiazole, dihydroisothiazole, tetrahydroisothiazole, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzoimidazole, perhydrobenzoimidazole etc.

### [Salts]

In the present invention, non-toxic salts includes all such salts. For example, non-toxic salts includes general salts, acid addition salts, hydrate salts etc.

The compounds of the present invention of the formula (I) may be converted into the corresponding salts by known method. Non toxic and water-soluble salts are preferable.

Suitable salts include the salts of alkali metal (potassium, sodium etc.), alkaline-earth metal (calcium, magnesium etc.), ammonium salts, salts of organic amine which is pharmacologically permitted (tetramethyl ammonium, triethylamine, methylamine, dimethylamine, cyclopentylamine, benzylamine, phenethylamine, piperidine, monoethanolamine, diethanolamine, tris (hydroxymethyl)aminomethane, lysine, arginine, N-methyl-D-glucane etc.).

The compounds of the present invention of the formula (I) may be converted into the corresponding acid-addition salts by known method. Non toxic and water-soluble salts are preferable.

Suitable acid-addition salts include the salts with inorganic acids such as hydrochloric acid, hydrobromide, sulfate, phosphate, nitrate, and the salts with organic acids such as acetic acid, trifluoroacetic acid, lactic acid, tartaric acid, oxalic acid, fumaric acid, maleic acid, citric acid, benzoic acid, methanesulfonic acid, ethanesulfonic acid, benzenesulfonic acid, toluenesulfonic acid, isethionic acid, glucuronic acid and gluconic acid.

The compounds of the present invention of the formula (I) or salts thereof may be converted into a corresponding hydrate by methods known per se.

In the compounds of the present invention of formula (I), the following compounds are preferred.
the formula (I-1) 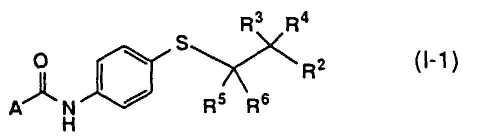 (wherein all symbols are the same meanings as hereinbefore described.),
the formula (I-2) 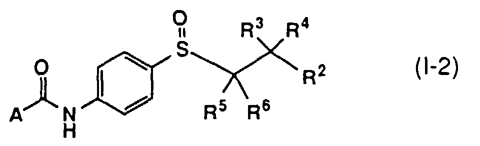 (wherein all symbols are the same meanings as hereinbefore described.),
the formula (I-3) 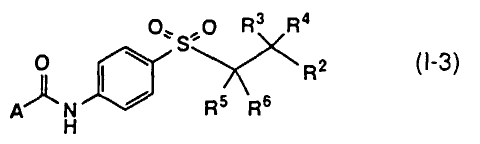 (wherein all symbols are the same meanings as hereinbefore described.),
the formula (I-4) 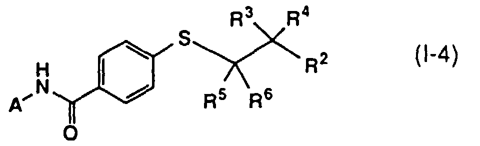 (wherein all symbols are the same meanings as hereinbefore described.),
the formula (I-5) 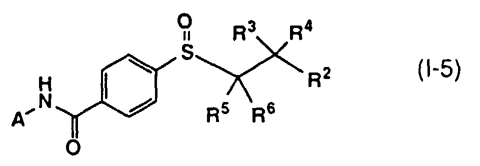 (wherein all symbols are the same meanings as hereinbefore described.),
the formula (I-6) 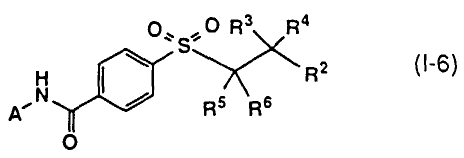 (wherein all symbols are the same meanings as hereinbefore described.),
the formula (I-7) 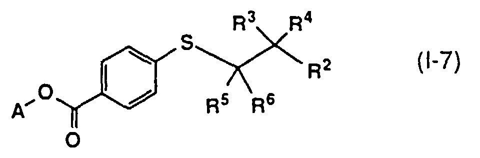 (wherein all symbols are the same meanings as hereinbefore described.),
the formula (I-8) 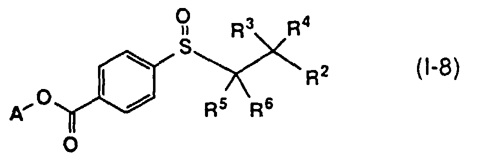 (wherein all symbols are the same meanings as hereinbefore described.),
the formula (I-9) 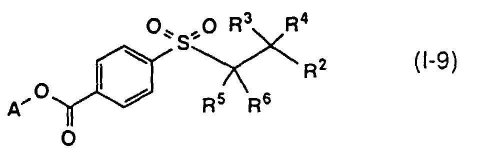 (wherein all symbols are the same meanings as hereinbefore described.),
the formula (I-10) 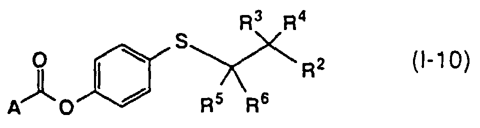 (wherein all symbols are the same meanings as hereinbefore described.),
the formula (I-11) 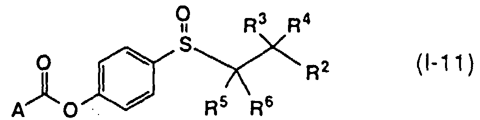 (wherein all symbols are the same meanings as hereinbefore described.),
the formula (I-12) 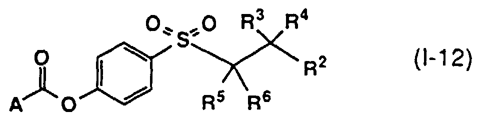 (wherein all symbols are the same meanings as hereinbefore described.),
the formula (I-13) 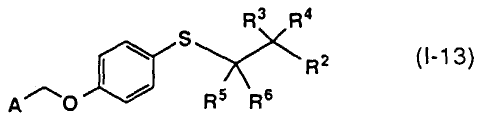 (wherein all symbols are the same meanings as hereinbefore described.),
the formula (I-14) 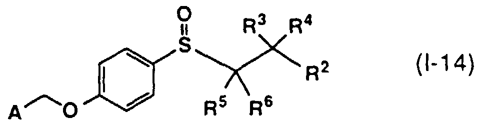 (wherein all symbols are the same meanings as hereinbefore described.),
the formula (I-15) 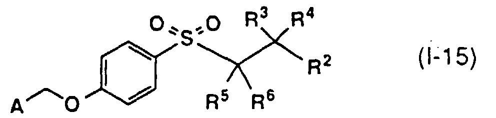 (wherein all symbols are the same meanings as hereinbefore described.),
the formula (I-16) 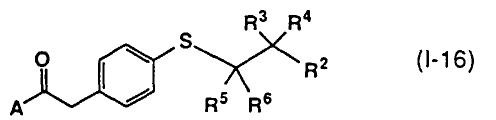 (wherein all symbols are the same meanings as hereinbefore described.),
the formula (I-17) 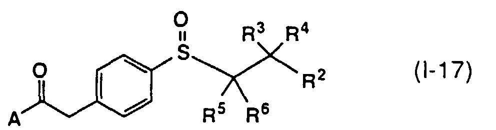 (wherein all symbols are the same meanings as hereinbefore described.),
the formula (I-18) 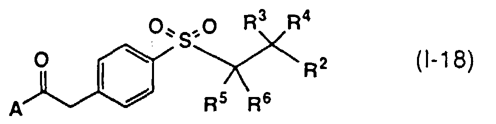 (wherein all symbols are the same meanings as hereinbefore described.),
the formula (I-19) 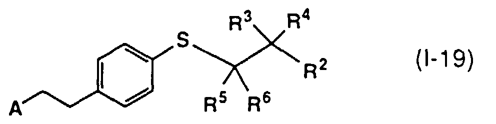 (wherein all symbols are the same meanings as hereinbefore described.),
the formula (I-20) 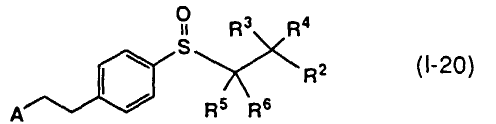 (wherein all symbols are the same meanings as hereinbefore described.),
the formula (I-21) 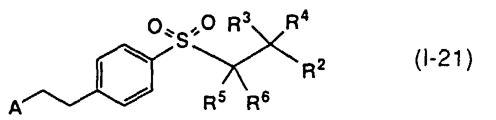 (wherein all symbols are the same meanings as hereinbefore described.),
the formula (I-22) 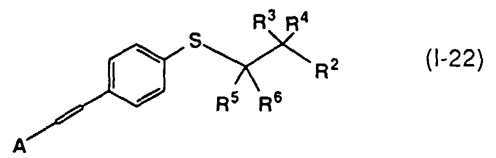 (wherein all symbols are the same meanings as hereinbefore described.),
the formula (I-23) 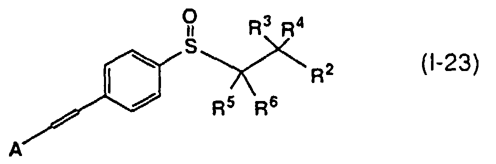 (wherein all symbols are the same meanings as hereinbefore described.),
the formula (I-24) 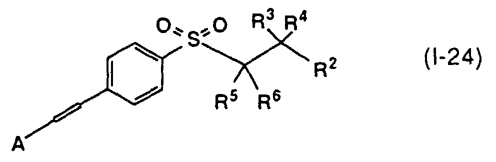 (wherein all symbols are the same meanings as hereinbefore described.),
the formula (I-25) 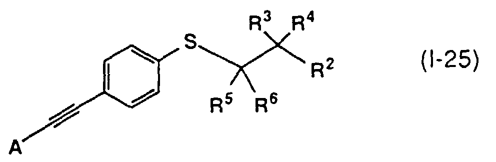 (wherein all symbols are the same meanings as hereinbefore described.),
the formula (I-26) 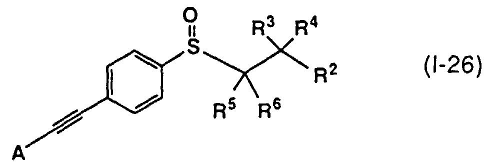 (wherein all symbols are the same meanings as hereinbefore described.),
the formula (I-27) 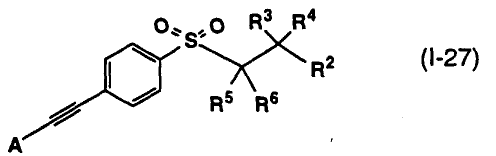 (wherein all symbols are the same meanings as hereinbefore described.),
the formula (I-28) 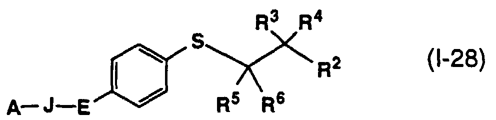 (wherein A, J and E taken together, represent methyl, halogen, trifluoromethyl, nitro, cyano, formyl, phenyl, hydroxy, NR¹⁶R¹⁷ (in which R¹⁶ and R¹⁷ are the same meanings as hereinbefore described.), heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are the same meanings as hereinbefore described.) or CONR²⁴R²⁵ (in which R²⁴ and R²⁵ are the same meanings as hereinbefore described.).), the other symbols are the same meanings as hereinbefore described.),
the formula (I-29) 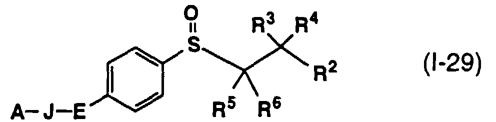 (wherein A, J and E taken together, represent methyl, halogen, trifluoromethyl, nitro, cyano, formyl, phenyl, hydroxy, NR¹⁶R¹⁷ (in which R¹⁶ and R¹⁷ are the same meanings as hereinbefore described.), heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are the same meanings as hereinbefore described.) or CONR²⁴R²⁵ (in which R²⁴ and R²⁵ are the same meanings as hereinbefore described.).), the other symbols are the same meanings as hereinbefore described.),
the formula (I-30) 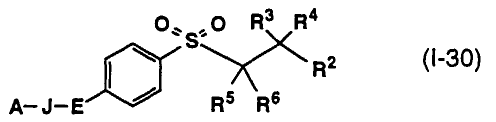 (wherein A, J and E taken together, represent methyl, halogen, trifluoromethyl, nitro, cyano, formyl, phenyl, hydroxy, NR¹⁶R¹⁷ (in which R¹⁶ and R¹⁷ are the same meanings as hereinbefore described.), heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are the same meanings as hereinbefore described.) or CONR²⁴R²⁵ (in which R²⁴ and R²⁵ are the same meanings as hereinbefore described.).), the other symbols are the same meanings as hereinbefore described.).

The compounds wherein n is 2, that is, the above compounds of the formulae (I-3), (I-6), (I-9), (I-12), (I-15), (I-18), (I-21), (I-24), (I-27) and (I-30) are more preferred. In the more preferred compounds, the compounds in which E is-CONH-, -CH₂-O-, -CH=CH-, ethynylene, and in which A, J and E taken together, represent heterocyclic ring, that is, the above compounds of the formulae (I-3), (I-15), (I-24), (I-27) and (I-30) (with proviso that A, J and E taken together, represent heterocyclic ring.) are particularly preferred.

Examples of representative compounds are shown in the following Tables and the compounds described in the Examples.

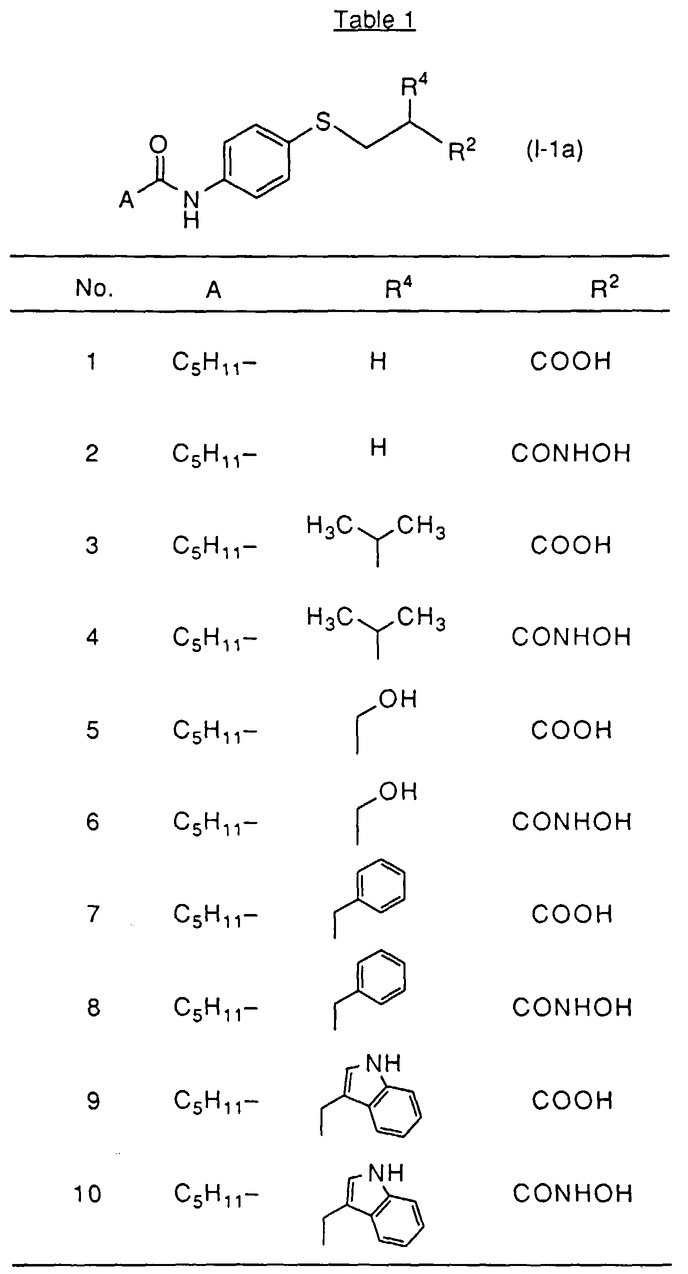

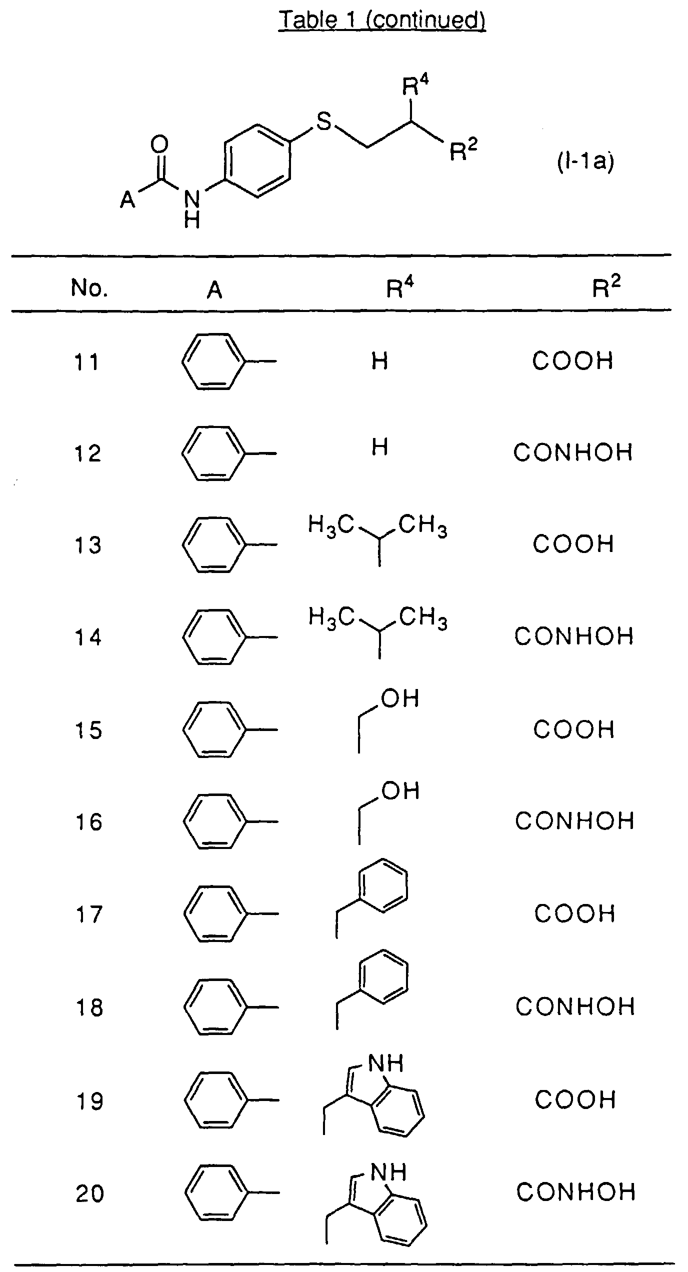

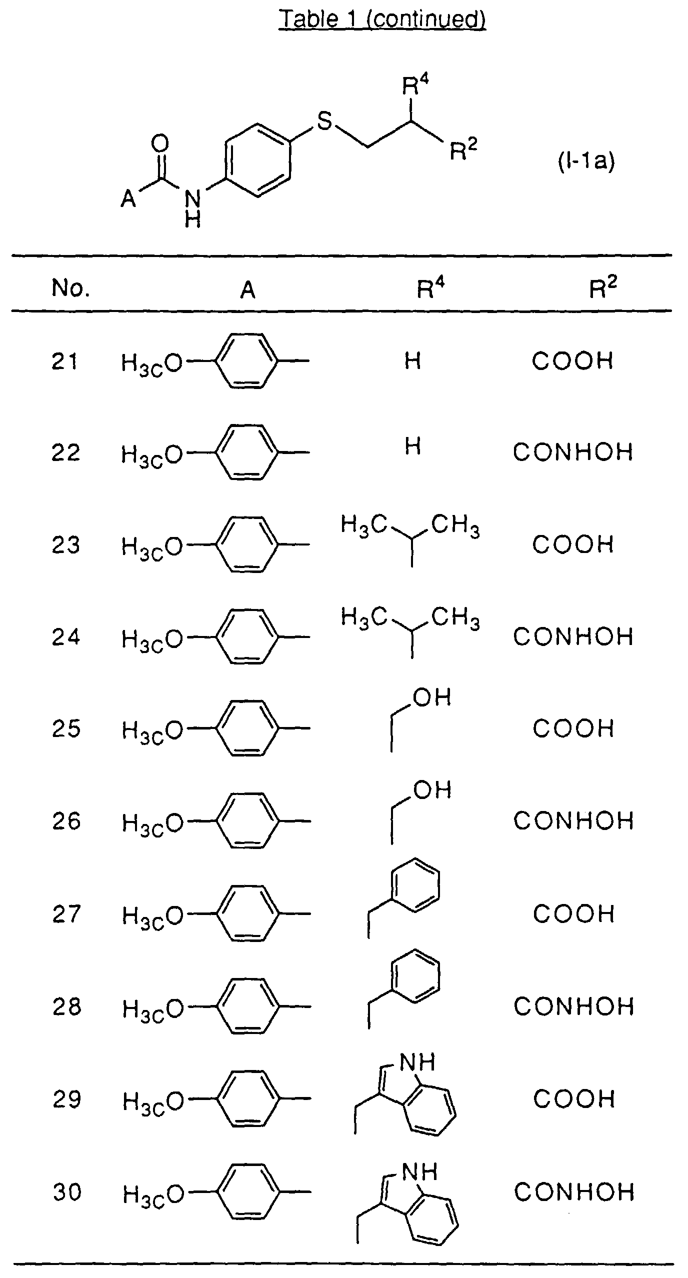

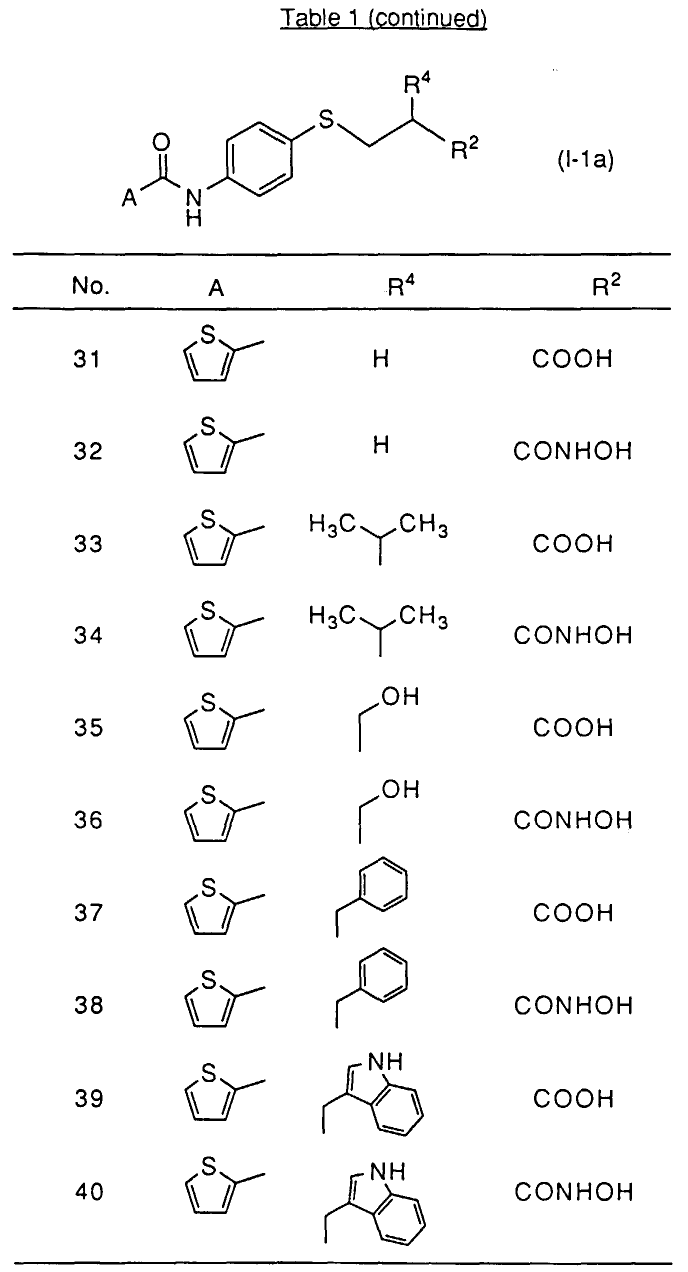

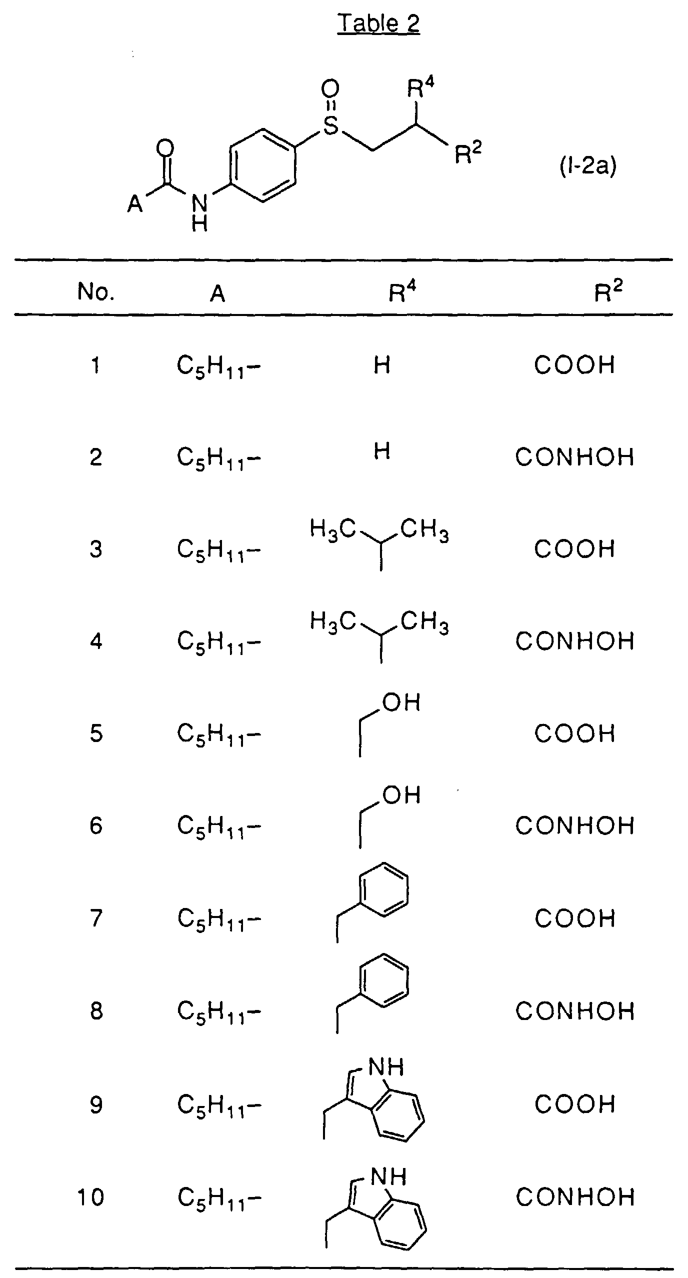

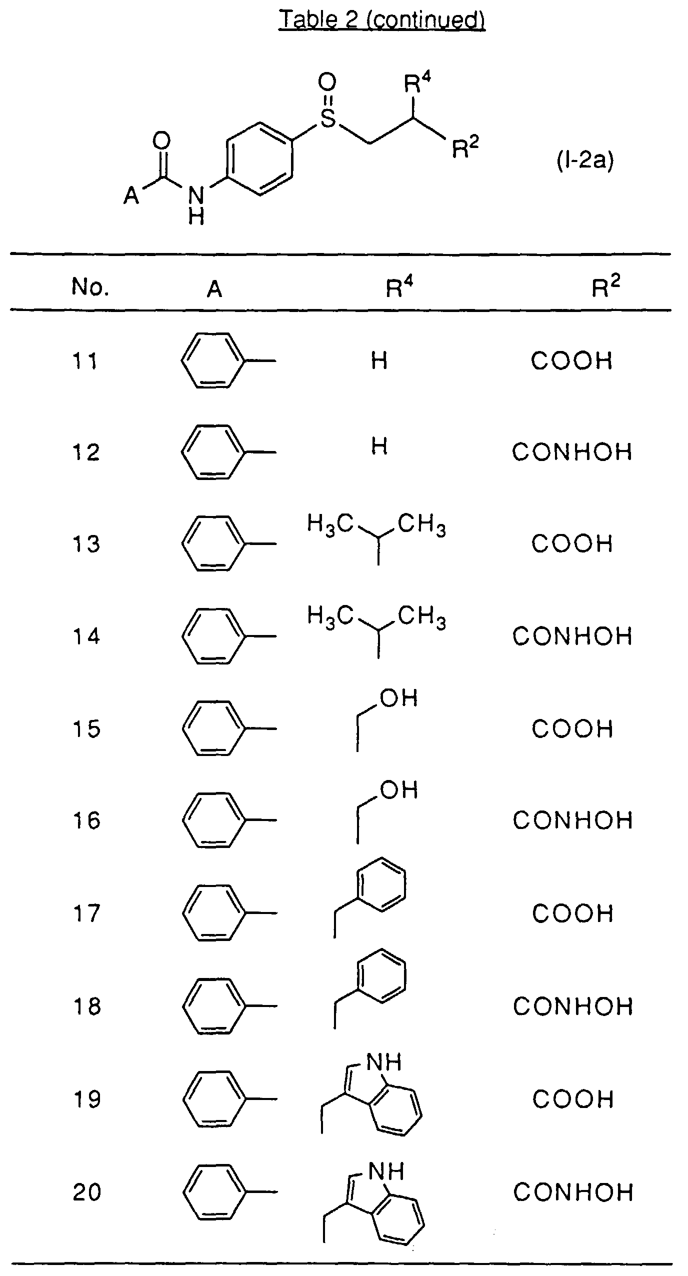

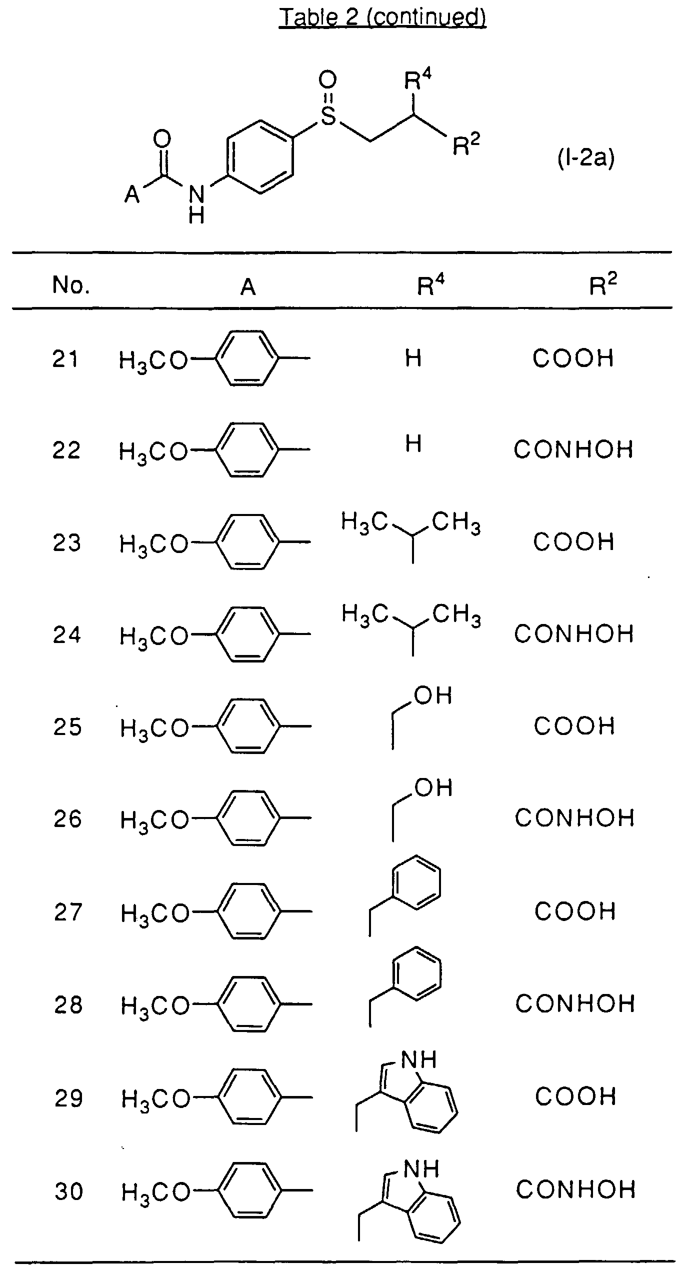

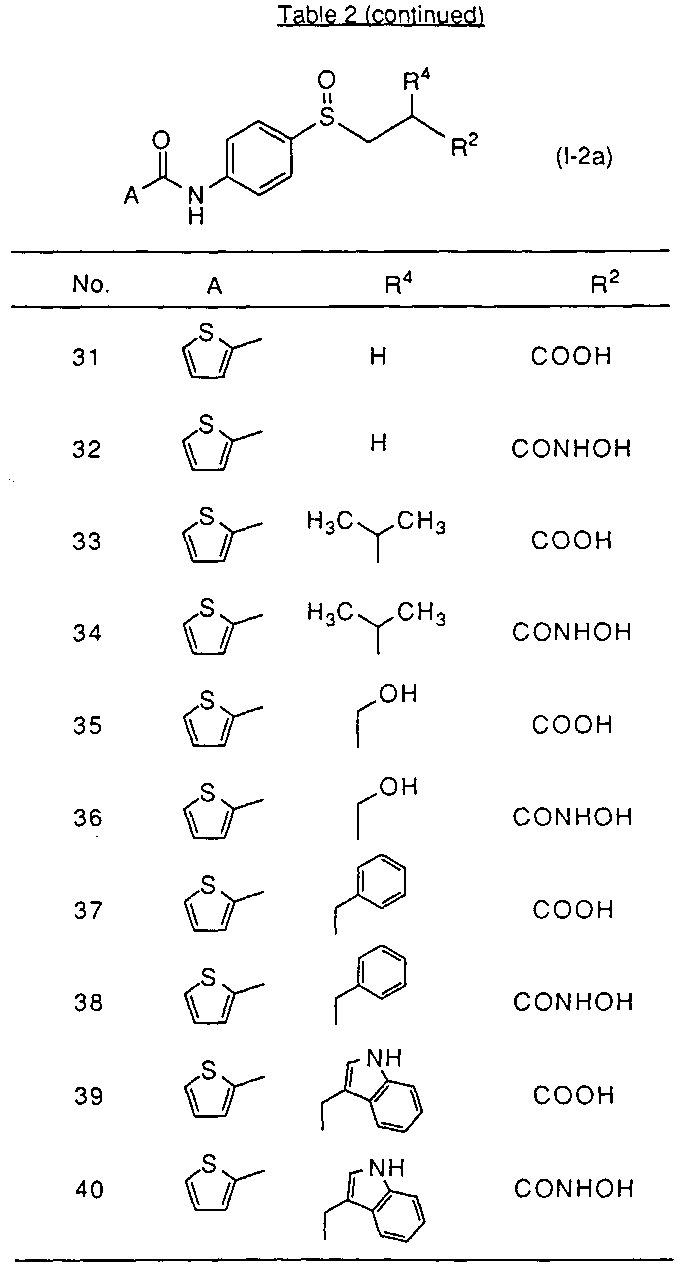

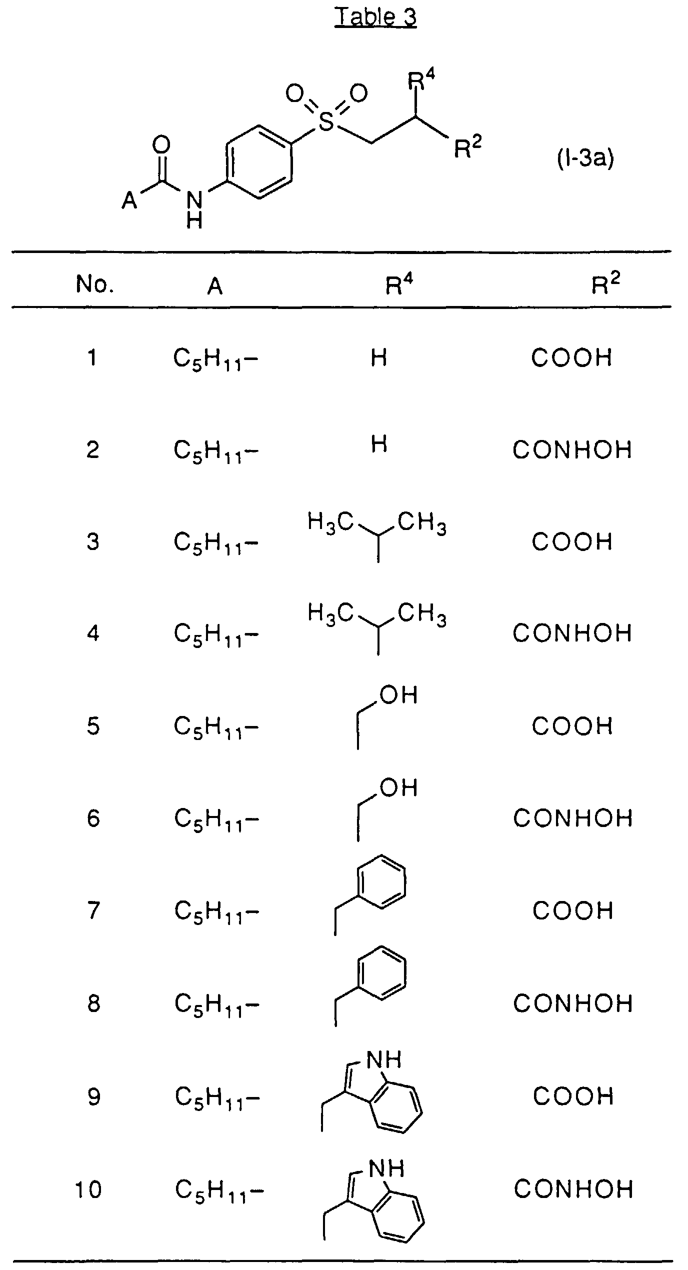

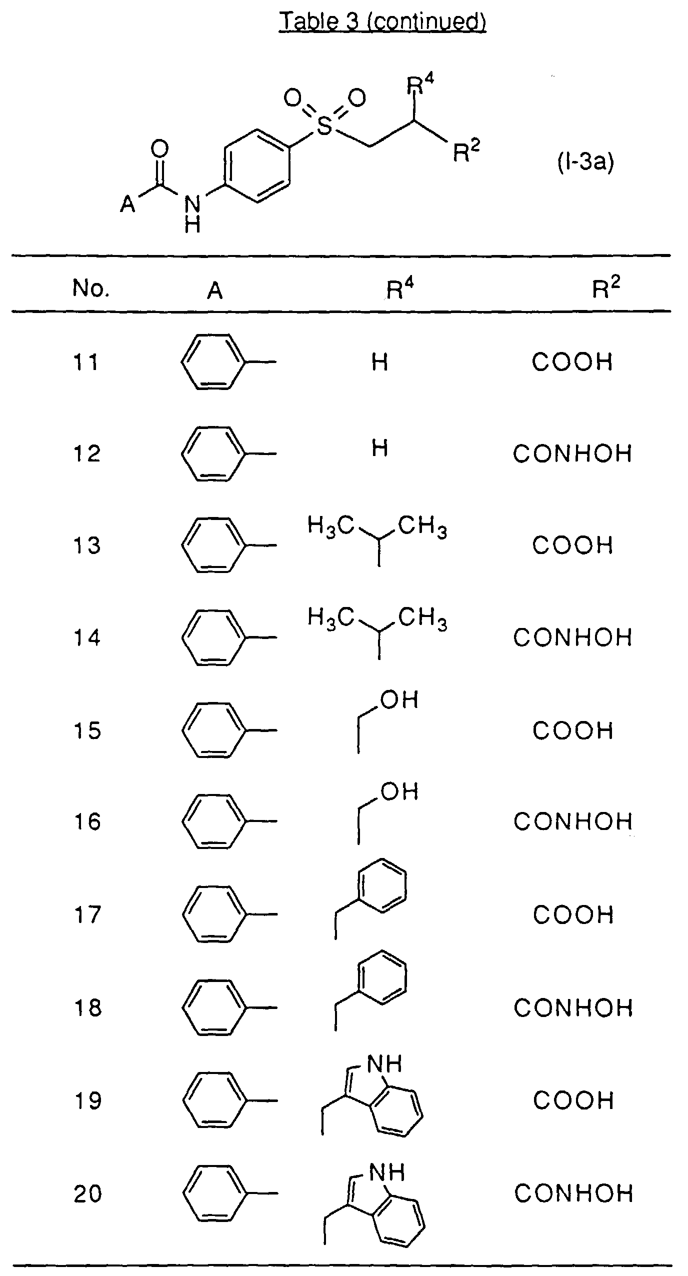

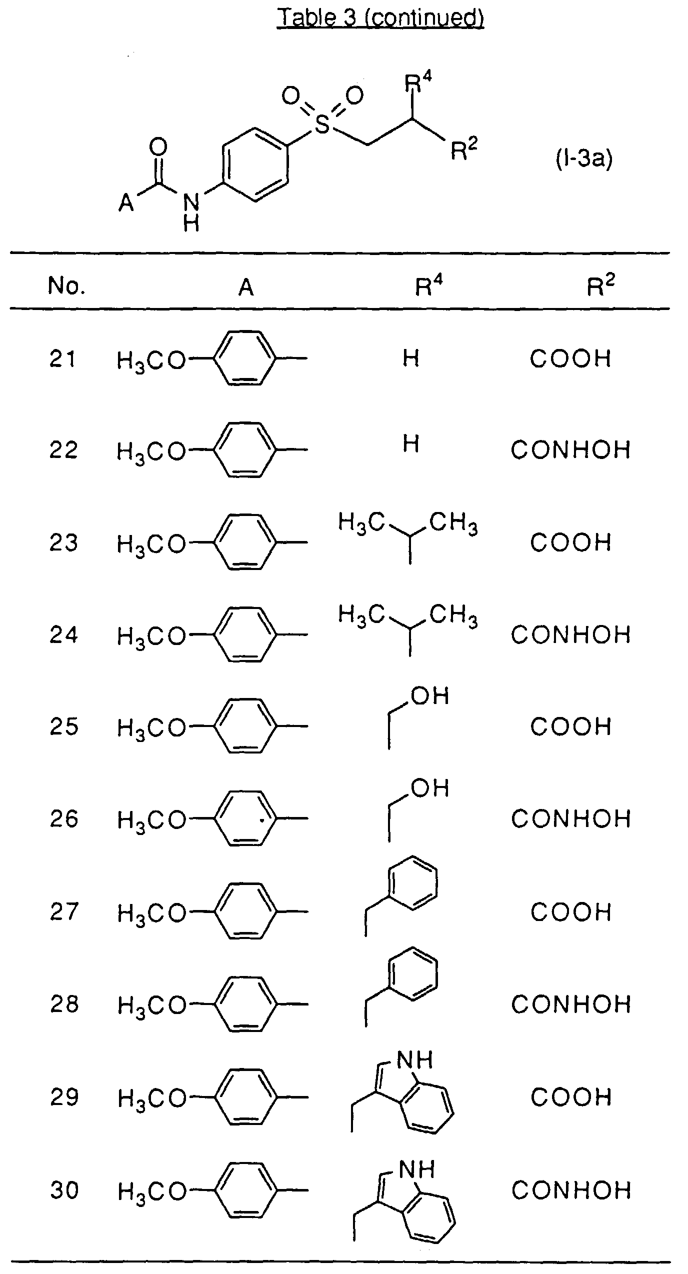

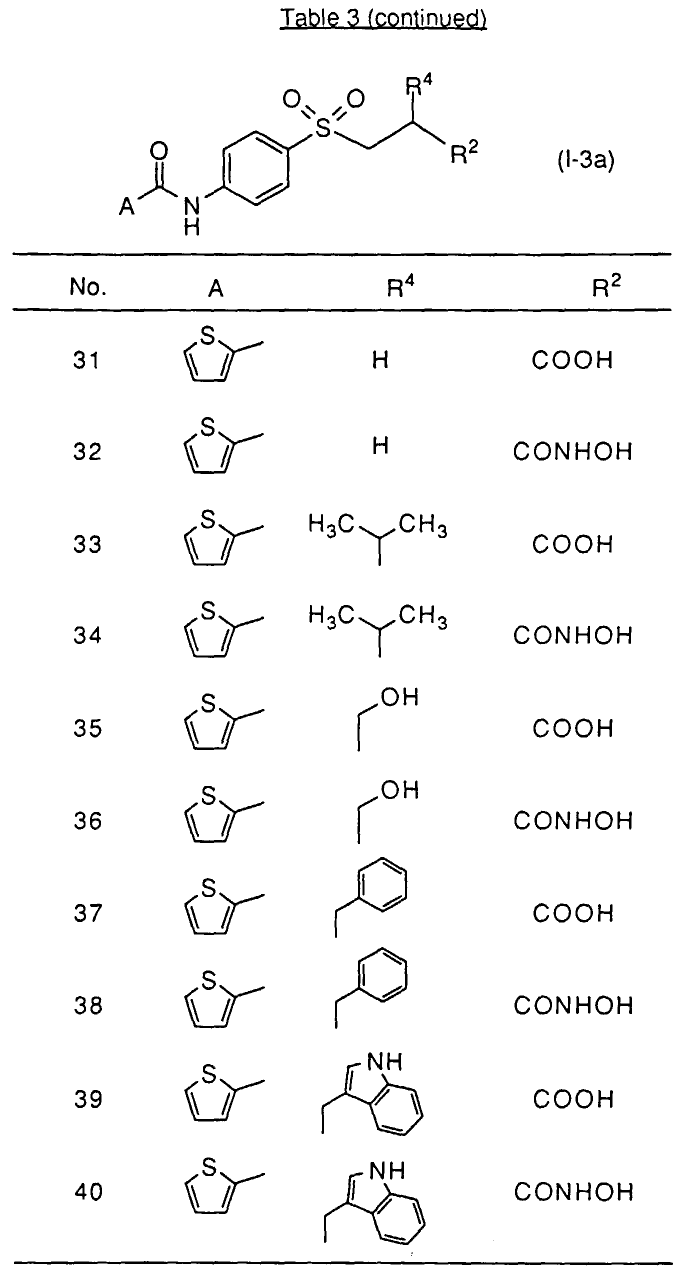

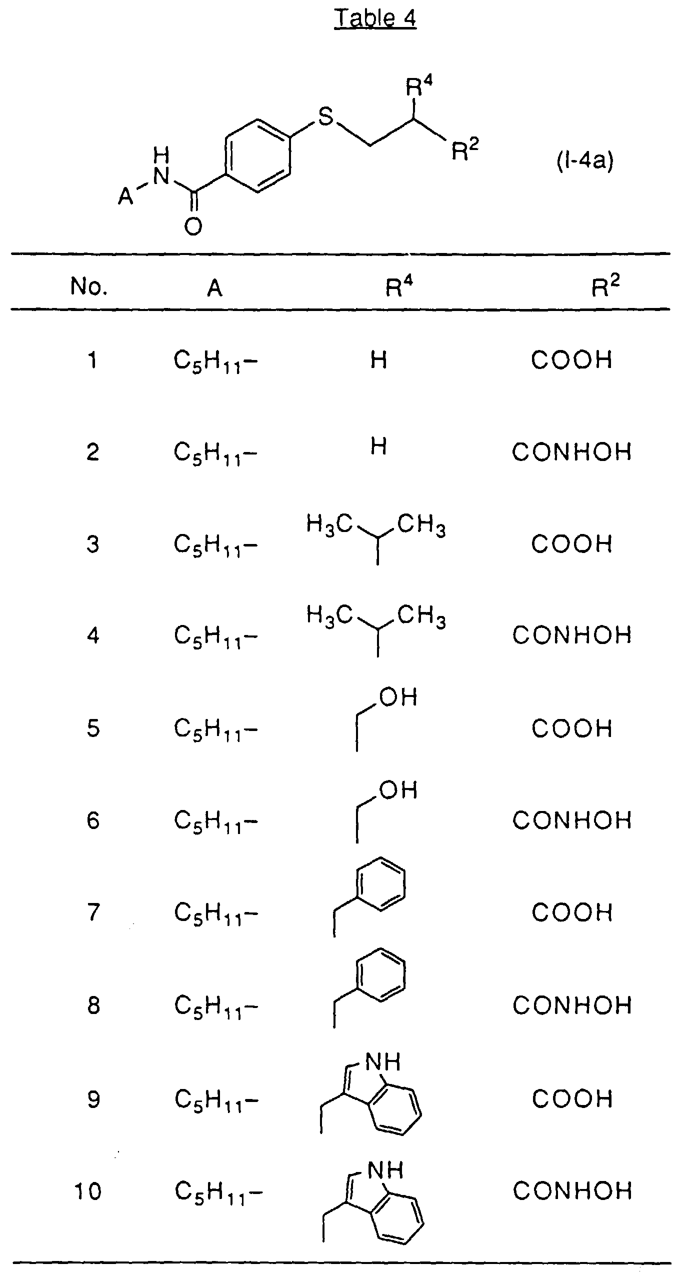

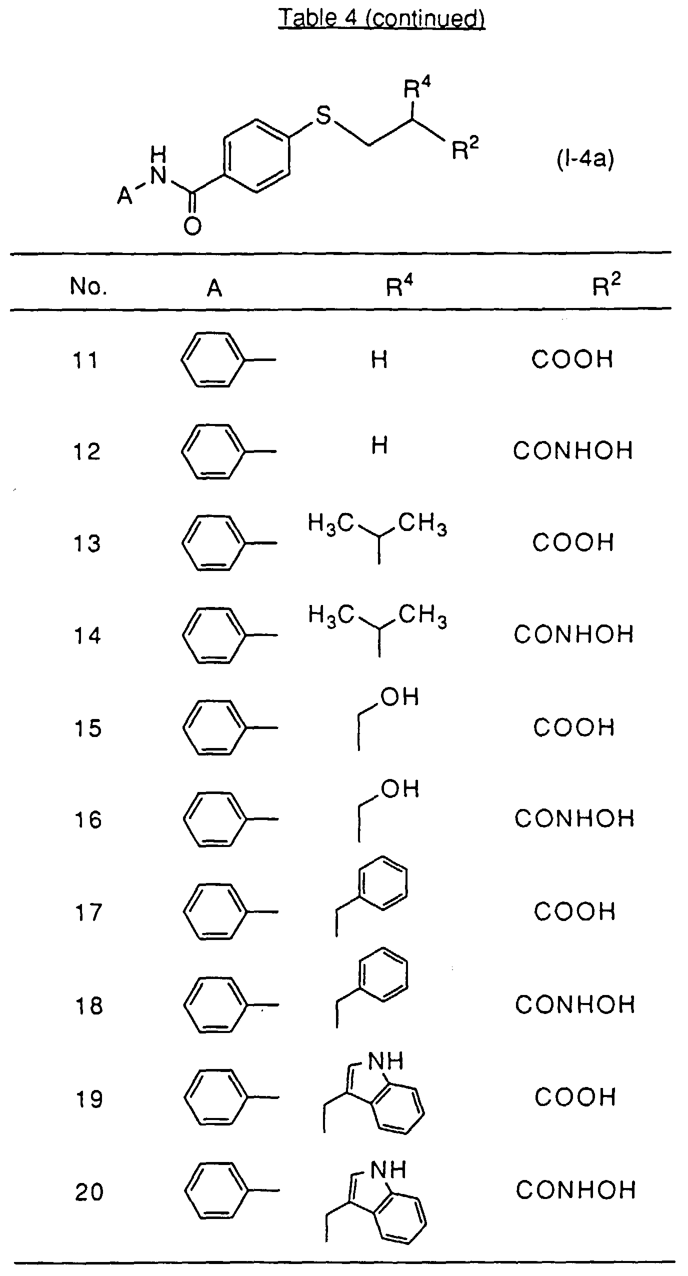

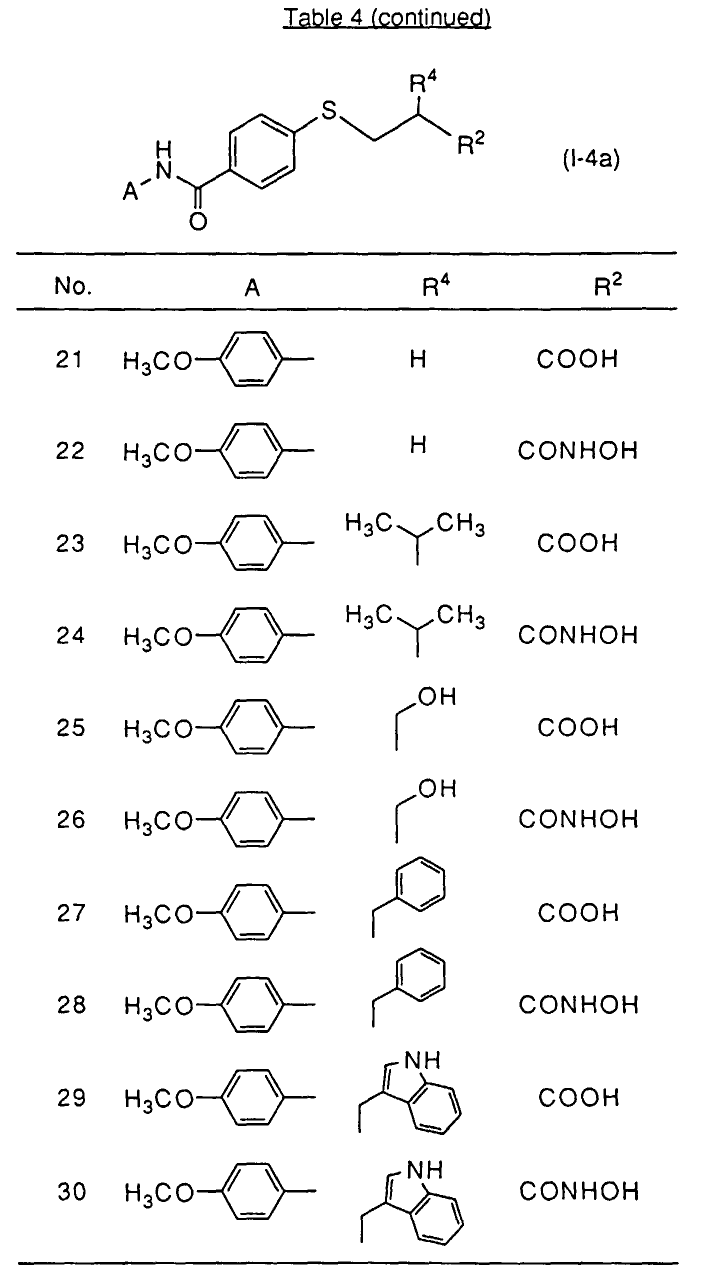

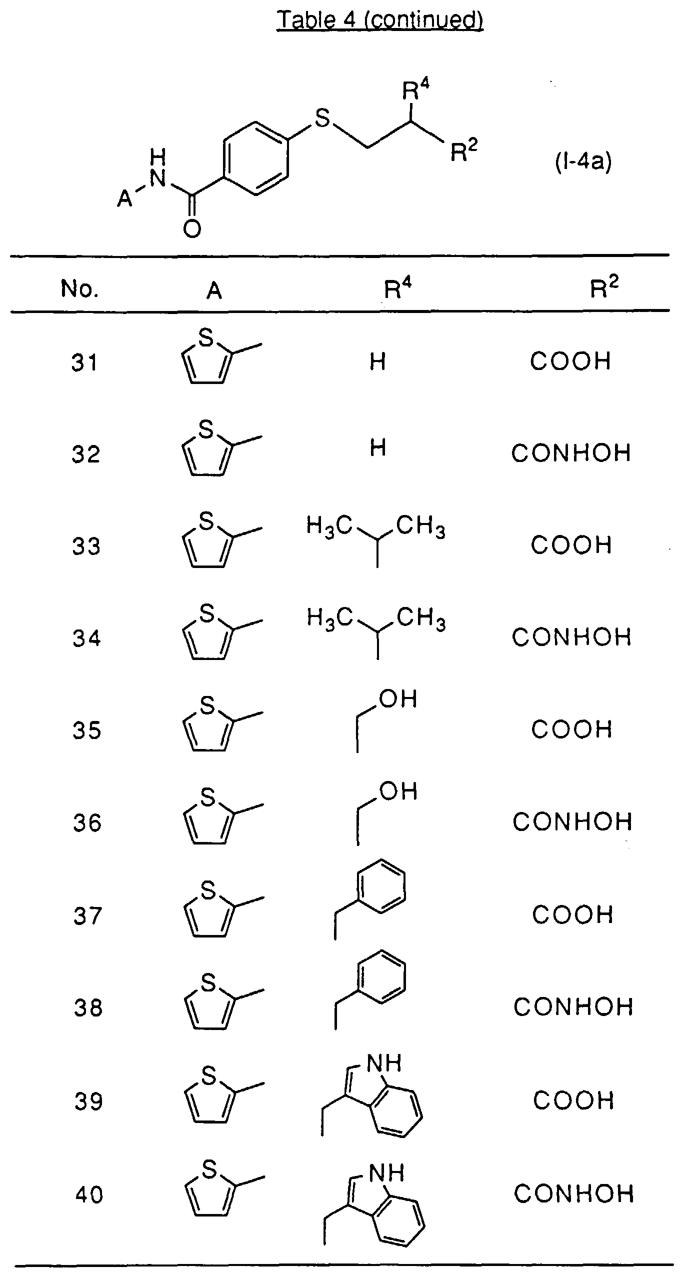

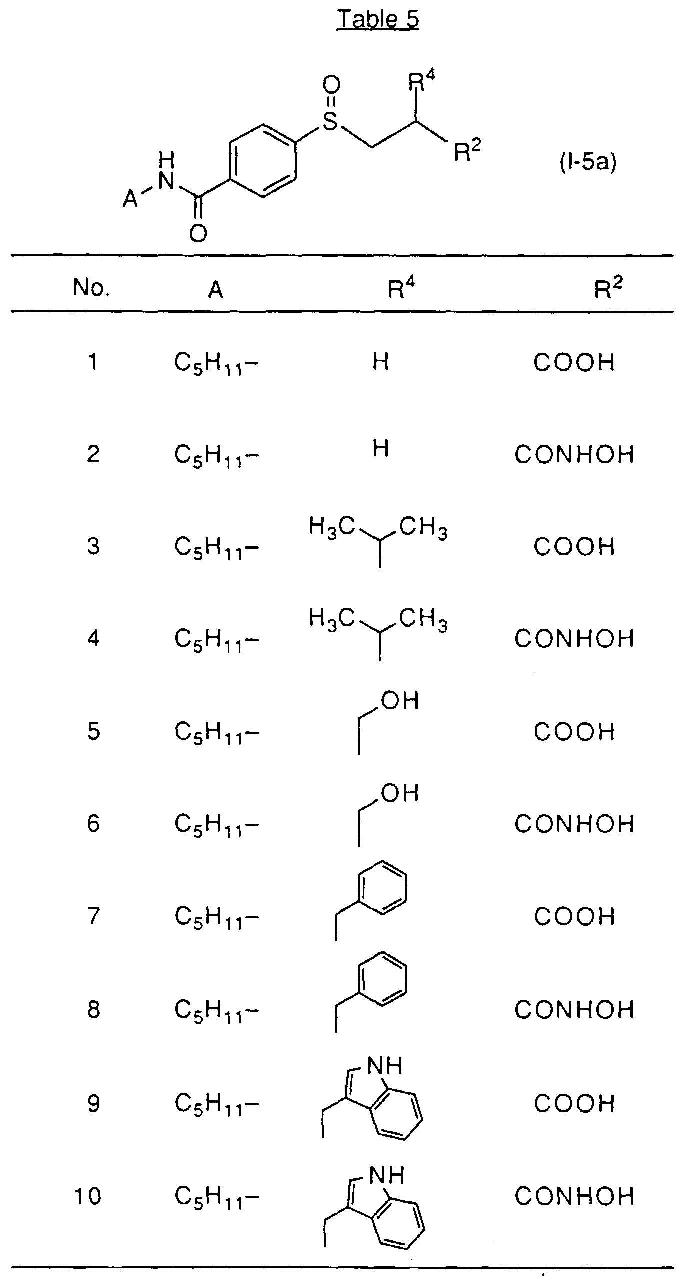

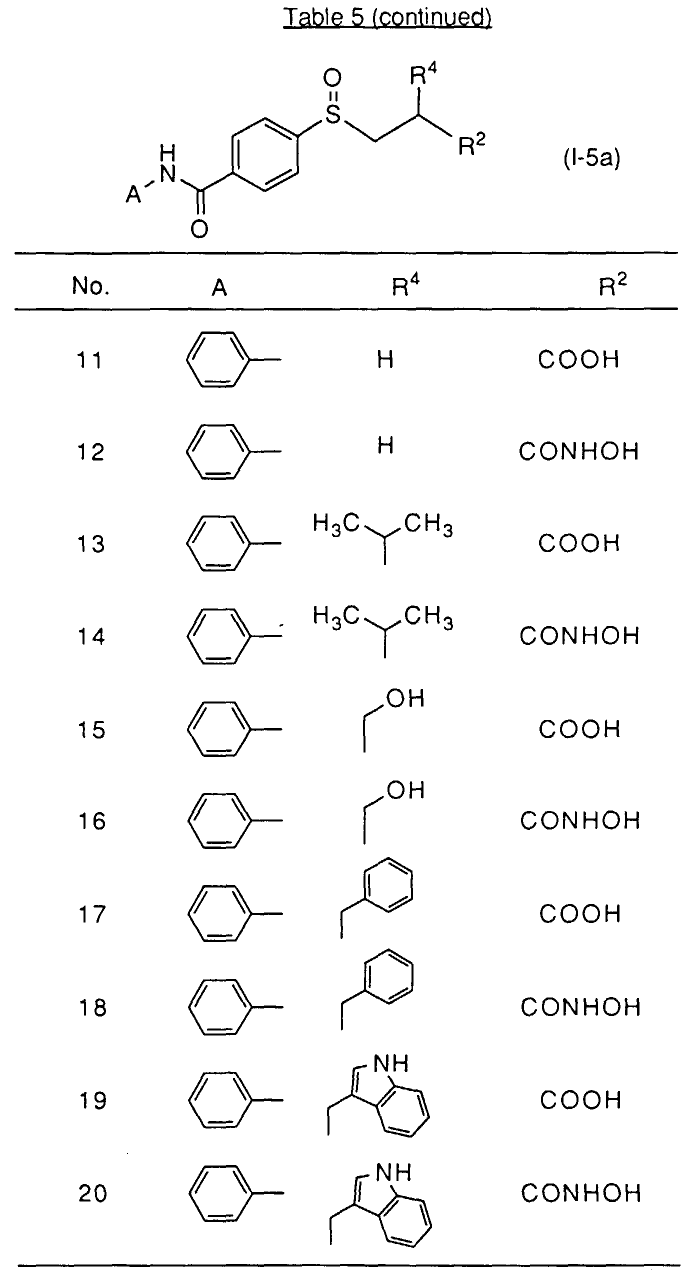

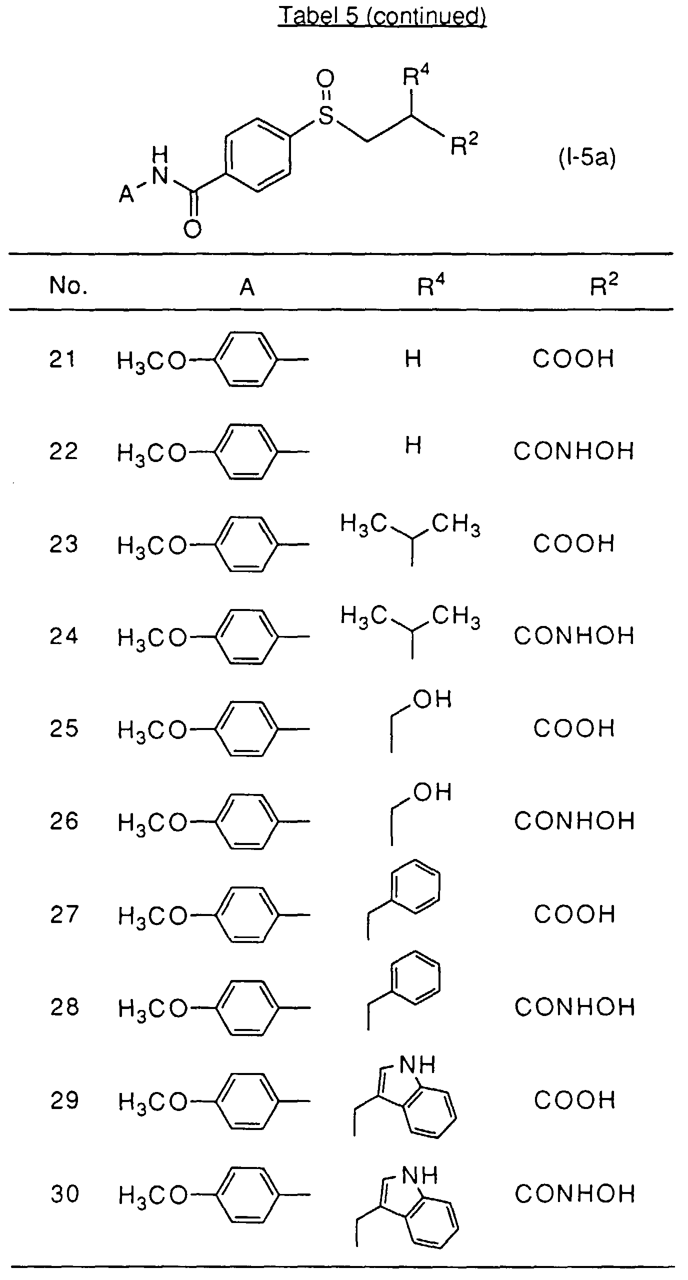

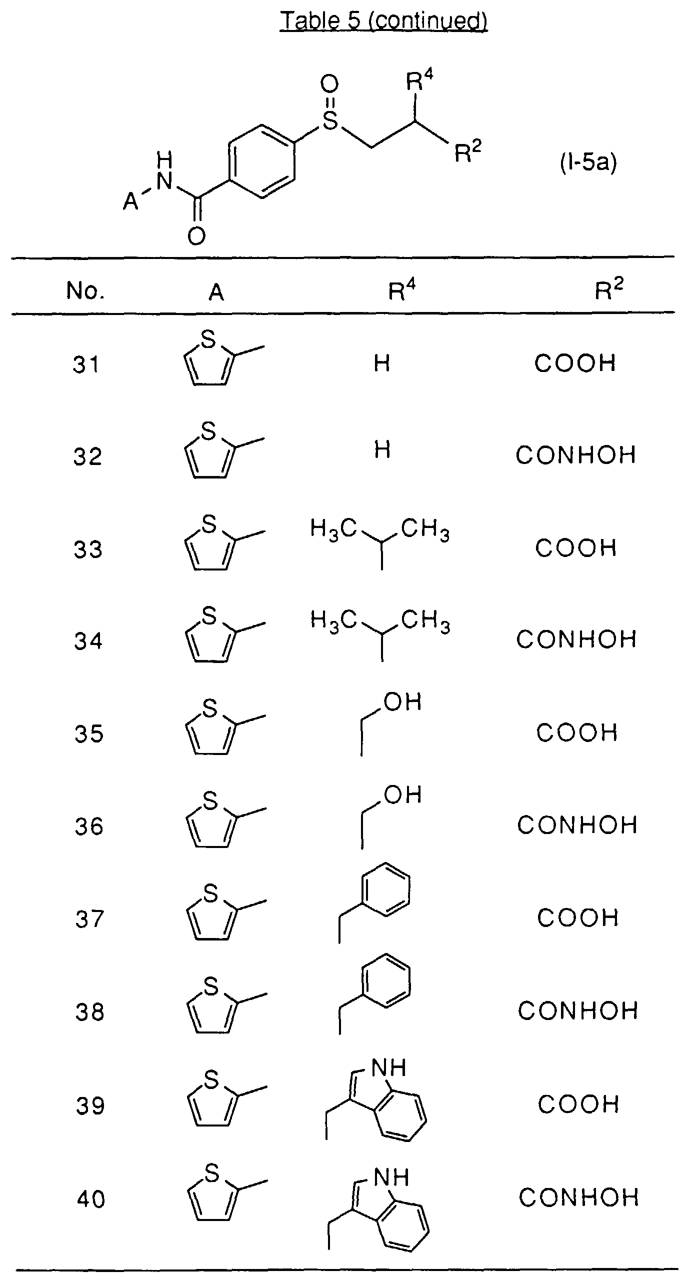

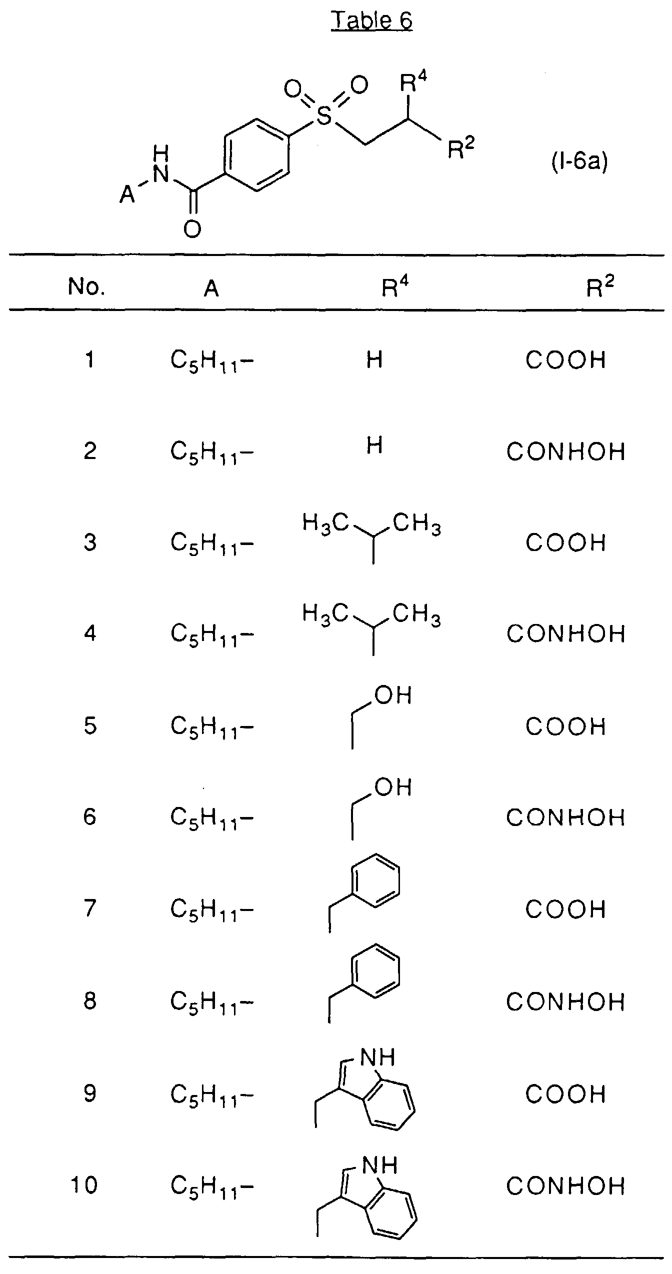

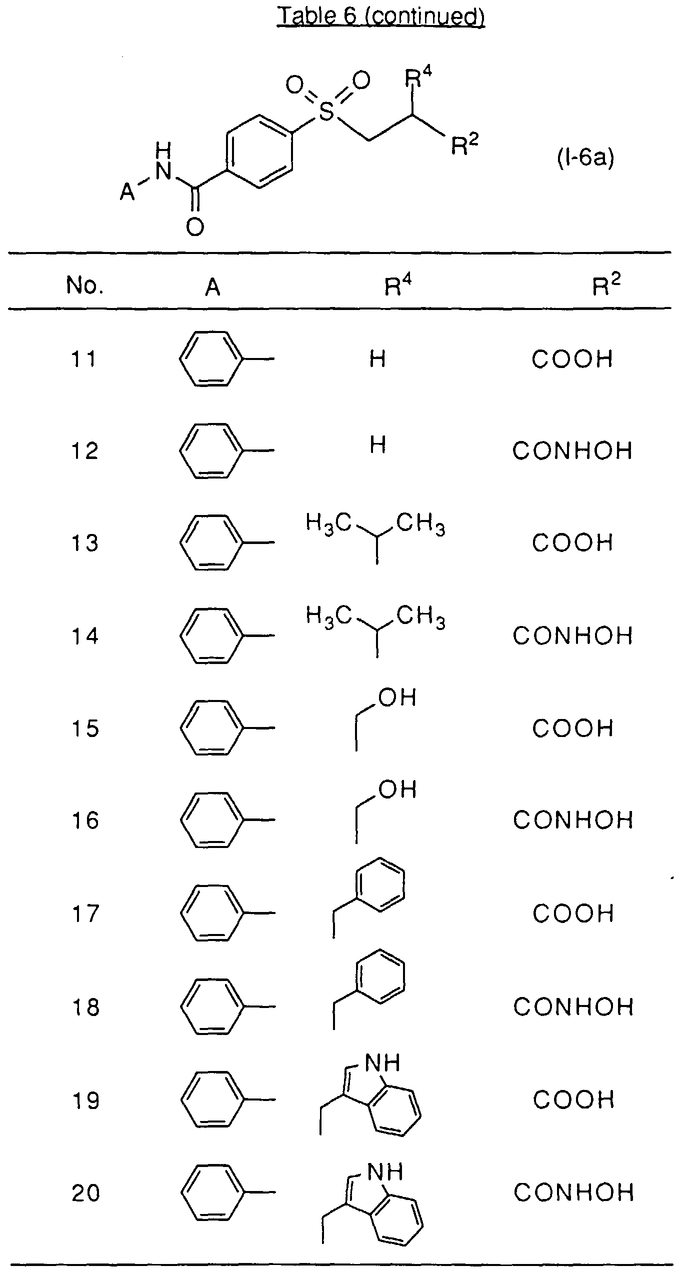

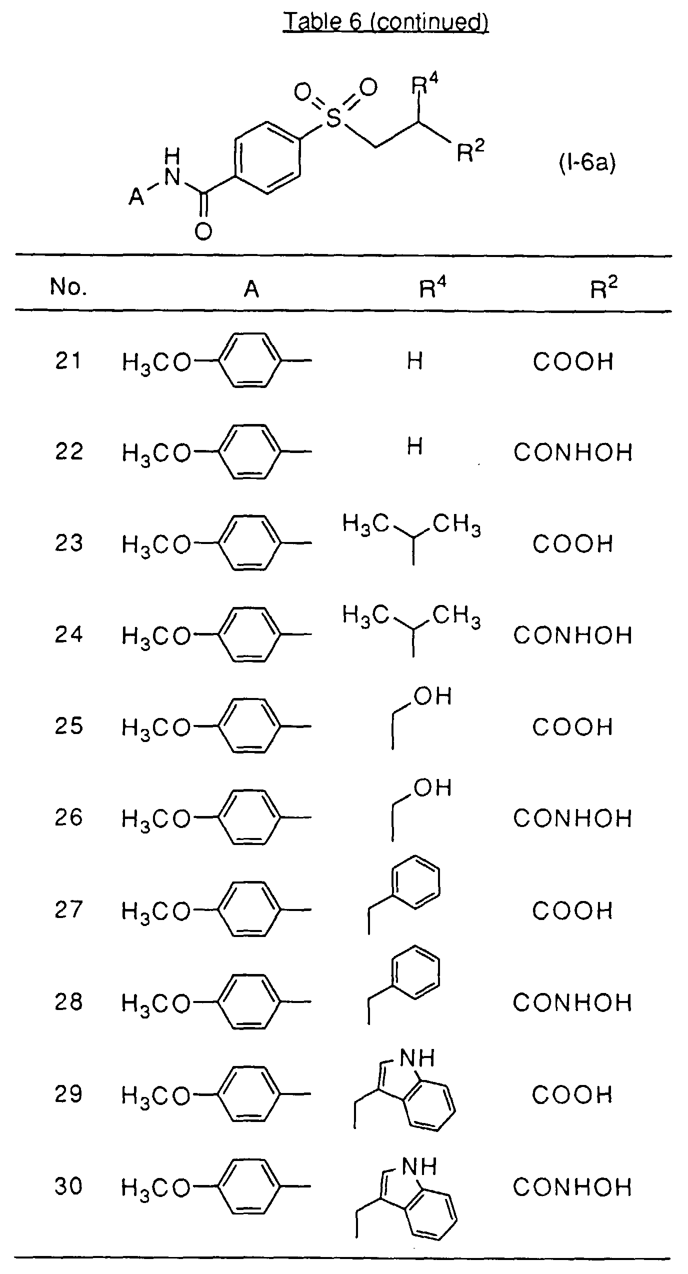

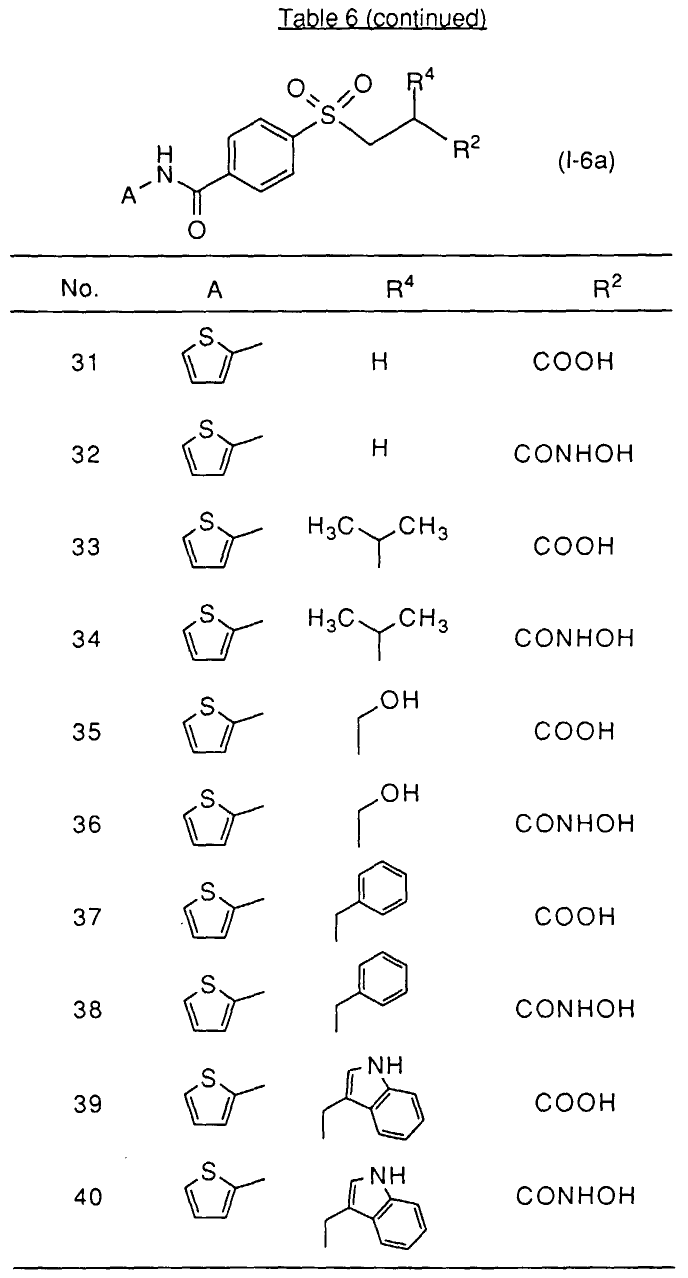

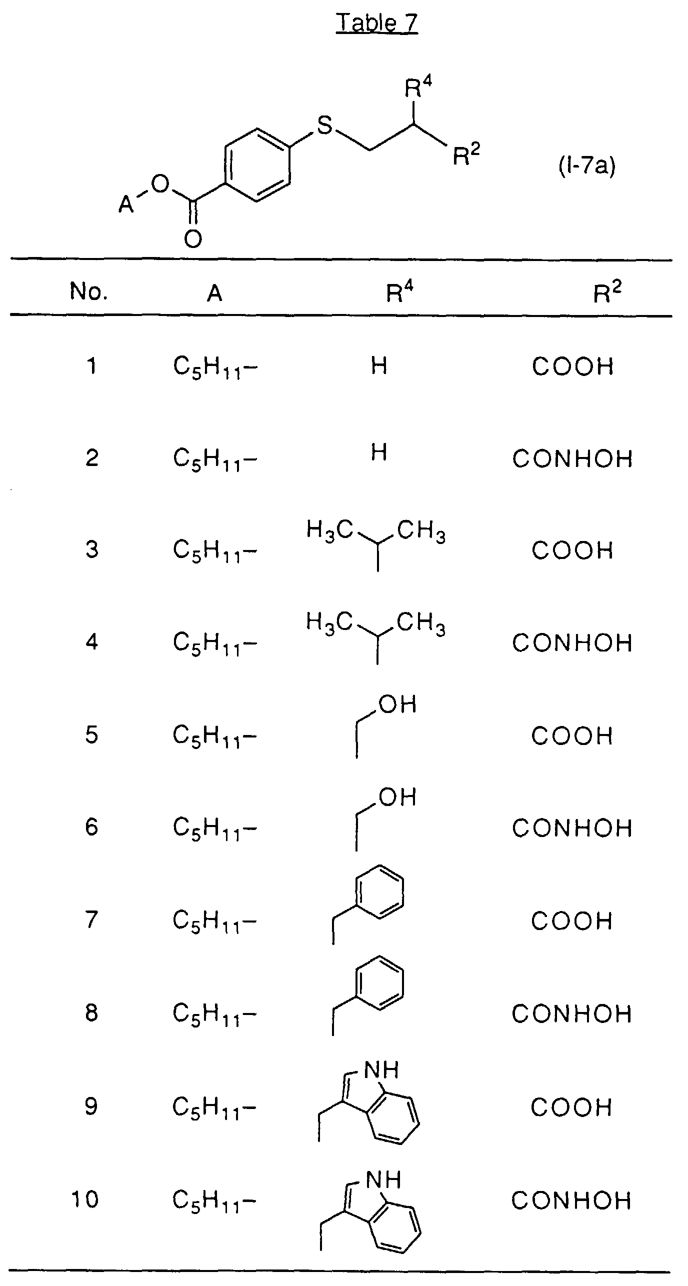

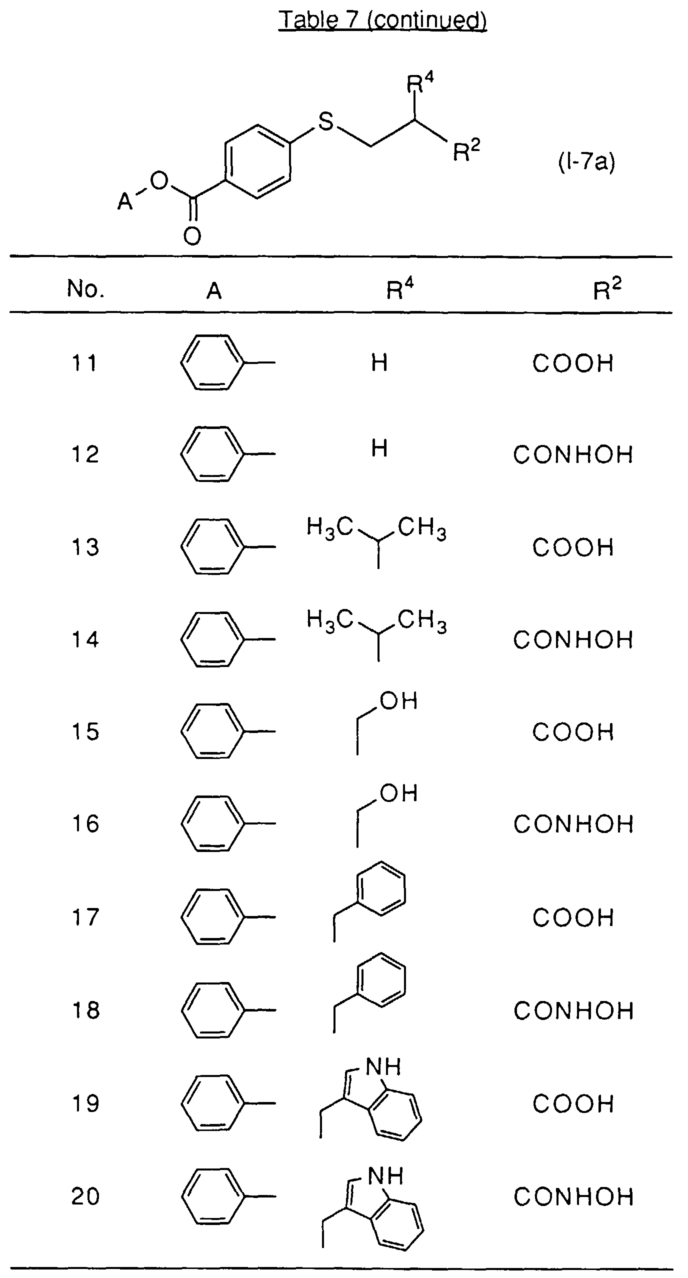

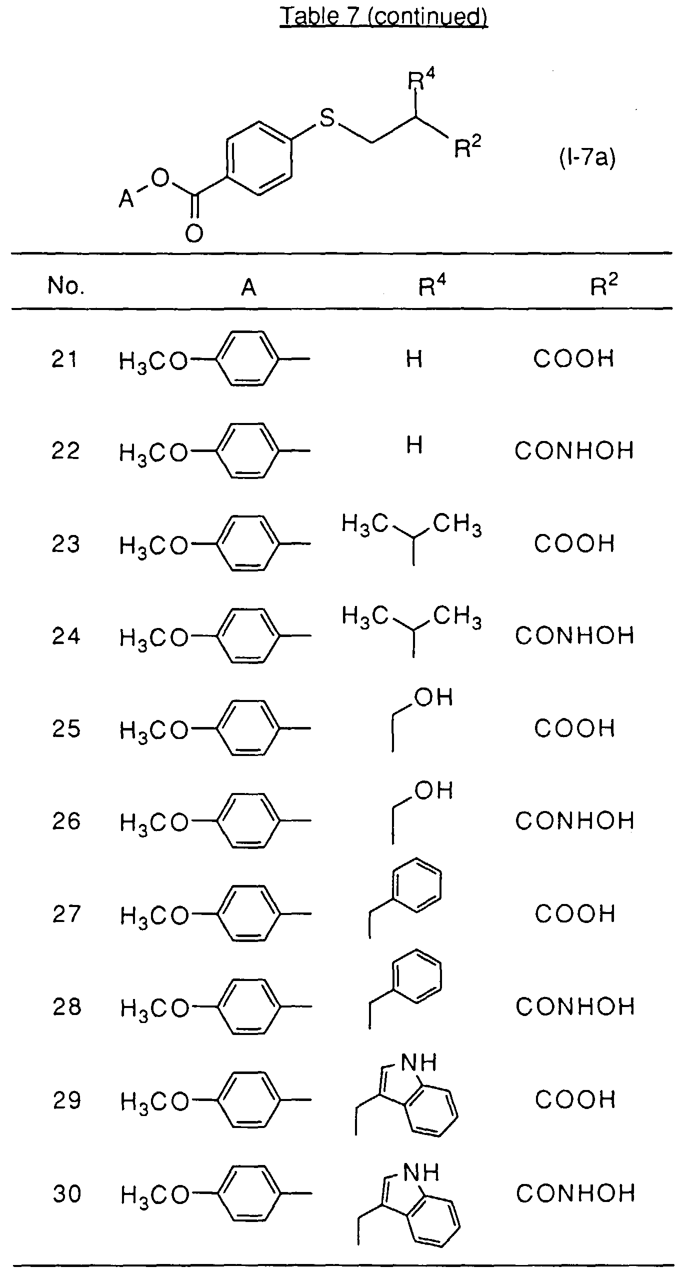

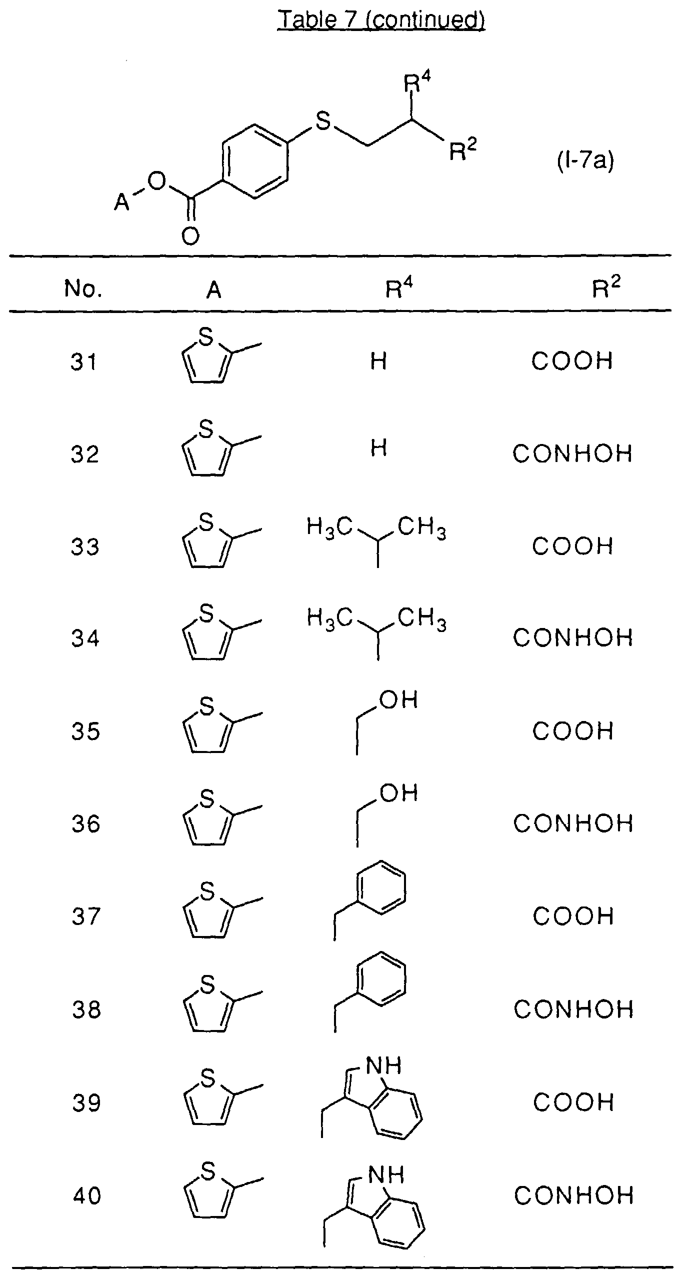

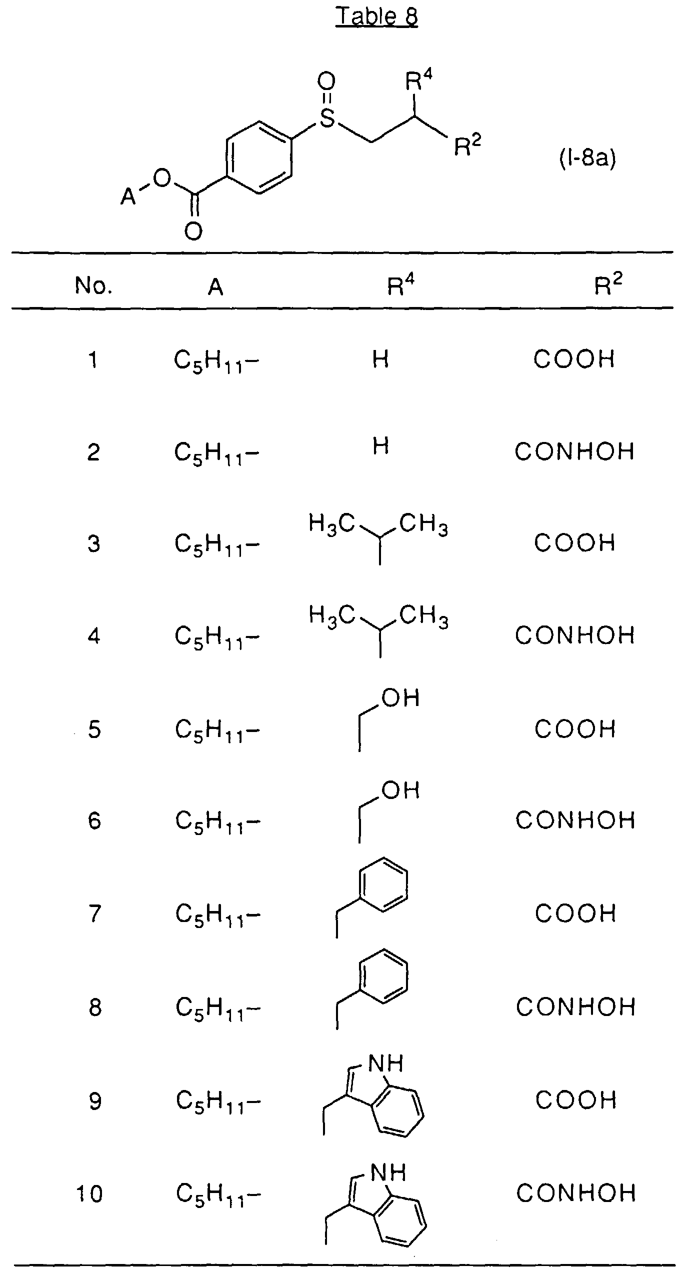

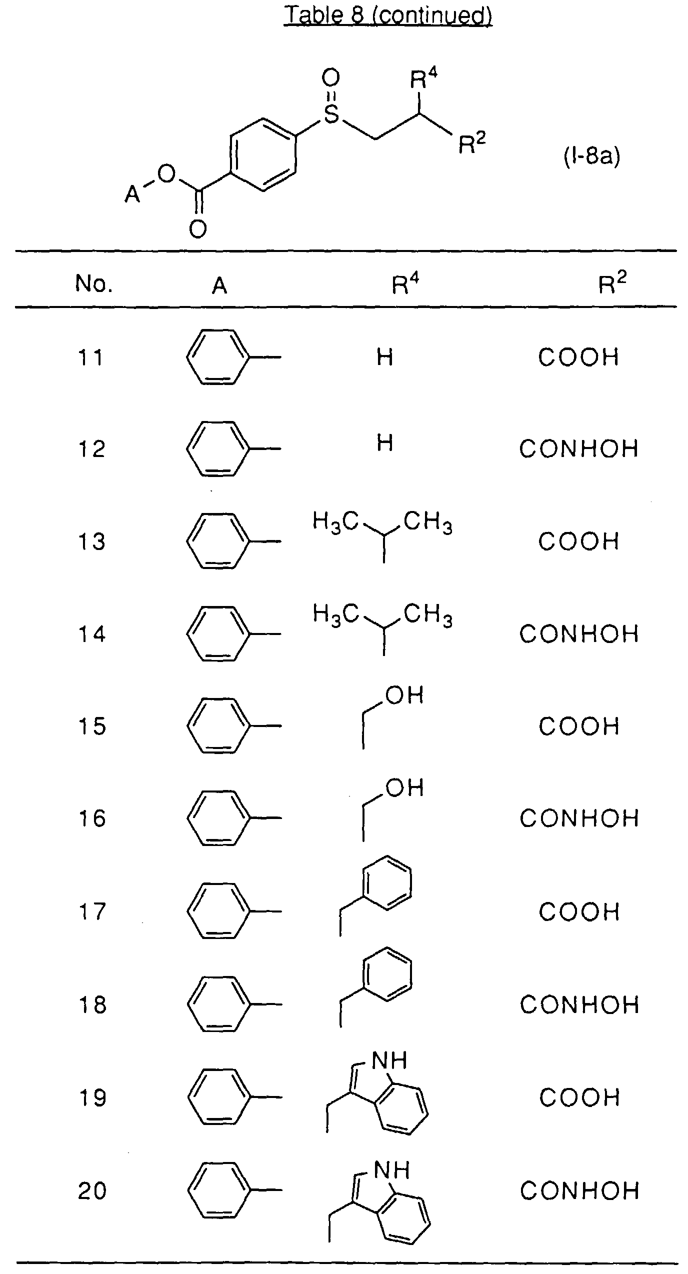

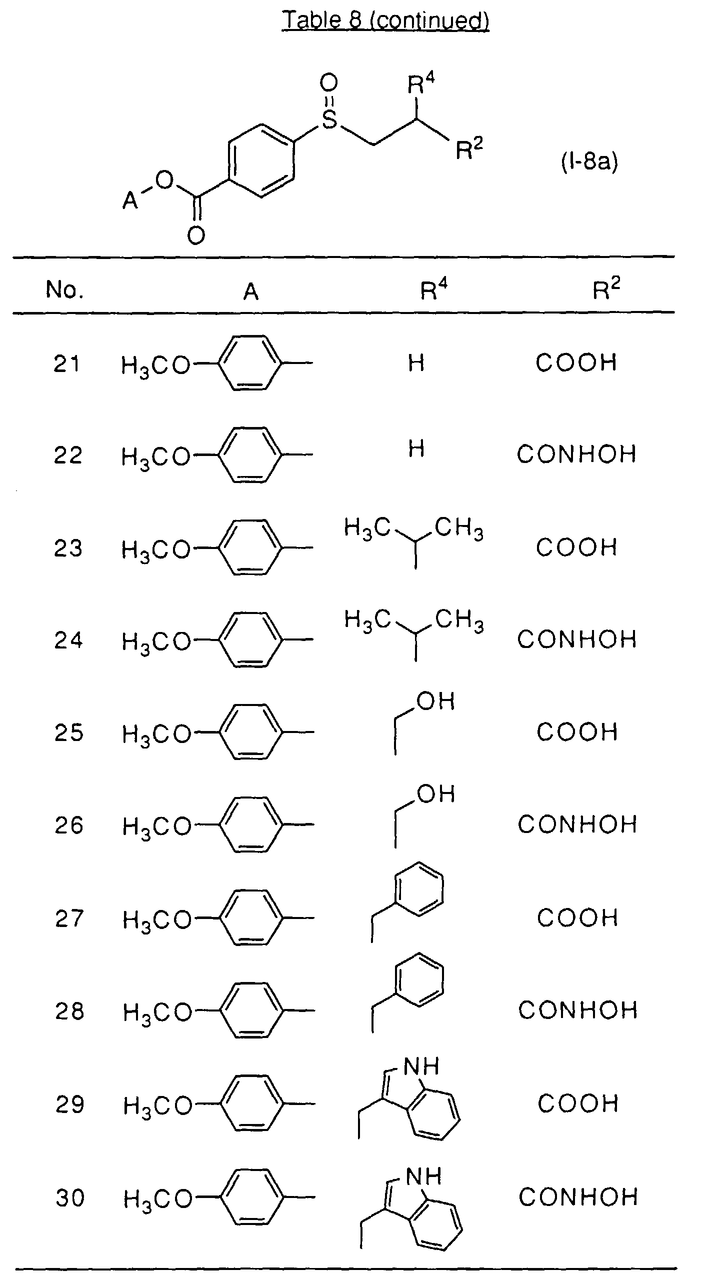

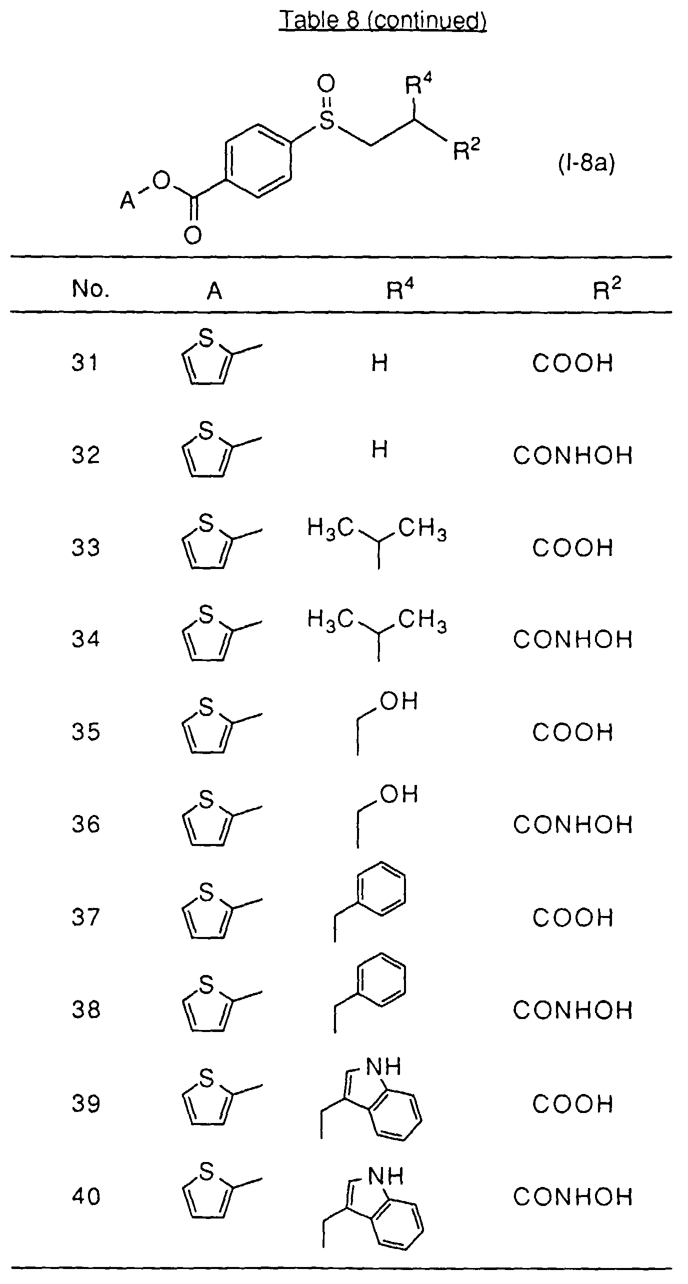

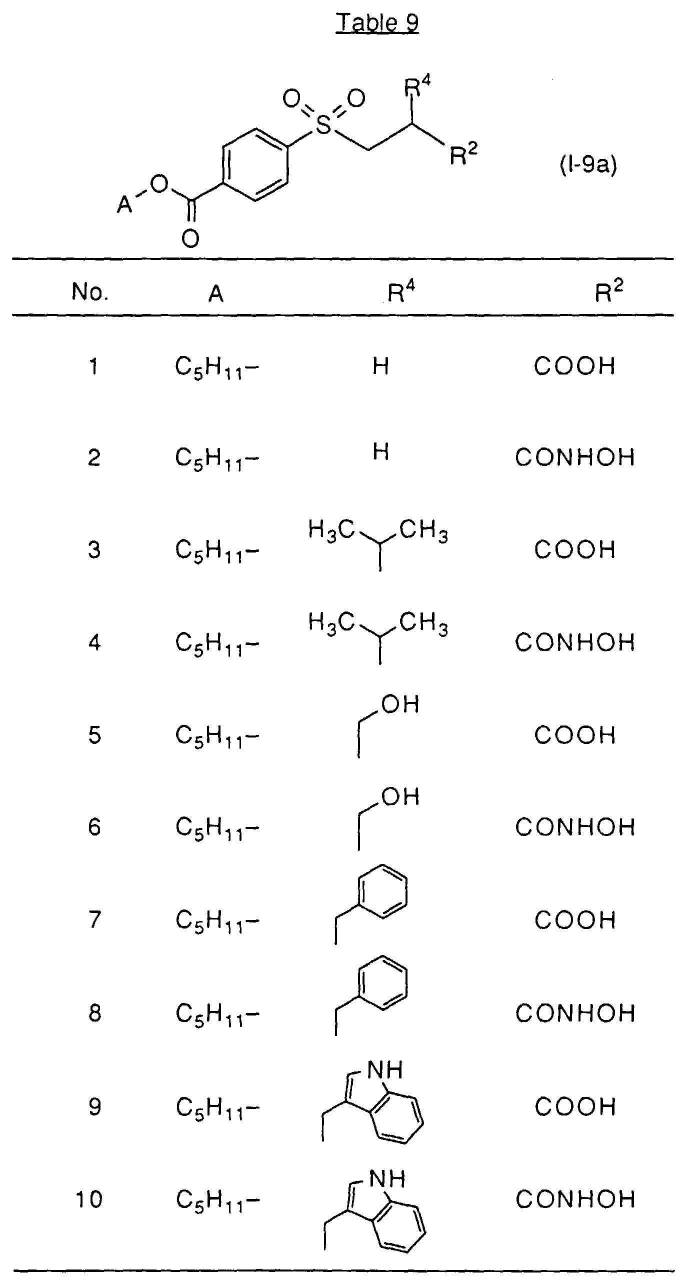

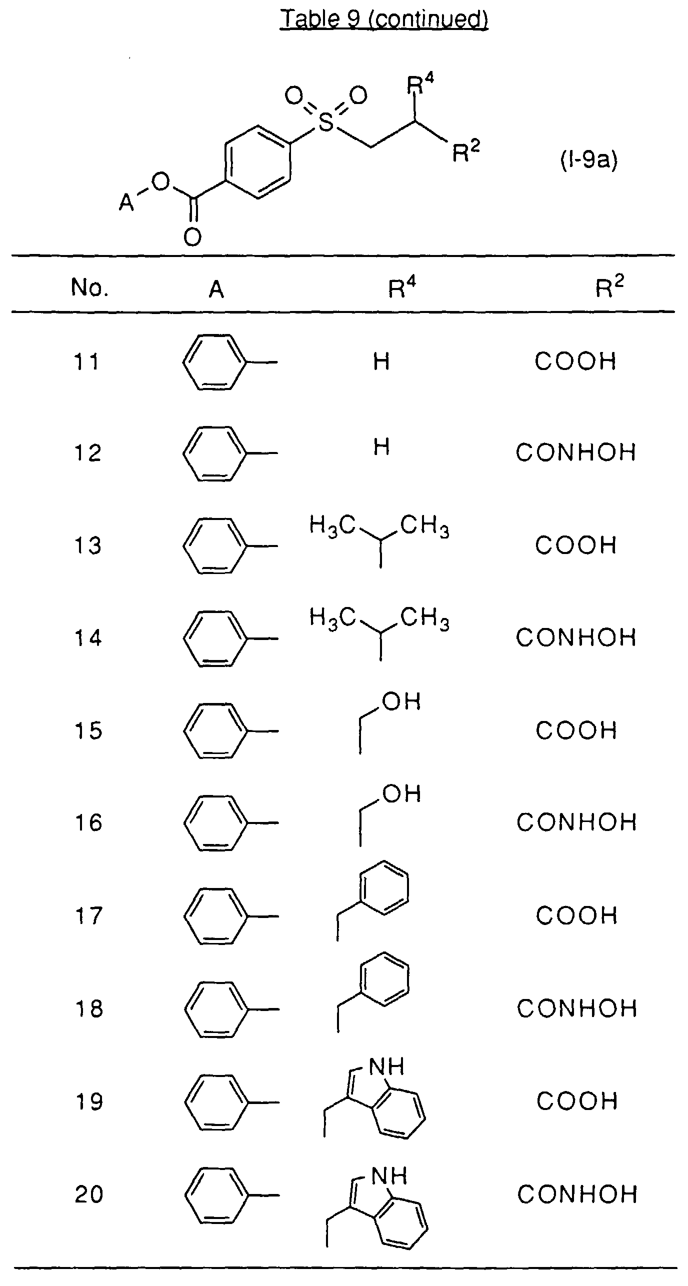

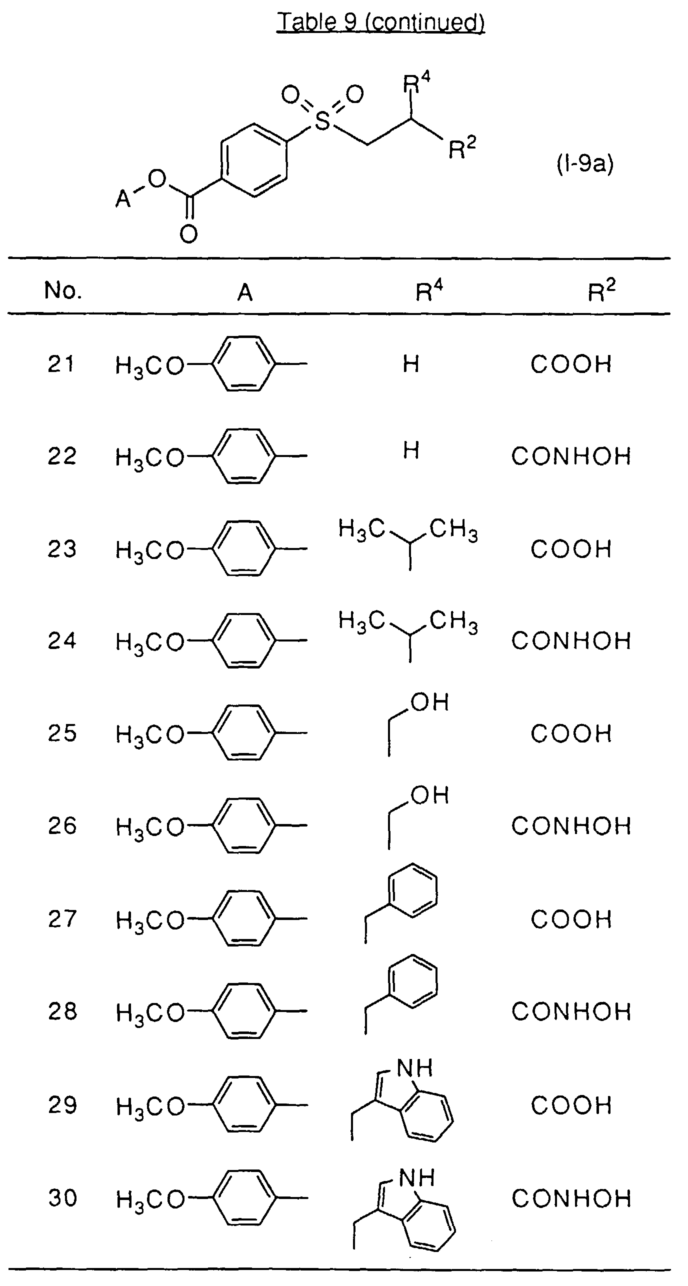

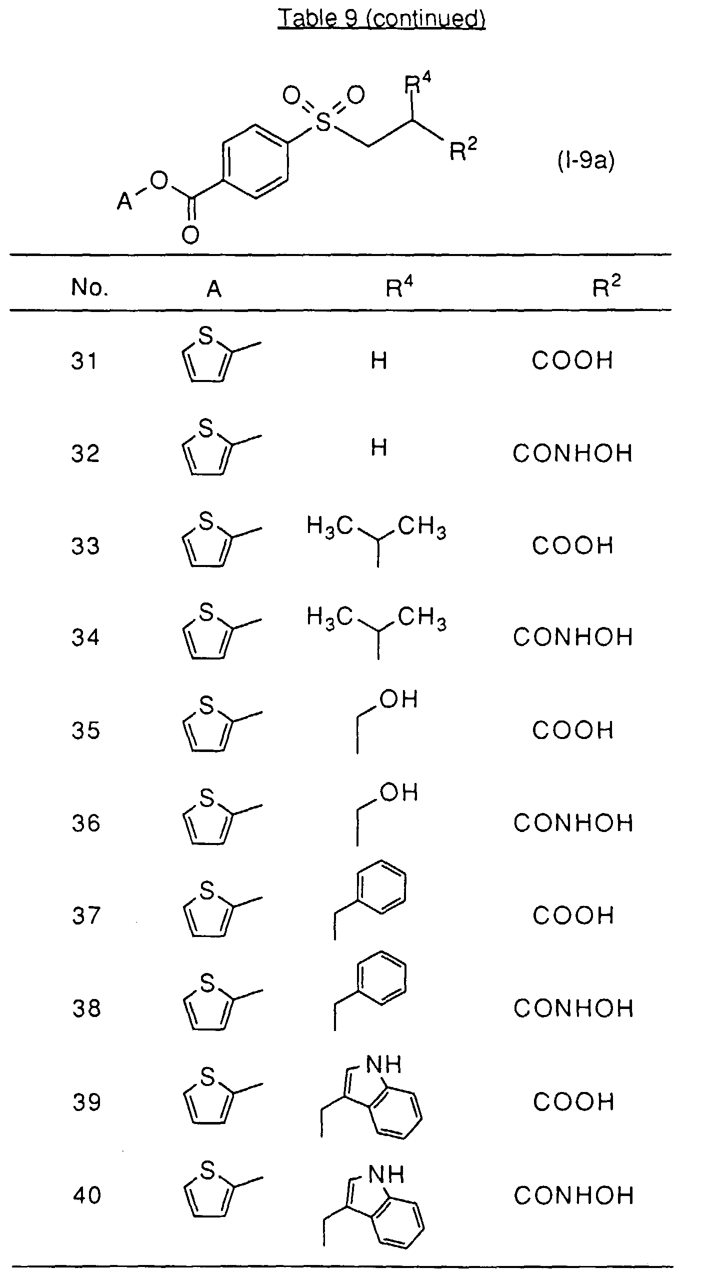

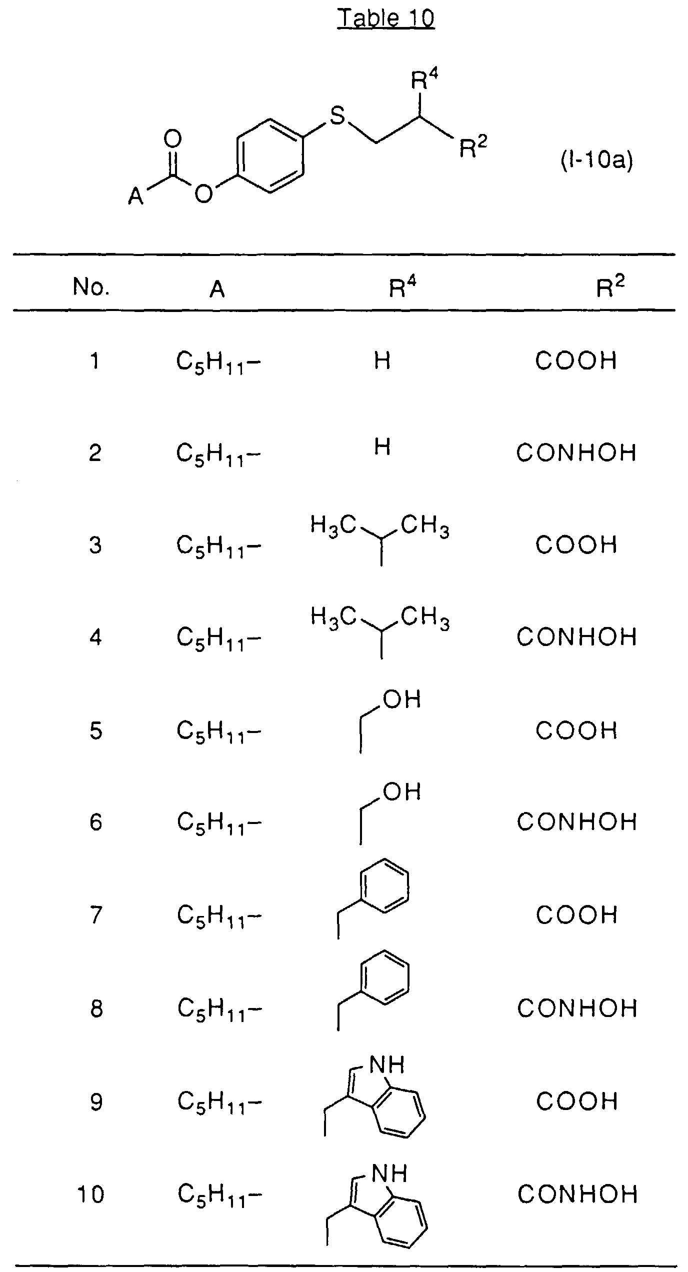

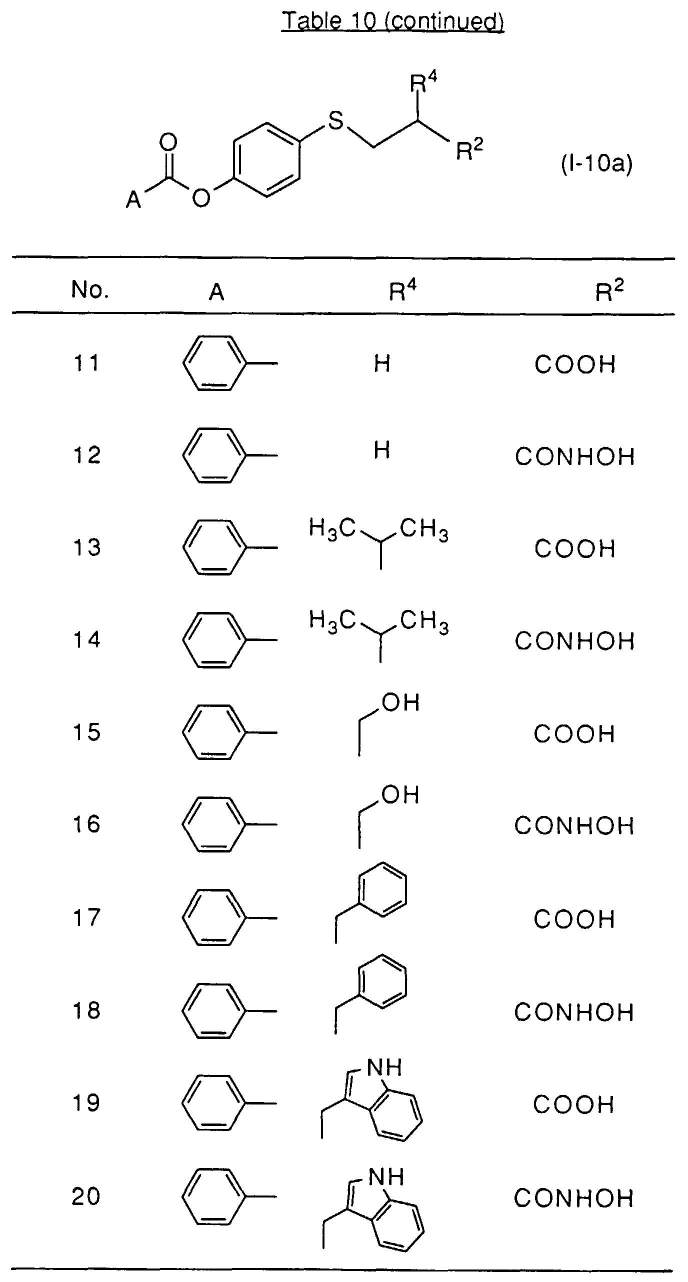

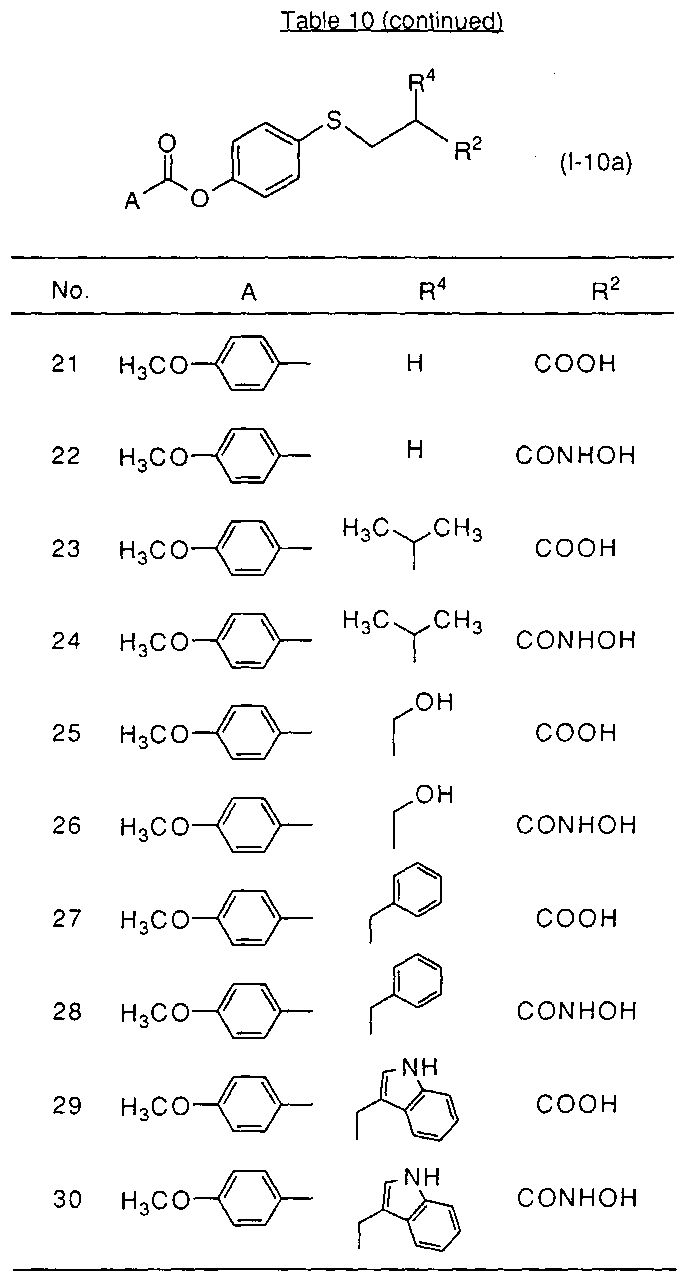

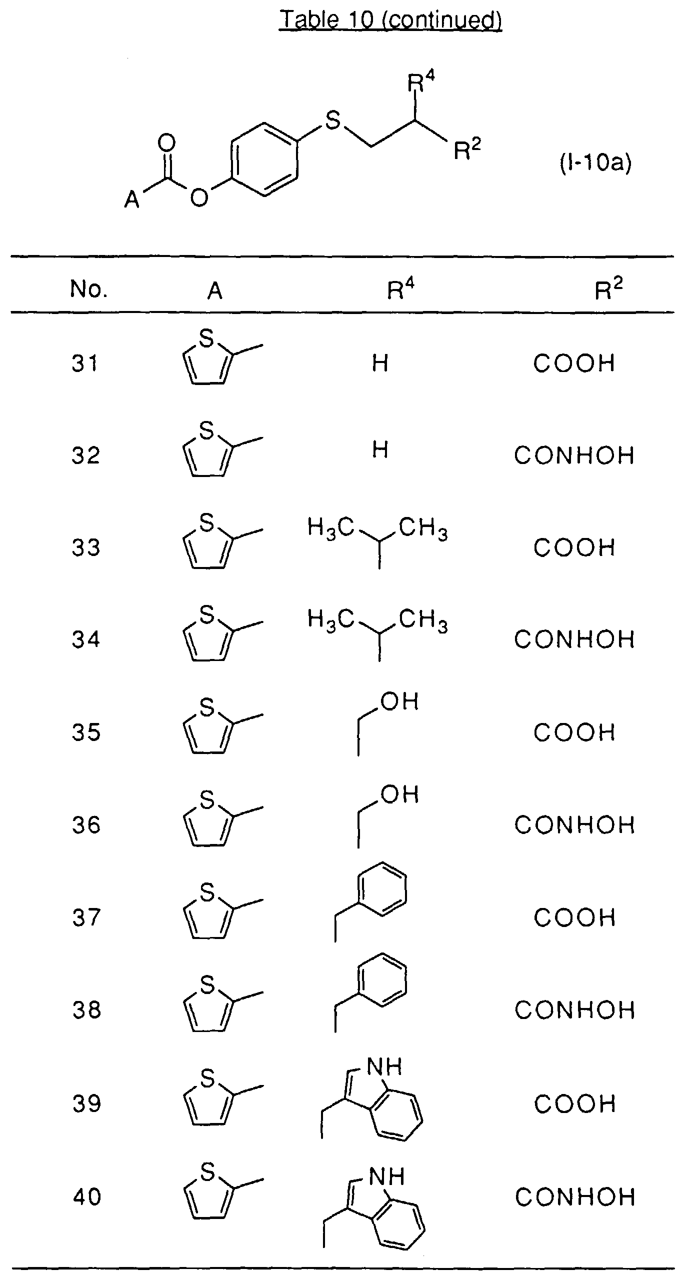

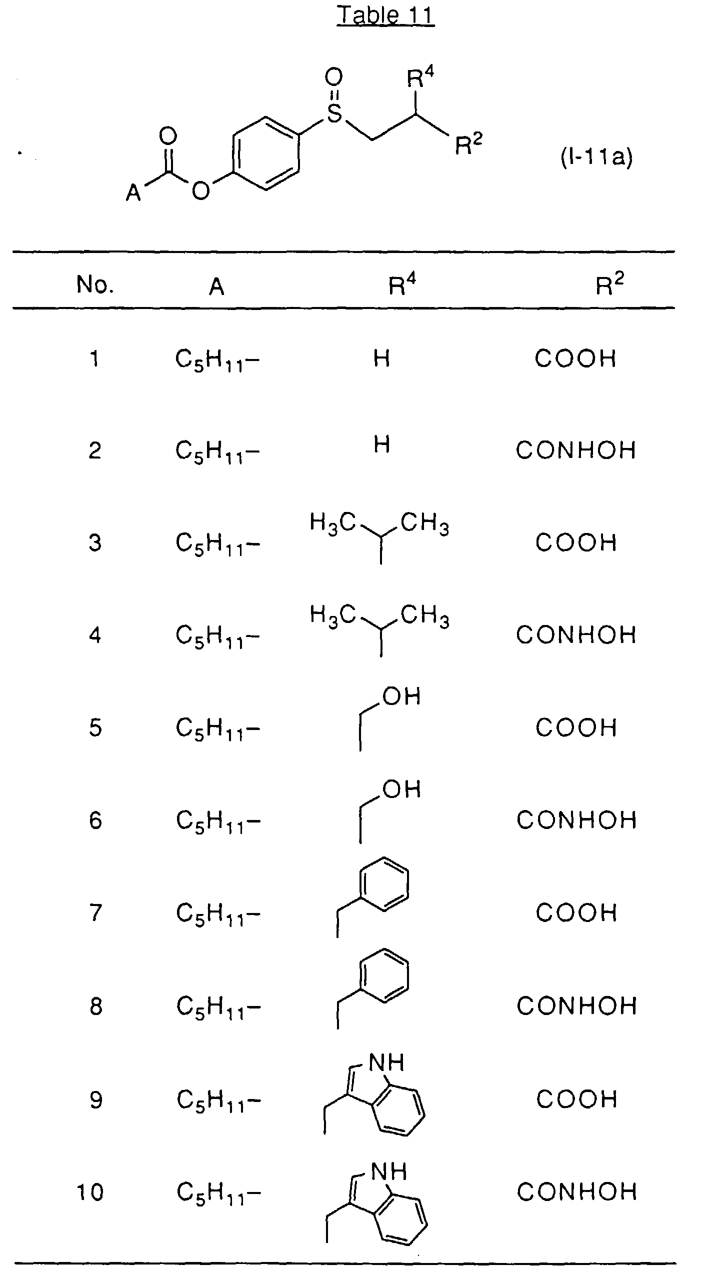

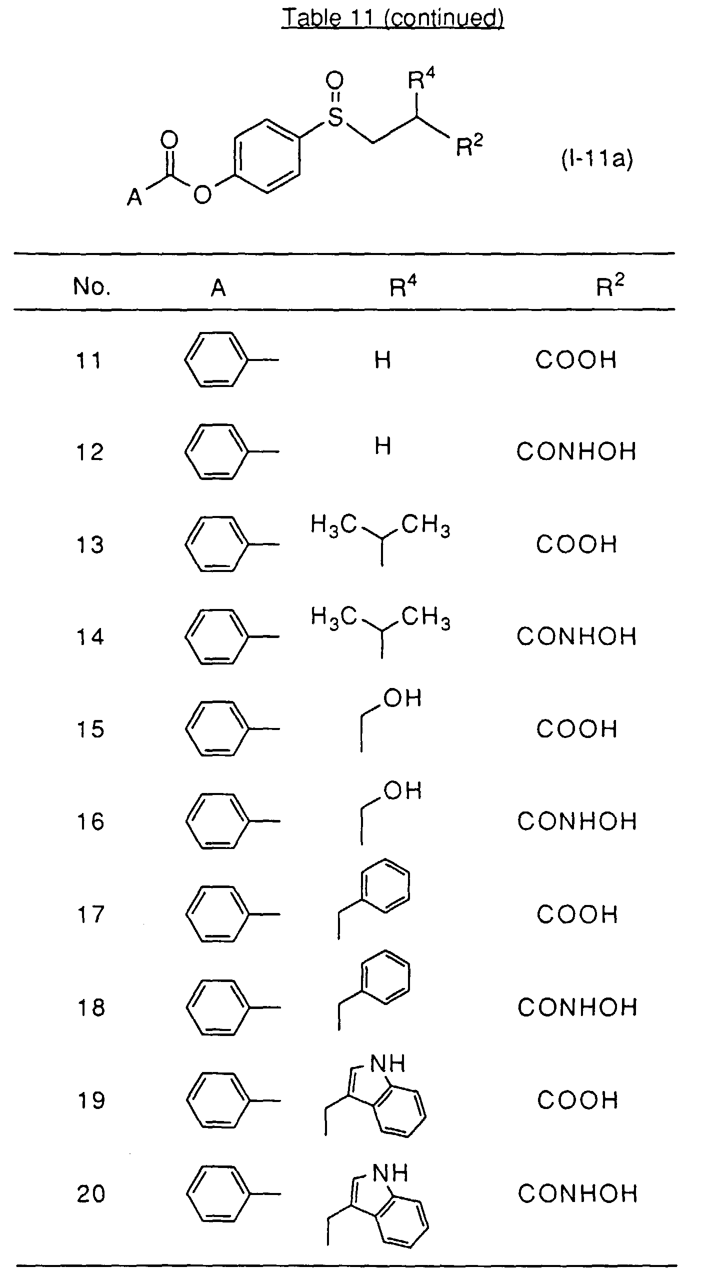

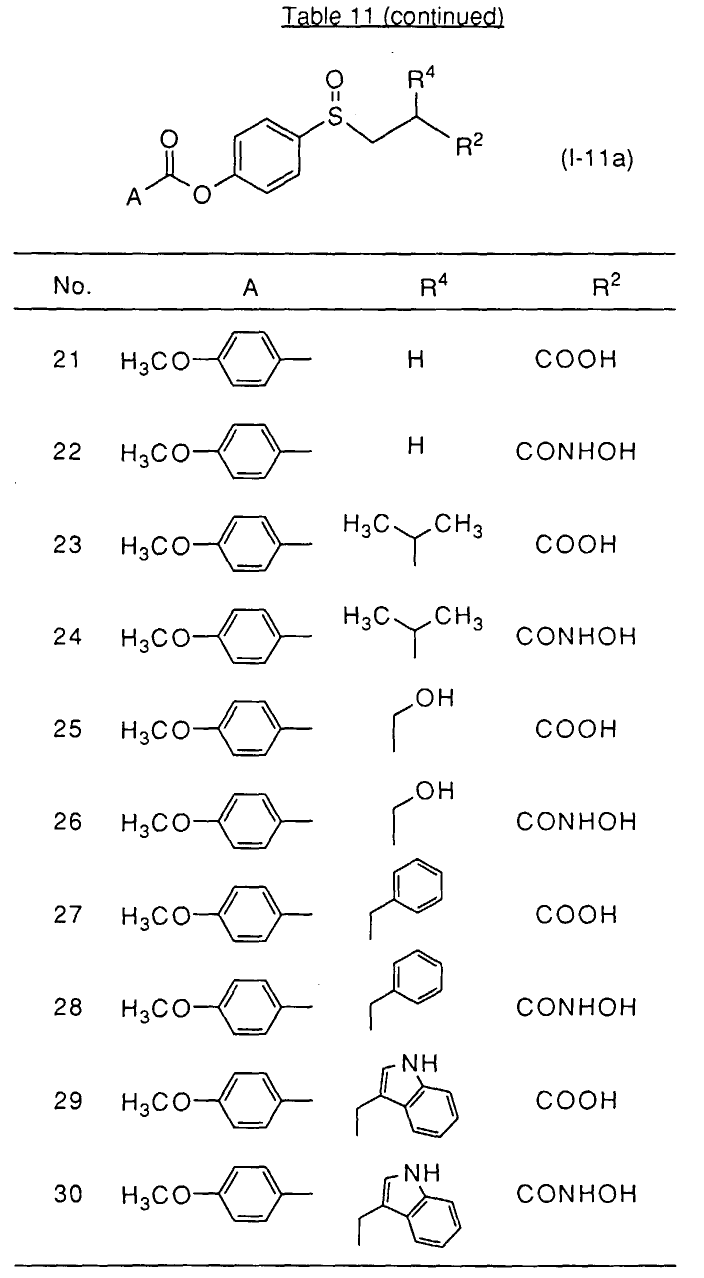

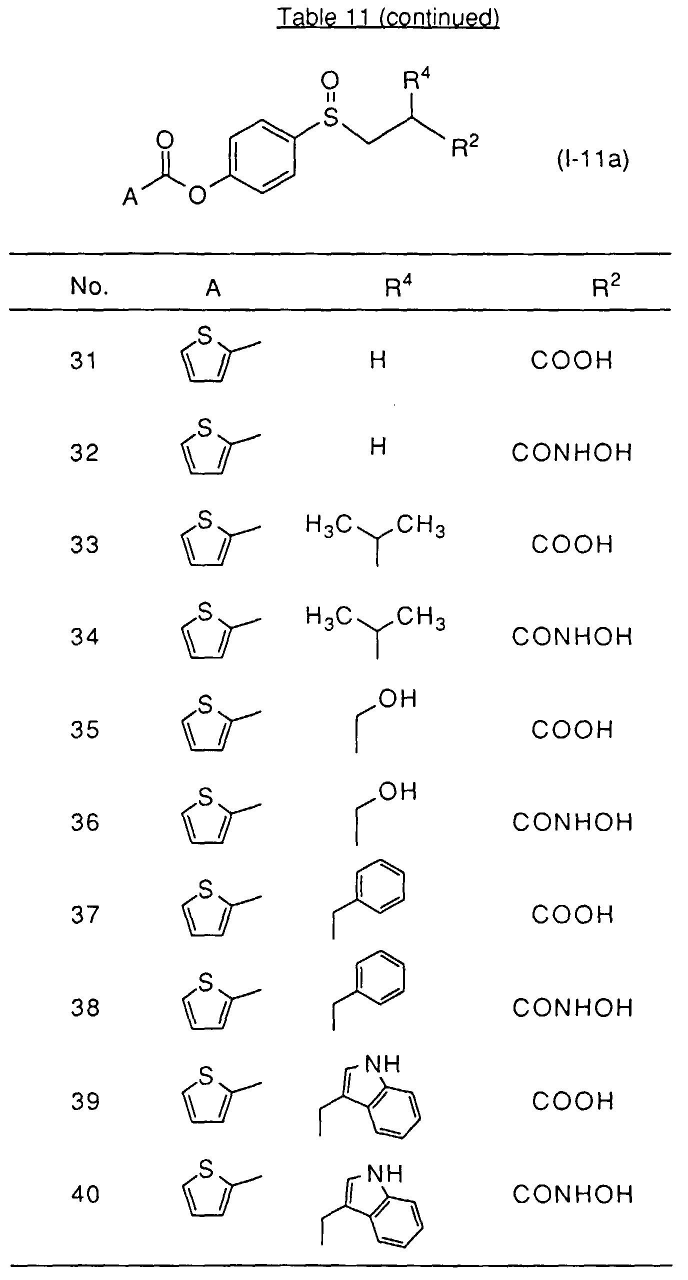

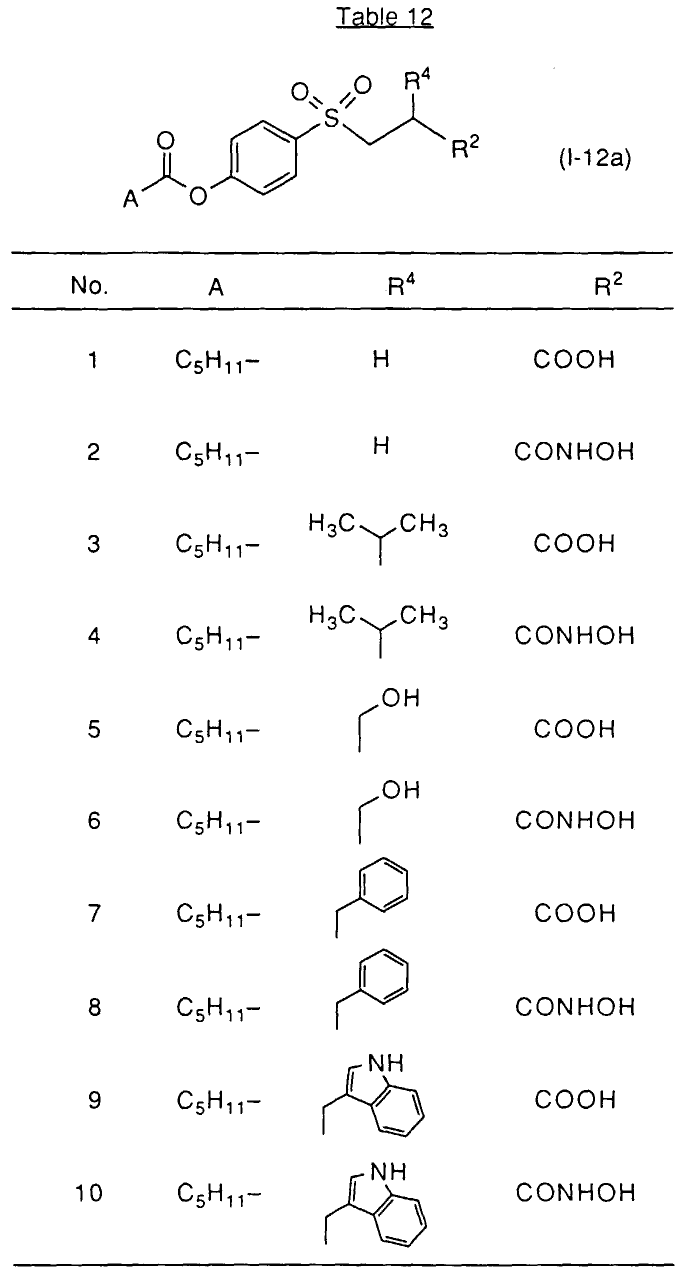

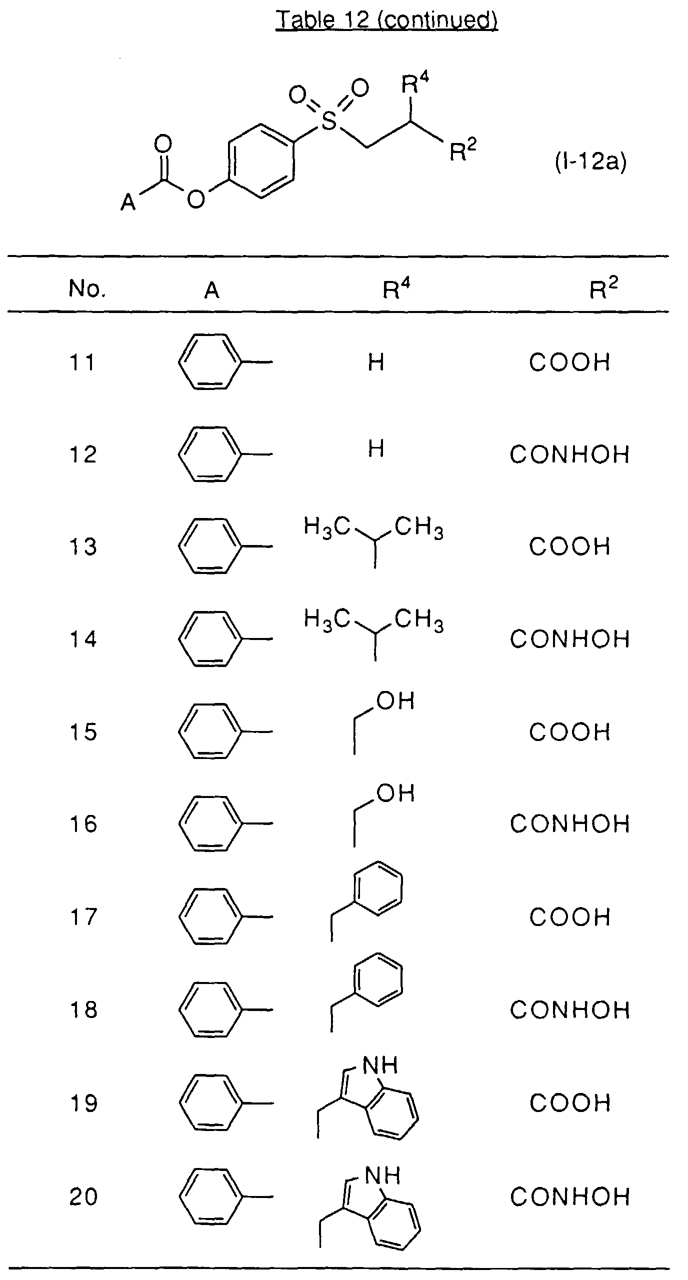

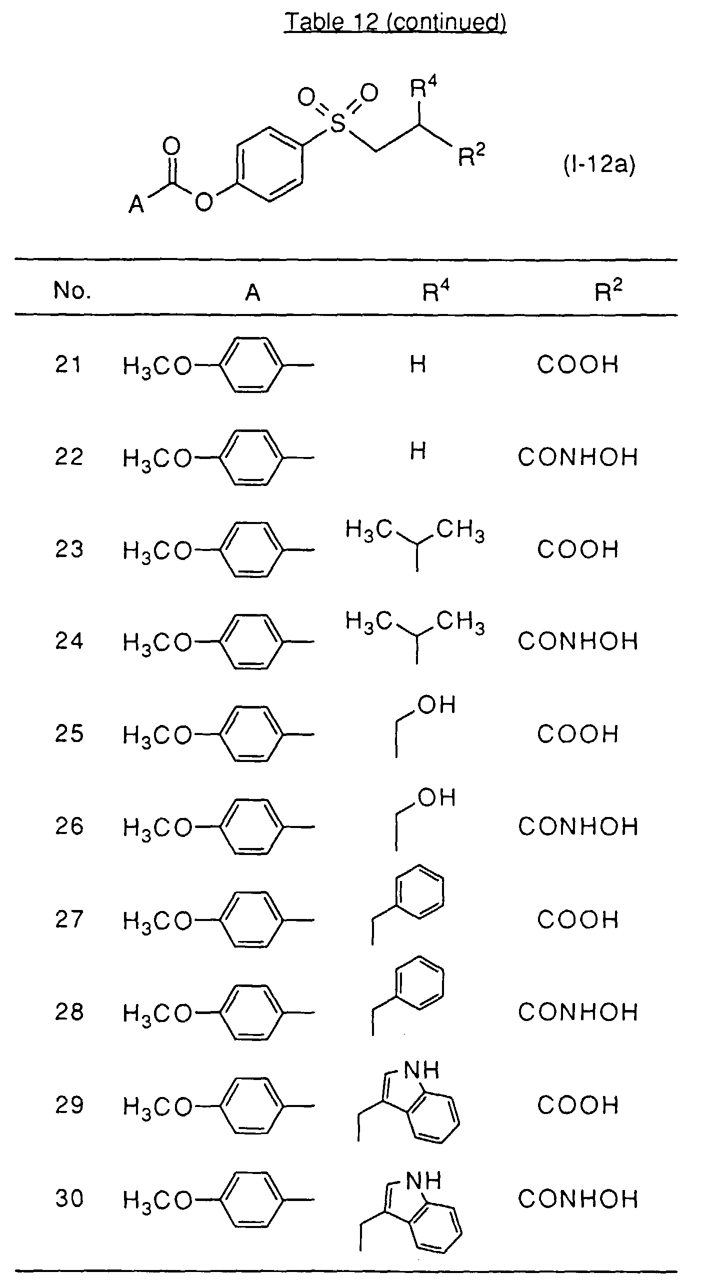

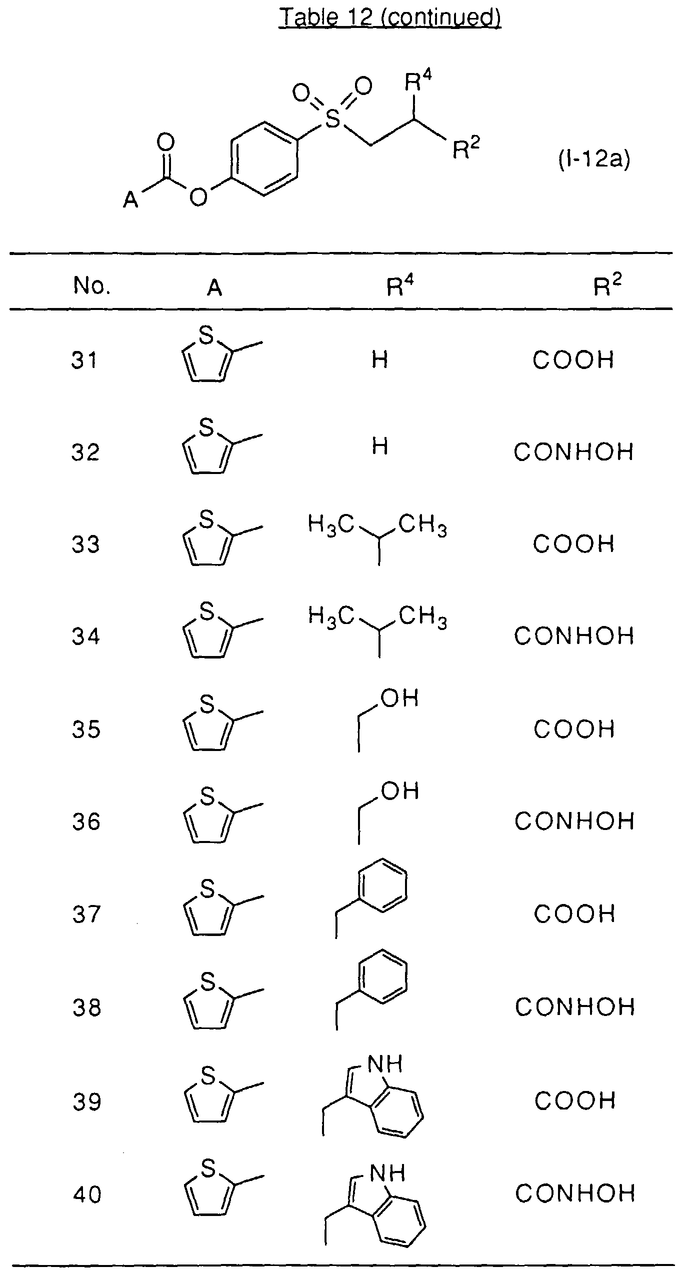

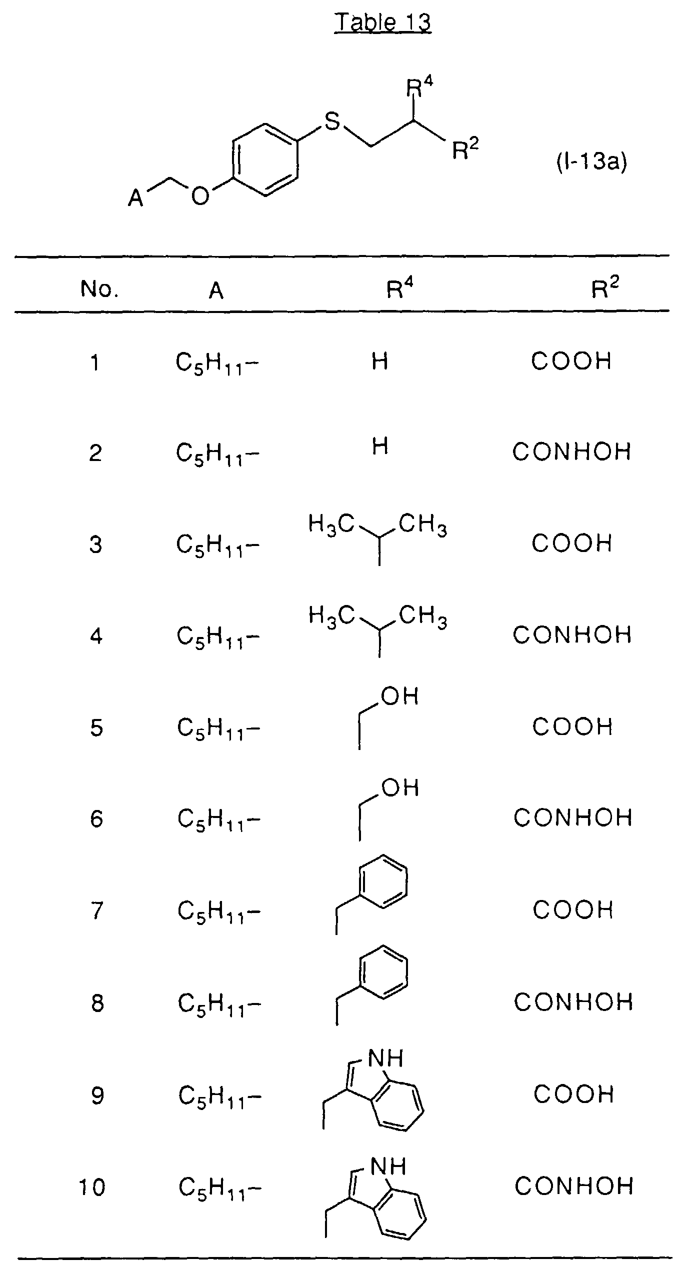

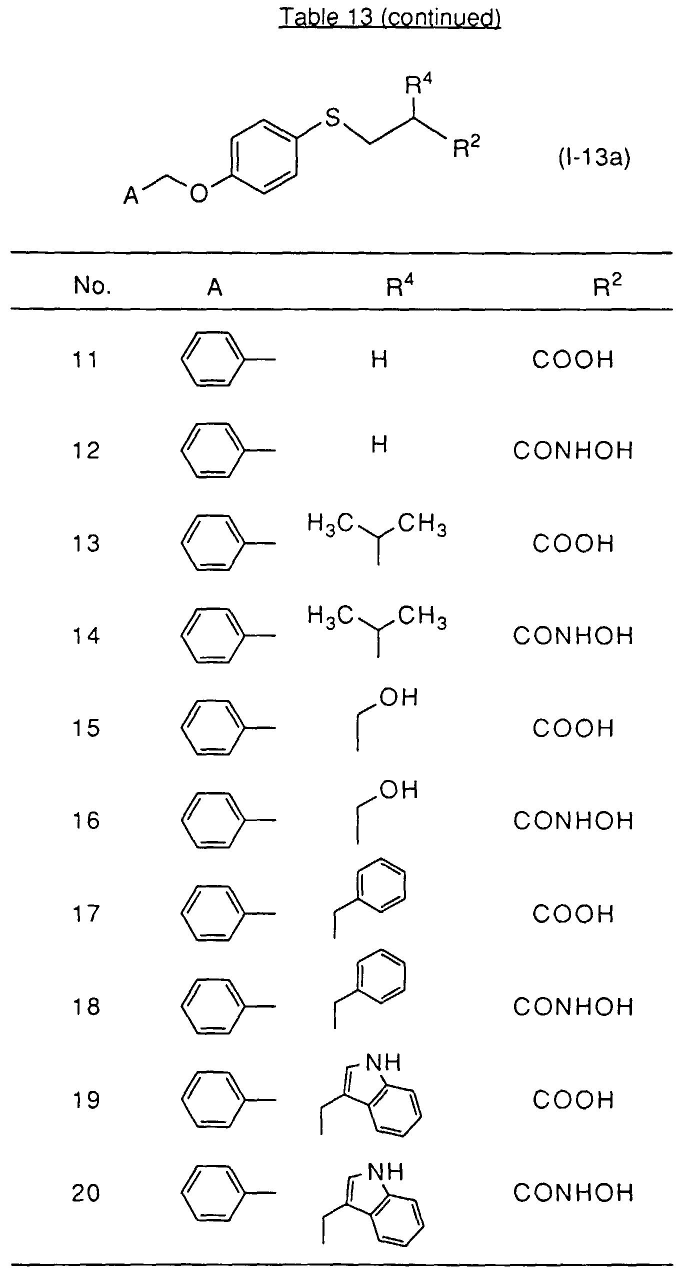

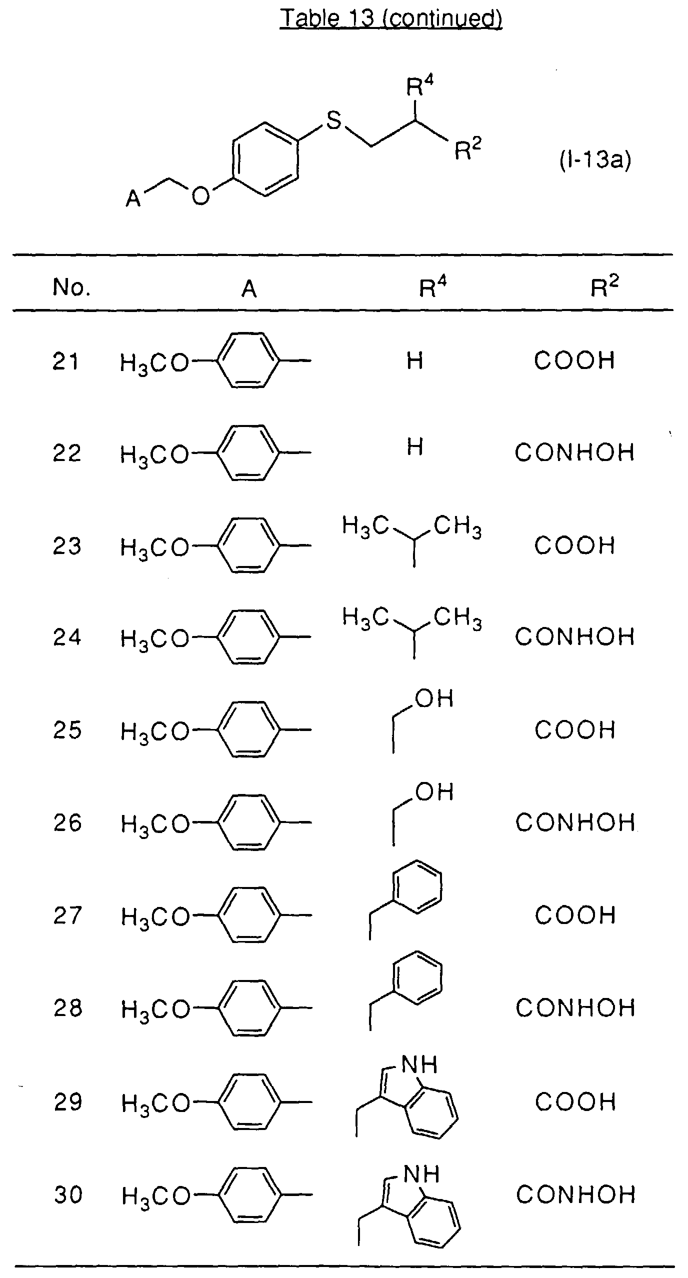

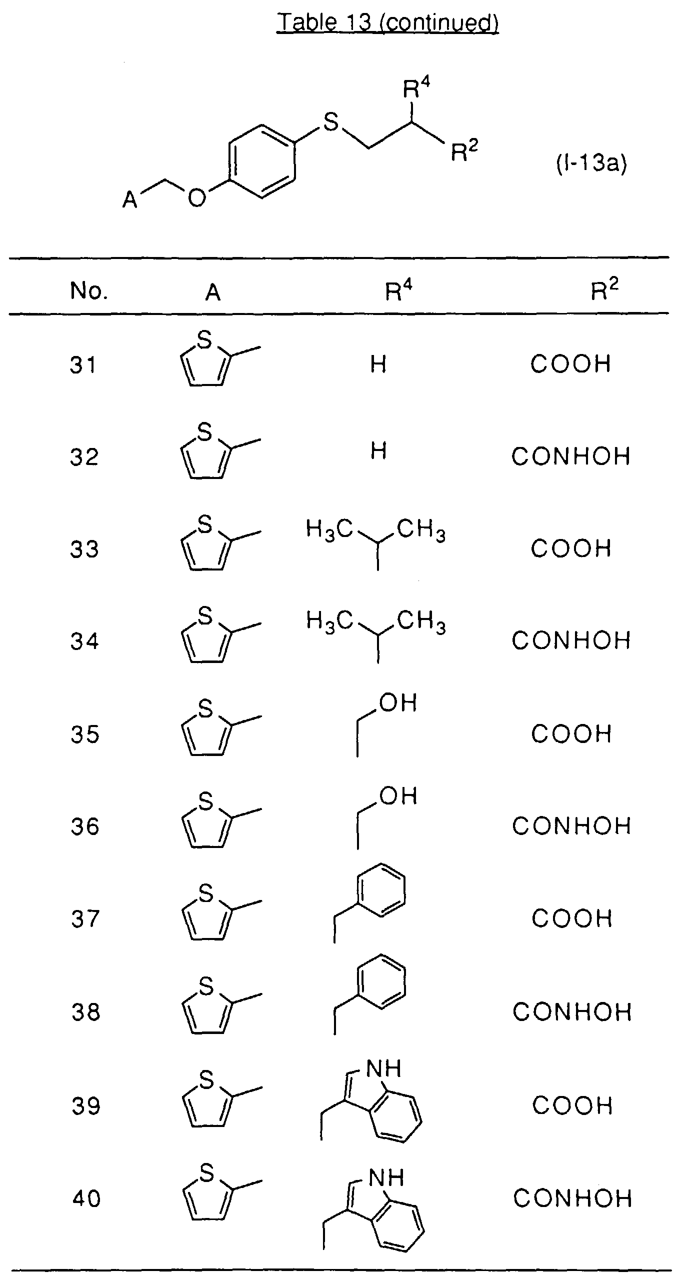

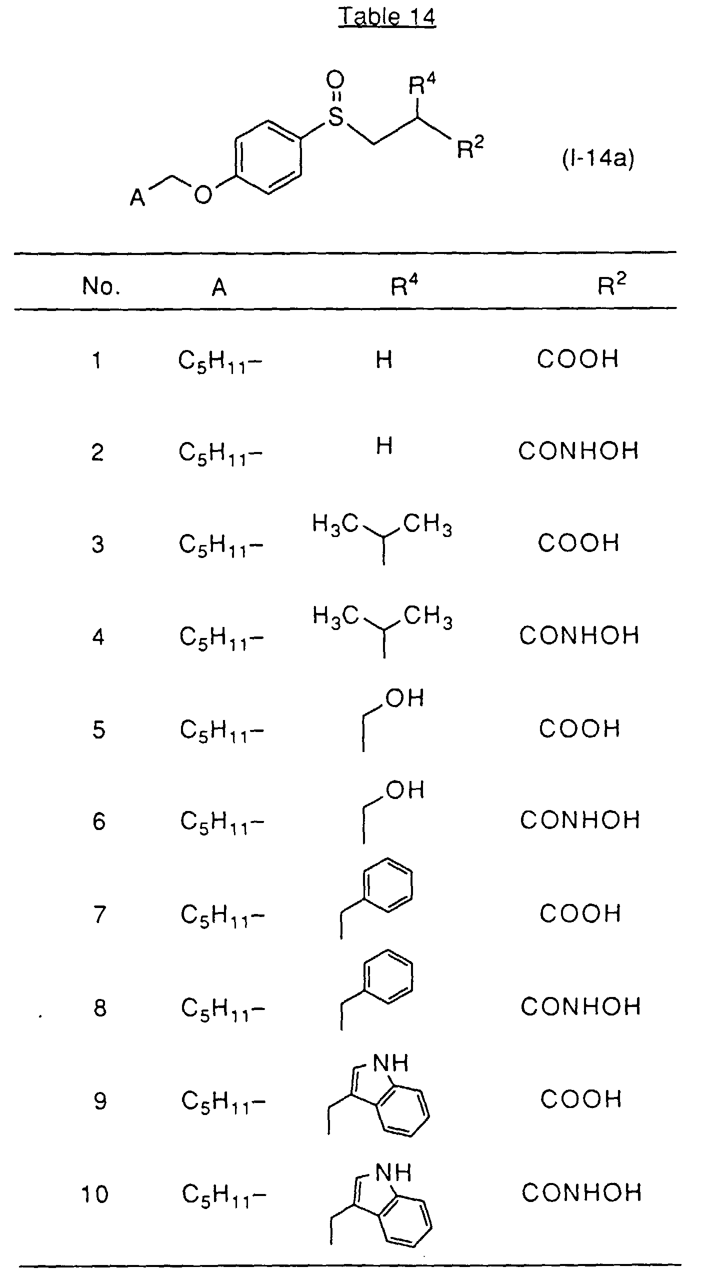

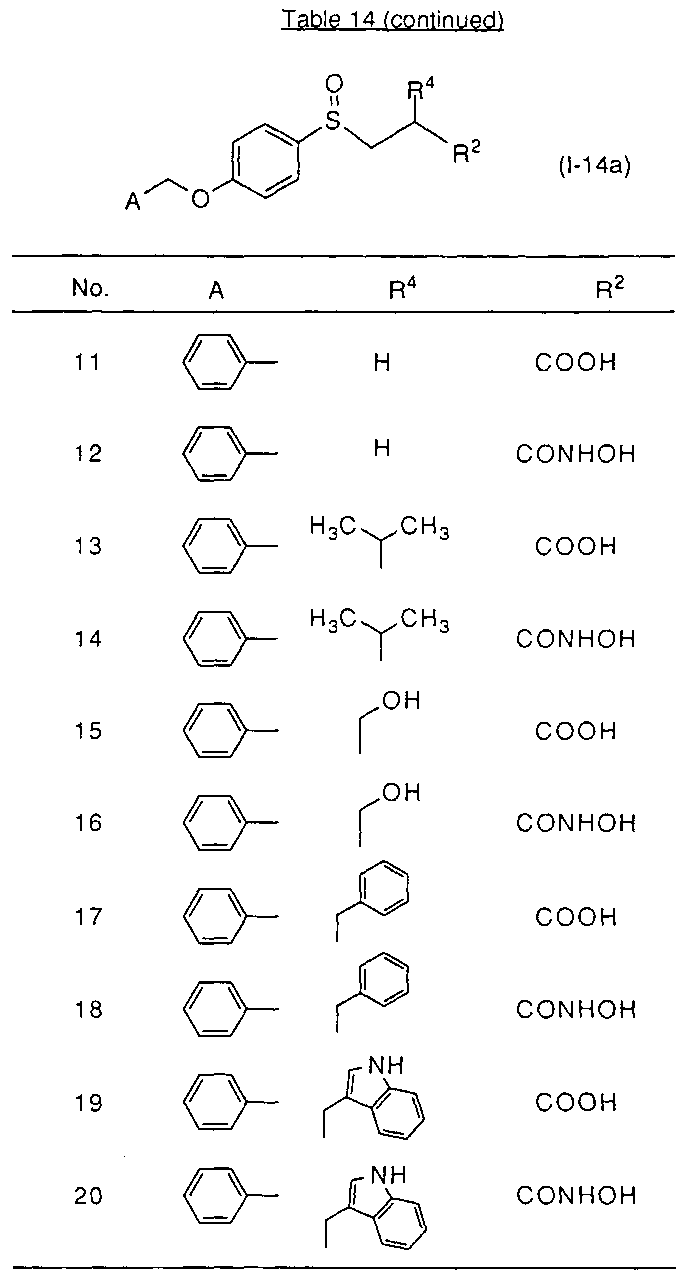

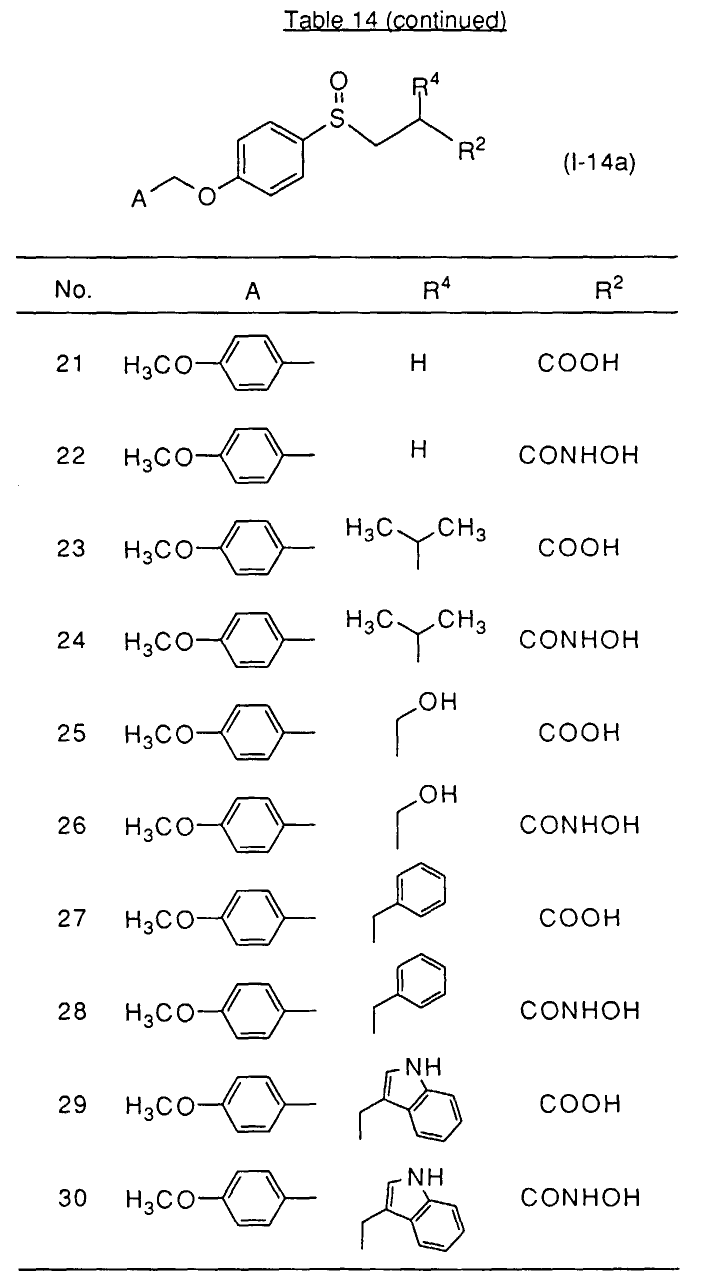

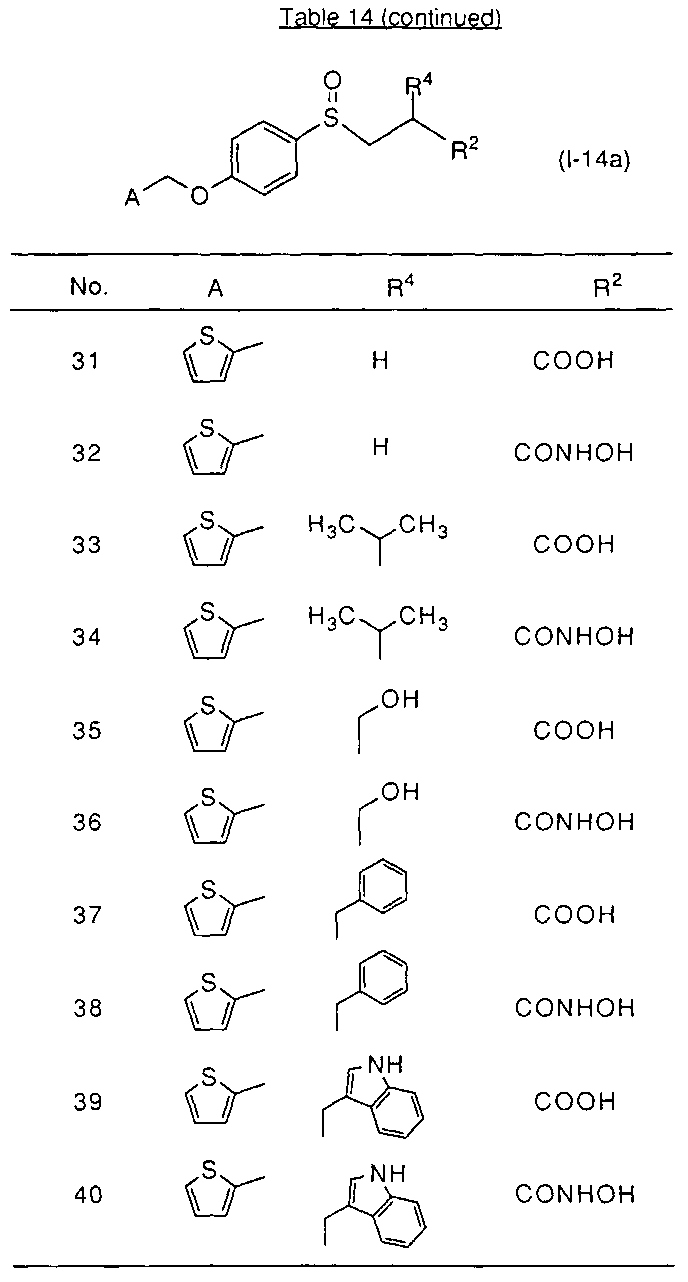

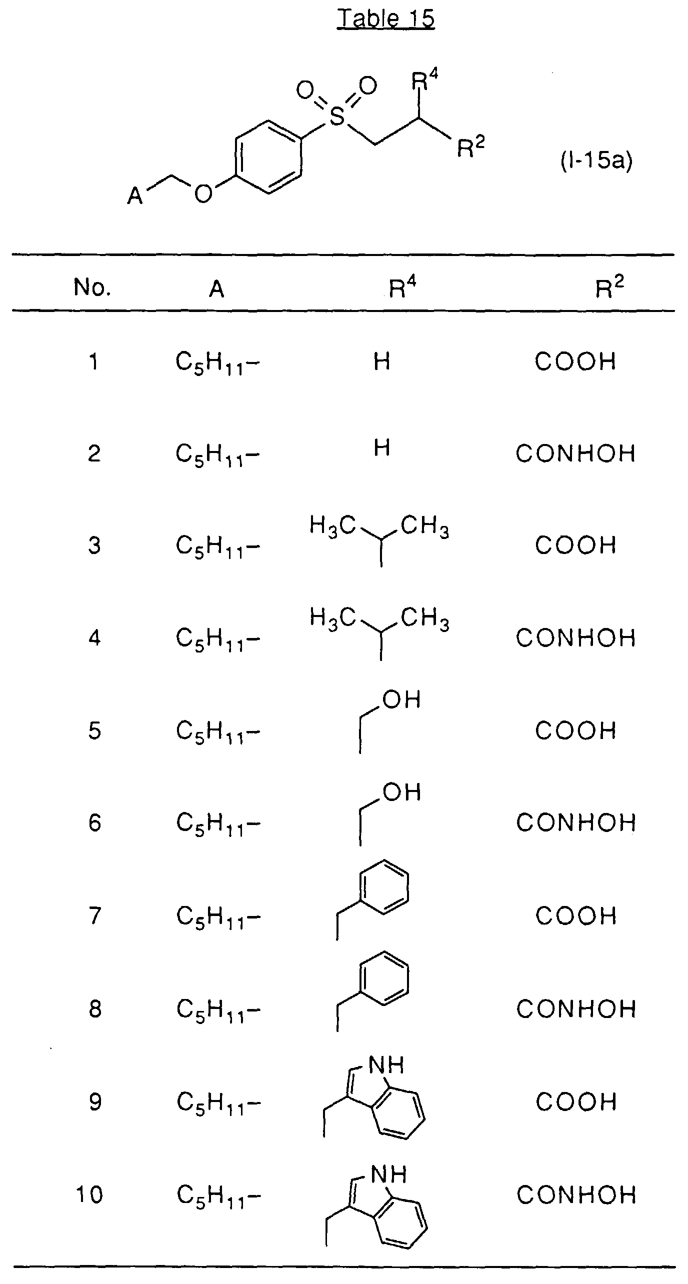

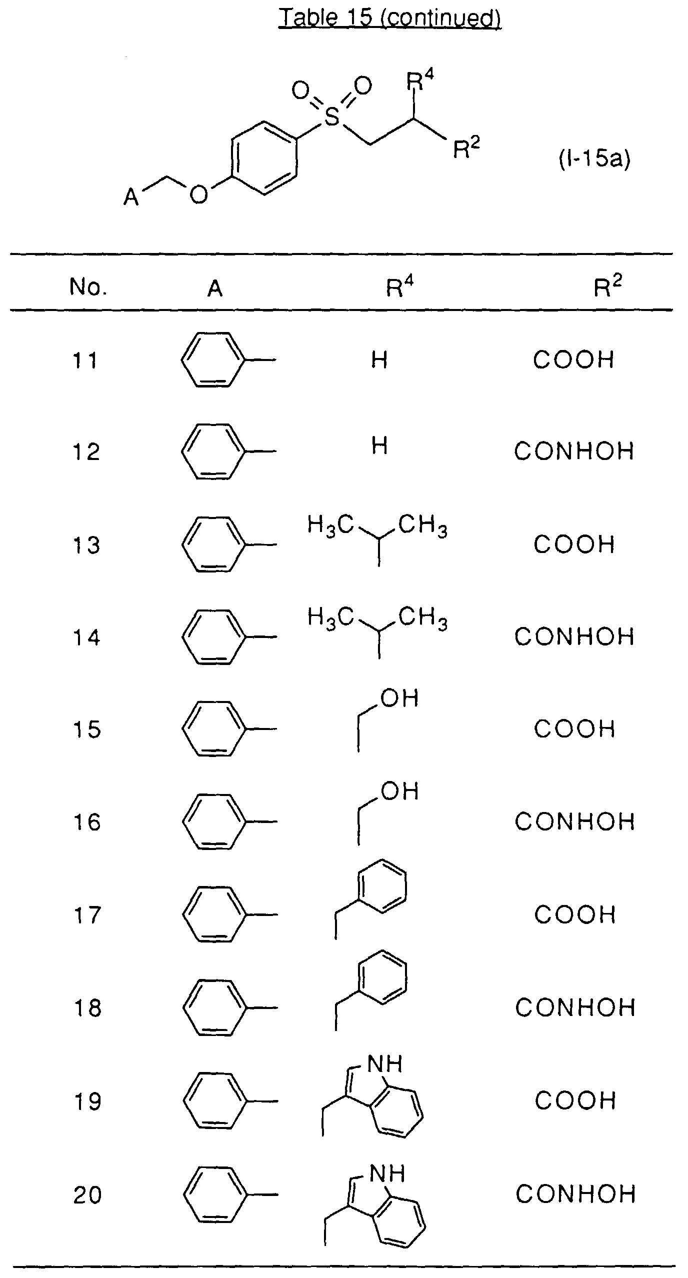

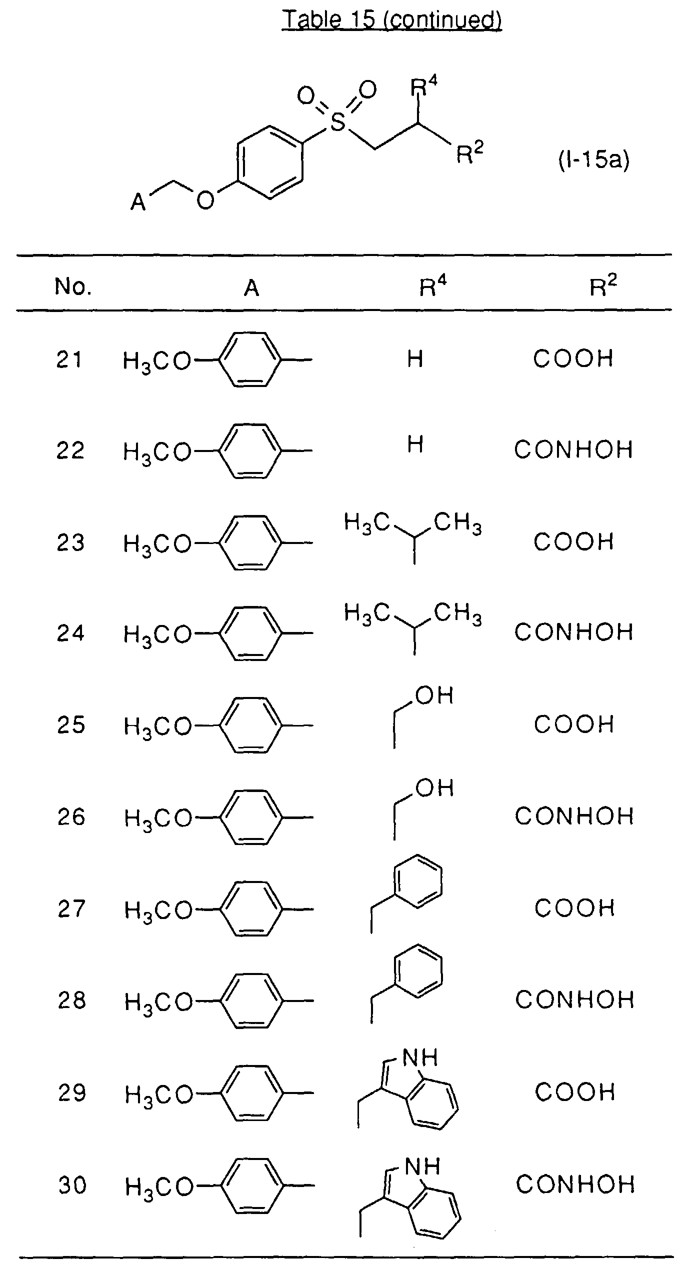

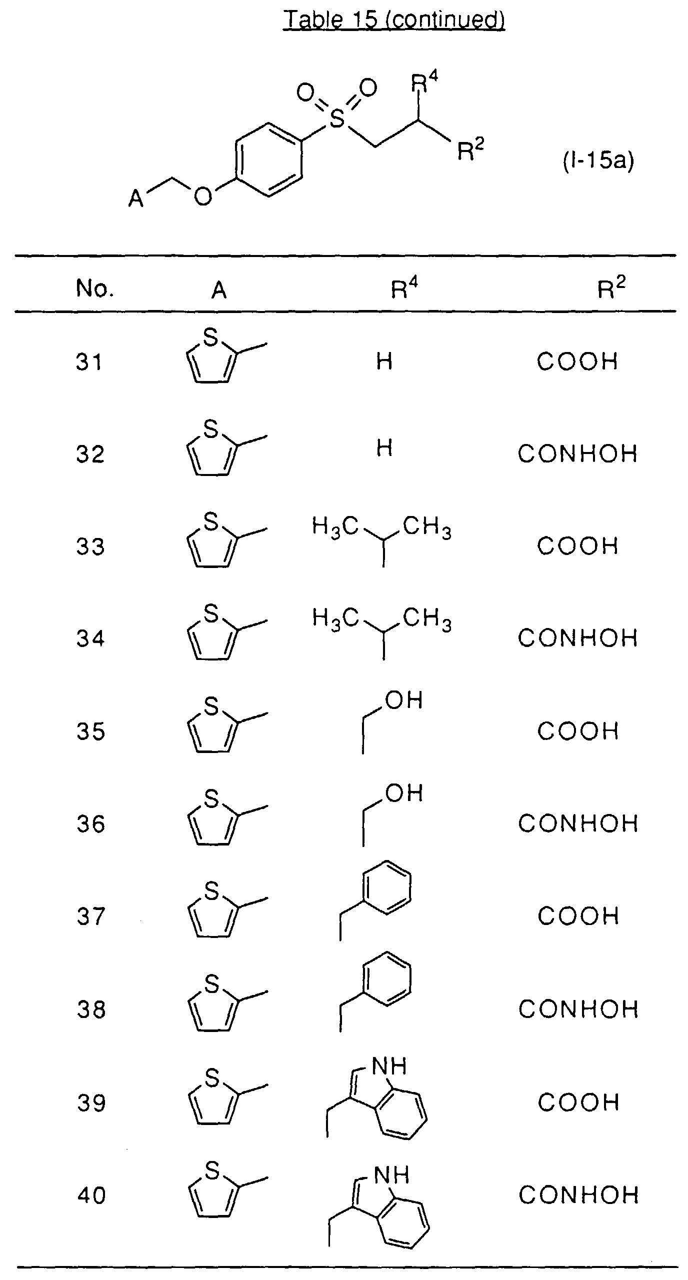

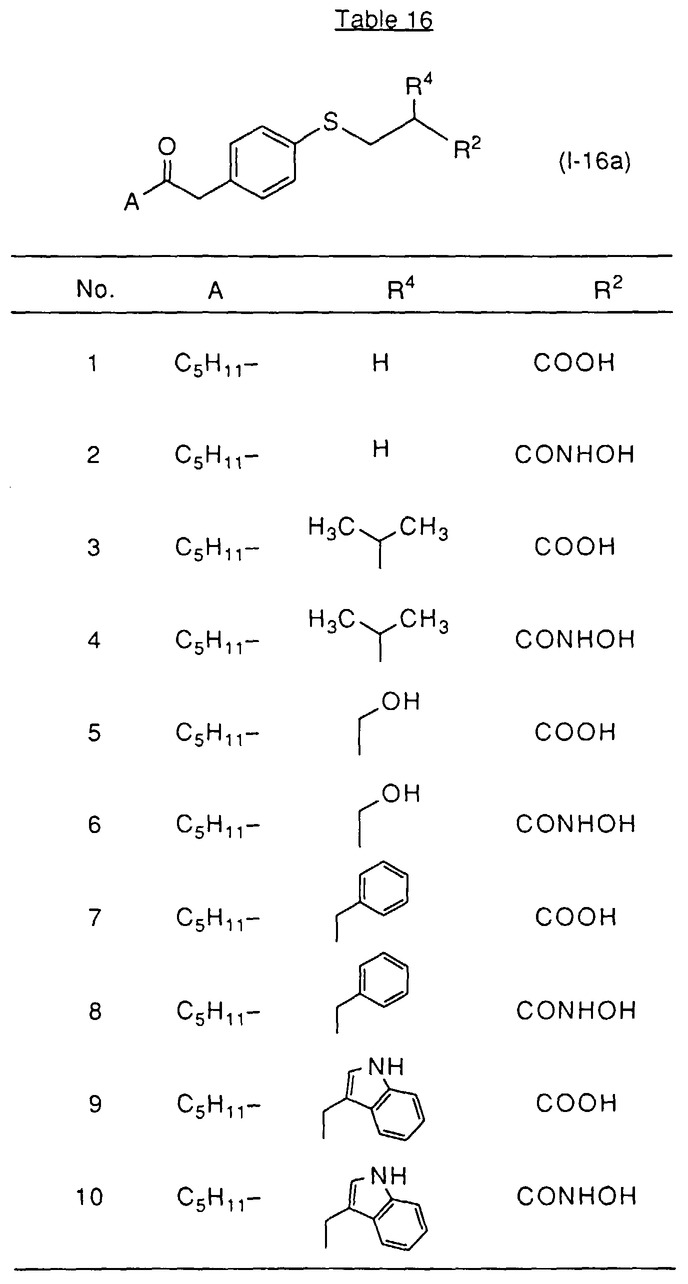

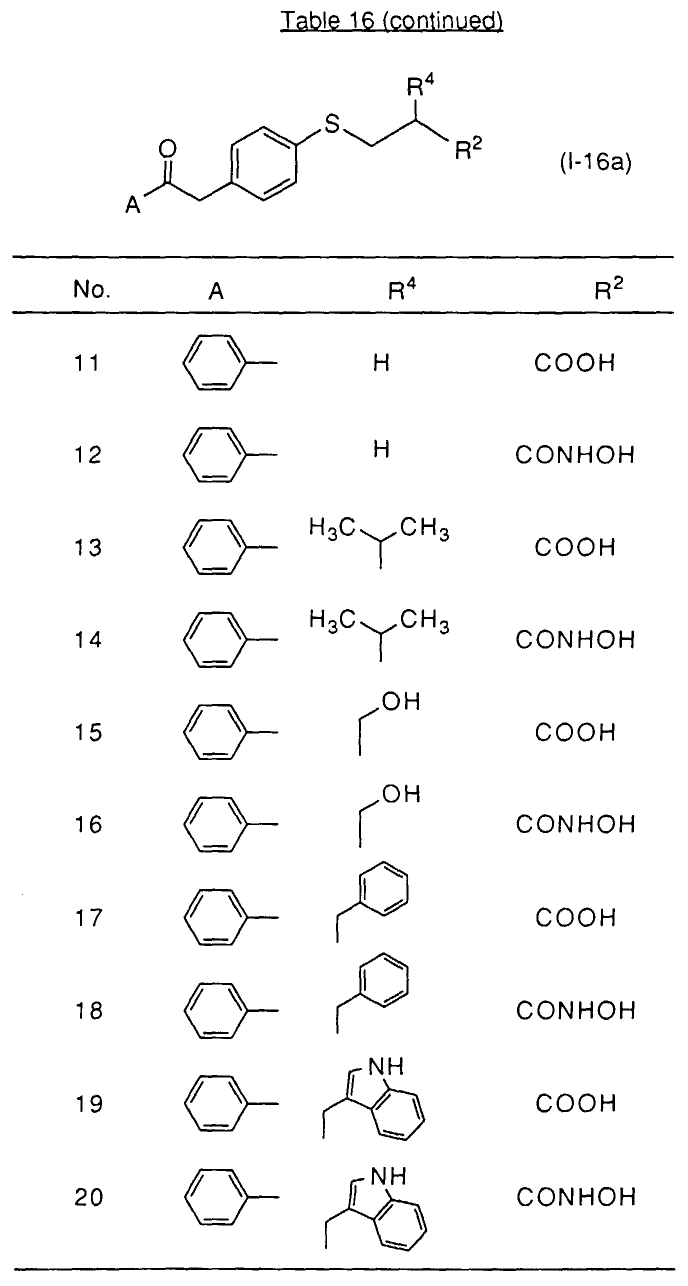

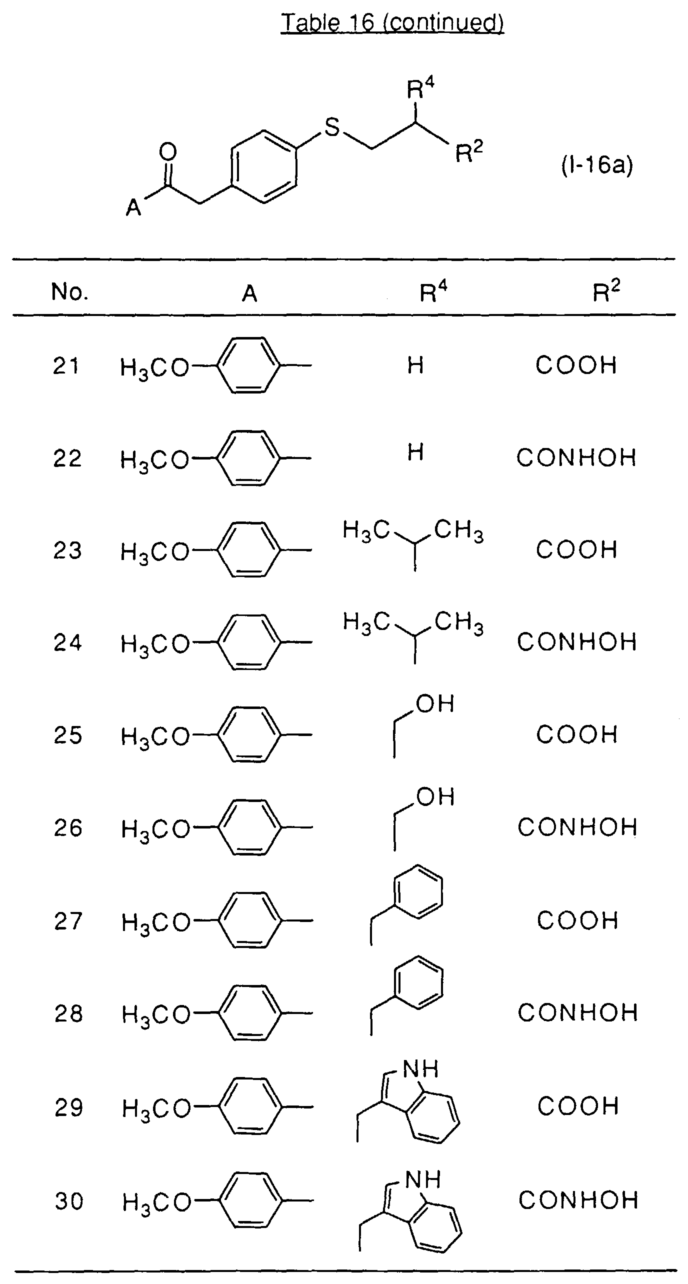

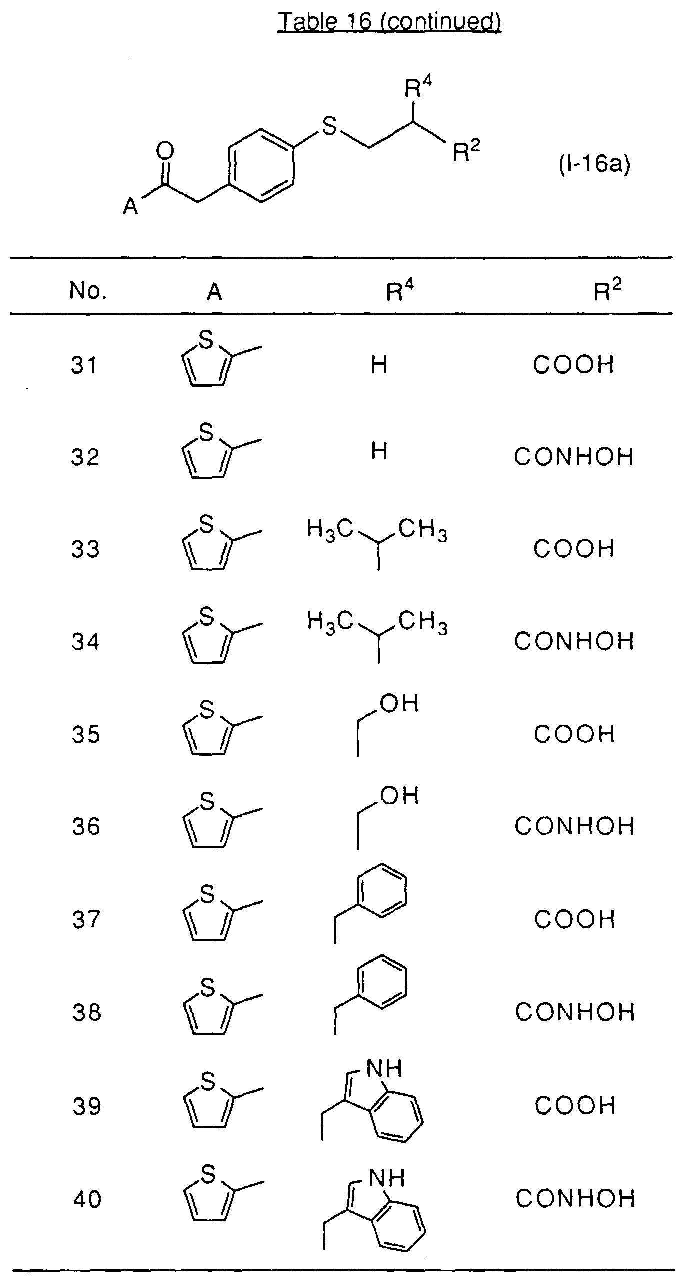

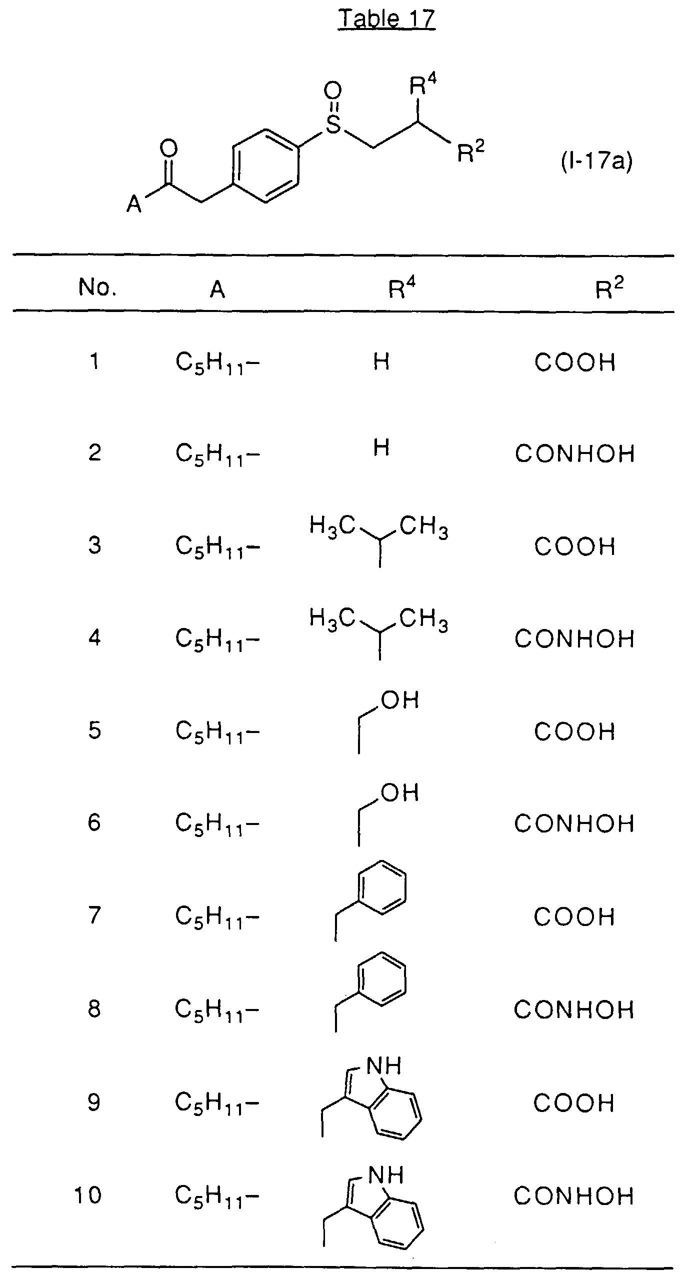

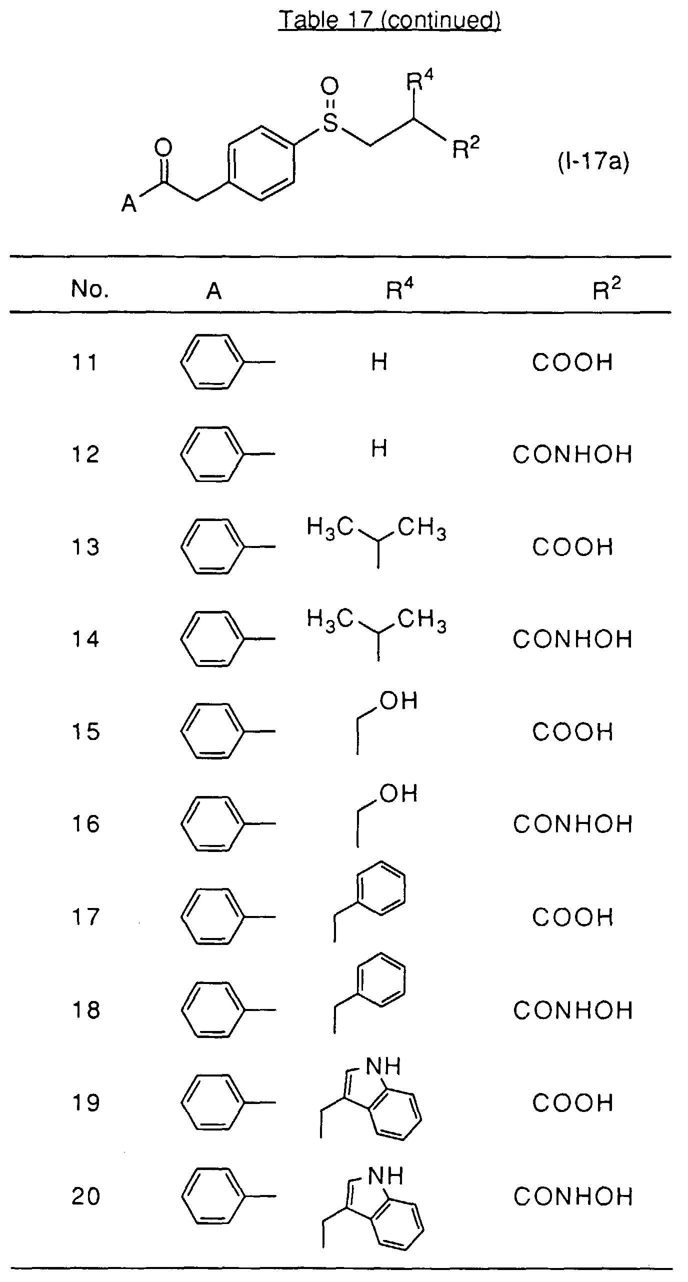

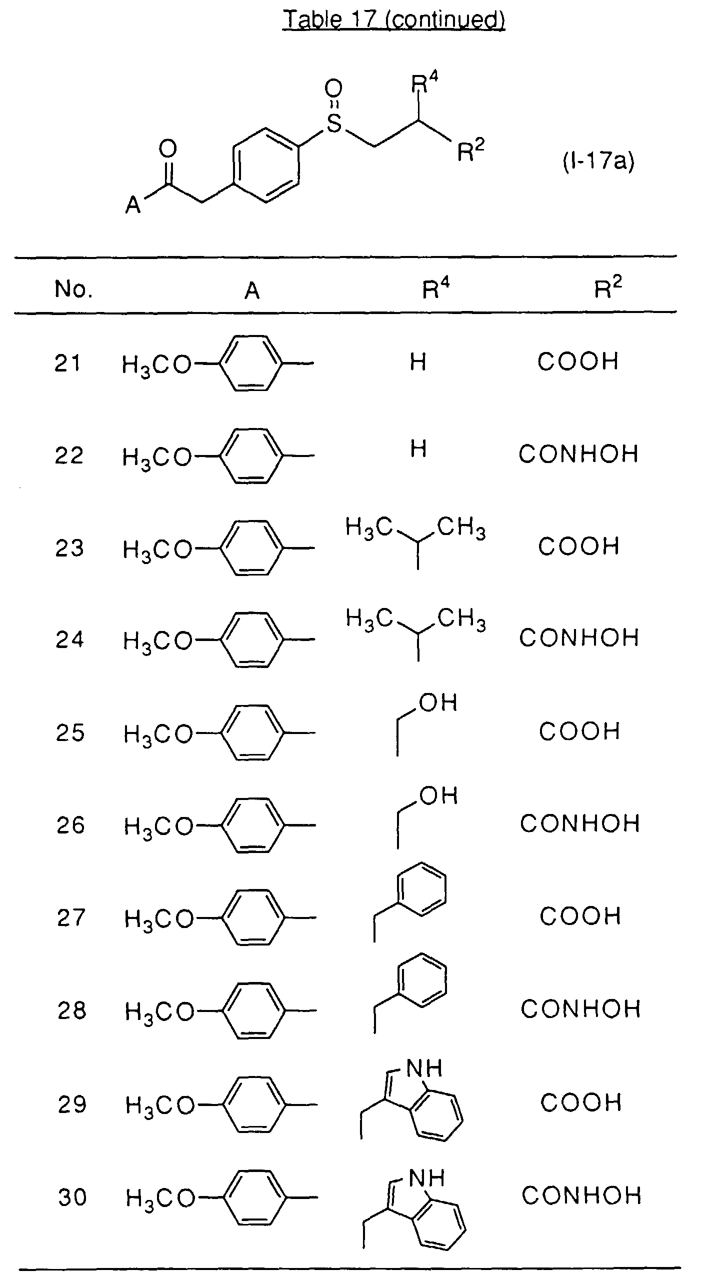

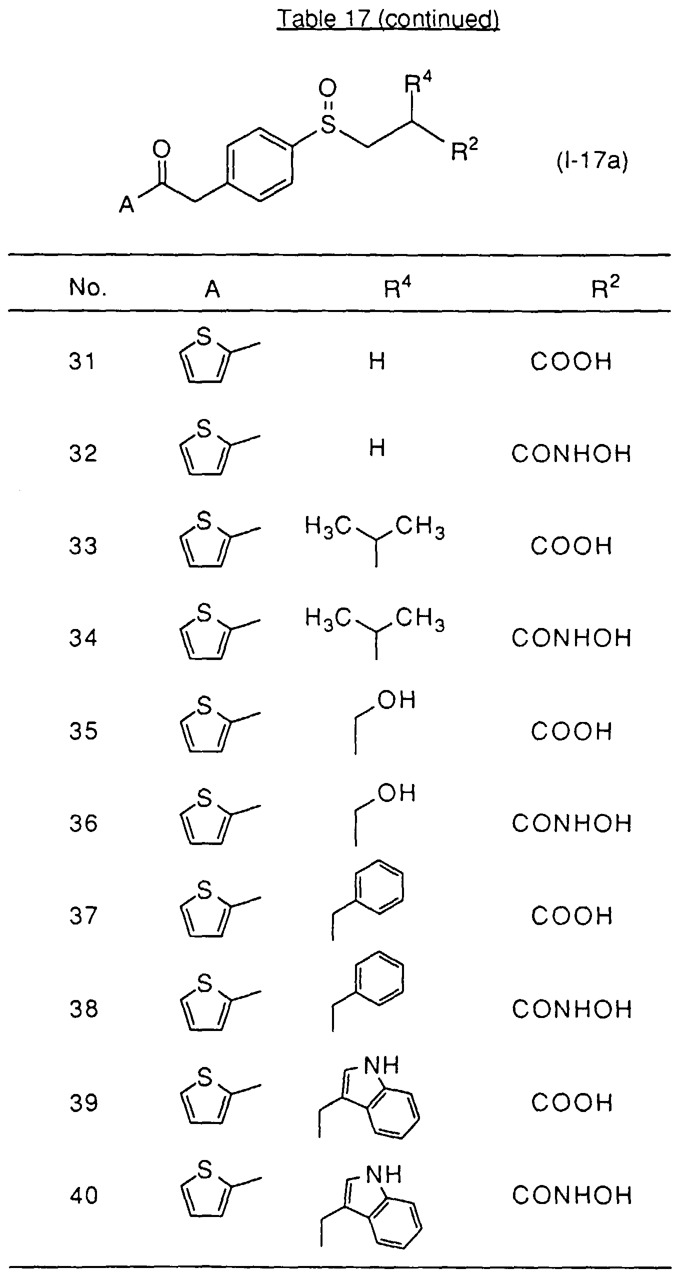

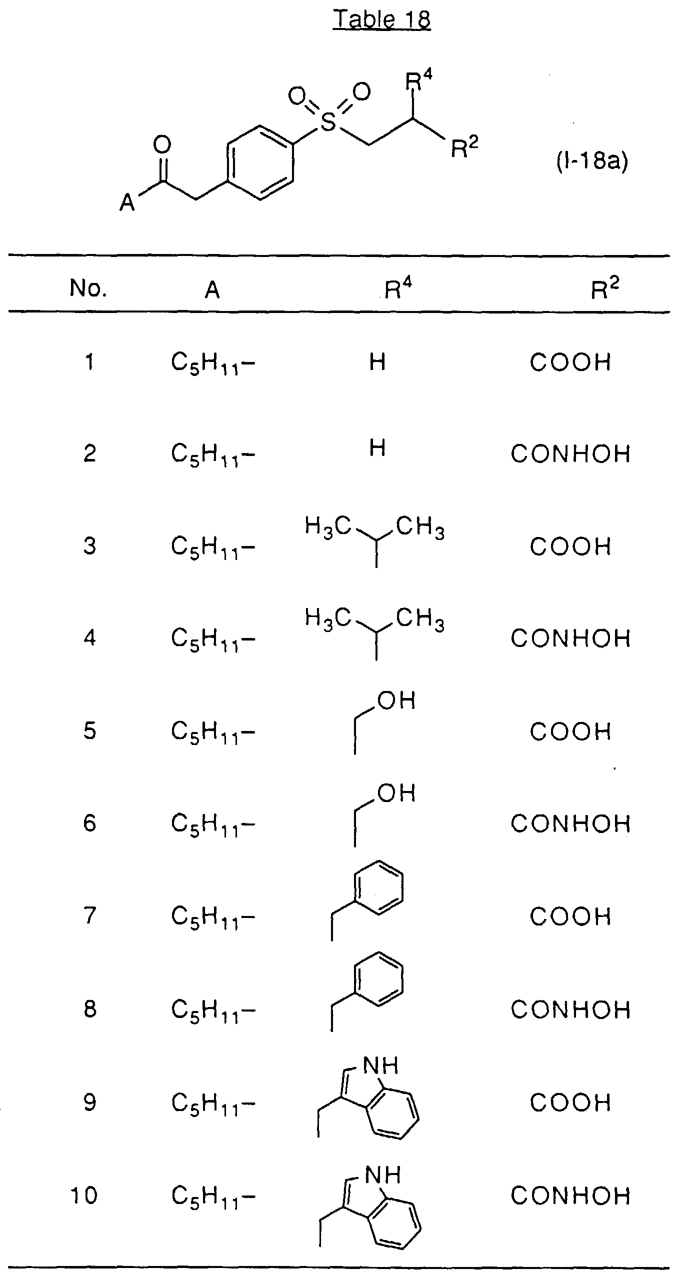

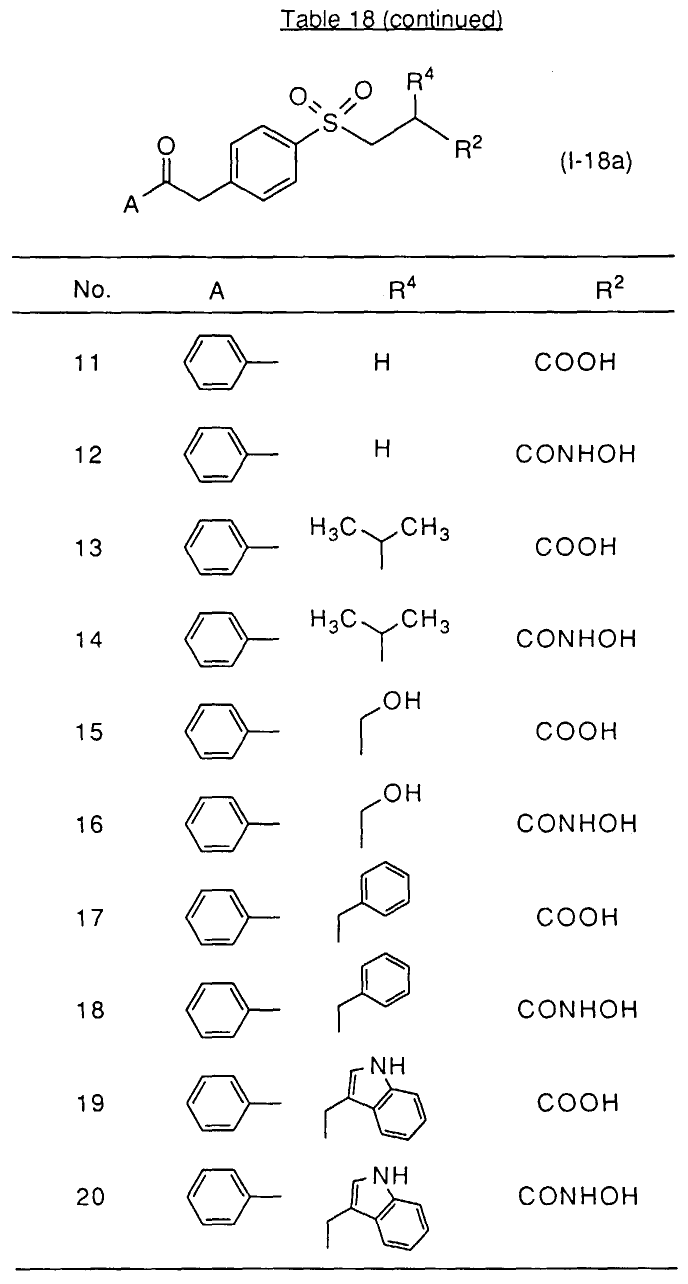

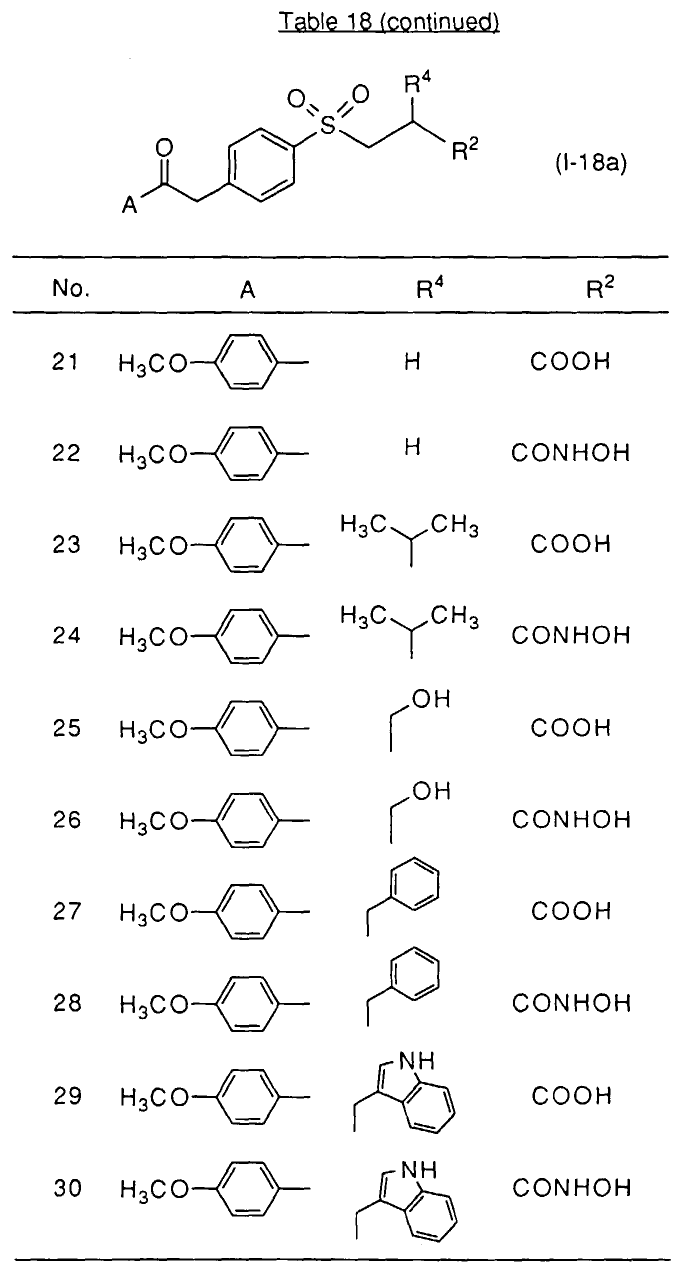

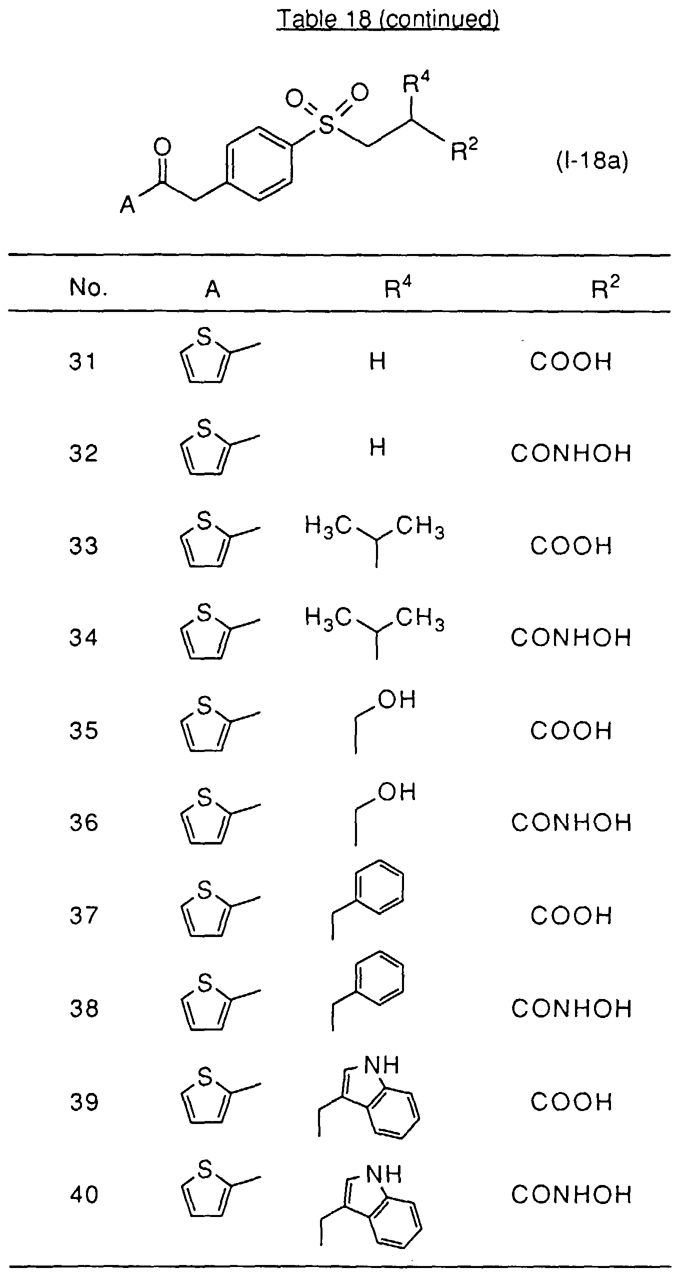

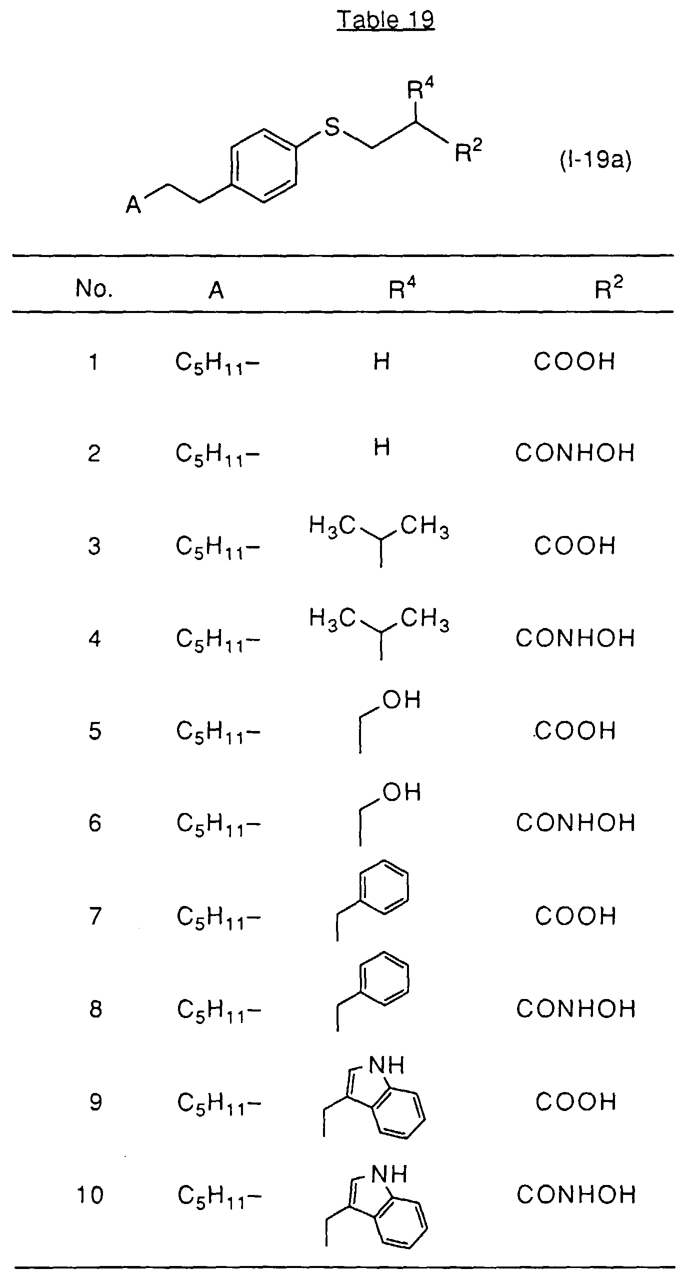

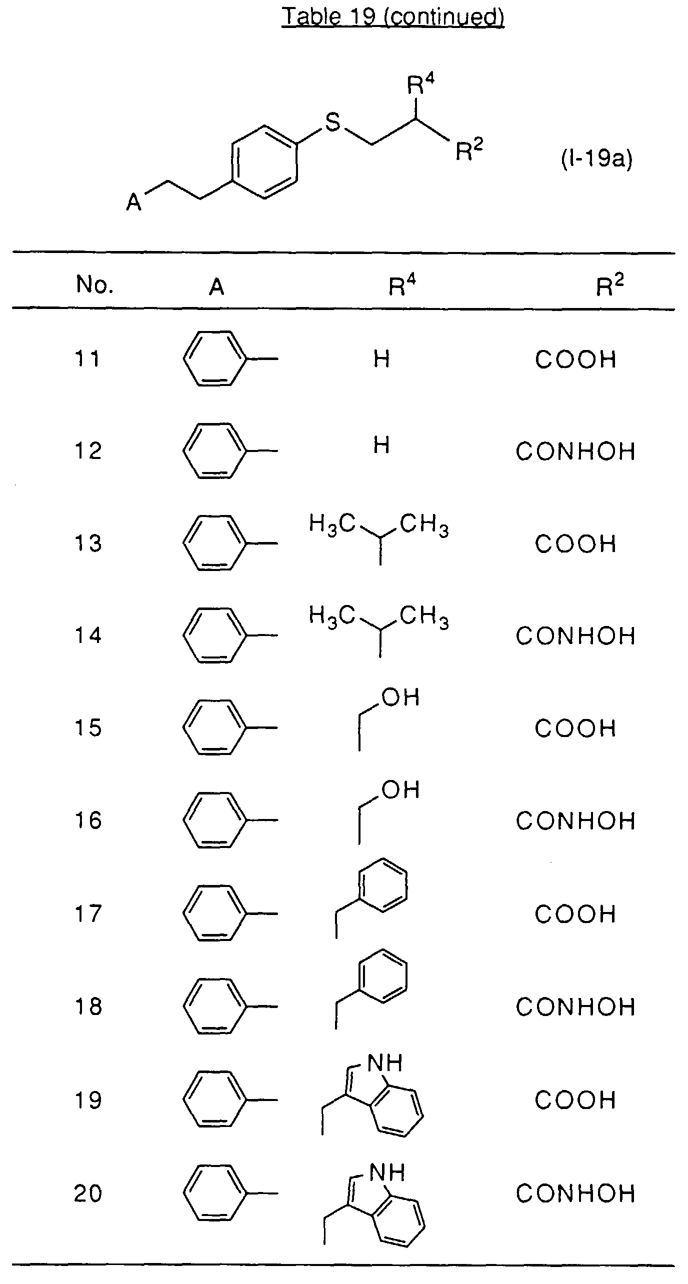

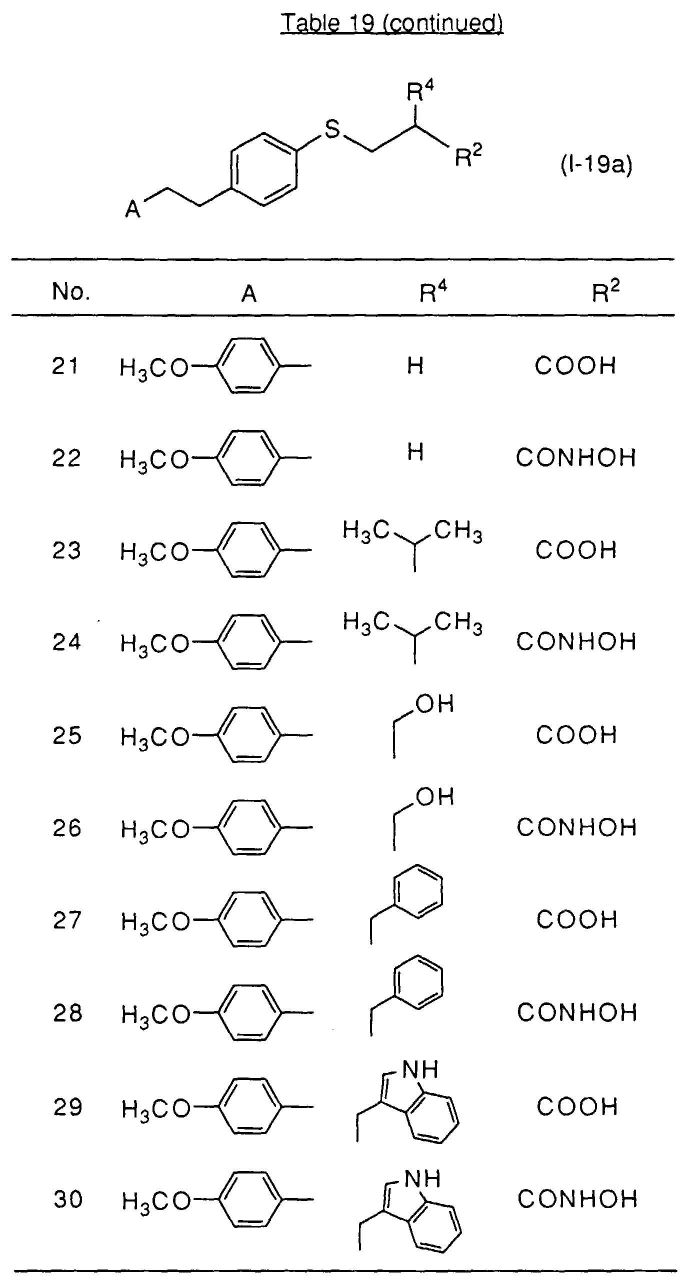

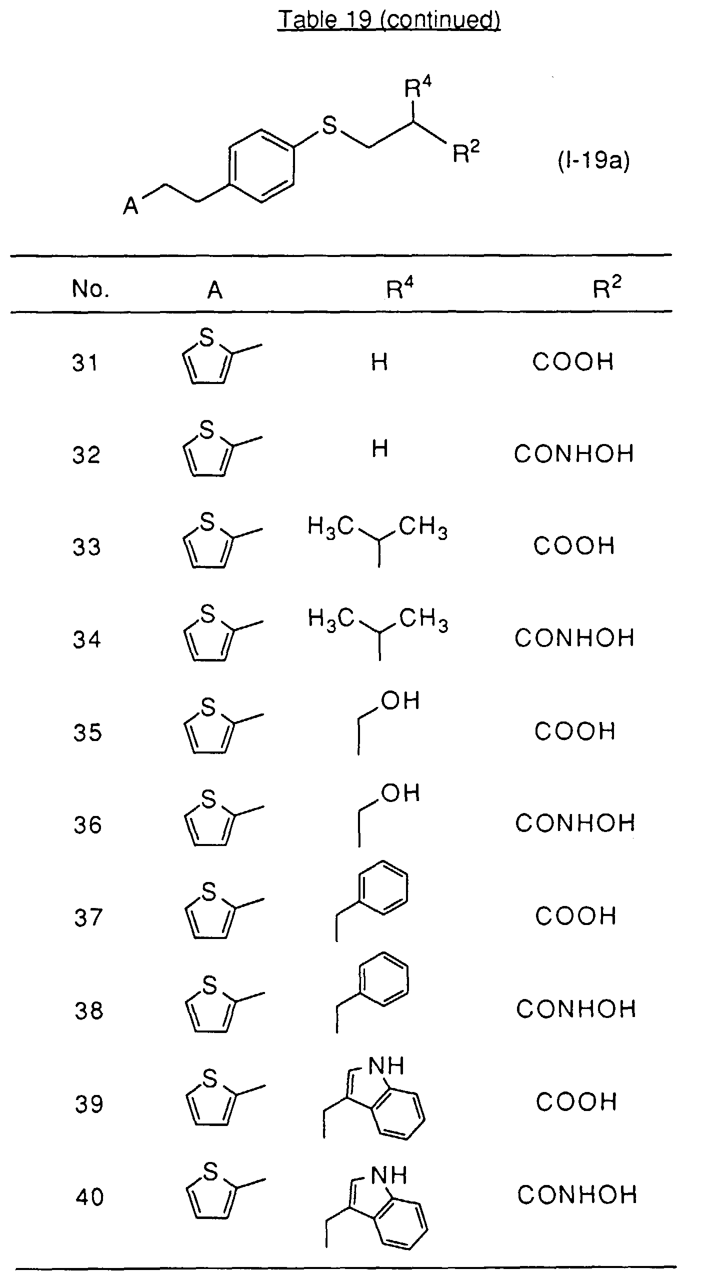

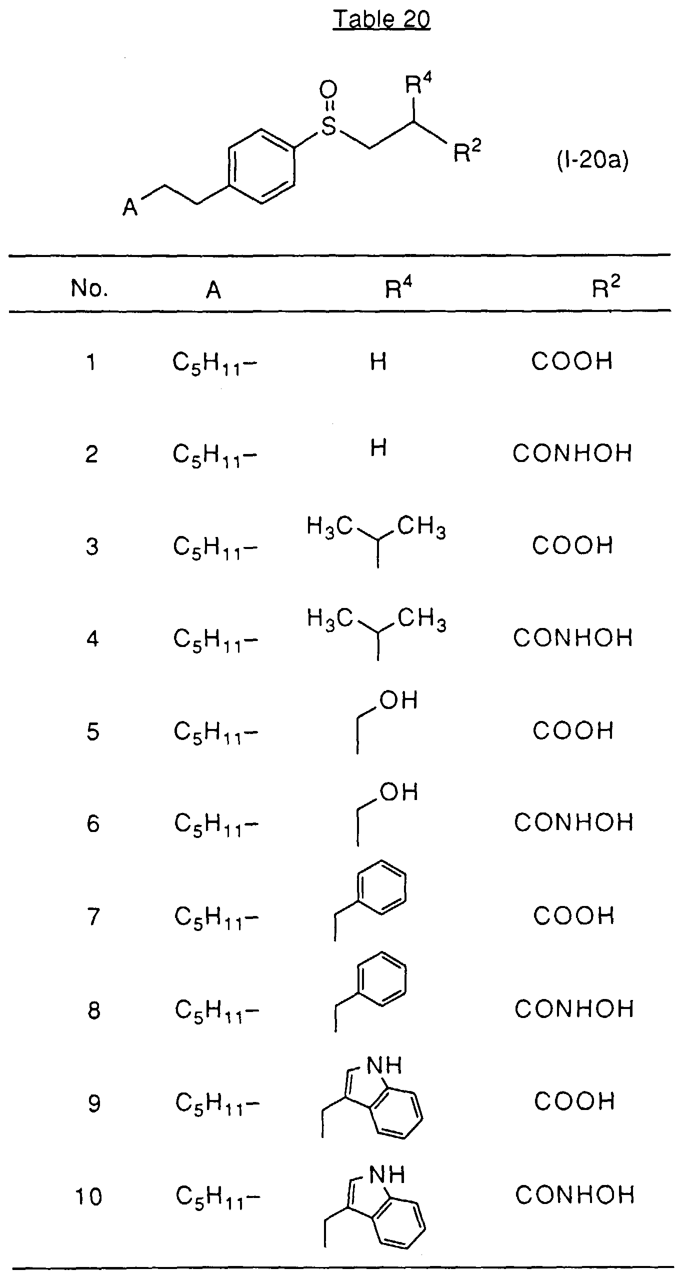

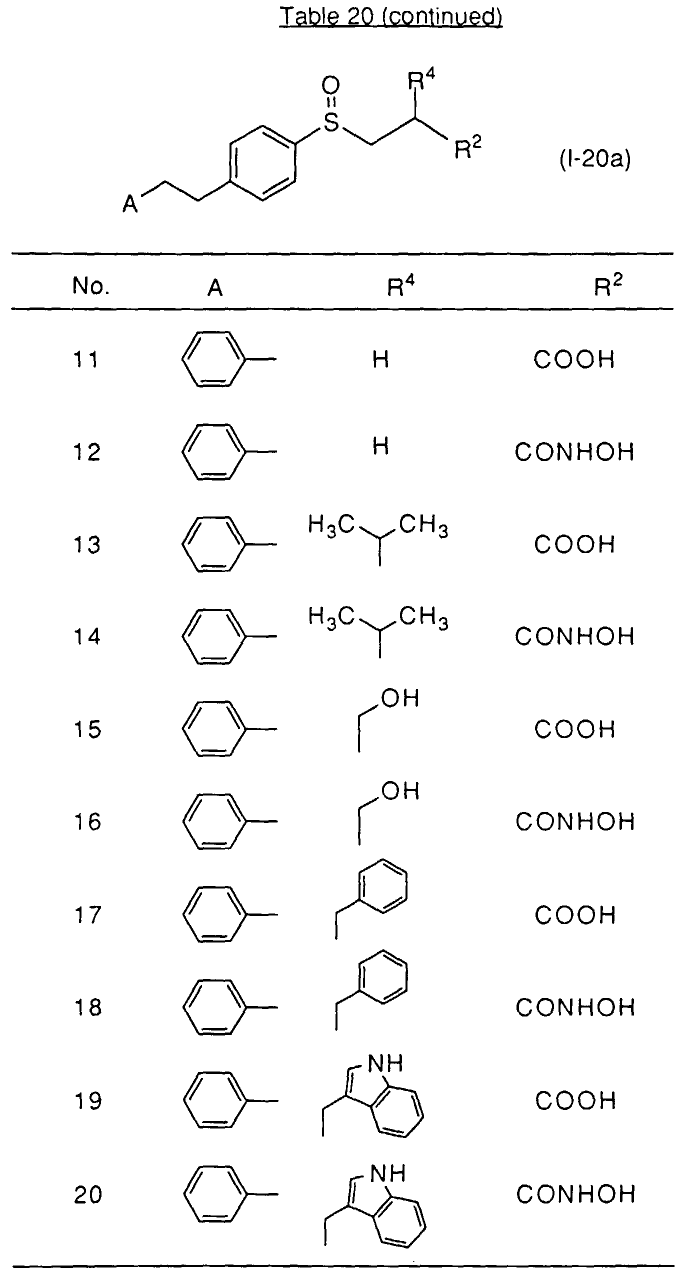

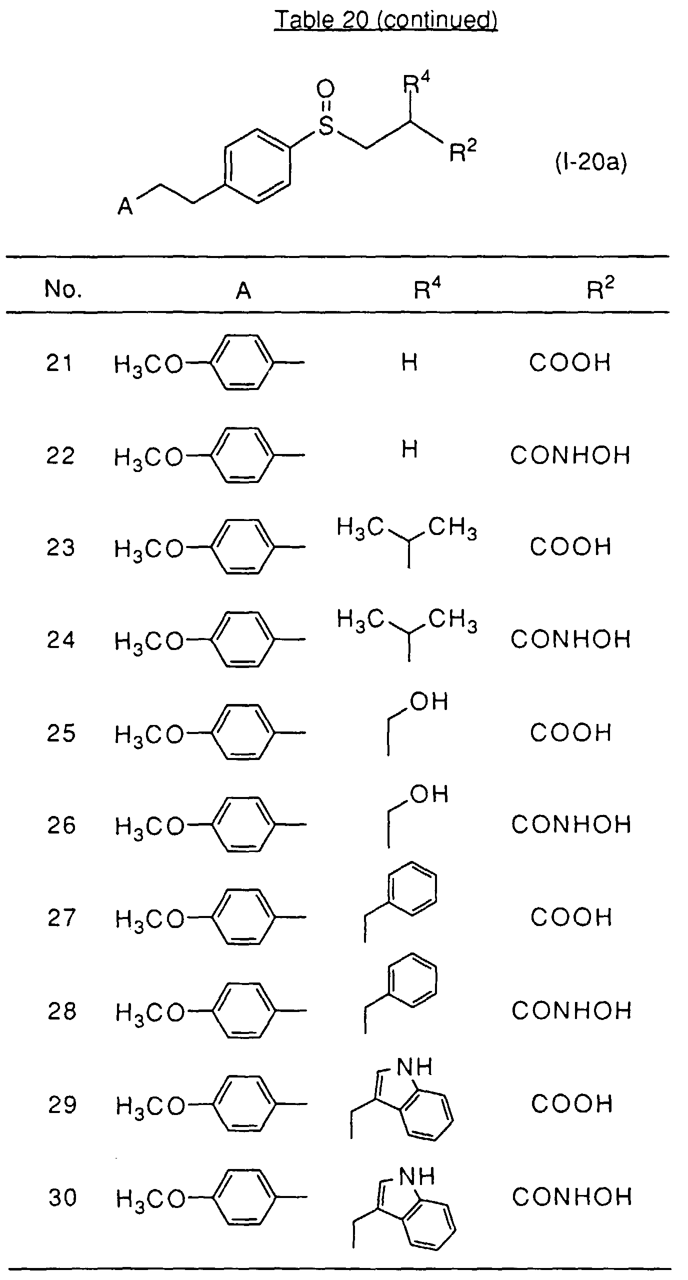

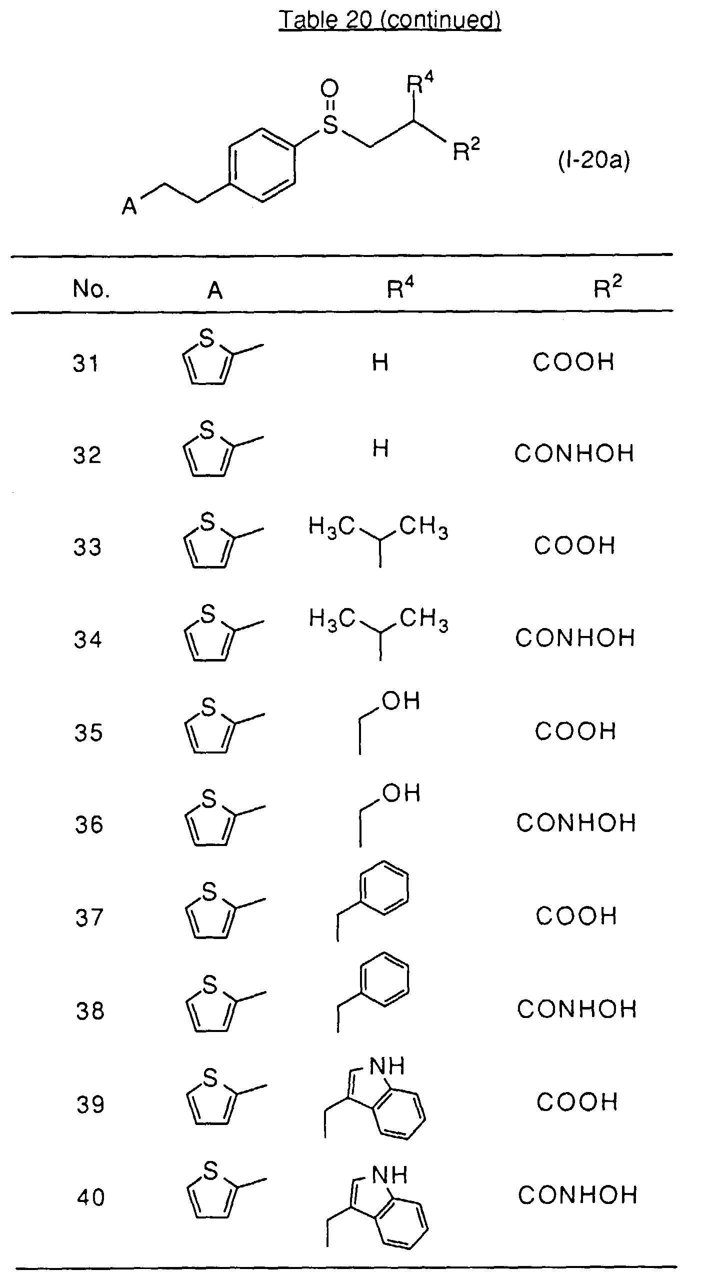

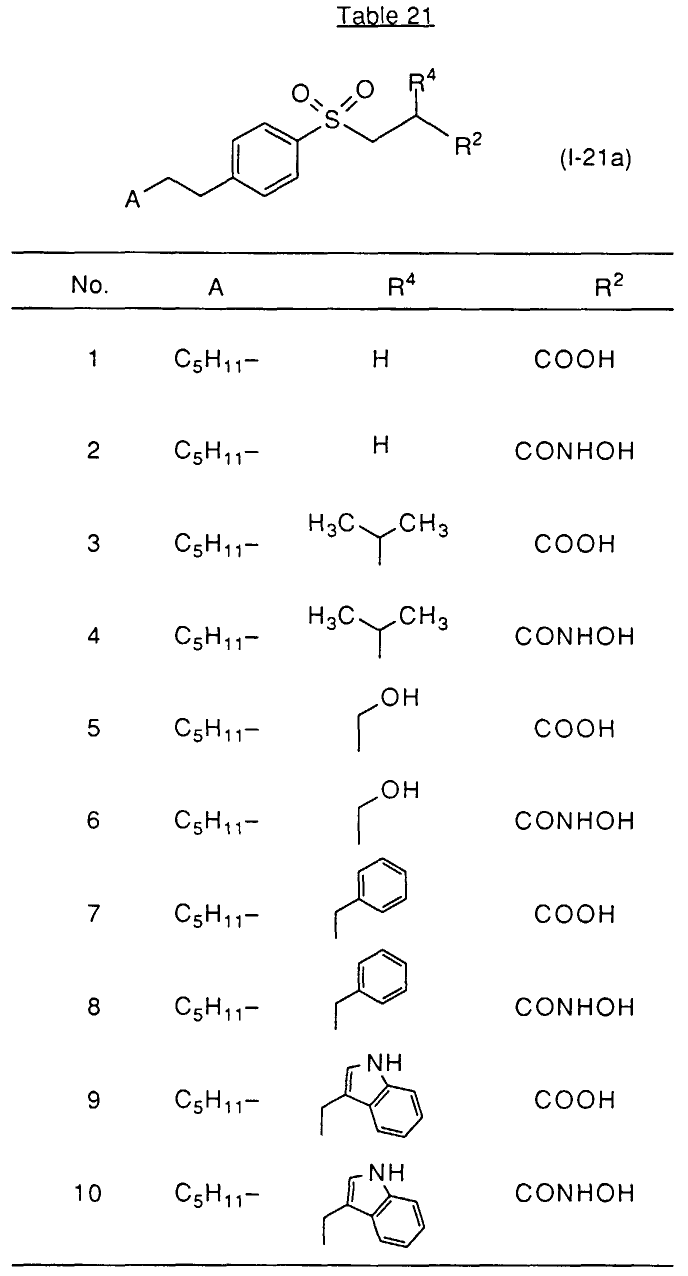

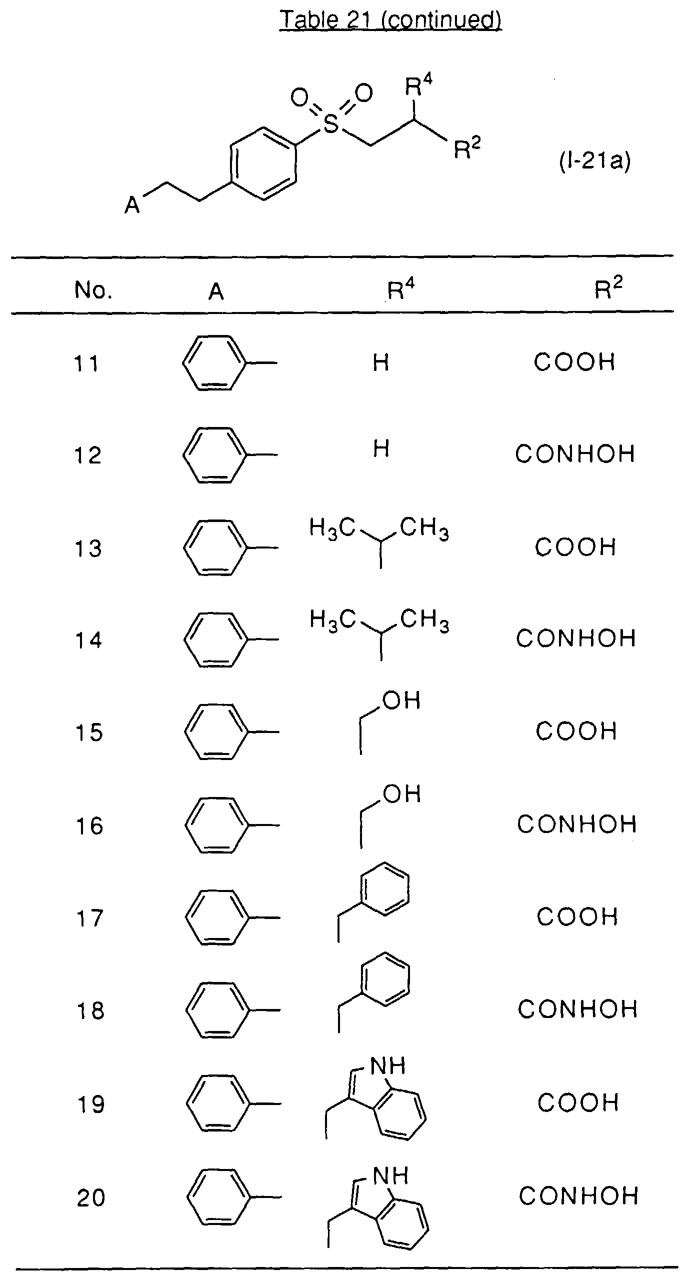

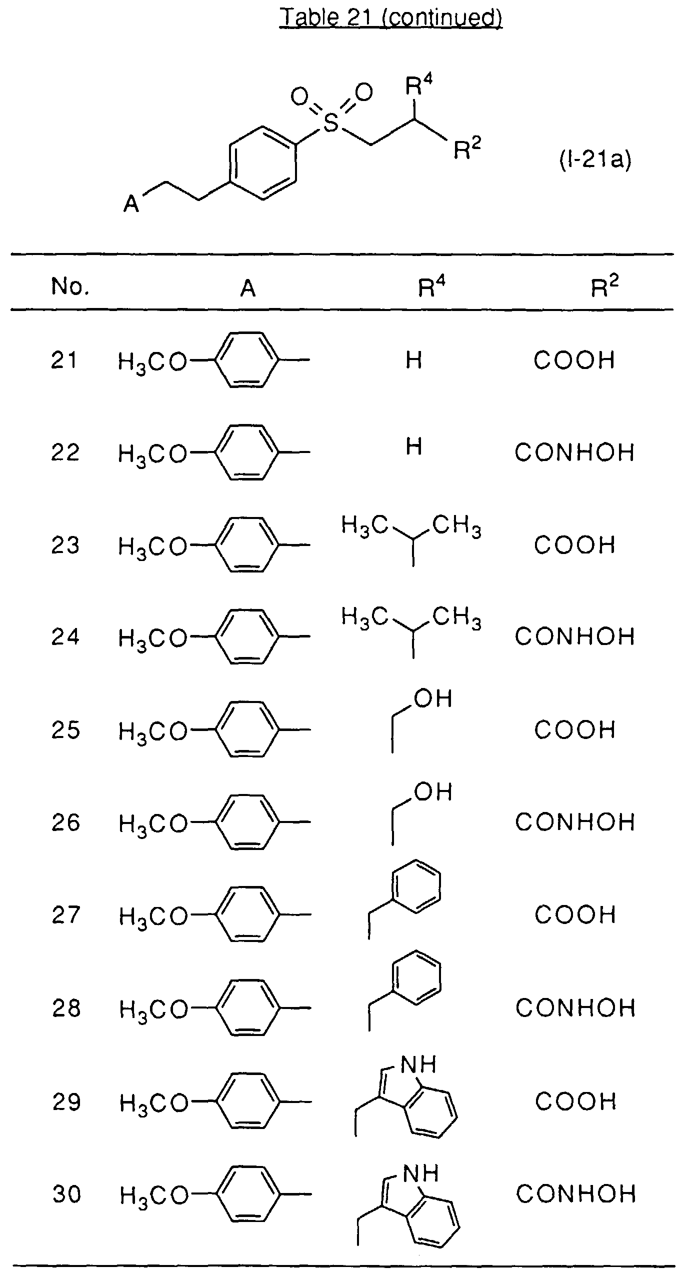

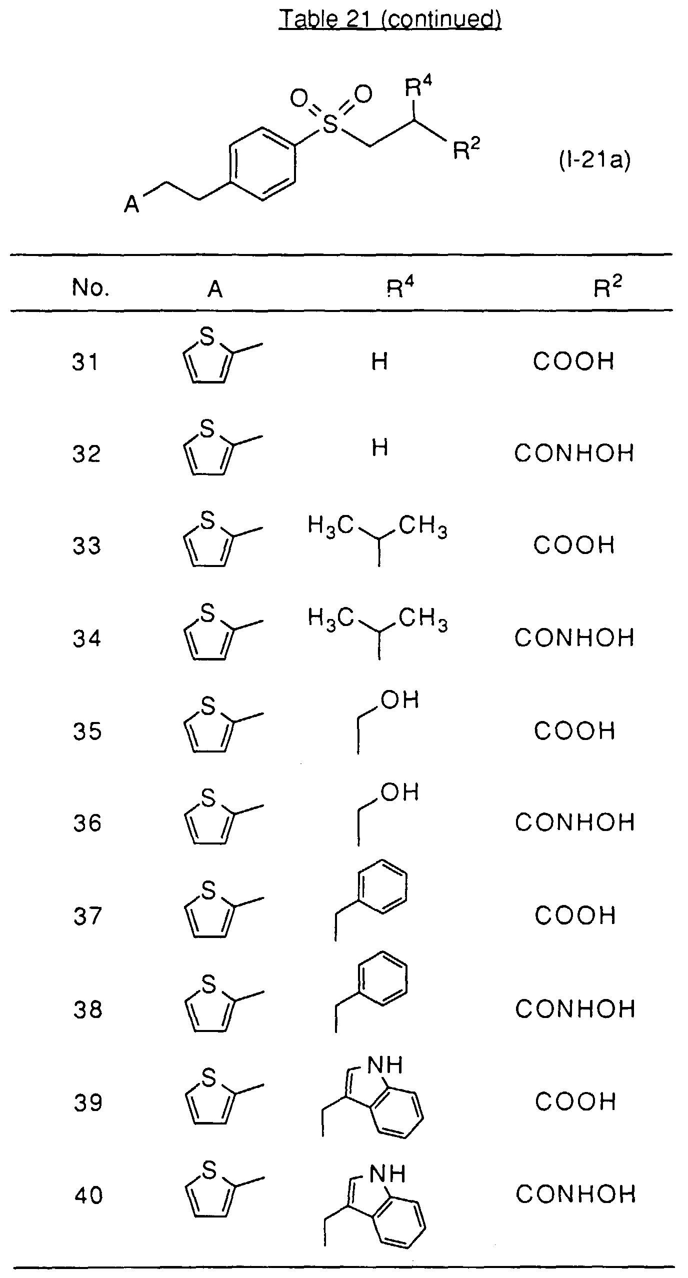

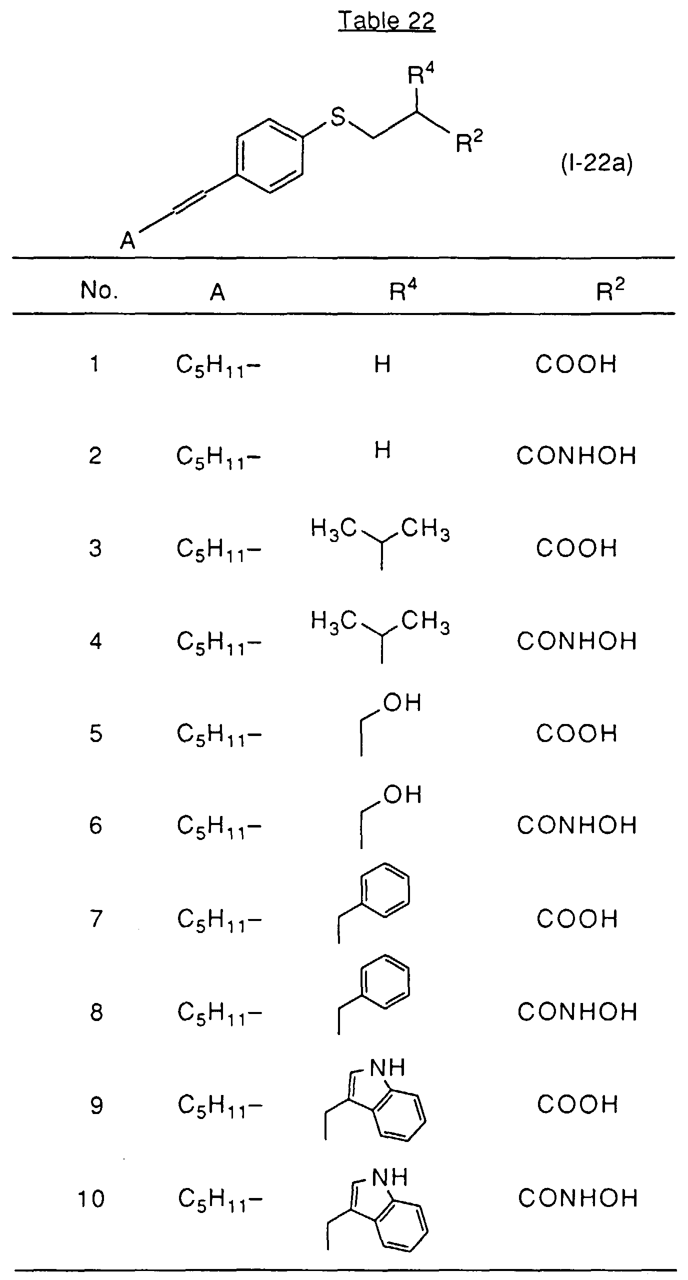

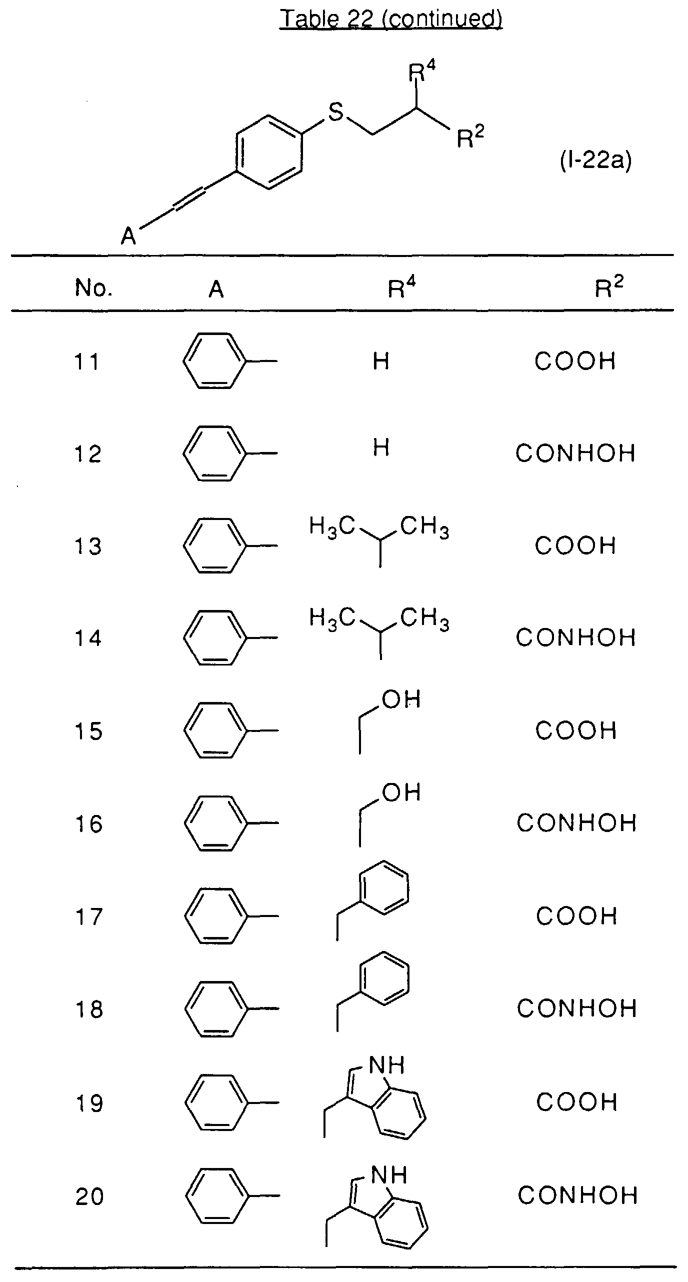

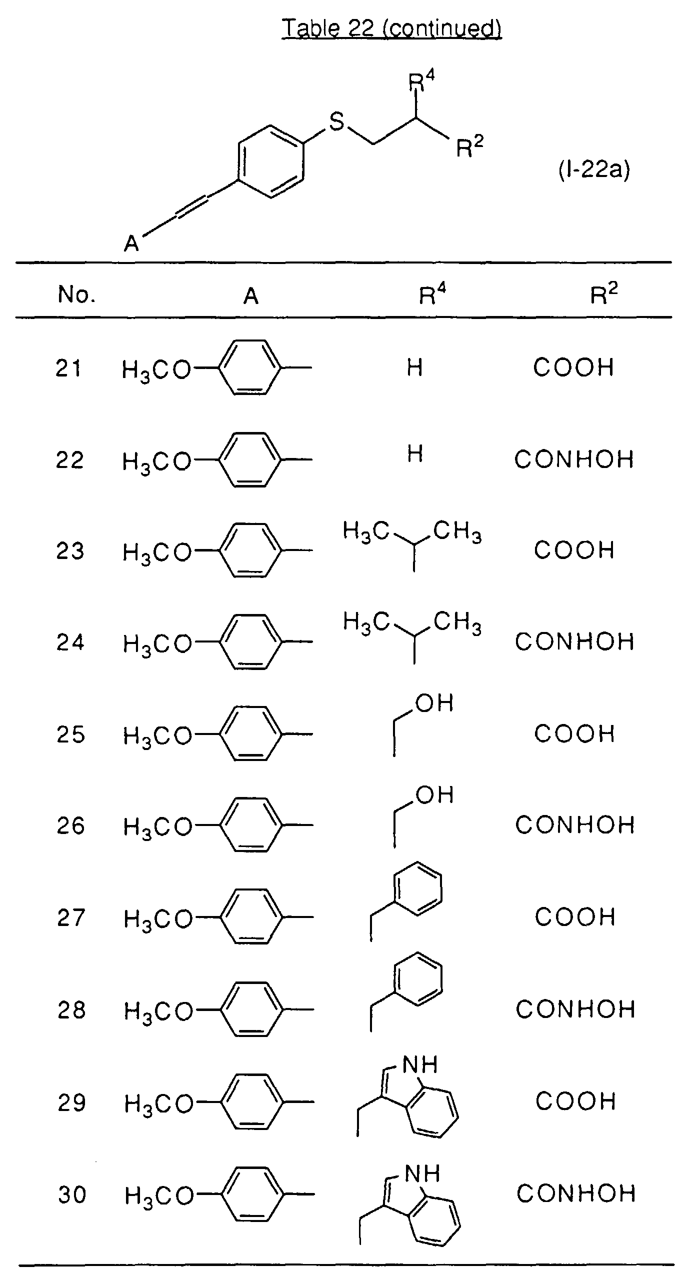

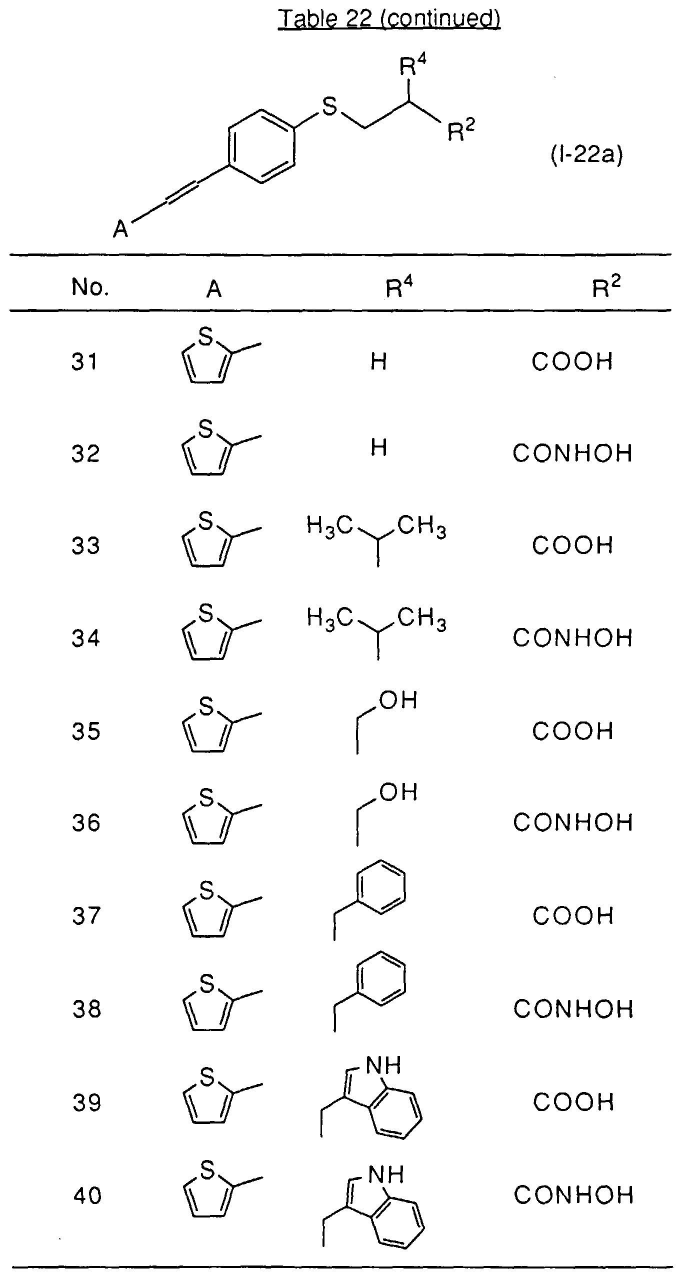

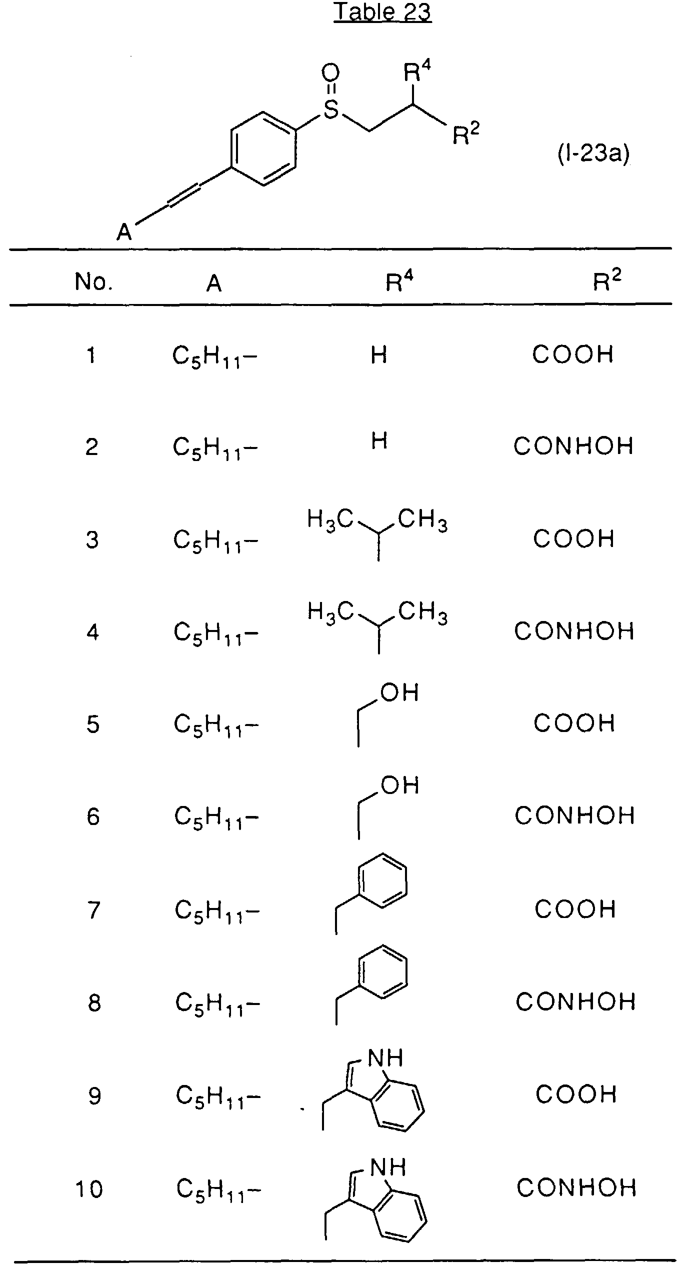

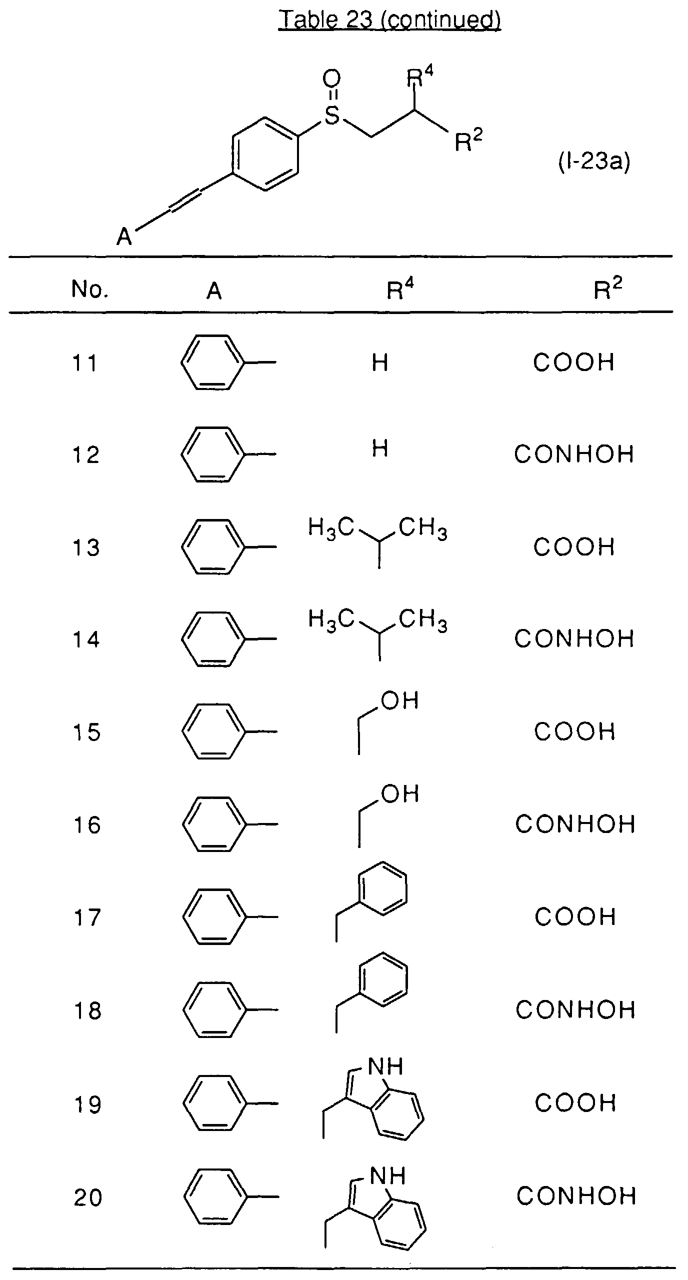

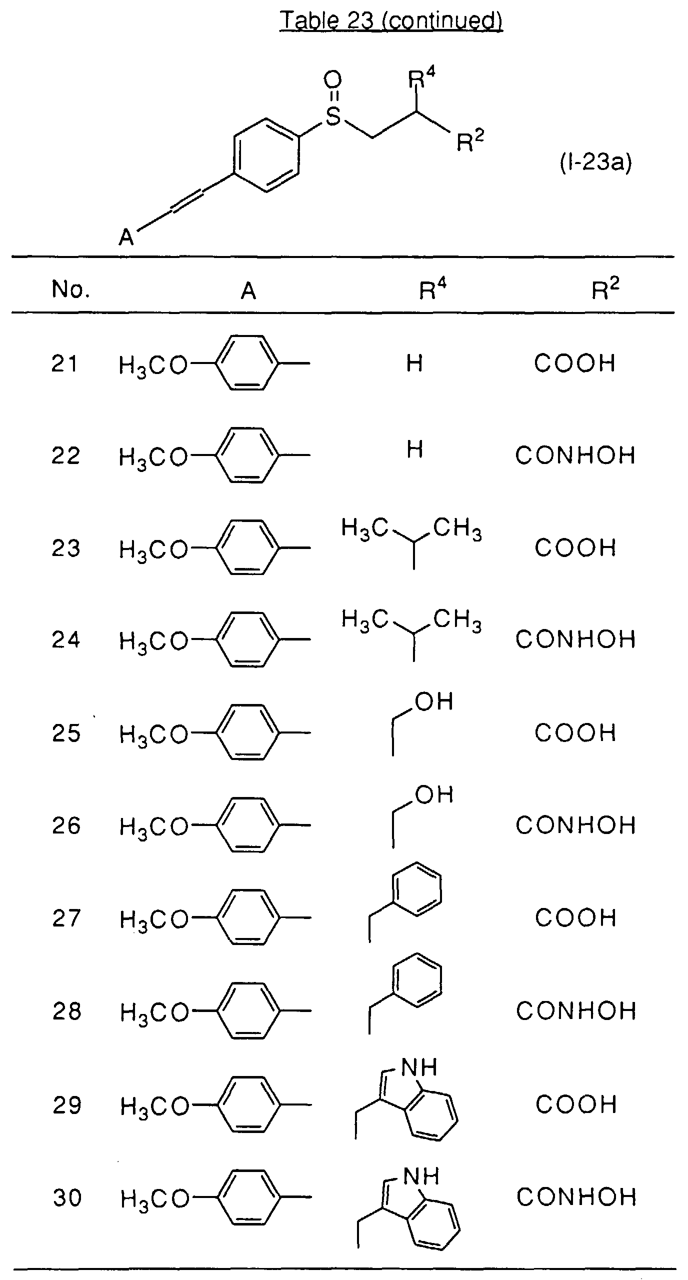

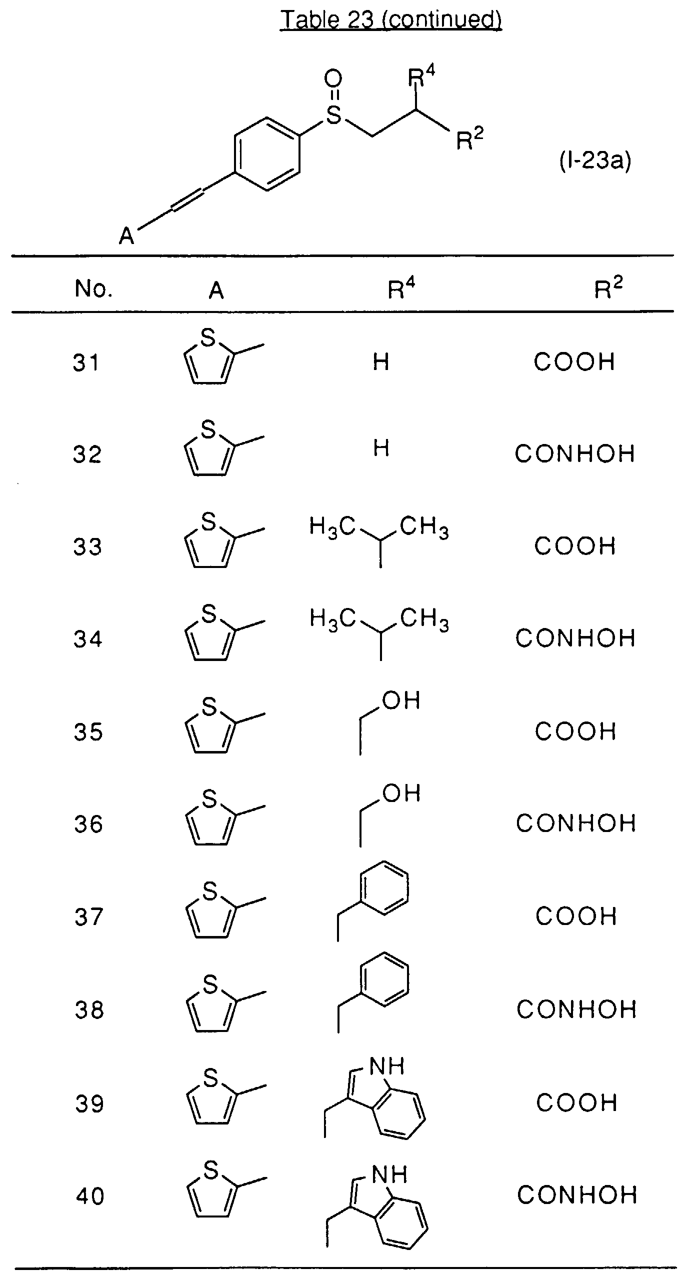

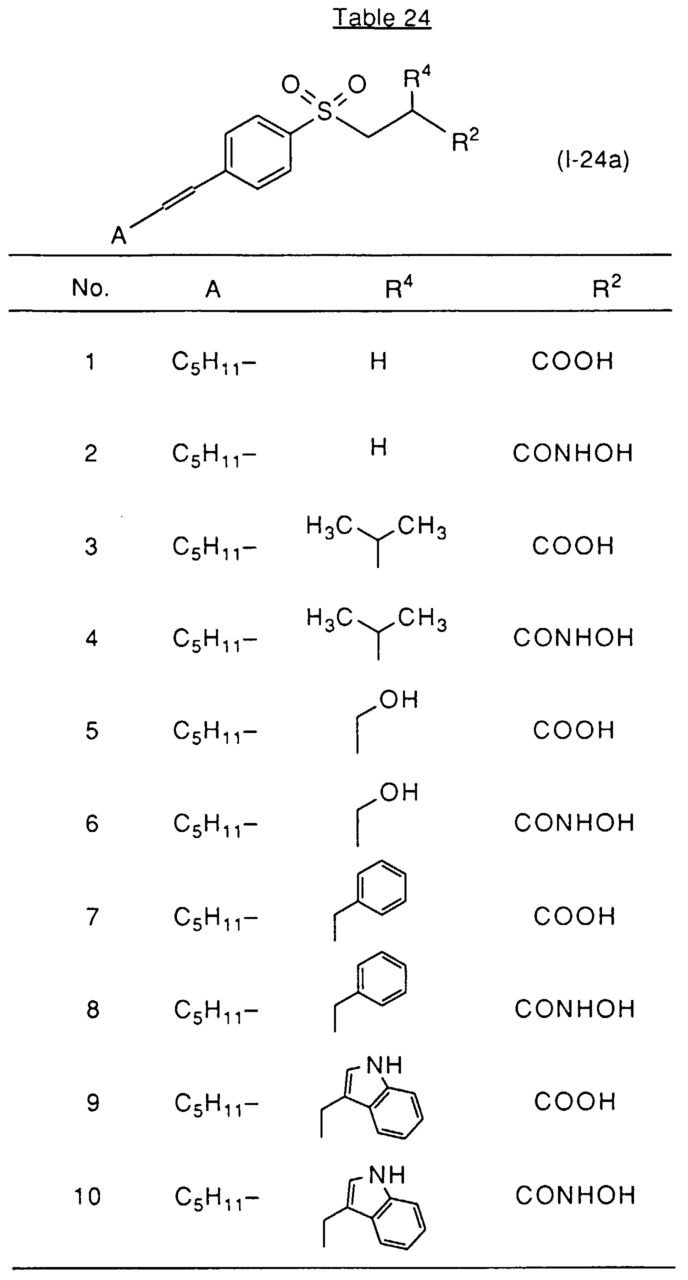

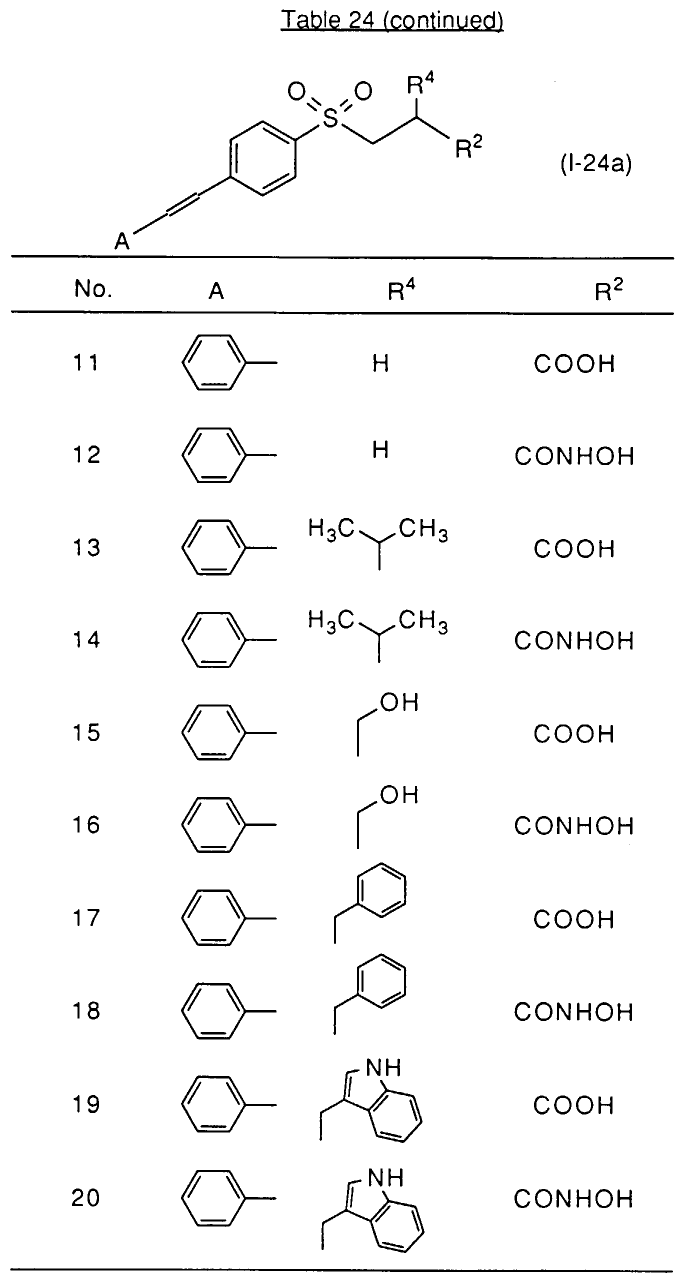

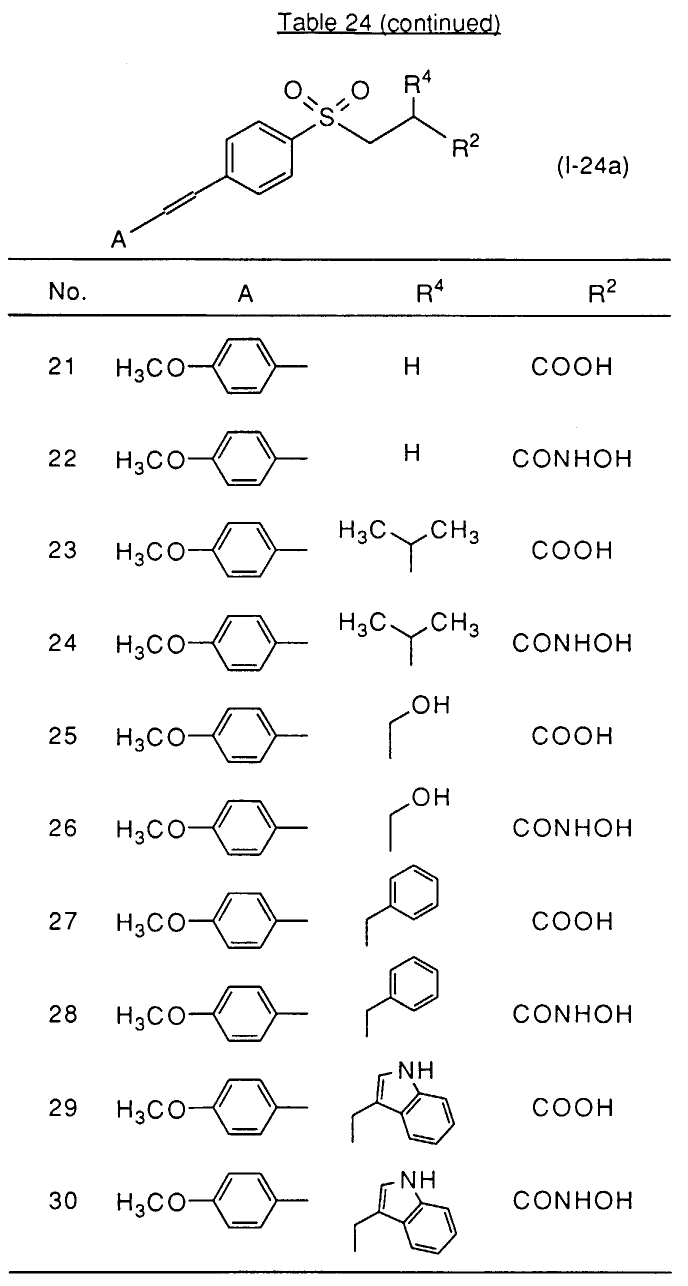

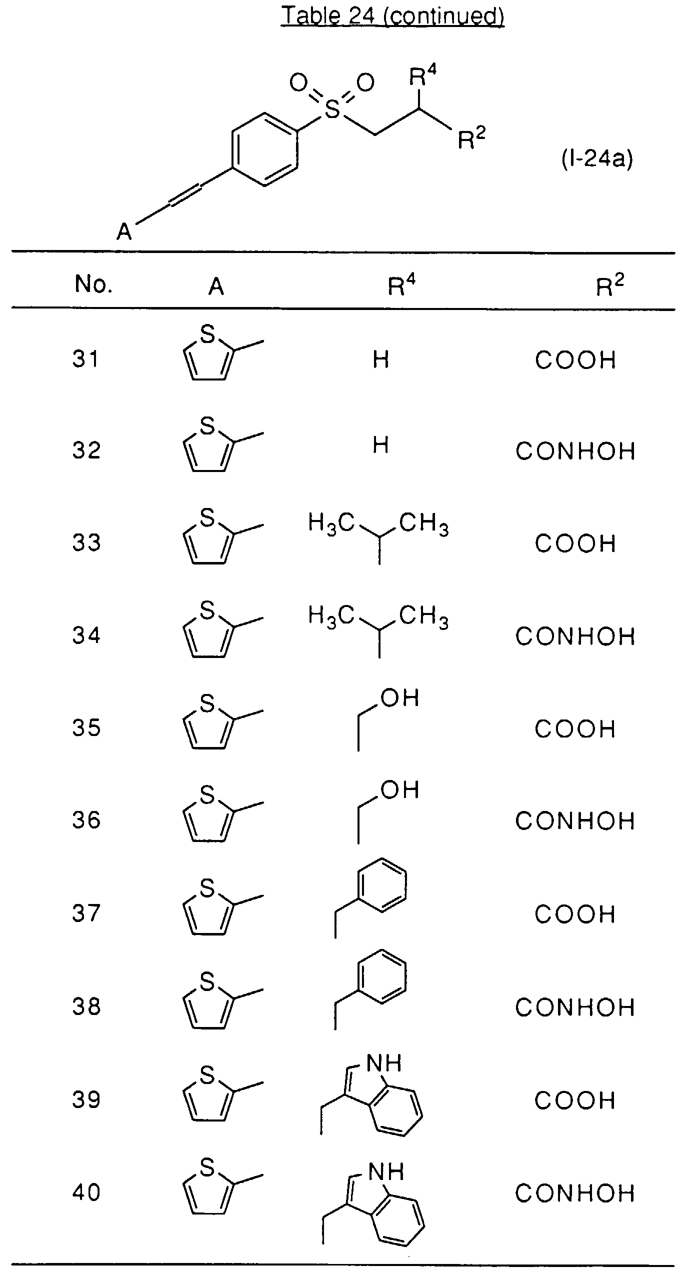

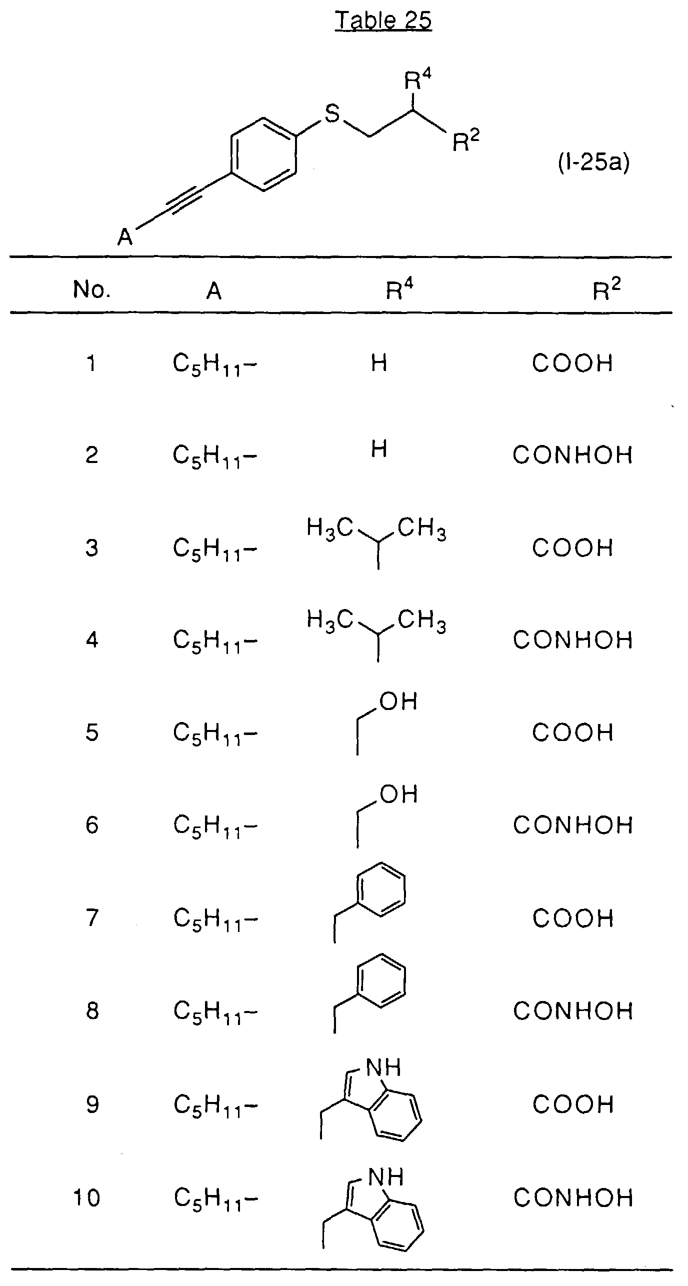

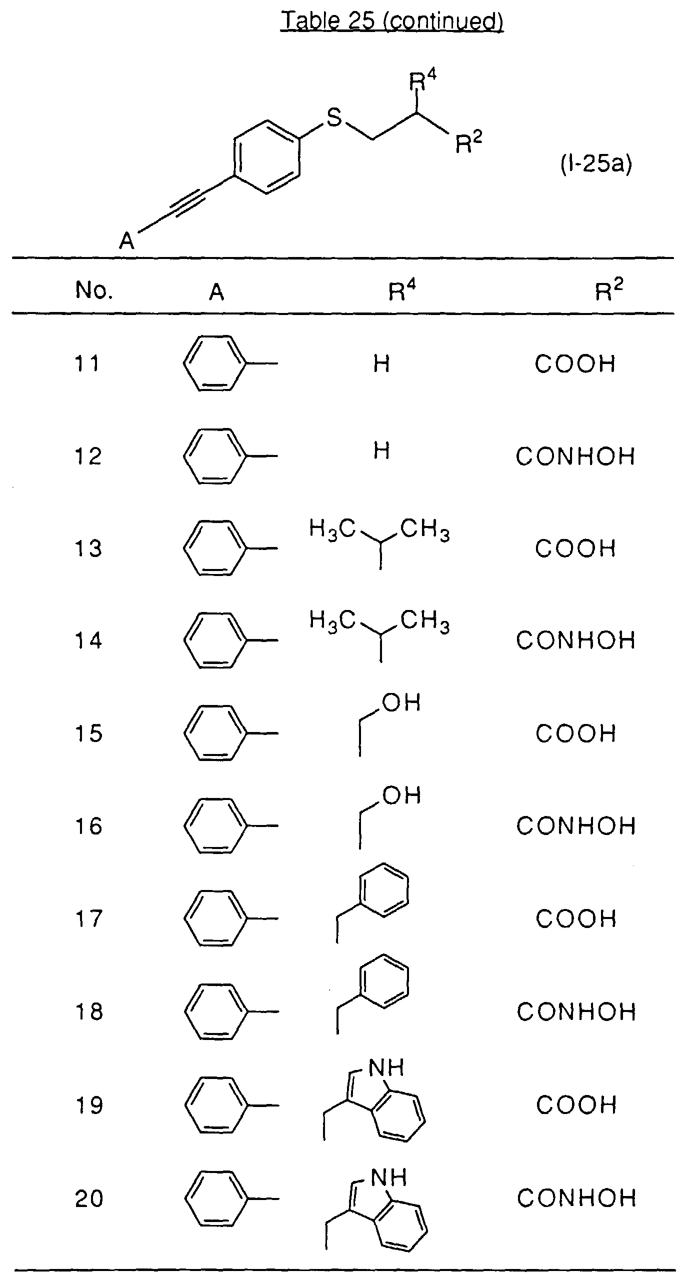

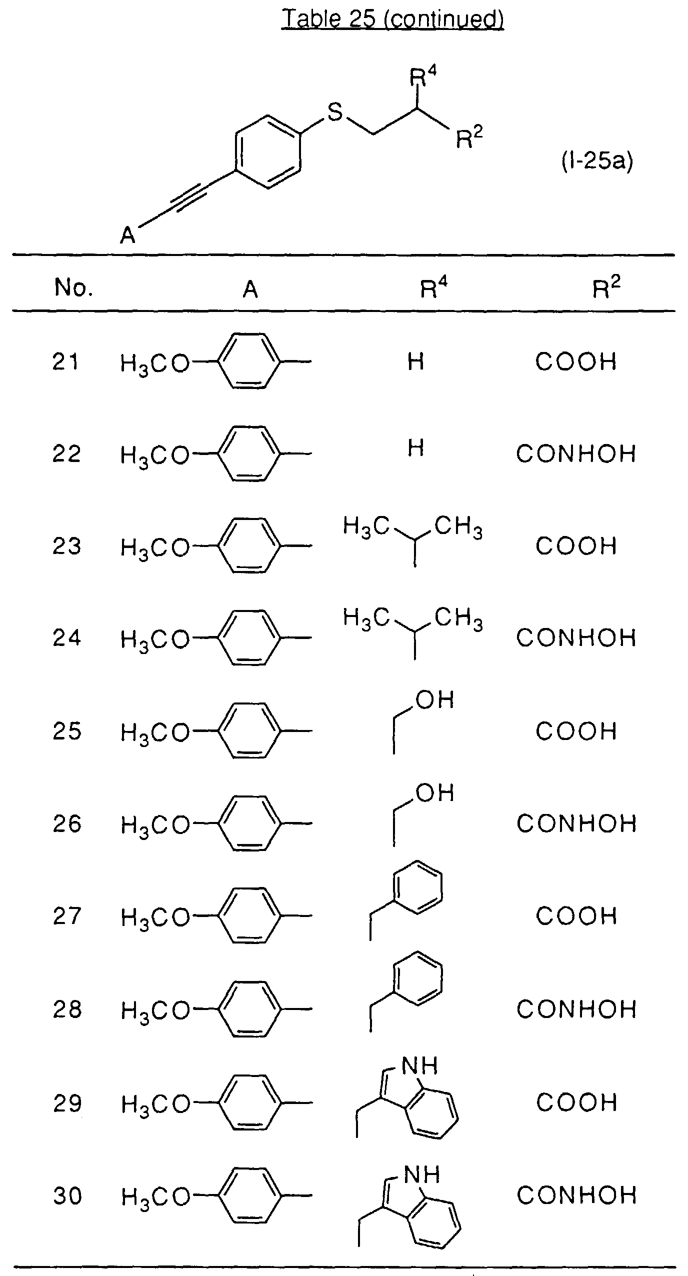

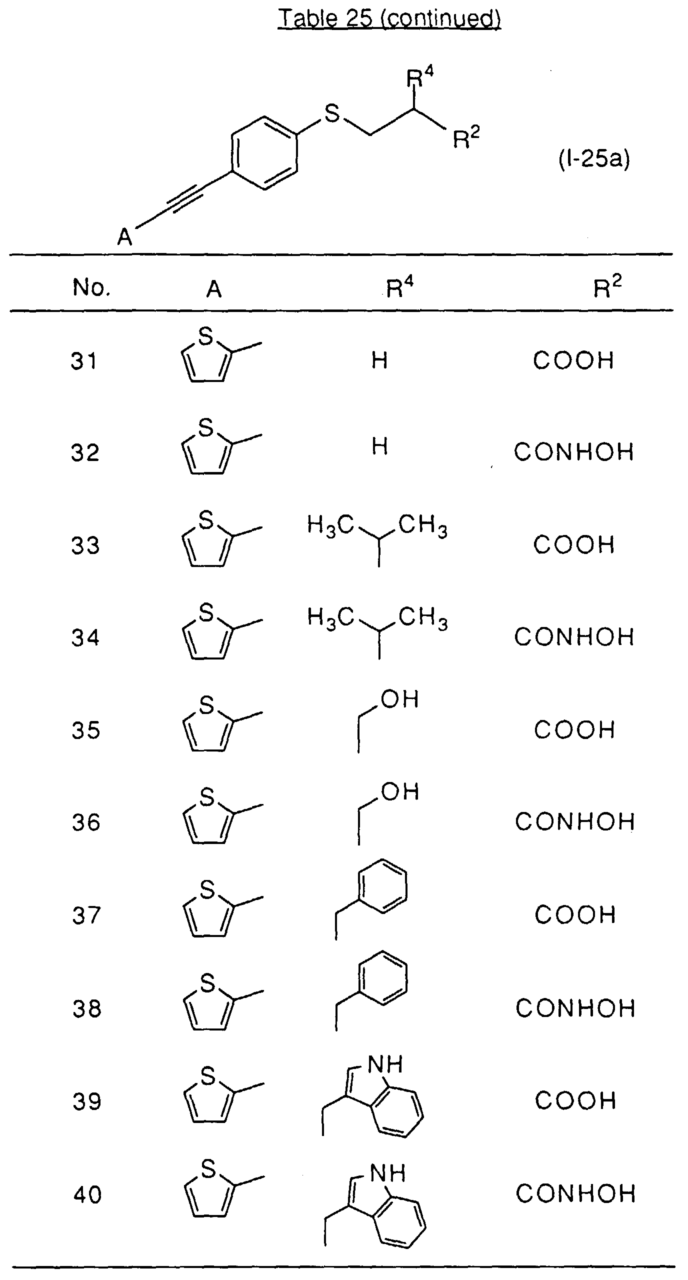

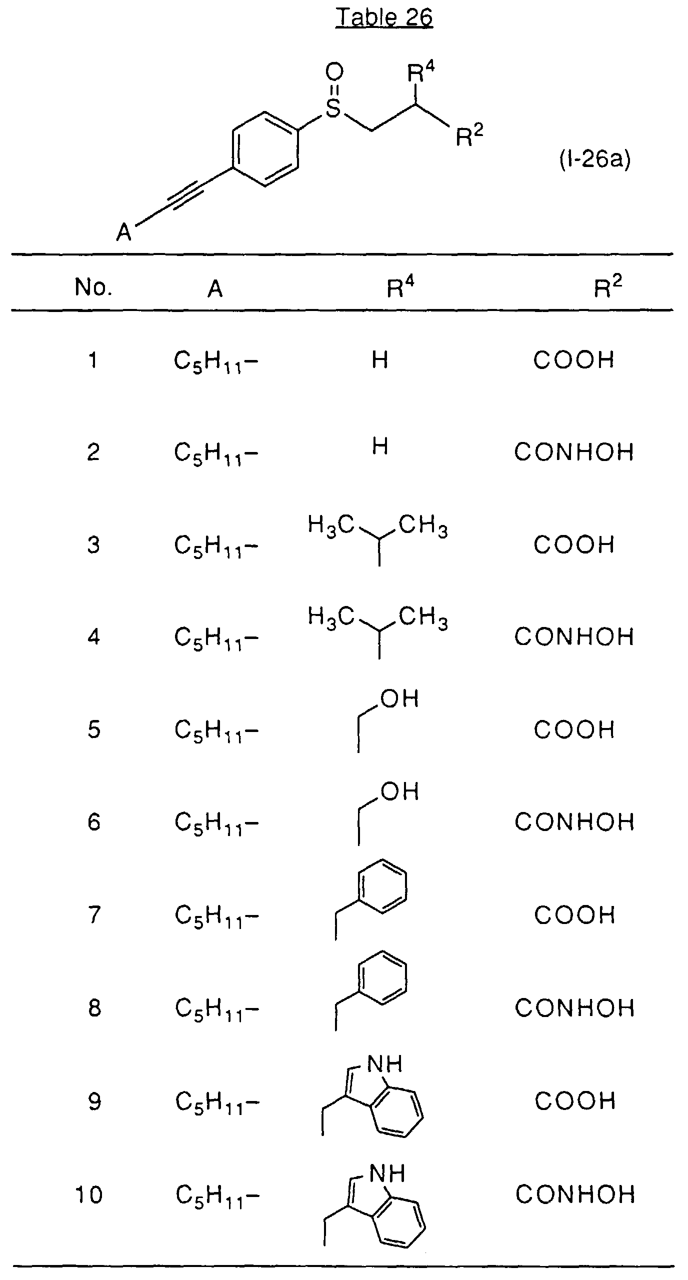

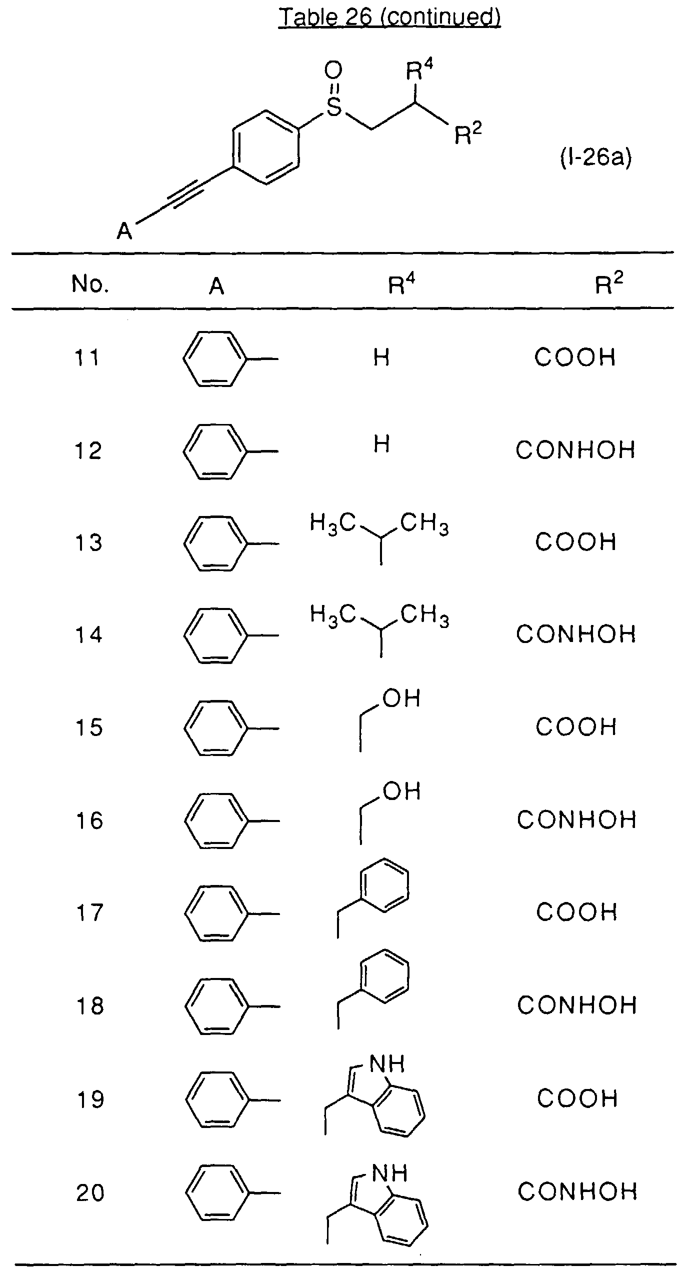

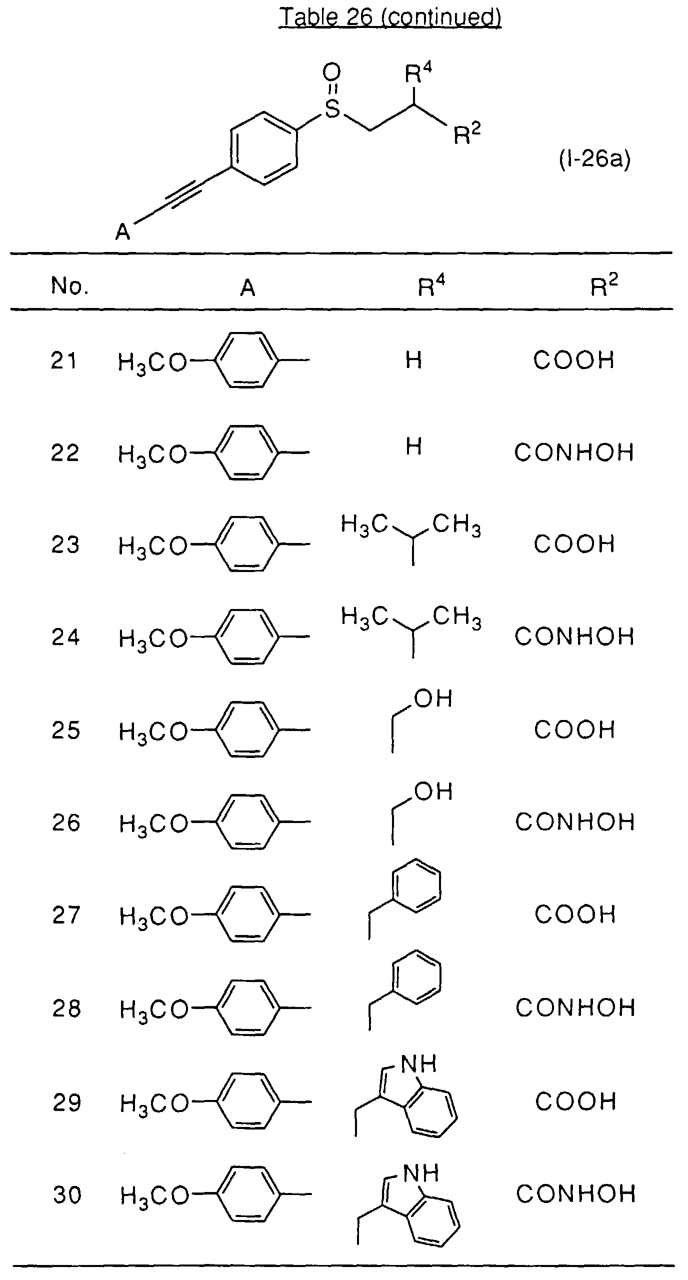

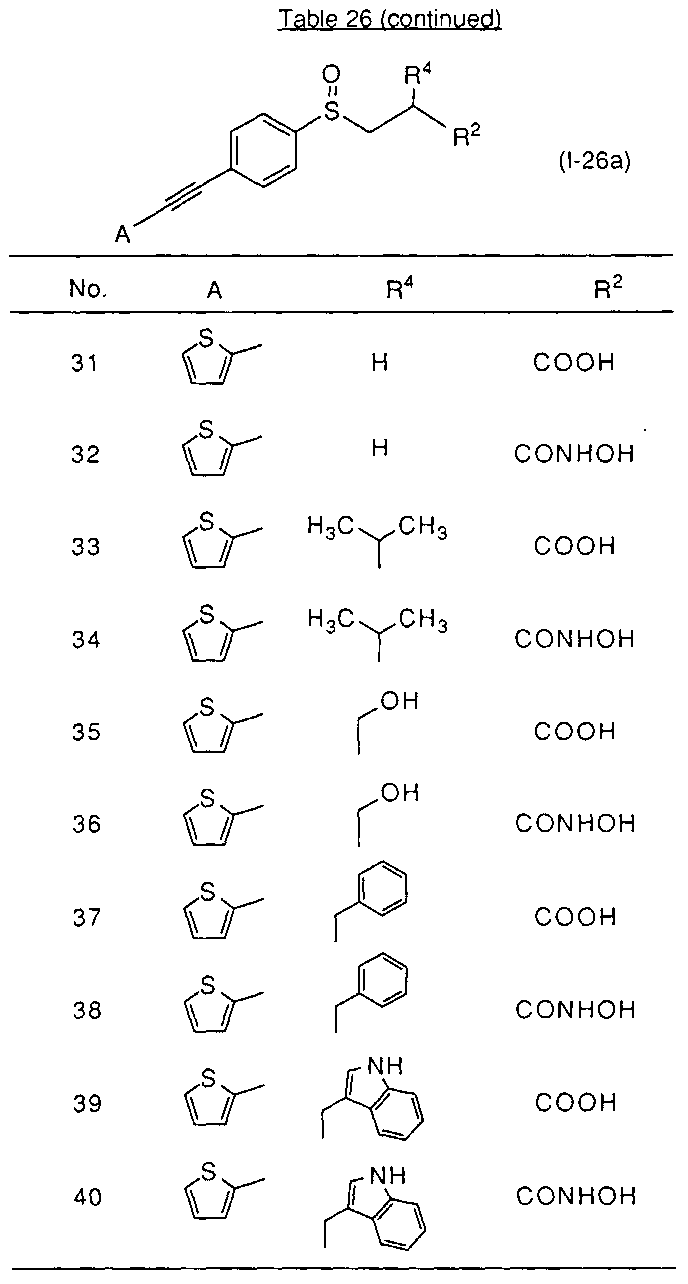

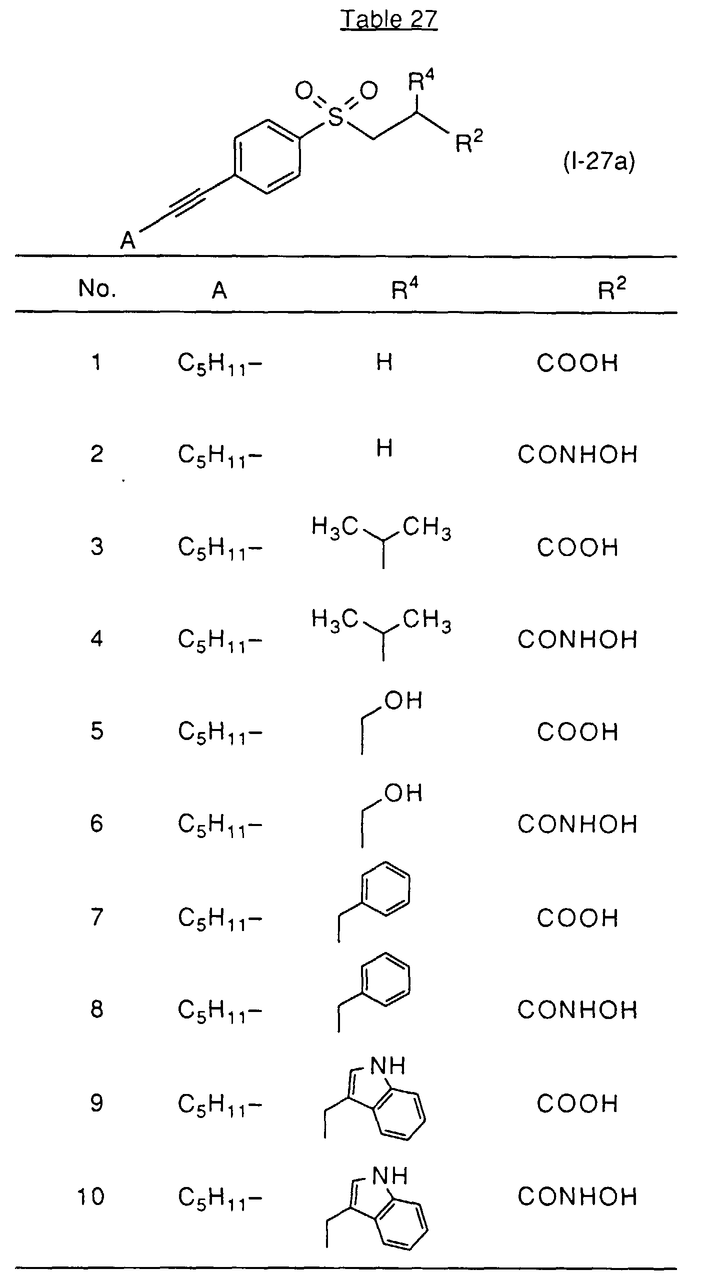

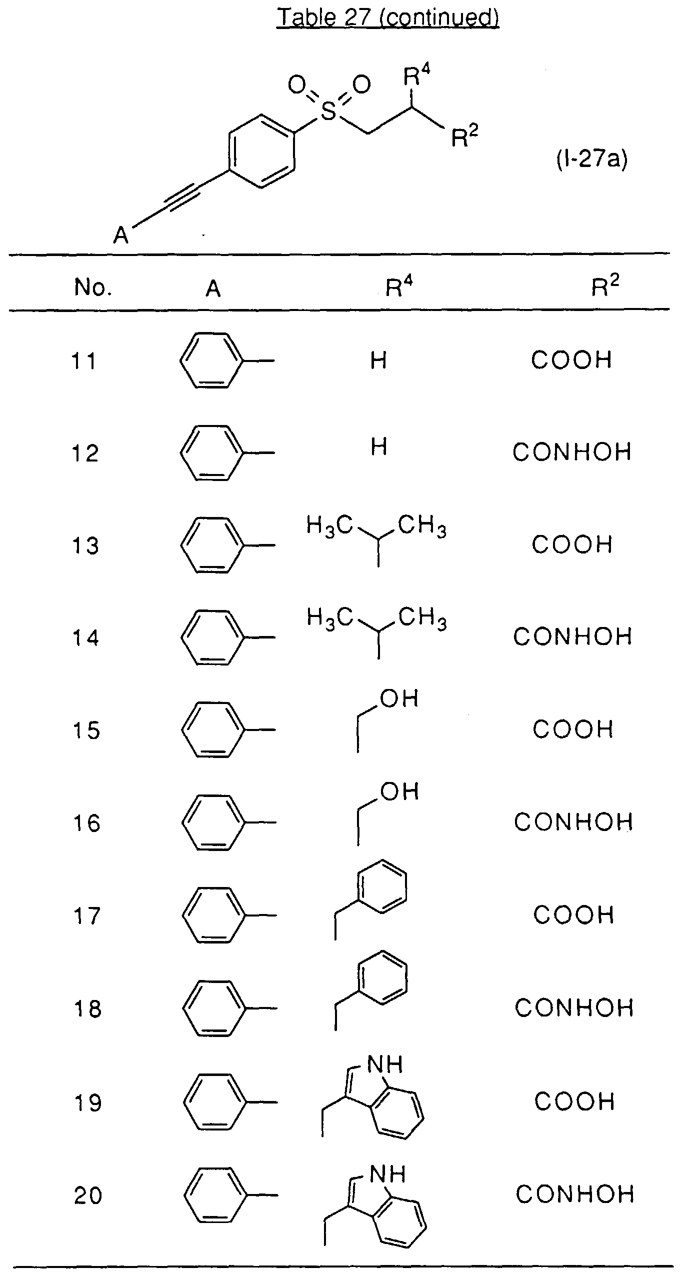

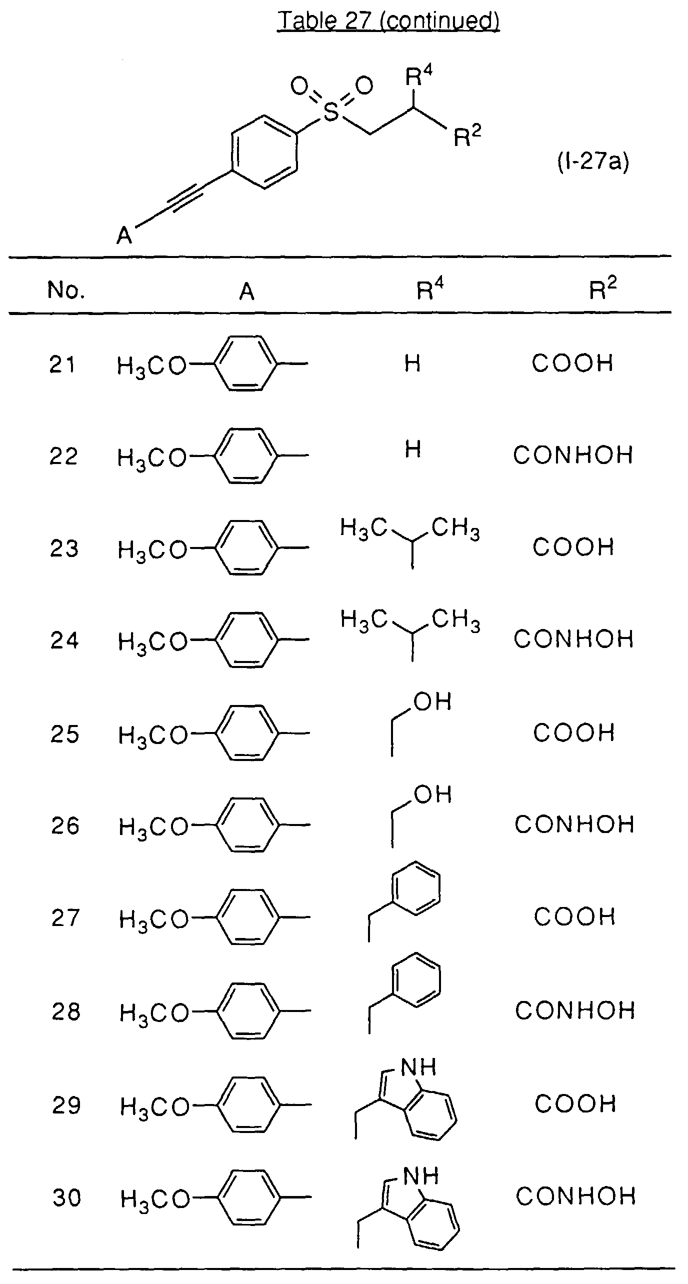

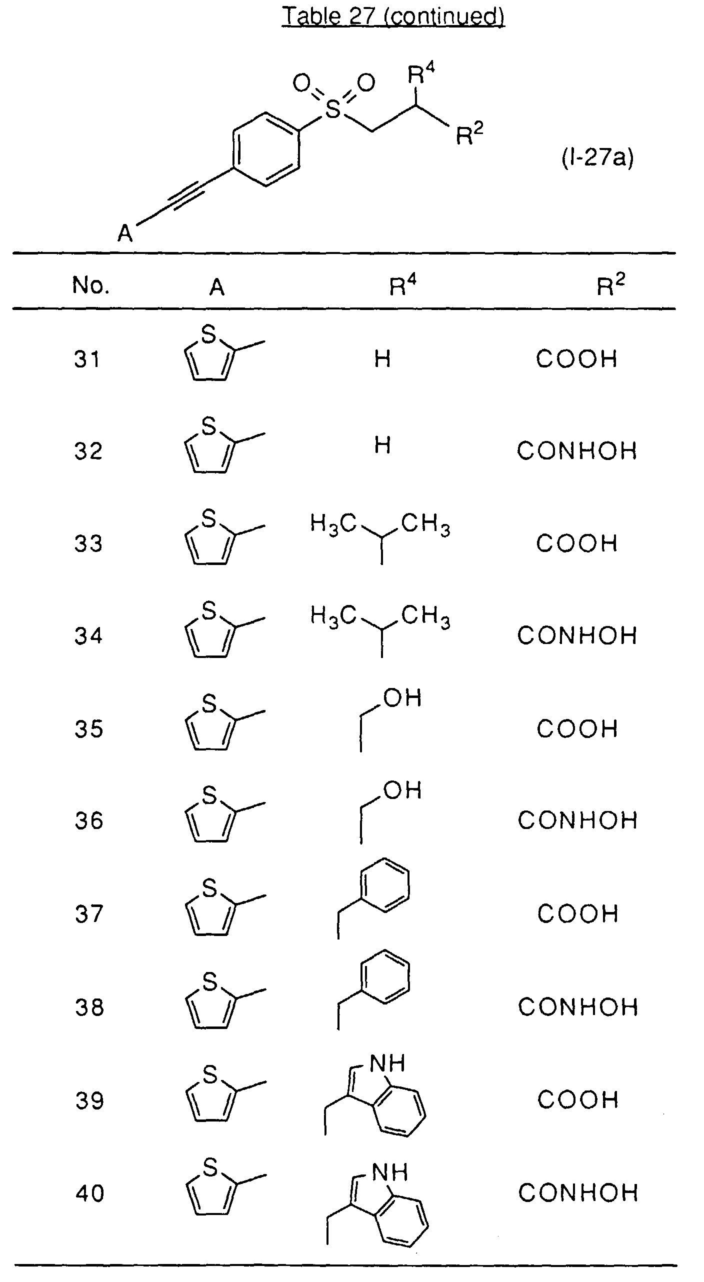

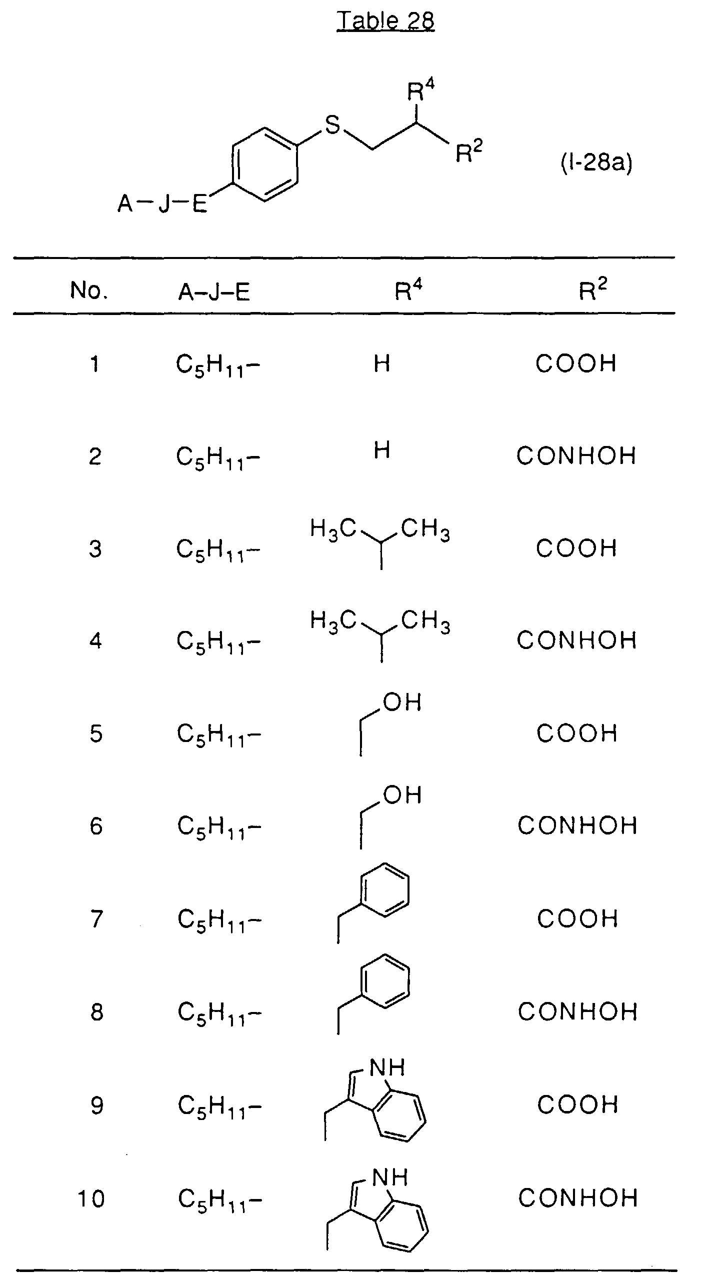

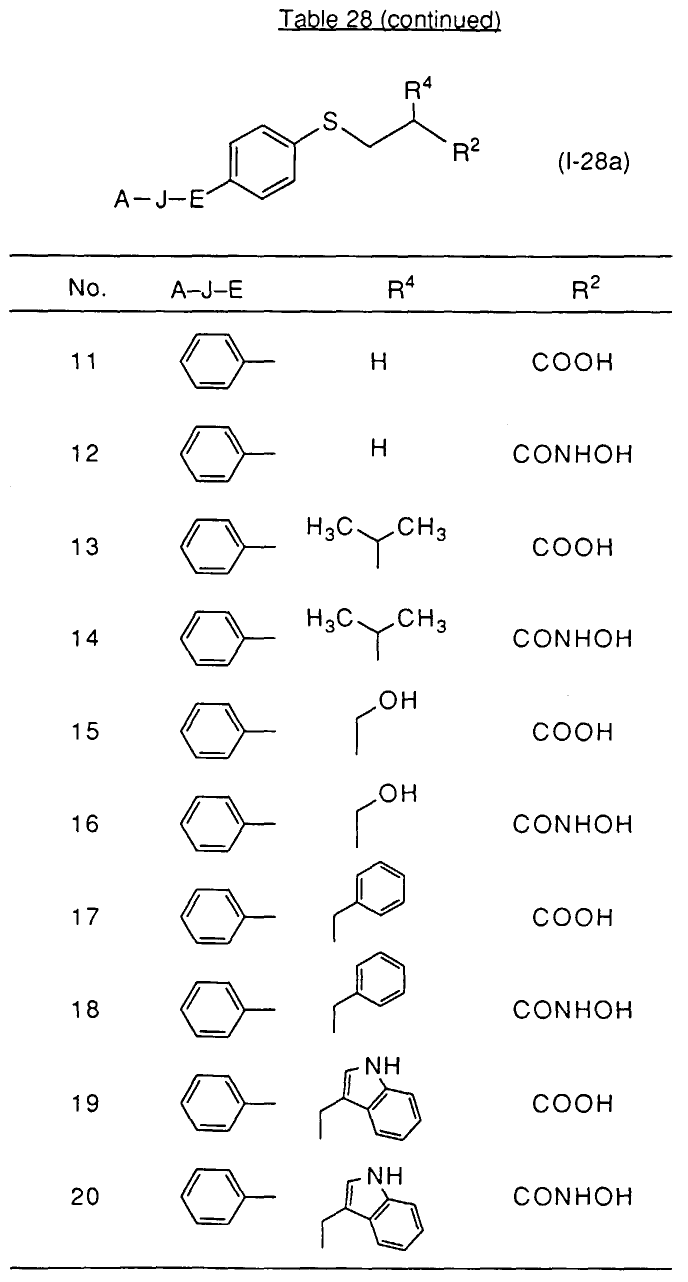

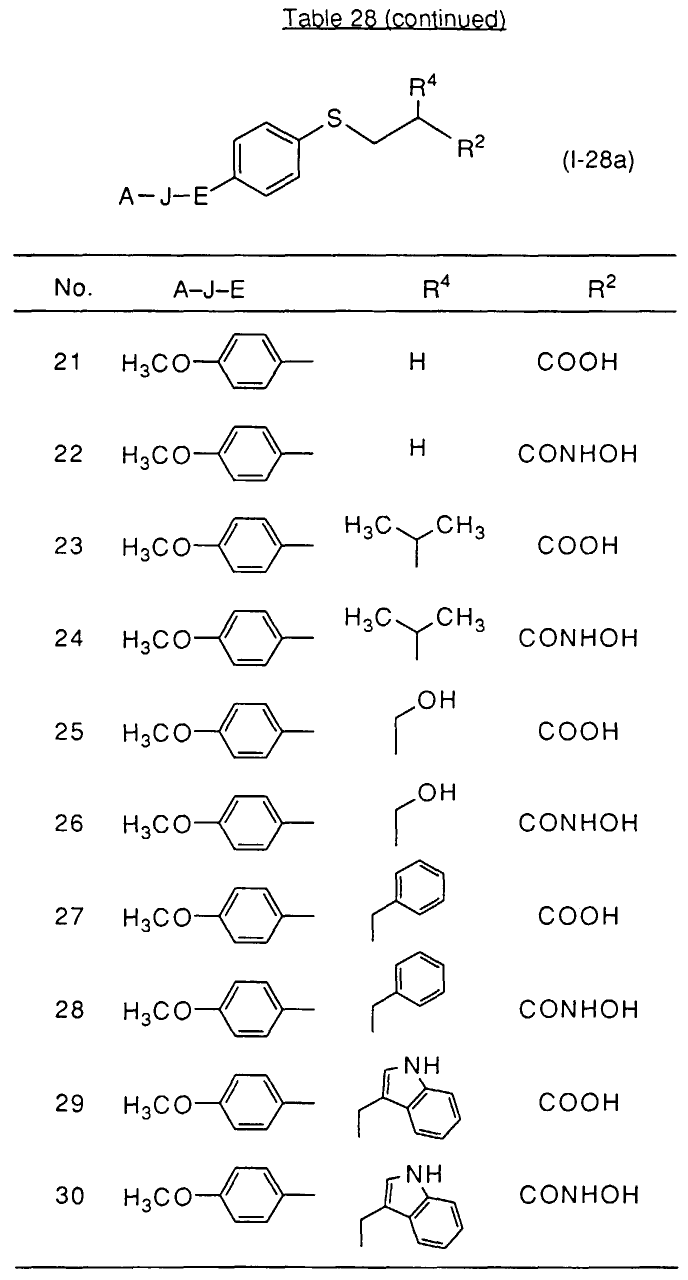

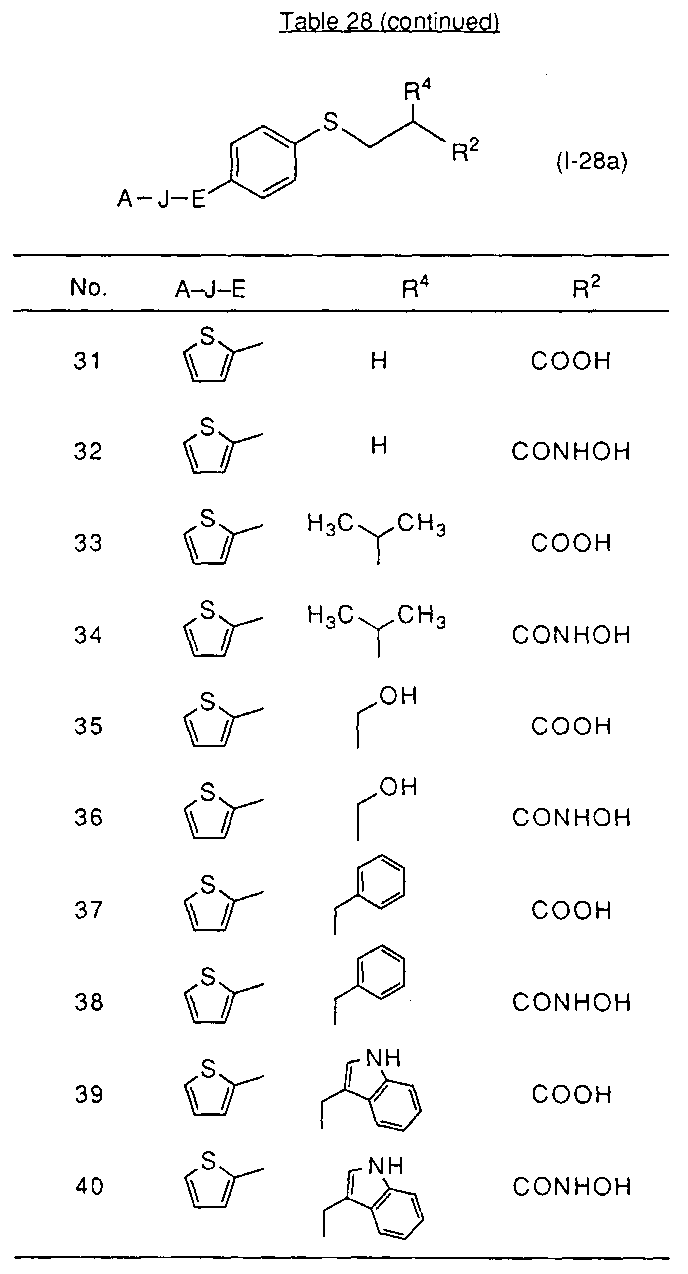

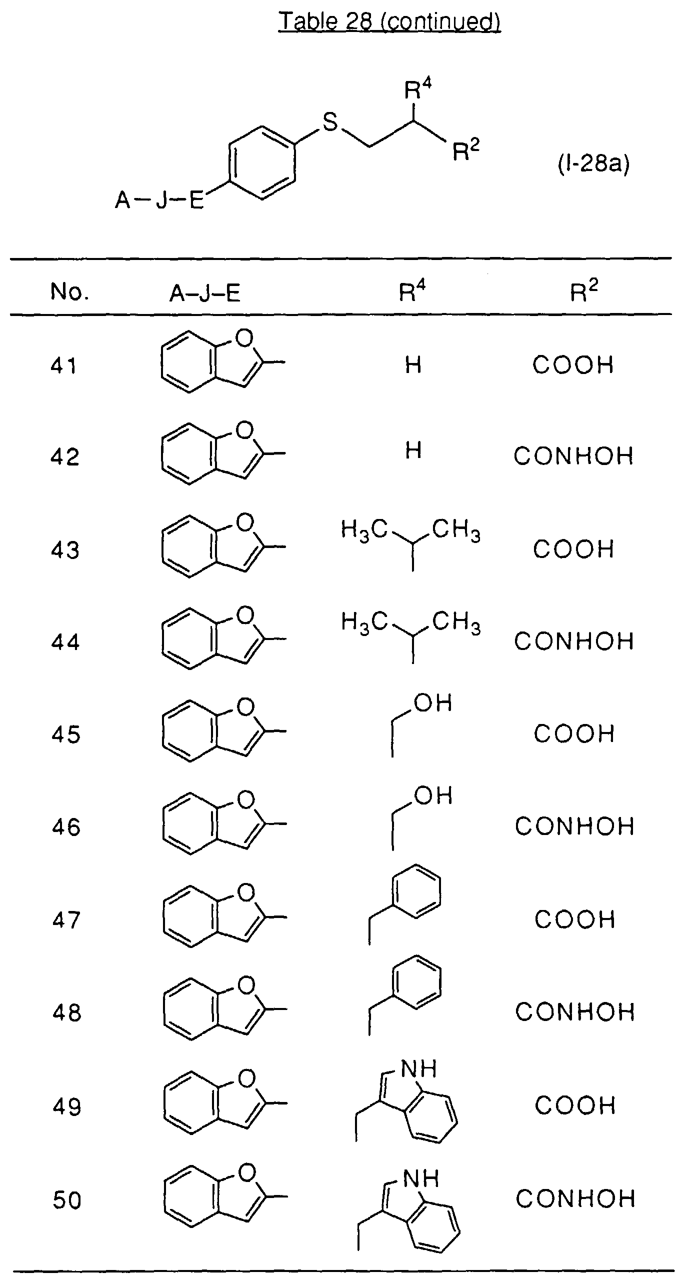

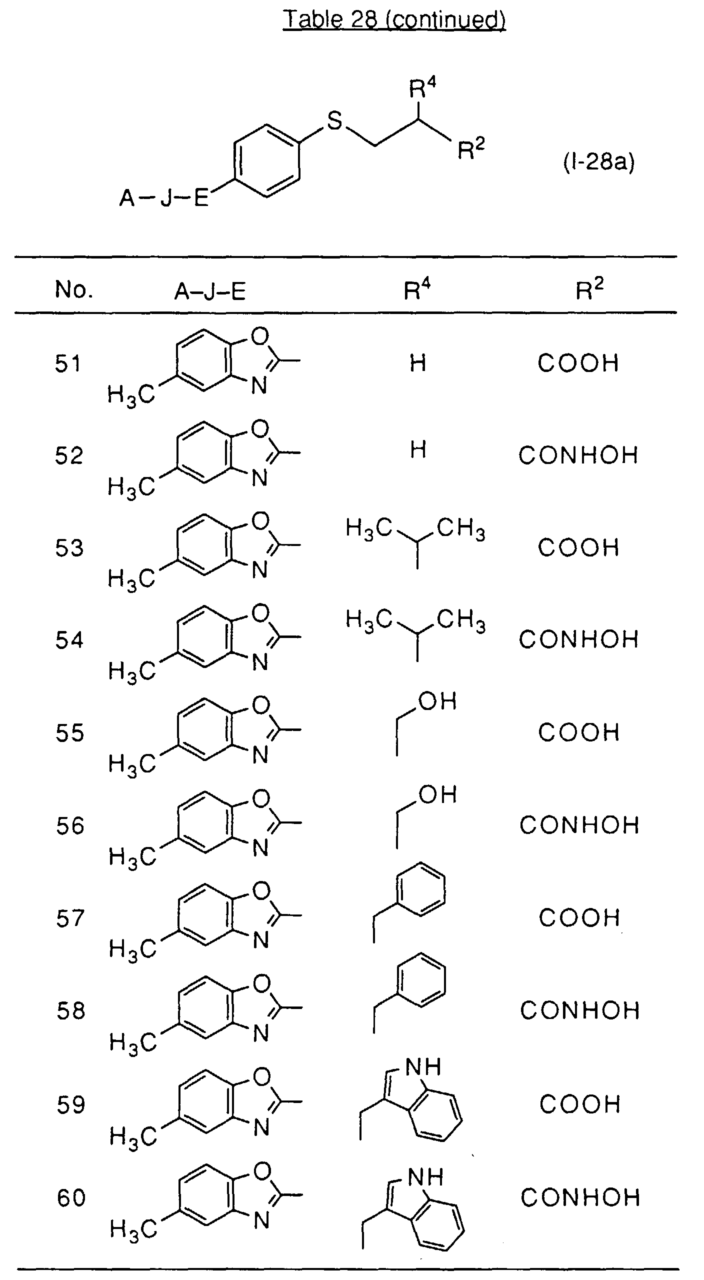

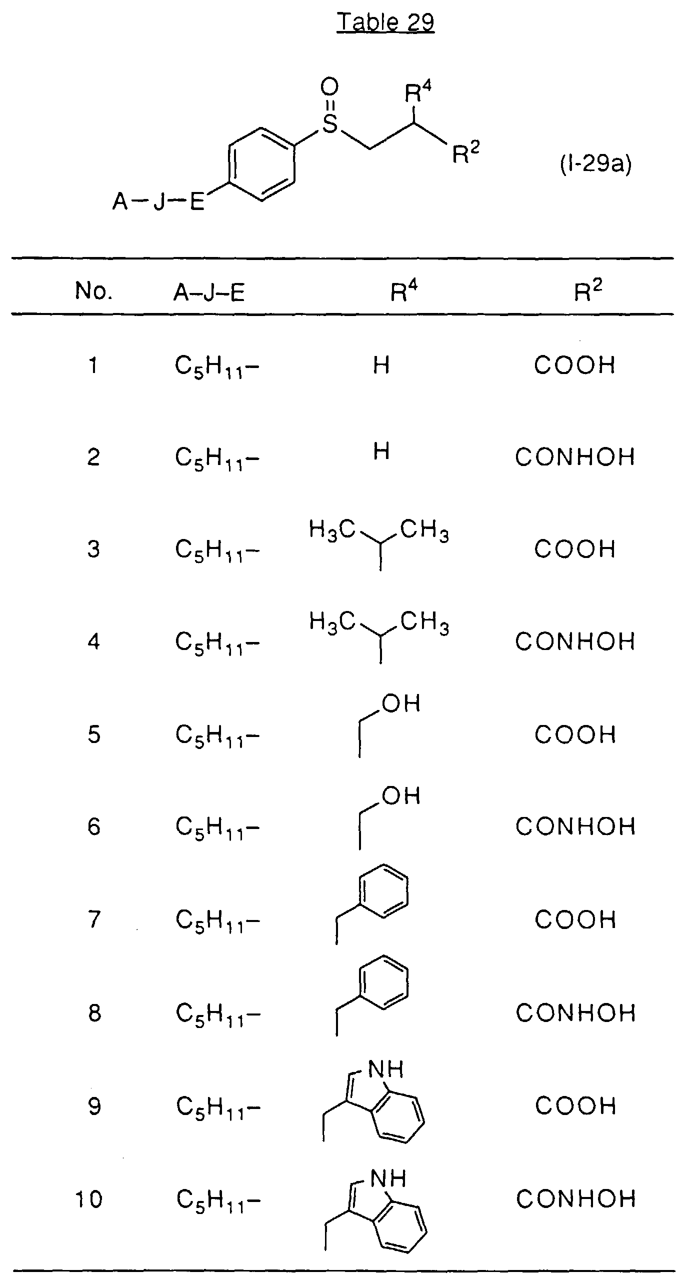

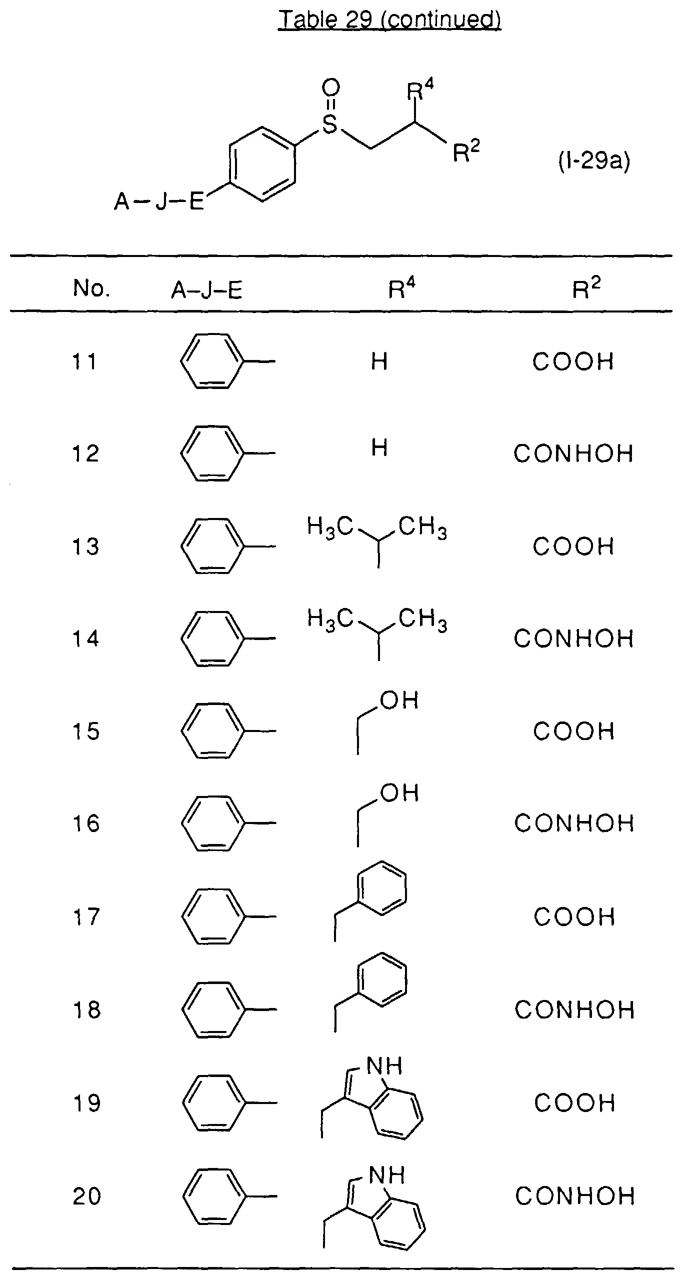

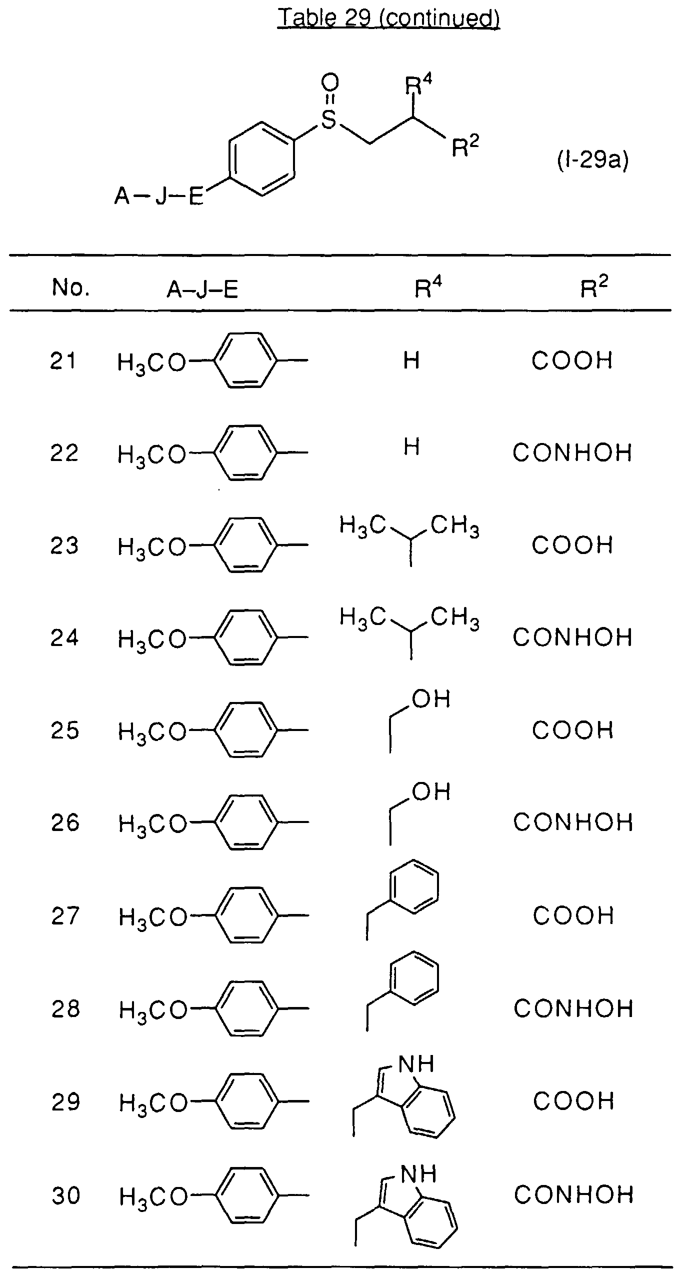

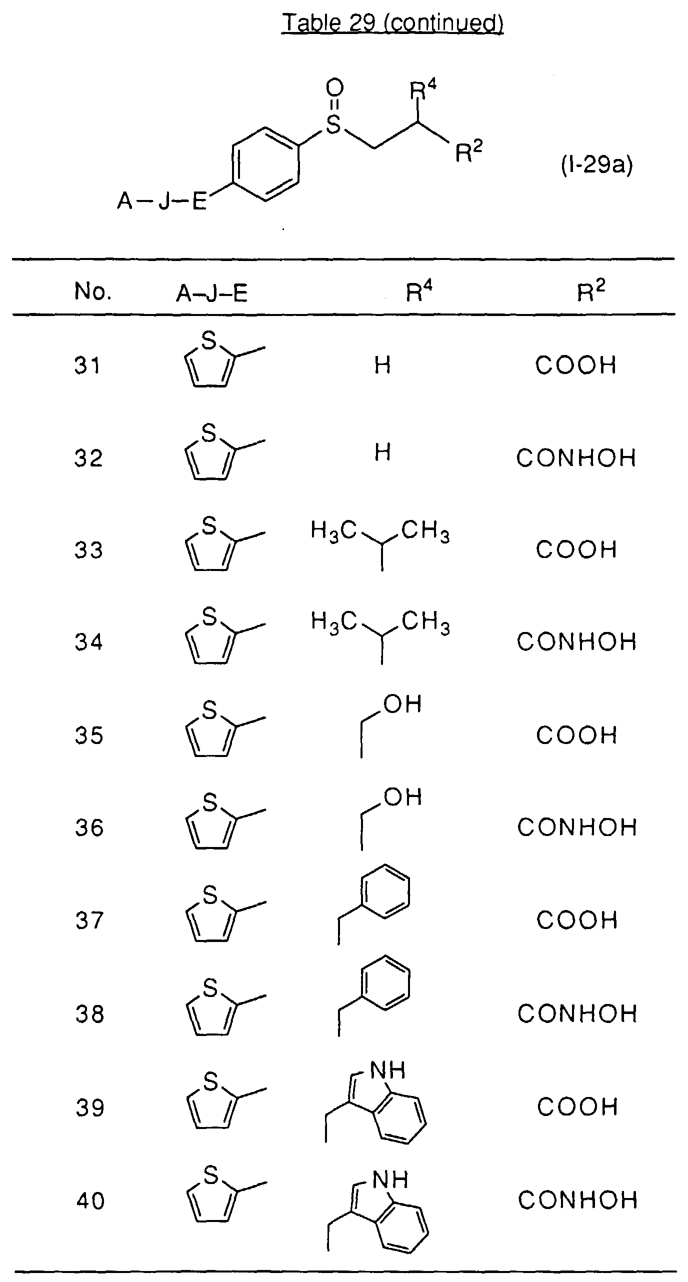

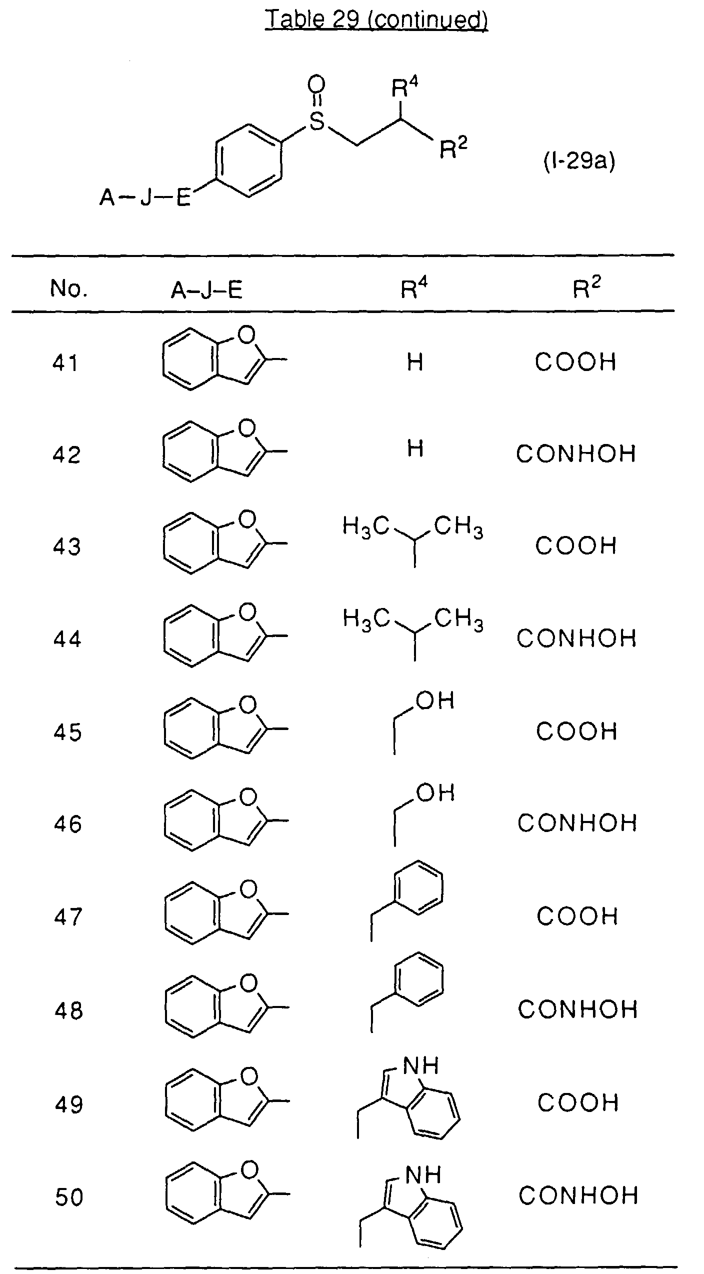

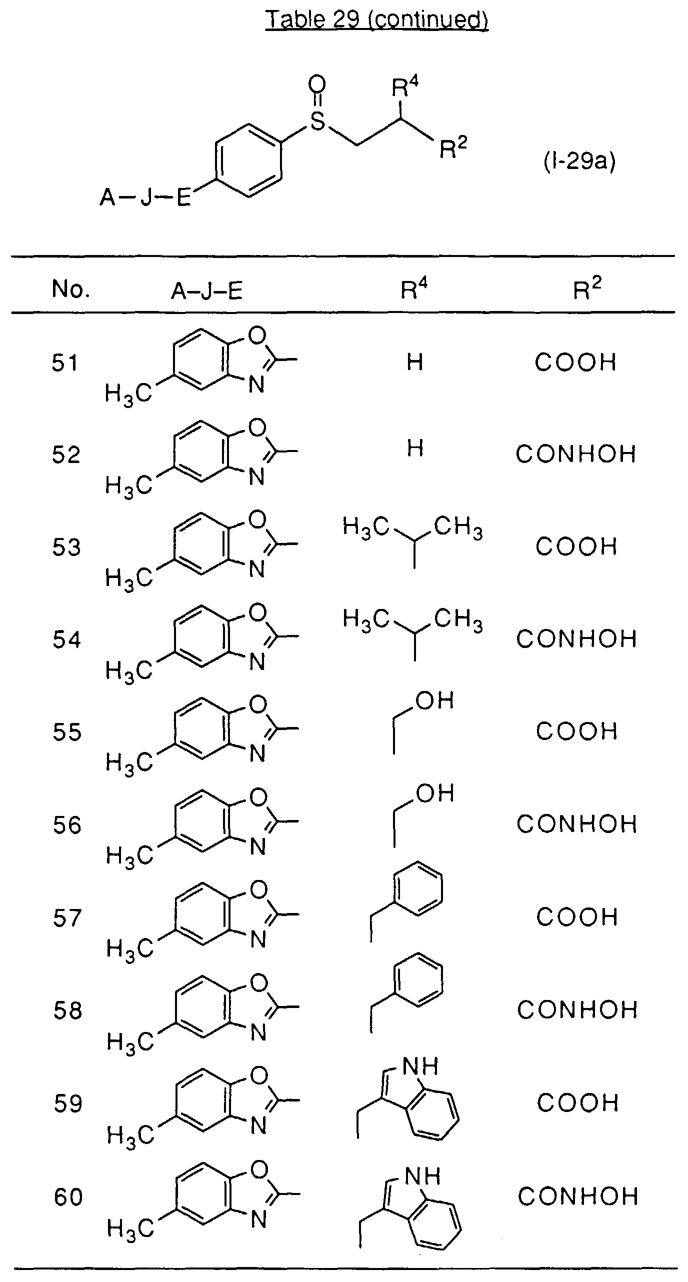

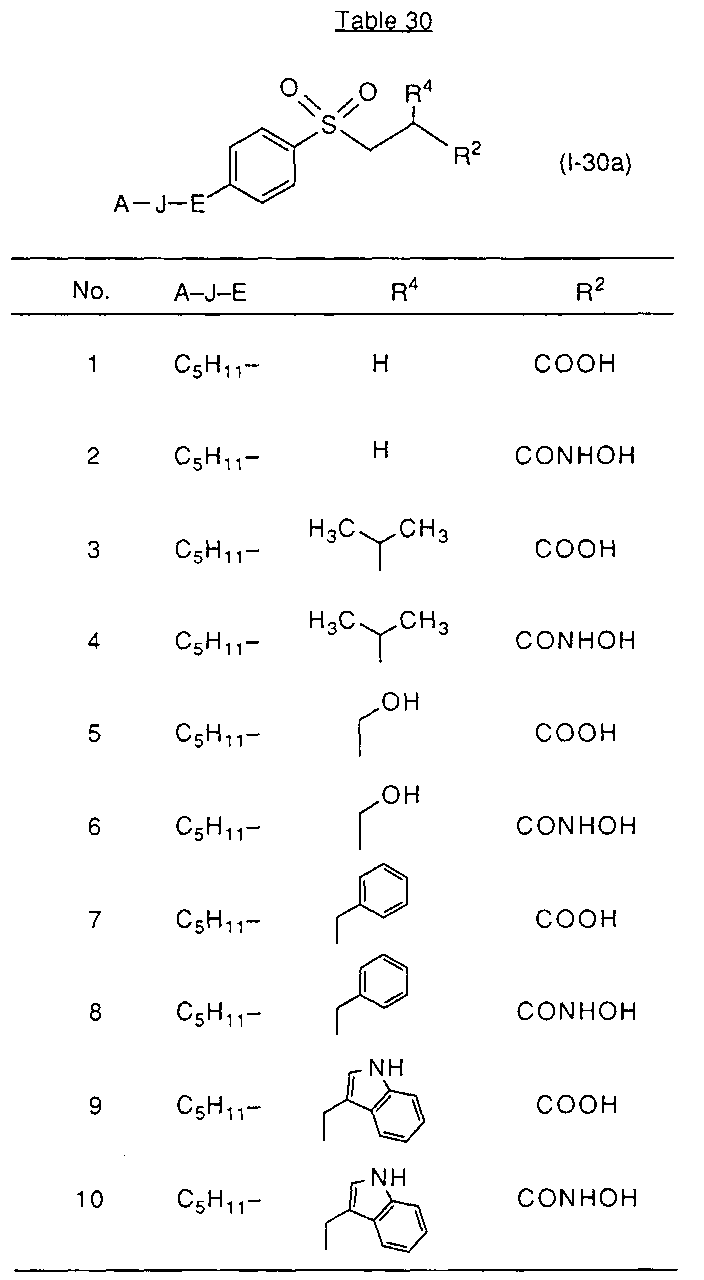

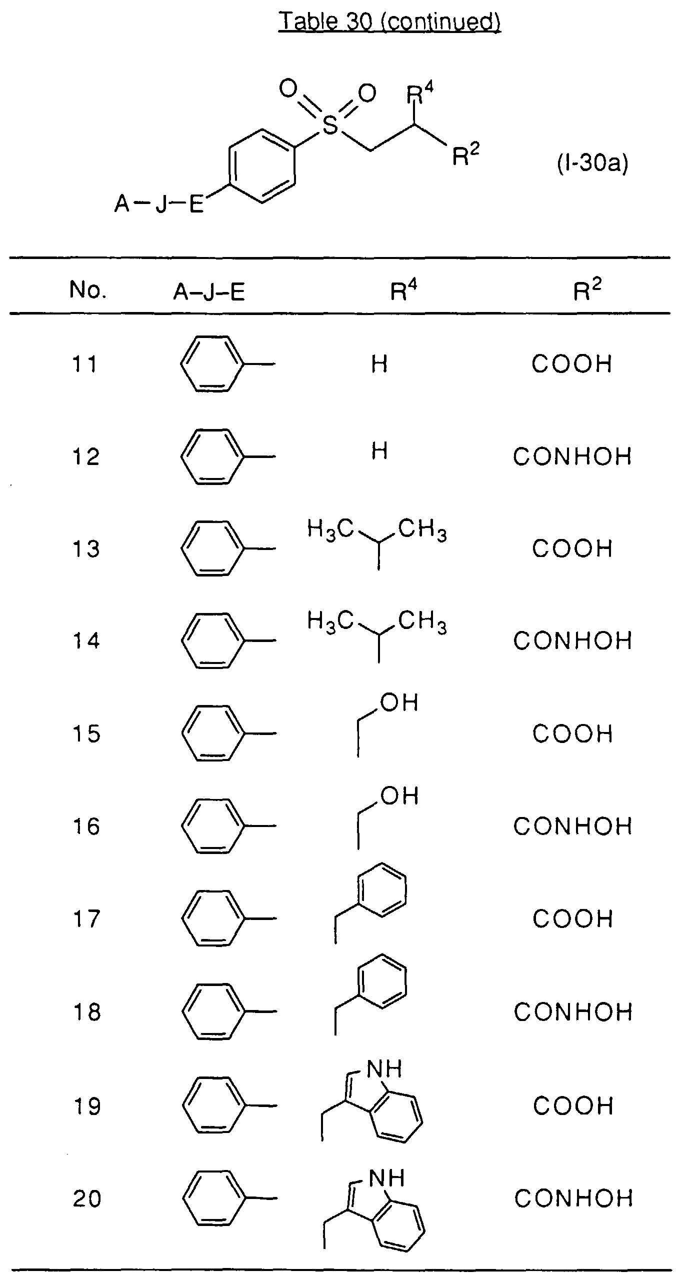

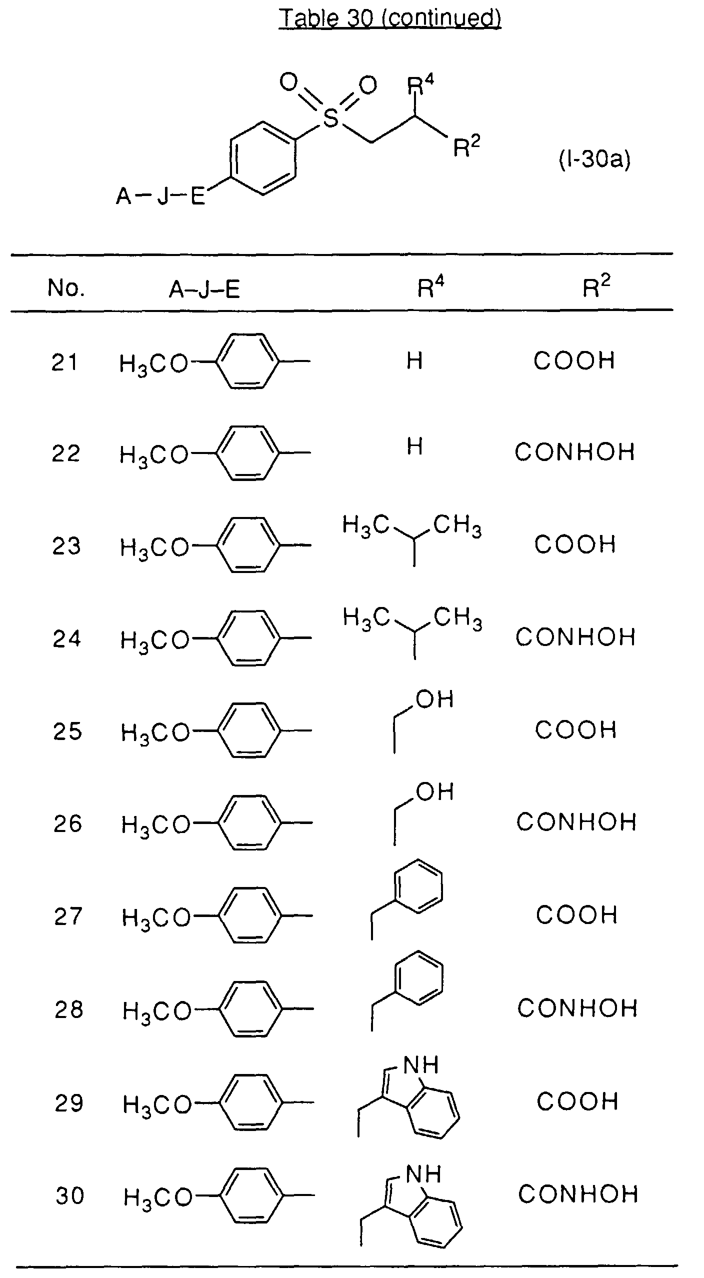

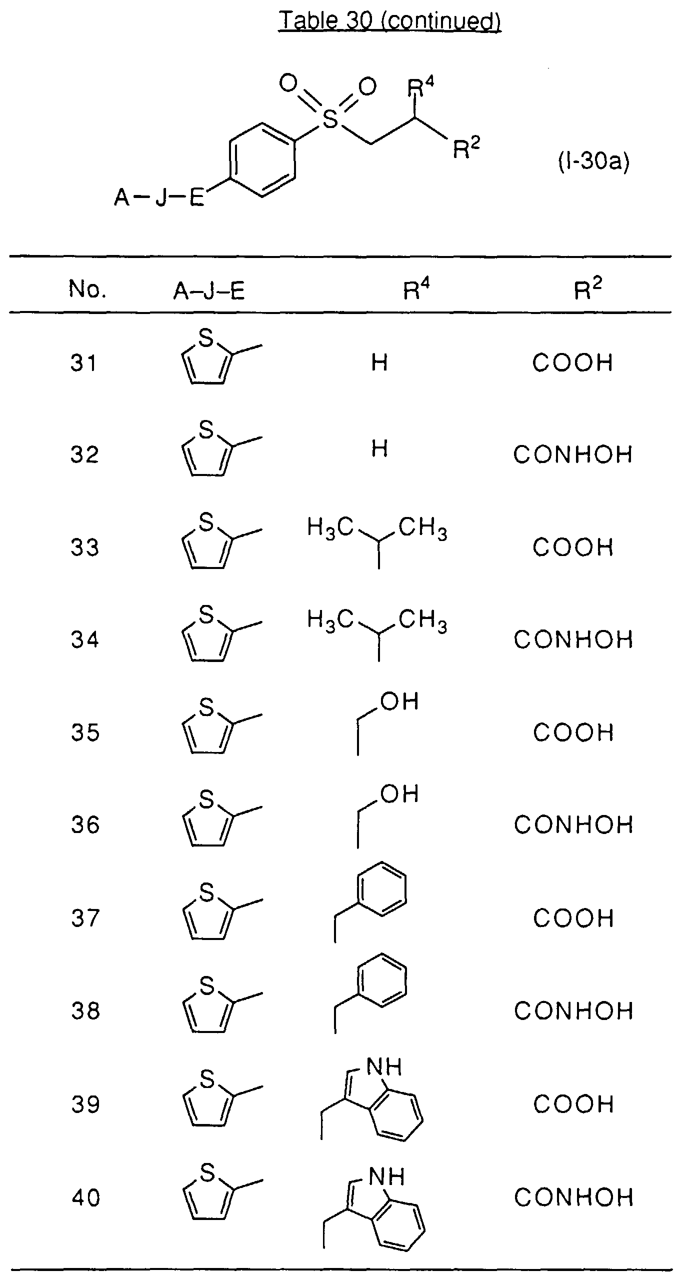

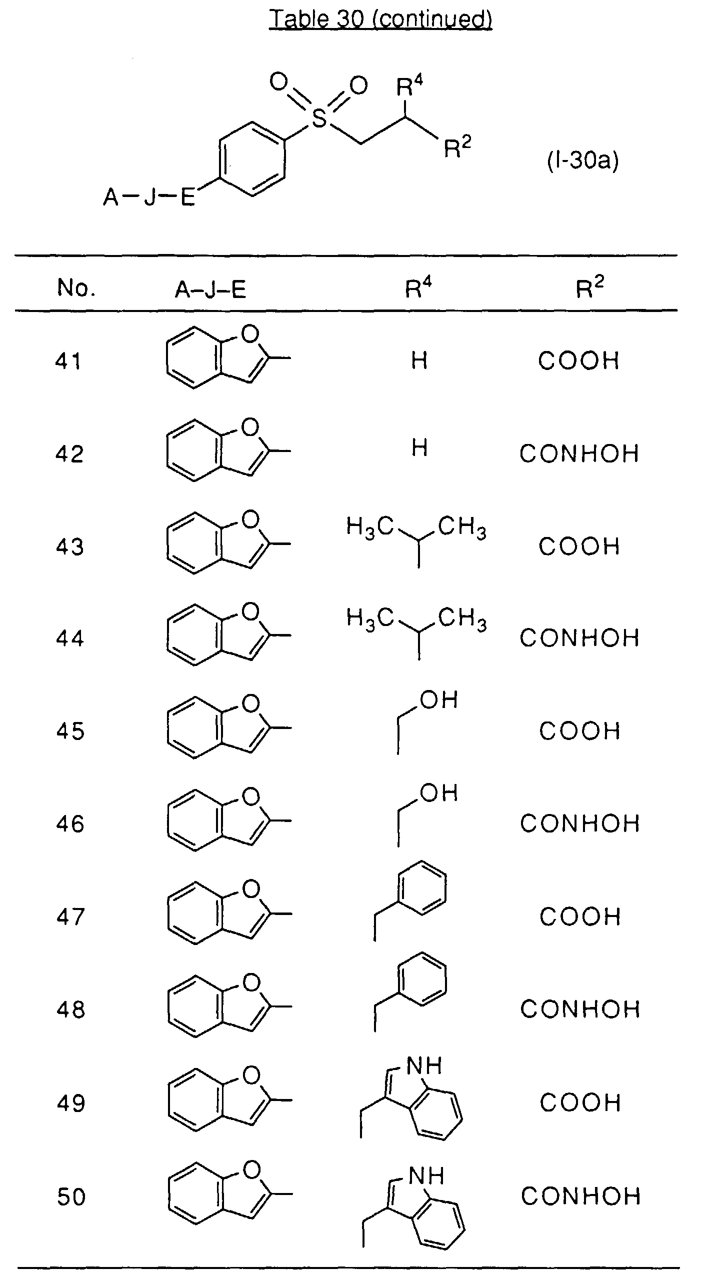

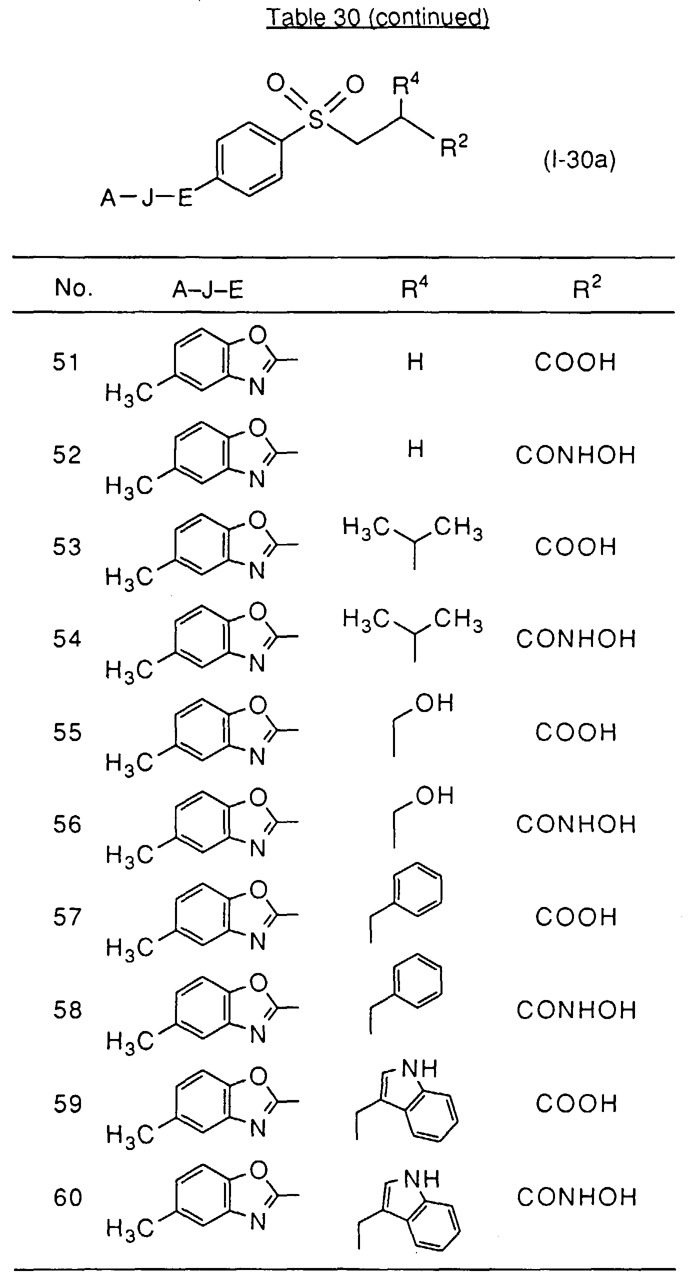

### [Processes for the Preparation of the compound of the present invention]

The compounds of the present invention of the formula (I) may be prepared by the following methods, the methods described in the Examples, or known methods.

In the compounds of the present invention of the formula (I),
(A-1) : the compound in which n is 0, R² is -COOR⁷⁻¹ (in which R⁷⁻¹ is C1-8 alkyl, phenyl, or C1-4 alkyl substituted by phenyl, -OCOR²³ or -CONR²⁴R²⁵), substituents of Ar in A, and R³ and R⁴ are not -COOH, hydroxy, amino or a group containing them, and A, J and E taken together do not represent -COOH, i.e., the compound of the formula(I-A-1) 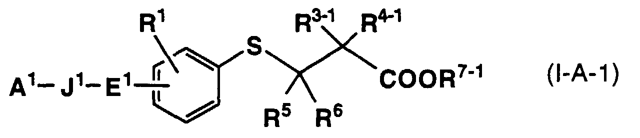 (wherein R³⁻¹ and R⁴⁻¹ have the same meanings as R³ and R⁴ respectively, with the proviso that R³⁻¹ and R⁴⁻¹ do not represent -COOH, hydroxy, amino or a group containing them; and A¹, J¹ and E¹ have the same meanings as A, J and E respectively, with the proviso that substituents of Ar in A are not -COOH, hydroxy or amino and A, J and E taken together do not represent -COOH, and the other symbols are as hereinbefore defined.) may be prepared by the following methods.
   (1) The compound in which E¹ is -CONR⁹-, may be prepared by amidation of the compound of the formula (II) 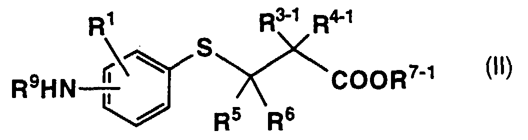 (wherein all the symbols are as hereinbefore defined.) with the compound of the formula (III) 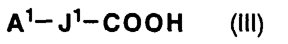 (wherein all the symbols are as hereinbefore defined.).
      The method of amidation is known. It includes the method
      (1) via an acid halide,
      (2) via a mixed acid anhydride,
      (3) using a condensing agent etc.

      These methods are explained as follows.
      (1) The method via an acid halide may be carried out, for example, by reacting a carboxylic acid with an acid halide (e. g., oxalyl chloride, thionyl chloride etc.) in an organic solvent (e.g., chloroform, methylene chloride, diethyl ether, tetrahydrofuran etc.) or without a solvent at from -20°C to the reflux temperature, and then by reacting the obtained acid halide with an amine in the presence of a tertiary amine (e. g., pyridine, triethylamine, dimethylaniline, dimethylaminopyridine etc.) in an organic solvent (e. g., chloroform, methylene chloride, diethyl ether, tetrahydrofuran etc.) at a temperature of from 0°C to 40°C.
      (2) The method via a mixed acid anhydride may be carried out, for example, by reacting a carboxylic acid with an acid halide (e. g., pivaloyl chloride, tosyl chloride, mesyl chloride, ethyl chloroformate, isobutyl chloroformate etc.) in the presence of a tertiary amine (e. g., pyridine, triethylamine, dimethylaniline, dimethylaminopyridine etc.) in an organic solvent (e. g., chloroform, methylene chloride, diethyl ether, tetrahydrofuran etc.) or without a solvent at a temperature of from -20°C to 40°C, and then by reacting the obtained mixture of acid anhydride with a corresponding amine in an organic solvent (e. g., chloroform, methylene chloride, diethyl ether, tetrahydrofuran etc.), at a temperature of from 0°C to 40°C.
      (3) The method using a condensing agent (e. g., 1,3-dicyclohexylcarbodiimide (DCC), 1-ethyl-3-[3-(dimethylamino)propyl] carbodiimide (EDC), 2-chloro-1-methylpyridinium iodide etc.) may be carried out, for example, by reacting a carboxylic acid with an amine using a condensing agent in the presence or absence of a tertiary amine (e. g., pyridine, triethylamine, dimethylaniline, dimethylaminopyridine etc.), in an organic solvent (e. g., chloroform, methylene chloride, dimethyl formamide, diethyl ether etc.) or without a solvent at a temperature of from 0°C to 40°C.

      The reactions (1), (2) and (3) hereinbefore described may be preferably carried out in an atmosphere of inert gas (e. g., argon, nitrogen etc.) under anhydrous conditions.
   (2) The compound in which E¹ is -NR⁹CO-, may be prepared by amidation of the compound of formula (IV) 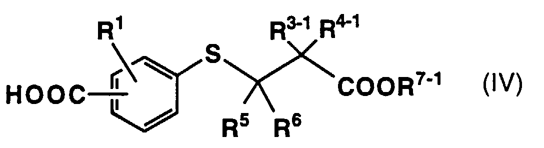 (wherein all the symbols are as hereinbefore defined.) with the compound of the formula (V) 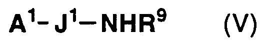 (wherein all the symbols are as hereinbefore defined.).
      The amidation may be carried out by methods hereinbefore described.
   (3) The compound in which E¹ is -OCO-, may be prepared by esterification of the compound of the formula (IV) 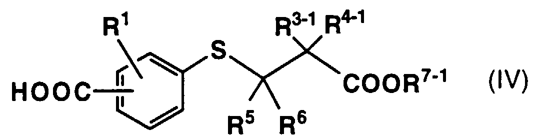 (wherein all the symbols are as hereinbefore defined.) with the compound of the formula (VI) 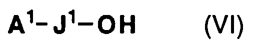 (wherein all the symbols are as hereinbefore defined.)
      The method of esterification is known. It includes the method
      (1) via an acid halide,
      (2) via a mixed acid anhydride,
      (3) using a condensing agent etc.

      These methods are explained as follows.
      (1) The method via an acid halide may be carried out, for example, by reacting a carboxylic acid with an acid halide (e. g., oxalyl chloride, thionyl chloride etc.) in an organic solvent (e.g., chloroform, methylene chloride, diethyl ether, tetrahydrofuran etc.) or without a solvent at from -20°C to the reflux temperature, and then by reacting the obtained acid halide with an alcohol in the presence of a tertiary amine (e. g., pyridine, triethylamine, dimethylaniline, dimethylaminopyridine etc.) in an organic solvent (e. g., chloroform, methylene chloride, diethyl ether, tetrahydrofuran etc.) at a temperature of from 0°C to 40°C.
      (2) The method via a mixed acid anhydride may be carried out, for example, by reacting a carboxylic acid with an acid halide (e. g., pivaloyl chloride, tosyl chloride, mesyl chloride etc.) or an acid derivative (e. g., ethyl chloroformate, isobutyl chloroformate etc.) in the presence of a tertiary amine (e. g., pyridine, triethylamine, dimethylaniline, dimethylaminopyridine etc.) in an organic solvent (e. g., chloroform, methylene chloride, diethyl ether, tetrahydrofuran etc.) or without a solvent at a temperature of from 0°C to 40°C, and then by reacting the obtained mixture of acid anhydride with an alcohol in an organic solvent (e. g., chloroform, methylene chloride, diethyl ether, tetrahydrofuran etc.), at a temperature of from 0°C to 40°C.
      (3) The method using a condensing agent (e. g., 1 ,3-dicyclohexylcarbodiimide (DCC), 1-ethyl-3-[3-(dimethylamino)propyl] carbodiimide (EDC), 2-chloro-1-methylpyridinium iodide etc.) may be carried out, for example, by reacting a carboxylic acid with an alcohol using a condensing agent in the presence or absence of a tertiary amine (e. g., pyridine, triethylamine, dimethylaniline, dimethylaminopyridine etc.), in an organic solvent (e. g., chloroform, methylene chloride, dimethyl formamide, diethyl ether etc.) or without a solvent at a temperature of from 0°C to 40°C.

      The reactions (1), (2) and (3) hereinbefore described may be preferably carried out in an atmosphere of inert gas (e. g., argon, nitrogen etc.) under anhydrous conditions.
   (4) The compound in which E¹ is -COO-, may be prepared by esterification of the compound of the formula (VII) 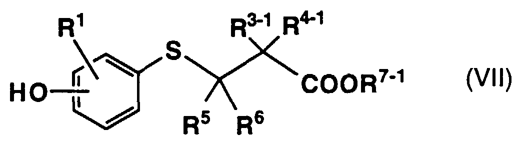 (wherein all the symbols are as hereinbefore defined.) with the compound of the formula (III) 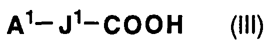 (wherein all the symbols are as hereinbefore defined.).
      The esterification may be carried out by methods hereinbefore described.
   (5) The compound in which E¹ is -CH₂-O-, may be prepared by etherification of the compound of the formula (VII) 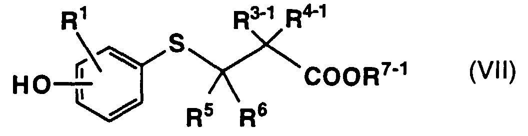 (wherein all the symbols are as hereinbefore defined.) with the compound of the formula (VIII) 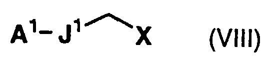 (wherein X is halogen or trifluoromethanesulfonyloxy, the other symbols are as hereinbefore defined.).
      The etherification is known and may be carried out, for example, in an organic solvent (e.g., dimethylformamide, acetone etc.), in the presence of base (e.g., potassium carbonate etc.), at a temperature of from 0°C to 40°C.
   (6) The compound in which E¹ is -CO-CH₂-, -(CH₂)₂-, -CH=CH- or -C≡C-, or in which A¹, J¹ and E¹ taken together represent heterocyclic ring (this heterocyclic ring may be substituted by 1-4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and ²⁵ are as hereinbefore defined.) or CONR²⁴R²⁵ (in which R²⁴ and R²⁵ are as hereinbefore defined.)), may be prepared by reduction of the compound of the formula (AA) 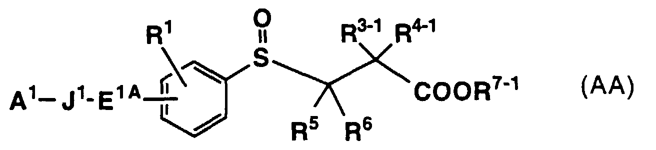 (wherein E^{1A} is -CO-CH₂-, -(CH₂)₂-, -CH=CH- or -C≡C-, or A¹, J¹ and E^{1A} taken together represent heterocyclic ring (this heterocyclic ring may be substituted by 1-4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are as hereinbefore defined.) or CONR²⁴R²⁵ (in which R²⁴ and R²⁵ are as hereinbefore defined.), and the other symbols are as hereinbefore defined.).
      The reduction is known and may be carried out, for example, using a hydrogen donor (e.g., triethylsilane, trichlorosilane etc.) in an organic solvent (e.g., trifluoroacetic acid etc.) at a temperature of from 0°C to 40°C, or this reaction may be also carried out by hydrogenolysis hereinafter described.
   (7) The compound in which A¹, J¹ and E¹ taken together represent methyl, halogen, trifluoromethyl, nitro, cyano, formyl, phenyl, hydroxy, or NR¹⁶R¹⁷, may be prepared by the reaction of the compound of the formula (IX) 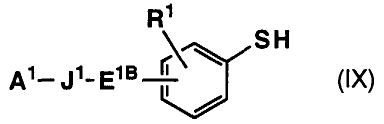 (wherein A¹, J¹ and E^{1B} taken together represent methyl, halogen, trifluoromethyl, nitro, cyano, formyl, phenyl, hydroxy, or NR¹⁶R¹⁷ (in which R¹⁶ and R¹⁷ are as hereinbefore defined.), and the other symbols are as hereinbefore defined.) with the compound of the formula (XA), formula (XB), formula (XC) or formula (XD) 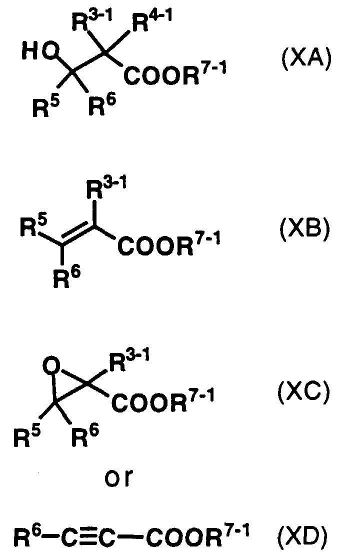 (wherein all the symbols are as hereinbefore defined.).

   This reaction is known and may be carried out, for example, in an organic solvent (e.g., diisopropylethylamine, tetrahydrofuran, ethanol, chloroform, acetonitrile etc.) in the presence of base catalyst (e.g., triethylamine, tetrabutylammonium fluoride, morpholine, n-butyllithium etc.), at a temperature of from 0°C to 40°C
(A-2) : the compound in which n is 1 or 2, R² is -COOR⁷⁻¹ (in which R⁷⁻¹ is as hereinbefore defined.), substituents of Ar in A, and R³ and R⁴ are not -COOH, hydroxy, amino or a group containing them, and A, J and E taken together do not represent -COOH, i.e., the compound of the formula (I-A-2) 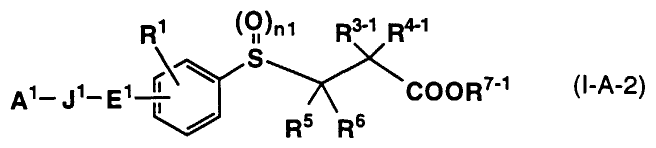 (wherein n1 is 1 or 2, and all the other symbols are as hereinbefore defined.) may be prepared by the following methods.
   (1) The compound in which n1 is 1 or 2, E¹ is -CONR⁹-, -NR⁹CO-, -OCO-,-COO-, or -OH₂-O-, and A¹, J¹ and E¹ taken together do not represent -COOH, may be prepared by oxidation of the compound of the formula (I-A-1a) 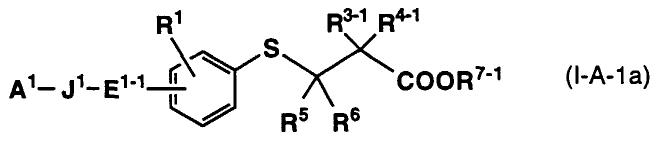 (wherein E¹⁻¹ is -CONR⁹-, -NR⁹CO-, -OCO-, -COO-, or -CH₂-O-, or A, J¹ and E¹⁻¹ taken together represent methyl, halogen, trifluoromethyl, nitro, cyano, formyl, phenyl, hydroxy, or NR¹⁶R¹⁷ (in which R¹⁶ and R¹⁷ are as hereinbefore defined.), and the other symbols are as hereinbefore defined.).
      The oxidation is known and may be carried out, for example, in an organic solvent (e.g., methylene chloride, chloroform etc.) in the presence of peracid (e.g., m-chloroperbenzoic acid etc.) at a temperature of from 0°C to 40°C ; or using oxidizing agent (e.g., periodic acid · 2 hydrate etc.) in a solvent (carbon tetrachloride, acetonitrile, water, ethanol or mixture solvent thereof etc.), in the presence or absence of catalyst (e.g., ruthenium (III) chloride hydrate etc.) at a temperature of from 0°C to reflux temperature.
   (2) The compound in which n1 is 1 or 2, E¹ is -C≡O-, or A, J and E taken together represents heterocyclic ring (this heterocyclic ring may be substituted by 1-4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are as hereinbefore defined.) and CONR²⁴R²⁵ (in which R²⁴ and R²⁵ are as hereinbefore defined.)), may be prepared by reaction of the compound of the formula (XI) 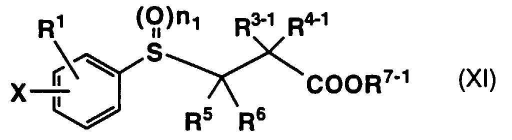 (wherein all the symbols are as hereinbefore defined.) with the compound of the formula (XII) 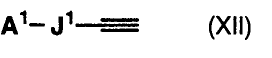 (wherein all the symbols are as hereinbefore defined.) or the corresponding heterocyclic ring compound.
      This reaction is known and may be carried out, for example, using a catalyst (e.g., tetrakis(triphenylphosphine)palladium etc.) in an organic solvent (e.g., acetonitrile, tetrahydrofuran etc.) in the presence of base (e.g., triethylamine etc.) and copper(I) iodide, at a temperature of from 0°C to reflux temperature.
   (3) The compound in which n1 is 2, E¹ is -CO-CH₂-, may be prepared by reaction of the compound of the formula (XIII) 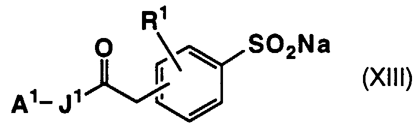 (wherein all the symbols are as hereinbefore defined.) with the compound of the formula (XA), formula (XB), formula (XC) or formula (XD) 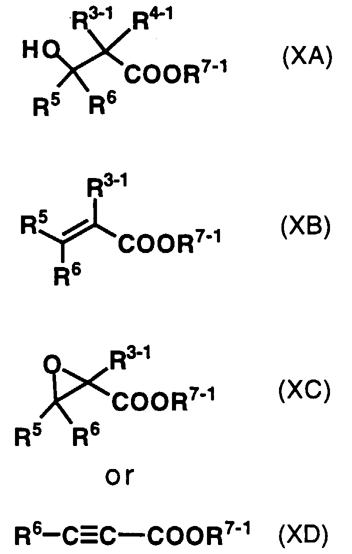 (wherein all the symbols are as hereinbefore defined.) ; or may be prepared by treating the compound prepared in (2) above with trifluoroacetic acid.
      This reaction is known and may be carried out, for example, by refluxing with heating, in an organic solvent (e.g., ethanol, mixture solvent of water and benzene etc.), in the presence of acetic acid or poly ethylene glycol.
   (4) The compound in which n1 is 2, E¹ is -CH=CH-, may be prepared by dehydration of the compound of the formula (XIV) 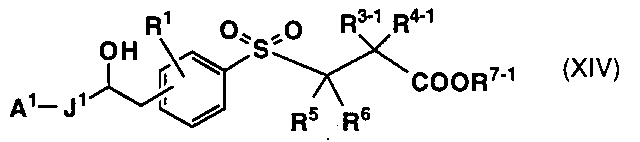 (wherein all the symbols are as hereinbefore defined.).
      The dehydration is known and may be carried out, for example, by refluxing with heating, in an organic solvent (e.g., toluene, benzene etc.), in the presence of catalytic quantity of p-toluenesulfonic acid.
   (5) The compound in which n1 is 2, and E¹ is -(CH₂)₂-, may be prepared by reduction of the compound of the formula (XIV) 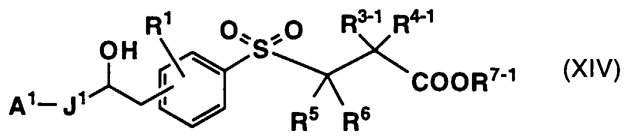 (wherein all the symbols are as hereinbefore defined.).
      The reduction may be carried out by method as hereinbefore described, or this reaction may be carried out by hydrogenolysis as hereinafter described.
   (6) The compound in which n1 is 1, and E¹ is -CO-CH₂-, -(CH₂)₂-, or -CH=CH-, may be prepared by reduction of the compound (n1 is 2, and E¹ is -CO-CH₂-, -(CH₂)₂-, or -CH=CH-.) prepared in (3) ∼ (5) above.
      The reduction may be carried out by method as hereinbefore described, or this reaction may be carried out by hydrogenolysis as hereinafter described.
      The compound in which n1 is 1, and E¹ is -CO-CH₂-, may be prepared by treating the compound (wherein n is 1, E¹ is -C≡C-.) prepared in (2) above with trifluoroacetic acid.
   (7) In the compounds hereinbefore prepared in (1) ∼ (6), the compound in which R³⁻¹ is hydrogen, and R⁴⁻¹ is 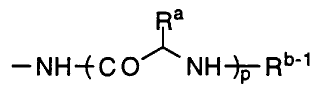 (in which R^{b-1} is -COOR²² or -COR²² (in which R²² is as hereinbefore defined.), and the other symbols are as hereinbefore defined.), may be prepared by amidation of the compound of the formula (BB) 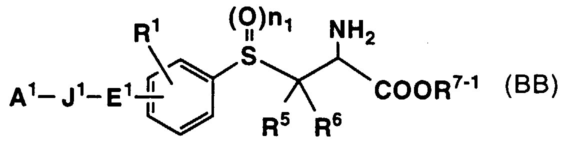 (wherein all the symbols are as hereinbefore defined.) with the corresponding carboxylic acid of the formula (CC) 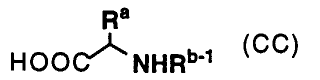 (wherein all the symbols are as hereinbefore defined.).

   If necessary, it may be prepared by amidation of the corresponding carboxylic acid of the formula (CC) with the compound obtained by deprotection of protecting group (R^{b-1}) of amino under acidic condition of compound prepared in above reaction.
   The amidation may be carried out by methods as hereinbefore described. The deprotection of protecting group of amino under acidic condition may be carried out by methods as hereinafter described.
(B) The compounds in which at least one of -COOR⁷ in R², and R³, R⁴, and substituents of Ar in A, and a group represented by A, J and E taken together, represents -COOH or a group containing -COOH ; or at least one of R³, R⁴, and substituents of Ar in A, represents hydroxy, amino or a group containing them, i.e., the compounds of the formula (I-B) 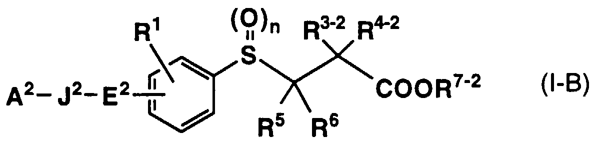 (wherein A², J², E², R³⁻², R⁴⁻², R⁷⁻² have the same meanings as A, J, ,E, R³, R⁴, R⁷ respectively, with proviso that at least one of -COOR⁷, and R³, R⁴, and substituents of Ar in A, and a group represented by A, J and E taken together, represents -COOH or a group containing them ; or at least one of R³, R⁴, and substituents of Ar in A, represents hydroxy, amino or a group containing them; and the other symbols are as hereinbefore defined.) may be prepared by deprotection under alkaline or acidic conditions, or hydrogenolysis of the compound of the formula (I-A-1) and the formula (I-A-2).
   Deprotection under alkaline conditions is known and may be carried out, for example, in an organic solvent (e.g., methanol, tetrahydrofuran, dioxane etc.), using an alkali metal hydroxide (e.g., sodium hydroxide, potassium hydroxide etc.), an alkali earth metal hydroxide (e.g., calcium hydroxide etc.) or a carbonate (e.g., sodium carbonate, potassium carbonate etc.), an aqueous solution thereof or a mixture thereof, at a temperature of from 0°C to 40°C.
   Deprotection under acidic conditions is known and may be carried out, for example, in a solvent (e.g., dichloro methane, dioxane, ethyl acetate, acetic acid ,water or a mixture solvent thereof etc.), using an organic acid (e.g., trifluoroacetic acid etc.) or an inorganic acid (e.g., hydrogen chloride, hydrogen bromide etc.) at a temperature of from 0°C to 120°C.
   Hydrogenolysis is known and may be carried out, for example, in a solvent (e.g., ether (e.g., tetrahydrofuran, dioxane, diemethoxyethane, diethyl ether etc.), alcohol (e.g., methanol, ethanol etc.), a benzene-type solvent (e.g., benzene, toluene etc.), ketone (etc. acetone, methylethylketone etc.), nitorile (acetonitrile etc.), amide (dimethylformamide etc.), water, ethyl acetate, acetic acid or a mixture of two or more thereof), in the presence of a catalyst (e.g., palladium on carbon, palladium black, palladium hydroxide, platinum dioxide, Raney-nickel etc.), in the presence or absence of an inorganic acid (e.g., hydrochloric acid, sulfuric acid, hypochlorous acid, boric acid, tetrafluoroboric acid etc.) or an organic acid (e.g., acetic acid, p-toluenesulfonic acid, oxalic acid, trifluoroacetic acid, formic acid etc.), at ordinary or elevated pressure of hydrogen gas or ammonium formate at a temperature of from 0°C to 200°C.
(C) The compounds, in which R² is -CONHOR⁸⁻¹ (in which R⁸⁻¹ is C1-8 alkyl, phenyl, or C1-4 alkyl substituted by phenyl.); substituents of Ar in A, R³ and R⁴ do not represent -COOH, hydroxy, amino or a group containing them; and A, J and E taken together do not represent -COOH, i.e., the compounds of the formula (I-C) 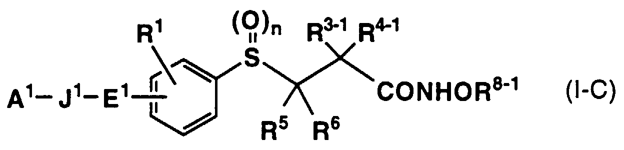 (wherein all the symbols are as hereinbefore defined.) may be prepared by theLE>LE>! following methods.
   (1) The compound in which n is 0, may be prepared by condensation of the compound of the formula (XV) 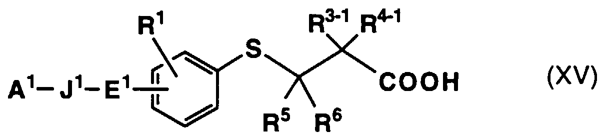 (wherein all the symbols are as hereinbefore defined.) with the compound of formula (XVI) 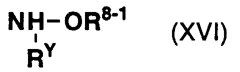 (wherein R^{Y} is hydrogen or protection group of amino, and the other symbols are as hereinbefore defined.).
      The condensation is known and may be carried out, for example, in an organic solvent (e.g., chloroform, methylene chloride, dimethylformamide, tetrahydrofuran etc.) or without a solvent, optionally using a tertiary amine (e.g., pyridine, triethylamine, dimethylaniline, dimethylaminopyridine etc.), using a condesing agent (e.g., 1-ethyl-3-[3-(dimethylamino)propyl]carbodiimide (EDC) etc.), at a temperature of from 0°C to 40°C.
   (2) The compound in which n is 1 or 2, may be prepared by oxidation of the compound of the formula (I-C-1) prepared in (1) above 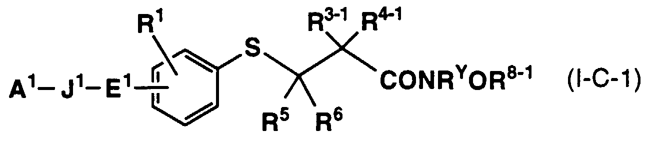 (wherein all the symbols are as hereinbefore defined.), if necessary, followed by deprotection of R^{Y}.

   The oxidation may be carried out by methods as hereinbefore described.
   The deprotection of R^{Y} is known and may be carried out, for example, in a solvent (e.g., methylene chloride, dioxane, ethyl acetate, acetic acid ,water or a mixture solvent thereof etc.), using an organic acid (e.g., trifluoroacetic acid etc.) or an inorganic acid (e.g., hydrogen chloride, hydrogen bromide etc.) at a temperature of from 0°C to 120°C ; or
   in a solvent (e.g., ether (e.g., tetrahydrofuran, dioxane, diemethoxyethane, diethyl ether etc.), alcohol (e.g., methanol, ethanol etc.), a benzene-type solvent (e.g., benzene, toluene etc.), ketone (etc. acetone, methylethylketone etc.), nitorile (acetonitrile etc.), amide (dimethylformamide etc.), water, ethyl acetate, acetic acid or a mixture of two or more thereof), in the presence of a catalyst (e.g., palladium on carbon, palladium black, palladium hydroxide, platinum dioxide, Raney-nickel etc.), in the presence or absence of an inorganic acid (e.g., hydrochloric acid, sulfuric acid, hypochlorous acid, boric acid, tetrafluoroboric acid etc.) or an organic acid (e.g., acetic acid, p-toluenesulfonic acid, oxalic acid, trifluoroacetic acid, formic acid etc.), at ordinary or elevated pressure of hydrogen gas or ammonium formate at a temperature of from 0°C to 200°C. When an acid is used, a salt thereof may be used.
(D) The compounds in which R⁸ in R² is hydrogen, in which at least one of R³, R⁴, and substituents of Ar in A, or a group represented by A, J and E taken together, represents -COOH or a group containing -COOH, and in which at least one of R³, R⁴, and substituents of Ar in A, represents hydroxy, amino or a group containing them, i.e., the compounds of the formula (I-D) 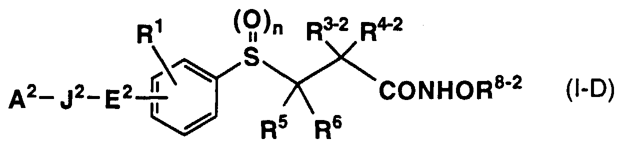 (wherein A², J², E², R³⁻² R⁴⁻² F⁸⁻² have the same meanings as A, J, E, R³, R⁴, R⁸ respectively, with the proviso that at least one of R³, R⁴, substituents of Ar in A, and a group represented by A, J and E taken together, represents -COOH or a group containing -COOH; at least one of R³, R⁴, and substituents of Ar in A, represents hydroxy, amino or a group containing them ; or R⁸ is hydrogen, and the other symbols are as hereinbefore defined.) may be prepared by deprotection under alkali or acidic conditions, or hydrogenolysis of the compound of the formula (I-C).

The deprotection under alkali or acidic conditions, or hydrogenolysis, may be carried out by the methods as hereinbefore described.

The compounds of formulae (II), (IV), (VII), (XI) or (XIV) may be prepared by known methods, methods described in the following schemes 1, 2, 3, 4 and 5 or methods described in the Examples.

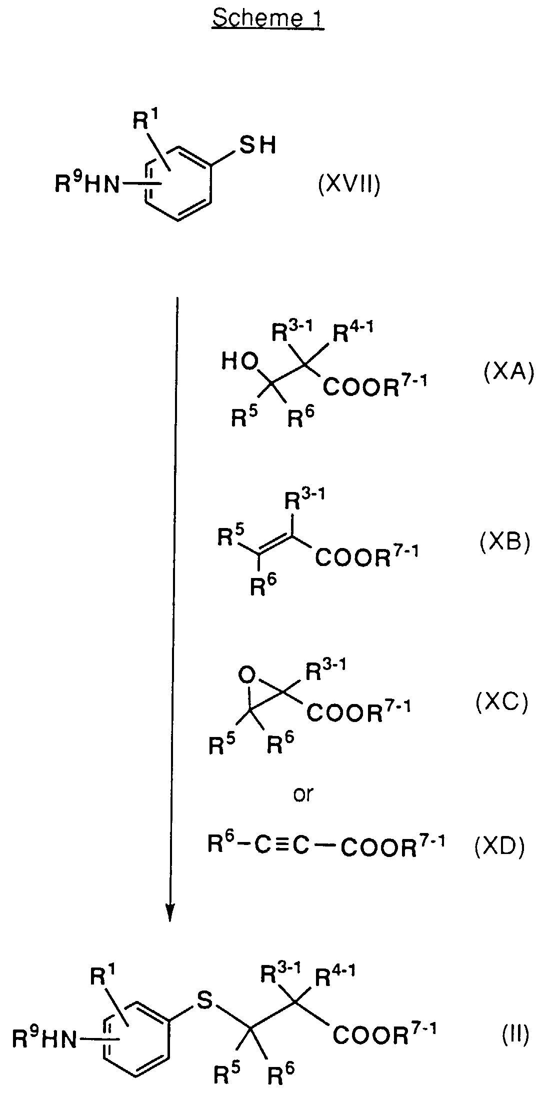

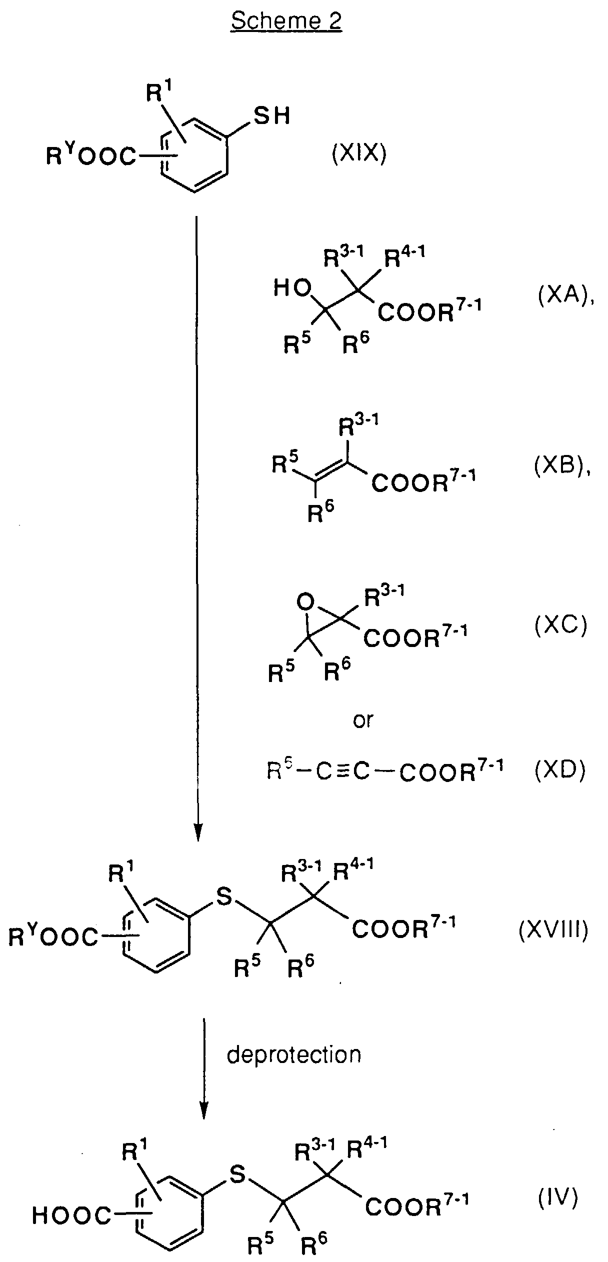

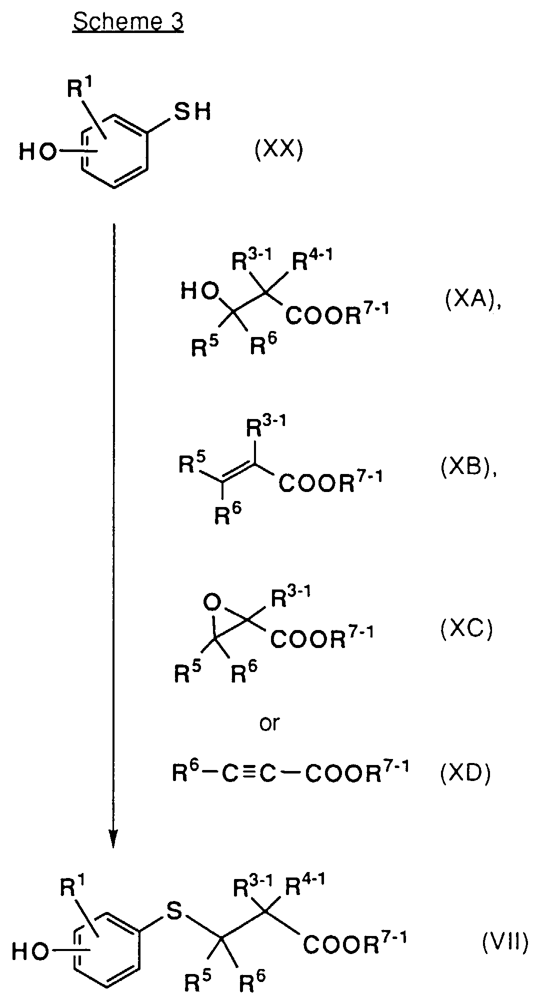

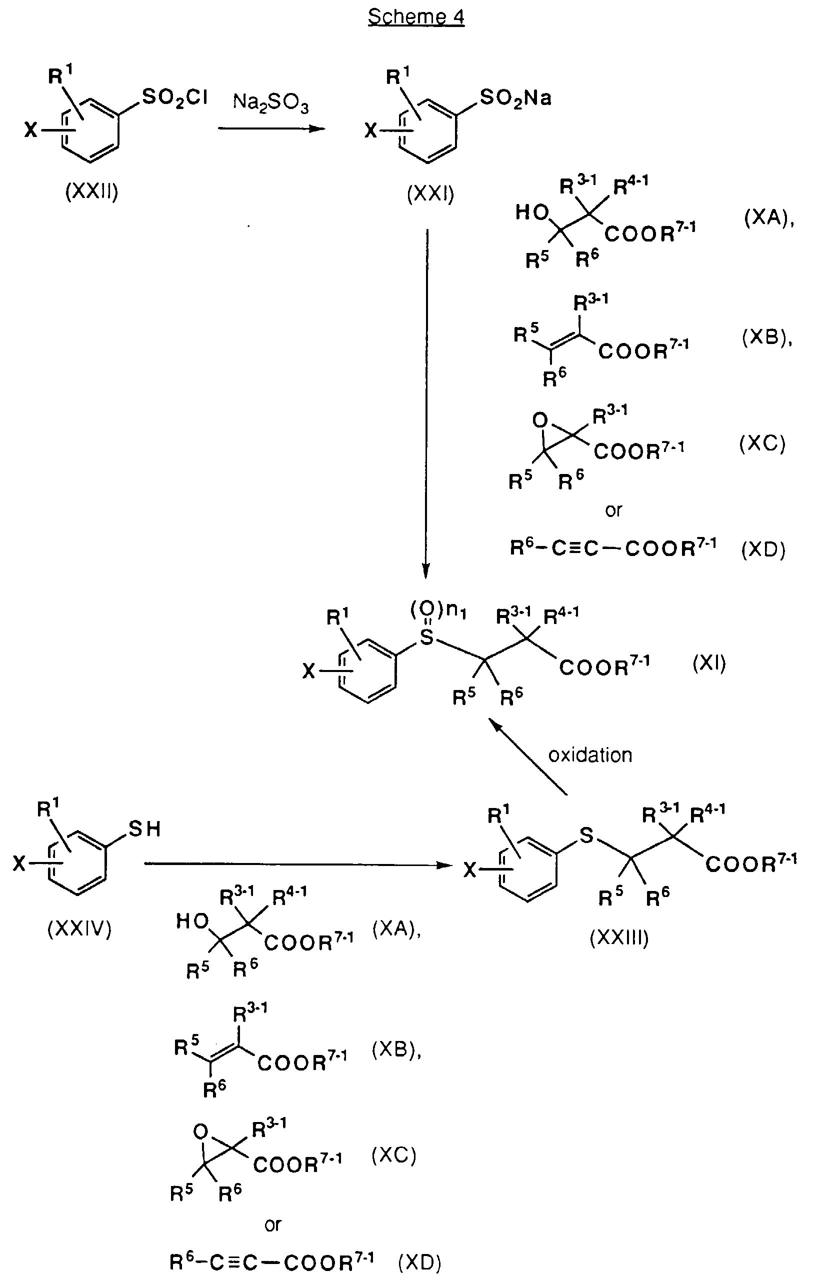

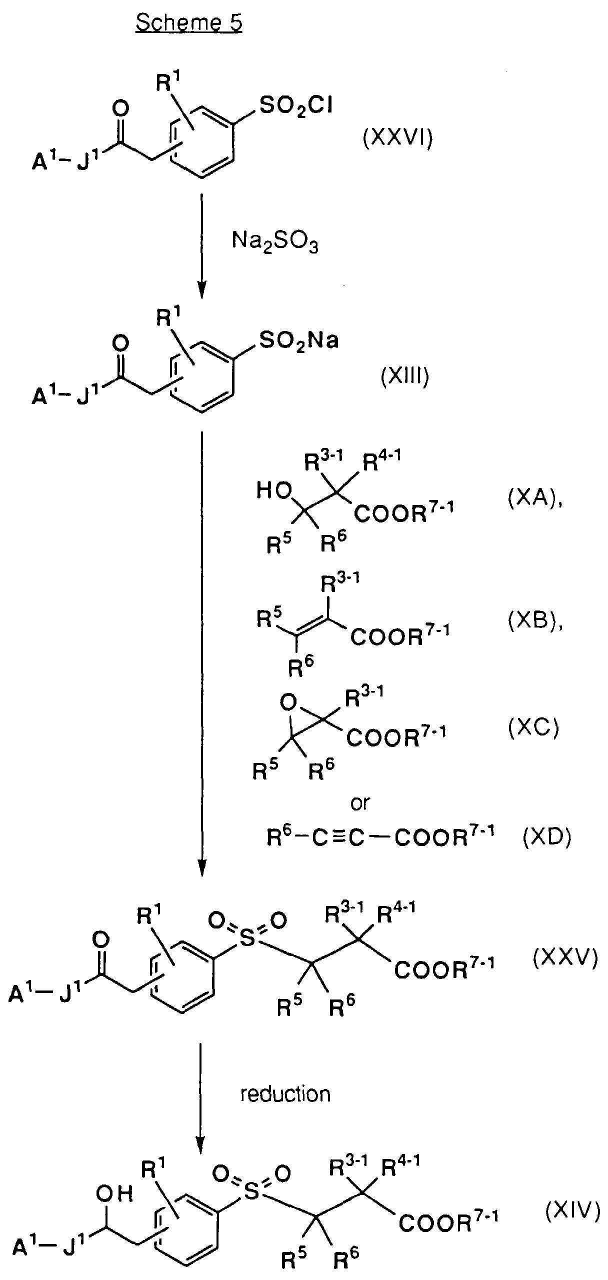

In the schemes, R^{Z} is protection group of carboxylic acid, the other symbols are the same meanings as hereinbefore described.

Each reaction in the above schemes, may be carried out by known methods. In the above schemes, the compounds used as starting materials are known per se, or may be easily prepared by known methods. The other starting materials and reagents in the present invention are known per se or may be prepared by known methods.

In each reaction in the present invention, products may be purified by conventional techniques. For example, purification may be carried out by distillation at atmospheric or reduced pressure, by high performance liquid chromatography, by thin layer chromatography or by column chromatography using silica gel or magnesium silicate, by washing or by recrystallization. Purification may be carried out after each reaction, or after a series of reactions.

### Possibility of Industrial use

### [Pharmacological Activity]

The potency of inhibitory activity of the compounds of the formula (I) against matrix metalloproteinases is confirmed by the following experiment. For example, with respect to inhibitory activity against gelatinase A, the following results are obtained .

### (1) Inhibitory activity against gelatinase A.

### Experimental method

The progelatinase A (7 µl; in assay buffer (90 µl) was purified from human normal skin dermal fibroblasts (HNDF). It was activated by the addition of 10 mM APMA (10 µl) for 1 hour at 37°C. The solution of activated gelatinase A (1 U/tube, 98 µl) was mixed with the solution of various concentrations of the test compound or the solution in which the test compound is not added, (2 µl), and 0.05 % FITC-gelatin (100 µl) and incubated for 2 hours at 37°C. The enzymatic reaction was terminated by the addition of 2 M Tris-HCl (pH 9.5) containing 94.7% ethanol (750 µl). The mixture was stirred and then allowed to stand for 30 minutes at 0°C. The mixture was centrifuged for 30 minutes at 900 x g. Inhibitory activity against gelatinase was determined by measuring the fluorescent intensity in supernatant (Ex = 495 nm, and Em = 520 nm). The results are shown in Table 31.

**Table 31**

| Example No. | IC50 (µM) |
|---|---|
| 8(2) | 0.54 |
| 8(7) | 0.40 |
| 19(1) | 0.011 |
| 28 | 0.013 |
| 28(1) | 0.0014 |
| 28(3) | 0.0029 |

In the above experimental method, APMA is p-aminophenylmercuric acetate and FITC is fluorescein isothiocyanate.

### [Toxicity]

On the other hand, the toxicity of the compounds of the present invention is very low and therefore it may be confirmed that the compounds are safe for pharmaceutical use.

### [Application for Pharmaceuticals]

Inhibition of matrix metalloproteinases is useful for prevention and / or treatment of rheumatoid diseases, arthrosteitis, unusual bone resorption, osteoporosis, periodontitis, interstitial nephritis, arteriosclerosis, pulmonary emphysema, cirrhosis, cornea injury, metastasis of, invasion of or growth of tumor cells, autoimmune diseases (e.g. Crohn's disease, Sjogren's syndrome), diseases caused by vascular emigration or infiltration of leukocytes, arterialization etc.

For the purpose above described, the compounds of formula (I) of the present invention, non-toxic salts, acid addition salts or hydrates thereof may normally be administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered in the form of, for example, solid compositions, liquid compositions or other compositions for oral administration, injections, liniments or suppositories for parenteral administration.

Solid compositions for oral administration include compressed tablets, pills, capsules, dispersible powders, and granules.

Capsules include hard capsules and soft capsules.

In such solid compositions, one or more of the active compound(s) may be admixed with at least one inert diluent (such as lactose, mannitol, glucose, hydroxypropyl cellulose, microcrystalline cellulose, starch, polyvinylpyrrolidone or magnesium metasilicate aluminate). The compositions may also comprise, as is normal practice, additional substances other than inert diluents: e.g., lubricating agents (such as magnesium stearate), disintegrating agents (such as cellulose calcium glycolate), stabilizing agents (such as lactose), and agents to assist dissolution (such as glutamic acid or aspartic acid). The tablets or pills may, if desired, be coated with a film of gastric or enteric material (such as sugar, gelatin, hydroxypropyl cellulose or hydroxypropylmethyl cellulose phthalate), or be coated with two or more films. And further, coating may include containment within capsules of absorbable materials such as gelatin.

Liquid compositions for oral administration include pharmaceutically acceptable emulsions, solutions, syrups and elixirs. In such liquid compositions, one or more of the active compound(s) may be contained in an inert diluent(s) commonly used in the art (e.g., purified water, ethanol). Besides inert diluents, such compositions may also comprise adjuvants (such as wetting agents or suspending agents), sweetening agents, flavouring agents, perfuming agents, and preserving agents.

Other compositions for oral administration include spray compositions which may be prepared by known methods and which comprise one or more of the active compound(s). Spray compositions may comprise additional substances other than inert diluents: e.g., stabilizing agents (such as sodium sulfate), isotonic buffer (such as sodium chloride, sodium citrate or citric acid). For preparation of such spray compositions, for example, the method described in the United States Patent No. 2,868,691 or 3,095,355 may be used.

Injections for parenteral administration include sterile aqueous or non-aqueous solutions, suspensions and emulsions. Aqueous solutions and suspensions may include distilled water for injection or physiological salt solution. Non-aqueous solutions and suspensions may include propylene glycol, polyethylene glycol, vegetable oil such as olive oil, alcohol such as ethanol or POLYSORBATE80 (registered trade mark). Injections may comprise additional ingredients other than inert diluents: e.g. preserving agents, wetting agents, emulsifying agents, dispersing agents, stabilizing agents (such as lactose), assisting agents such as agents to assist dissolution (e.g. glutamic acid or aspartic acid). They may be sterilized for example, by filtration through a bacteria-retaining filter, by incorporation of sterilizing agents in the compositions or by irradiation. They may also be manufactured in the form of sterile solid compositions which may be dissolved in sterile water or some other sterile diluent(s) for injection immediately before use.

Other compositions for parenteral administration include liquids for external use, and endermic liniments, ointment, suppositories for rectal administration and pessaries for vaginal administration which comprise one or more of the active compound(s) and may be prepared by methods known per se.

### Reference example and Example

The following reference examples and examples illustrate the present invention, but do not limit the present invention.

The solvents in the parentheses show the developing or eluting solvents and the ratios of the solvents used are by volume in chromatographic separations or TLC.

The solvents in the parentheses in NMR show the solvents used in measurement.

### Example 1

### 3-(4-aminophenylthio)propionic acid t-butyl ester

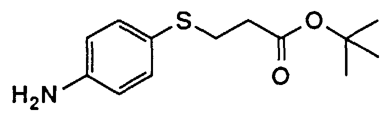

To a solution of 4-aminothiophenol (2.36 g) in tetrahydrofuran (THF; 20 ml), 2-propenoic acid t-butyl ester (3.51 ml) and 1.0M tetrabutylammonium fluoride in THF solution (340 µl) were added. The mixture solution was stirred for 30 minutes at room temperature to give the title compound. The compound was used for next reaction as such.

### Example 1(1)∼1(4)

The following compounds were obtained by the same procedure as Example 1, using the corresponding thiophenol derivatives and the corresponding carboxylic acid derivatives.

### Example 1(1)

### 3-(4-hydroxyphenylthio)propionic acid t-butyl ester

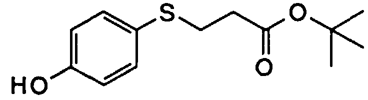

The prepared title compound was used for next reaction without purification.

### Example 1(2)

### 2-methyl-3-(4-hydroxyphenylthio)propionic acid t-butyl ester

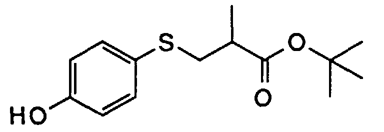

TLC : Rf 0.45 (hexane : ethyl acetate = 7 : 3),
NMR(CDCl₃ + CCl₄ (5 drops)) : δ 7.31 (2H, d, J=8.8Hz), 6.77 (2H, d, J=8.8Hz), 5.82 (1 H, s), 3.08 (1 H, dd, J=13.2Hz, 7.8Hz), 2.77 (1H, dd, J=13.2Hz, 6.6Hz), 2.53 (1H, m), 1.47 (9H, s), 1.20 (3H, d, J=7.2Hz).

### Example 1(3)

### 2-benzyl-3-(4-bromophenylthio)propionic acid t-butyl ester

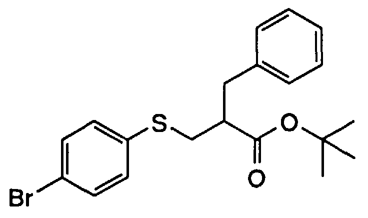

TLC : Rf 0.58 (hexane : ethyl acetate = 4 : 1),
NMR (CDCl₃) : δ 7.36 (2H, d, J=8.8Hz), 7.30-7.10 (7H, m), 3.11 (1H, dd, J=14.0Hz, 8.1Hz), 3.00-2.70 (4H, m), 1.36 (9H, s).

### Example 1(4)

### 3-(4-methoxyphenylthio)propionic acid t-butyl ester

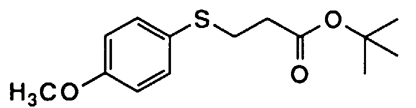

The prepared title compound was used for next reaction without purification.

### Example 2

### 3-[4-(benzoylamino)phenylthio]propionic acid t-butyl ester

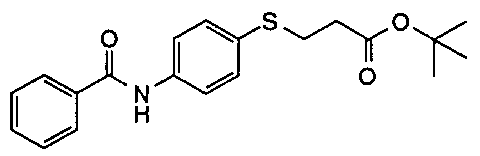

Triethylamine (3.3 ml) and benzoyl chloride (2.0 ml) were added to the compound prepared in Example 1 and the reaction solution was stirred for 30 minutes at room temperature. 1N hydrochloric acid was added to the reaction solution. The reaction solution was extracted with ethyl acetate. The extract was washed with a saturated aqueous solution of sodium bicarbonate, water, a saturated aqueous solution of sodium chloride, dried over anhydrous magnesium sulfate and concentrated. The residue was washed with ether to give the title compound (5.17 g).
TLC : Rf 0.51 (hexane : ethyl acetate = 7 : 3),
NMR (CDCl₃) : δ 7.92 (1H, s), 7.89-7.82 (2H, m), 7.60 (2H, d, J=8.8Hz), 7.56-7.42 (3H, m), 7.38 (2H, d, J=8.8Hz), 3.08 (2H, t, J=7.4Hz), 2.51 (2H, t, J=7.4Hz), 1.45 (9H, s).

### Example 3

### 3-(4-benzyloxyphenylthio)propionic acid t-butyl ester

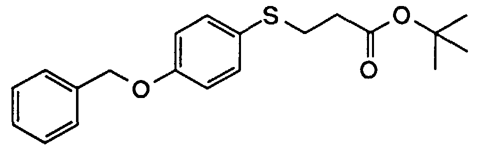

A mixture solution of the compound prepared in Example 1(1) (3.29 g), benzyl bromide (2.3 ml), potassium carbonate (3.58 g) and dimethylformamide (15 ml) was stirred overnight at room temperature. The reaction solution was extracted with ethyl acetate. The extract was washed with water and a saturated aqueous solution of sodium chloride, dried over anhydrous magnesium sulfate and concentrated to give the title compound (2.20 g) having the following physical data.
TLC : Rf 0.71 (hexane : ethyl acetate = 4 : 1),
NMR (CDCl₃) : δ 7.46-7.30 (7H, m), 6.90 (2H, d, J=9.0Hz), 5.04 (2H, s), 3.00 (2H, t, J=7.6Hz), 2.46 (2H, t, J=7.6Hz), 1.44 (9H, s).

### Example 4

### 3-[4-(benzoylamino)phenylsulfinyl]propionic acid t-butyl ester

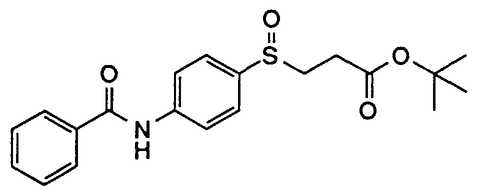

To a solution of the compound prepared in Example 2 (714 mg) in chloroform (7 ml), m-chloroperbenzoic acid (493 mg) was added and the reaction solution was stirred for 30 minutes at room temperature. The reaction solution was washed with a saturated aqueous solution of sodium bicarbonate and a saturated aqueous solution of sodium chloride, dried over anhydrous magnesium sulfate and concentrated. Ether was added to the residue and the crystal was filtered off and then the title compound (616 mg) having the following physical data was obtained.
TLC : Rf 0.37 (chloroform : methanol = 19 : 1),
NMR (ODCl₃) : δ 8.17 (1H, s), 7.94-7.86 (2H, m), 7.85 (2H, d, J=8.8Hz), 7.61 (2H, d, J=8.8Hz), 7.57-7.46 (3H, m), 3.15 (1H, ddd, J=6.6Hz, 8.8Hz, 13.2Hz), 2.93 (1H, ddd, J=5.8Hz, 8.2Hz, 13.2Hz), 2.73 (1H, ddd, J=6.6Hz, 8.2Hz, 14.4Hz), 2.43 (1H, ddd, J=5.8Hz, 8.8Hz, 14.4Hz), 1.43 (9H, s).

### Example 5

### 3-[4-(benzoylamino)phenylsulfonyl]propionic acid t-butyl ester

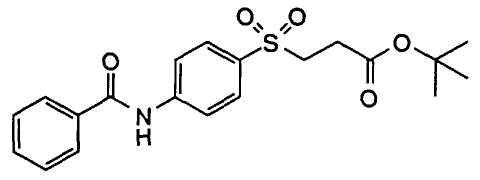

Under an atmosphere of argon, to a mixture solution of the compound prepared in Example 2 (714 mg) in carbon tetrachloride (2 ml), acetonitrile (2 ml) and water (4 ml), periodic acid · 2 hydrates (958 mg) and ruthenium (III) chloride · hydrate (8 mg) were added and reaction mixture was stirred for 2 hours. Water was added to the reaction mixture and it was extracted with ethyl acetate. The extract was washed with a saturated aqueous solution of sodium thiosulfate and a saturated aqueous solution of sodium chloride, dried over anhydrous magnesium sulfate and concentrated. The residue was purified by column chromatography (hexane : ethyl acetate = 3 : 2) to give the title compound (730 mg) having the following physical data.
TLC : Rf 0.20 (hexane : ethyl acetate = 7 : 3),
NMR (CDCl₃) : δ 8.11 (1 H, s), 7.94-7.86 (6H, m), 7.65-7.47 (3H, m), 3.37 (2H, t, J=7.4 Hz), 2.65 (2H, t, J=7.4 Hz), 1.41(9H,s).

### Example 5(1)∼5(2)

The following compounds were obtained by the same procedure as Example 5, using compounds prepared in Example 3 and Example 1(3)

### Example 5(1)

### 3-[4-(benzyloxy)phenylsulfonyl]propionic acid t-butyl ester

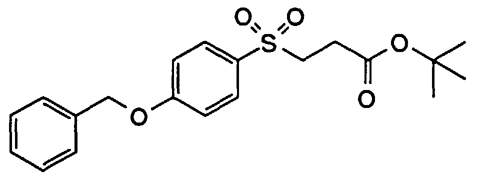

TLC : Rf 0.33 (hexane : ethyl acetate = 7 : 3),
NMR (CDCl₃) : δ 7.83 (2H, d, J=9.2Hz), 7.42-7.32 (5H, m), 7.09 (2H, d, J=9.2Hz), 5.14 (2H, s), 3.34 (2H, t, J=7.4Hz), 2.64 (2H, t, J=7.4Hz).

### Example 5(2)

### 2-benzyl-3-(4-bromophenylsulfonyl)propionic acid t-butyl ester

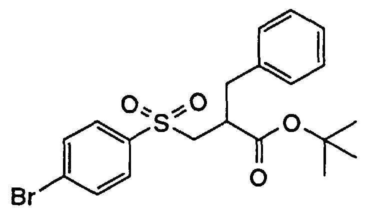

TLC : Rf 0.30 (hexane : ethyl acetate = 4 : 1),
NMR (CDCl₃) : δ 7.69 (2H, d, J=8.8Hz), 7.67 (2H, d, J=8.8Hz), 7.30-7.20 (3H, m), 7.07 (2H, s), 3.63 (1H, m), 3.10-2.90 (3H, m), 2.90 (1H, m), 1.33 (9H, s).

### Reference example 1

### 2-methyl-3-[4-(trifluoromethanesulfonyloxy)phenylthio]propionic acid t-butyl ester

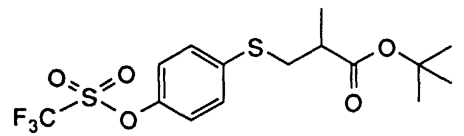

Under an atmosphere of argon and at -78°C, to a solution of the compound prepared in Example 1(2) (1 .50 g) in dichloromethane (10 ml), pyridine (1.13 ml) and anhydrous trifluoromethanesulfonic acid (1.13 ml) were added dropwise. The mixture solution was stirred for 2 hours at room temperature. A saturated aqueous solution of sodium bicarbonate (20 ml) was added to the reaction mixture and it was vigorously stirred for 1 hour at room temperature. The reaction mixture was extracted with ethyl acetate. The extract was washed with 1 N hydrochloric acid, a saturated aqueous solution of sodium bicarbonate, a saturated aqueous solution of sodium chloride, dried over anhydrous magnesium sulfate and concentrated. The residue was purified by column chromatography (hexane : ethyl acetate = 19 : 1) to give the title compound (2.05 g) having the following physical data.
TLC : Rf 0.29 (hexane : ethyl acetate = 19 : 1),
NMR (CDCl₃ + CCl₄ (5 drops)) : δ 7.40 (2H, d, J=9.0Hz), 7.19 (2H, d, J=9.0Hz), 3.24 (1H, dd, J=13.2Hz, 7.3Hz), 2.91 (1H, dd, J=13.2Hz, 6.8Hz), 2.59 (1H, m), 1.45 (9H, s), 1.24 (3H, d, J=6.8Hz).

### Reference example 2

### 2-methyl-3-(4-trifluoromethanesulfonyloxyphenylsulfonyl)propionic acid t-butyl ester

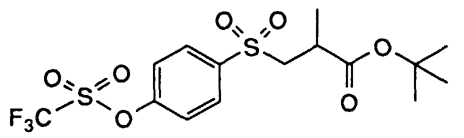

The title compound (748 mg) having the following physical data was obtained by the same procedure as Example 5, using the compound prepared in Reference example 1 (1.00 g).
mp : 50°C,
TLC : Rf 0.57 (hexane : ethyl acetate = 9 :1),
NMR (ODCl₃ + CCl₄ (5 drops)) : δ 8.05 (2H, d, J=8.6Hz), 7.49 (2H, d, J=8.6Hz), 3.71 (1H, dd, J=14.2Hz, 7.6Hz), 3.06 (1H, dd, J=14.2Hz, 5.1 Hz), 2.96 (1H, m), 1.42 (9H, s), 1.31 (3H, d, J=7.2Hz).

### Example 6

### 3-(4-bromophenylsulfonyl)propionic acid t-butyl ester

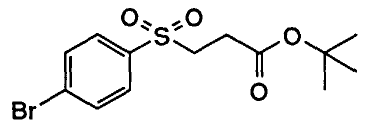

The mixture of 4-bromophenylsulfinic acid sodium salt (729 mg), 2-propenoic acid t-butyl ester (439 µl), 95% ethanol (4 ml) and acetic acid (372 µl) was stirred for 12 hours at room temperature. Further, 2-propenoic acid t-butyl ester (2.20 ml) was added to the mixture and it was refluxed for 4 hours. The reaction mixture was diluted with ethyl acetate. The mixture was washed with a saturated aqueous solution of sodium bicarbonate, a saturated aqueous solution of sodium chloride, dried over anhydrous magnesium sulfate and concentrated to give the title compound (872 mg) having the following physical data.
mp : 105°C,
TLC : Rf 0.31 (hexane : ethyl acetate = 4 : 1),
NMR (CDCl₃ + CCl₄ (5 drops)) : δ 7.78 (2H, d, J=8.8Hz), 7.72 (2H, d, J=8.8Hz), 3.38 (2H, t, J=7.3Hz), 2.65 (2H, t, J=7.3Hz), 1.40 (9H, s).

### Example 7

### 2-methyl-3-[4-(4-tolylethynyl)phenylsulfonyl]propionic acid t-butyl ester

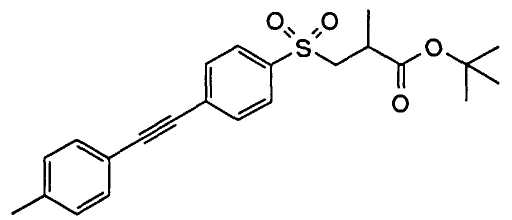

To a solution of the compound prepared in Reference example 2 (432 mg) and 4-ethynyltoluene (133 µl) in acetonitrile (10 ml), triphenylphosphine (31 mg), triethylamine (2 ml), copper iodide (8 mg) and 10% palladium - carbon (43 mg) were added successively. The mixture was refluxed for 3 hours and concentrated. The residue was purified by column chromatography (hexane : ethyl acetate = 17 : 3) to give the title compound (376 mg). The title compound (376 mg) was recrystallized from hexane to give the title compound (227 mg) having the following physical data.
TLC : Rf 0.41 (hexane : ethyl acetate = 4 : 1),
NMR (CDCl₃ + CCl₄ (5 drops)) : δ 7.88 (2H, d, J=8.6Hz), 7.67 (2H, d, J=8.6Hz), 7.45 (2H, d, J=8.3Hz), 7.19 (2H, d, J=8.3Hz), 3.68 (1H, dd, J=13.9Hz, 7.1Hz), 3.01 (1H, dd, J=13.9Hz, 5.4Hz), 2.89 (1H, m), 1.42 (9H, s), 1.29 (3H, d, J=7.2Hz).

### Example 7(1)∼7(5)

The following compounds were obtained by the same procedure as Example 7, using the compounds prepared in Example 6 or Example 5(2), and the corresponding acetylene derivatives.

### Example 7(1)

### 3-[4-(1-heptynyl)phenylsulfonyl]propionic acid t-butyl ester

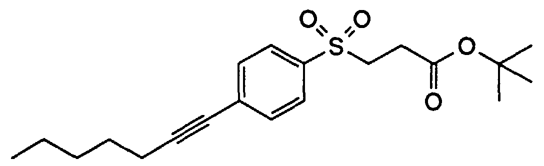

TLC : Rf 0.38 (hexane : ethyl acetate = 6 : 1),
NMR (CDCl₃ + CCl₄ (5 drops)) : δ 7.81 (2H, d, J=8.6Hz), 7.55 (2H, d, J=8.6Hz), 3.37 (2H, t, J=7.6Hz), 2.64 (2H, t, J=7.6Hz), 2.44 (2H, t, J=7.1 Hz), 1.59 (2H, m), 1.20-1.50 (4H, m), 1.41 (9H, s), 0.93 (3H, t, J=6.8Hz).

### Example 7(2)

### 3-[4-(phenylethynyl)phenylsulfonyl]propionic acid t-butyl ester

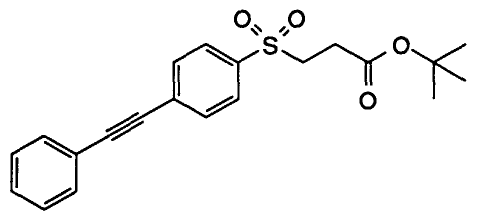

TLC : Rf 0.59 (hexane : ethyl acetate = 4 : 1),
NMR (CDCl₃) : δ 7.89 (2H, d, J=8.7H), 7.70 (2H, d, J=8.7Hz), 7.58-7.53 (2H, m), 7.40-7.37 (3H, m), 3.40 (2H, t, J=7.8Hz), 2.67 (2H, t, J=7.8Hz), 1.41 (9H, s).

### Example 7(3)

### 3-[4-(2-pyridylethynyl)phenylsulfonyl]propionic acid t-butyl ester

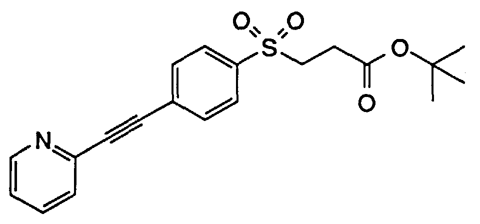

TLC : Rf 0.34 (hexane : ethyl acetate = 1 : 1),
NMR (CDCl₃) : δ 8.65 (1H, m), 7.92 (2H, d, J=8.6H), 7.78 (2H, d, J=8.6H), 7.70 (1H, m), 7.56 (1H, m), 7.31 (1H, m), 3.42 (2H, m), 2.69 (2H, m), 1.42 (9H, s).

### Example 7(4)

### 3-[4-(4-methoxyphenylethynyl)phenylsulfonyl]propionic acid t-butyl ester

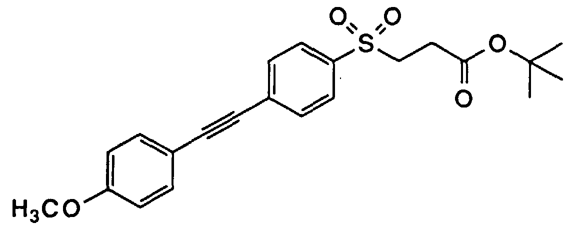

TLC : Rf 0.15 (hexane : ethyl acetate = 4 : 1),
NMR (CDCl₃) : δ 7.87 (2H, d, J=8.6H), 7.66 (2H, d, J=8.6H), 7.49 (2H, d, J=8.8H), 6.90 (2H, d, J=8.8H), 3.85 (3H, s), 3.40 (2H, m), 2.66 (2H, m), 1.41 (9H, s).

### Example 7(5)

### 2-benzyl-3-[4-(4-tolylethynyl)phenylsulfonyl]propionic acid t-butyl ester

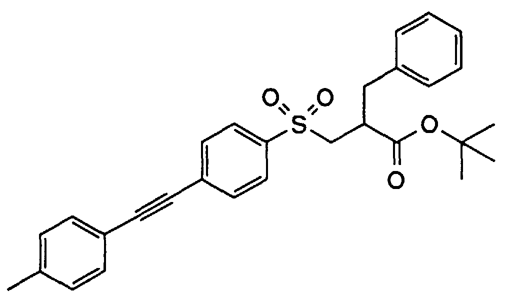

TLC : Rf 0.19 (hexane : ethyl acetate = 4 : 1),
NMR (CDCl₃) : δ 7.81 (2H, d, J=8.6Hz), 7.64 (2H, d, J=8.6Hz), 7.45 (2H, d, J=8.1Hz),7.30-7.15 (5H, m), 7.08 (2H, m), 3.64 (1H, m), 3.15-2.75 (4H, m), 2.40 (3H, s), 1.34 (9H, s).

### Example 8

### 3-[4-(benzoylamino)phenylthio]propionic acid

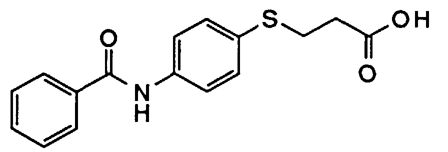

A solution of the compound prepared in Example 2 (560 mg) in trifluoroacetic acid (5 ml) was stirred for 1 hour at room temperature. The reaction solution was concentrated and benzene was added to the residue, and then the solution was concentrated again. The residue was washed with ether to give the title compound (440 mg) having the following physical data.
TLC : Rf 0.19 (chloroform : methanol = 9 : 1),
NMR (DMSO-d₆) : δ 12.80-11.80 (1H, br.s), 10.30 (1H, s), 7.95 (2H, dd, J=2.0Hz, 8.2Hz), 7.77 (2H, d, J=8.8Hz), 7.65-7.47 (3H, m), 7.36 (2H, d, J=8.8Hz), 3.09 (2H, t, J=7.4Hz), 2.50 (2H, t J=7.4Hz).

### Example 8(1)∼8(11)

The following compounds were obtained by the same procedure as Example 8 (deprotection under acidic condition; for example, a solution of trifluoroacetic acid, hydrochloric acid in dioxane or ethyl acetate, are used.), using the compounds prepared in Example 4, 5, 5(1), 7, 7(1), 7(2), 7(3), 7(4), 7(5) or 1(4).

### Example 8(1)

### 3-[4-(benzoylamino)phenylsulfinyl]propionic acid

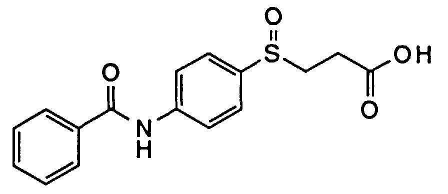

TLC : Rf 0.26 (chloroform : methanol : acetic acid = 90 : 10 : 1),
NMR (DMSO-d₆) : δ 12.80-11.80 (1H, br.s), 10.52 (1H, s), 8.01 (2H, d, J=8.8Hz), 7.99-7.93 (2H, m), 7.64 (2H, d, J=8.8Hz), 7.62-7.50 (3H, m), 3.20 (1H, ddd, J=6.8, 8.2, 13.6Hz), 2.95 (1H, ddd, J=6.2,8.2,13.6Hz), 2.58 (1H, ddd, J=6.8,8.2,16.8Hz), 2.33 (1H, ddd, J=6.2,8.2,16.8Hz).

### Example 8(2)

### 3-[4-(benzoylamino)phenylsulfonyl]propionic acid

TLC : Rf 0.33 (chloroform : methanol : acetic acid = 90 : 10 : 1),
NMR (DMSO-d₆) : δ 13.20-11.80 (1H, br.s), 10.68 (1H, s), 8.07 (2H, d, J=8.8Hz), 7.98 (2H, dd, J=1.8, 8.2Hz), 7.87 (2H, d, J=8.8Hz), 7.70-7.50 (3H, m), 3.48 (2H, t, J=7.2Hz), 2.53 (2H, t, J=7.2Hz).

### Example 8(3)

### 3-[4-(benzyloxy)phenylsulfonyl]propionic acid

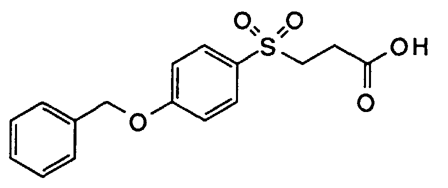

TLC : Rf 0.63 (chloroform : methanol : acetic acid = 90 : 10 : 1),
NMR (DMSO-d₆) : δ 12.70-12.40 (1H, br.s), 7.82 (2H, d, J=8.8Hz), 7.52-7.30 (5H, m), 7.28-7.20 (2H, d, J=8.8Hz), 5.22 (2H, s), 3.46 (2H, t, J=7.4Hz), 2.50 (2H, t, J=7.4Hz).

### Example 8(4)

### 2-methyl-3-[4-(4-tolylcarbonylmethyl)phenylsulfonyl]propionic acid

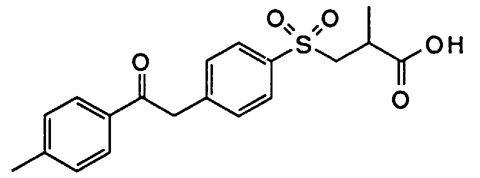

TLC : Rf 0.33 (chloroform : methanol : acetic acid = 40 : 1 : 0.2),
NMR (DMSO-d₆ + CCl₄ (5 drops)) : δ 12.58 (1H, s), 7.97 (2H, d, J=7.8Hz), 7.84 (2H, d, J=8.1Hz), 7.54 (2H, d, J=8.1Hz), 7.36 (2H, d, J=7.8Hz), 3.60 (1H, dd, J=14.2, 6.8Hz), 3.39 (1H, dd, J=14.2, 5.4Hz), 2.68 (1H, m), 2.39 (3H, s), 1.17 (3H, d, J=7.1Hz).

### Example 8(5)

### 2-methyl-3-[4-(4-tolylethynyl)phenylsulfonyl]propionic acid

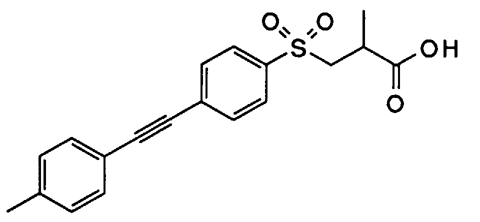

TLC : Rf 0.35 (chloroform : methanol = 10 : 1),
NMR (DMSO-d₆ + CCl₄ (5 drops)) : δ 12.57 (1H, s), 7.91 (2H, d, J=8.6Hz), 7.79 (2H, d, J=8.6Hz), 7.50 (2H, d, J=8.3Hz), 7.27 (2H, d, J=8.3Hz), 3.66 (1H, dd, J=14.4, 7.3Hz), 3.44 (1H, dd, J=14.4, 5.4Hz), 2.70 (1H, m), 2.36 (3H, s), 1.18 (3H, d, J=7.1Hz).

### Example 8(6)

### 3-[4-(1-heptynyl)phenylsulfonyl]propionic acid

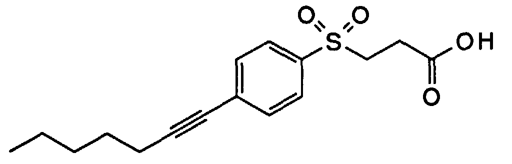

mp : 122 ∼ 123°C,
TLC : Rf 0.78 (chloroform : methanol : acetic acid = 9 :1: 0.5),
NMR (DMSO-d₆ + CCl₄ (5 drops)) : δ 12.54 (1H, s), 7.84 (2H, d, J=8.2Hz), 7.63 (2H, d, J=8.2Hz), 3.52 (2H, t, J=7.4Hz), 2.40-2.60 (4H, m), 1.57 (2H, m), 1 .37 (4H, m), 0.90 (3H, t J=7.0Hz).

### Example 8(7)

### 3-[4-(phenylethynyl)phenylsulfonyl]propionic acid

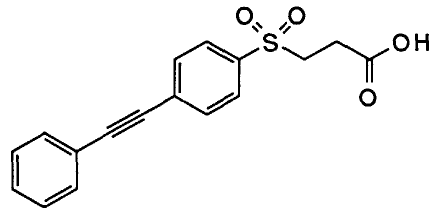

TLC : Rf 0.47 (chloroform : methanol = 4 : 1),
NMR (DMSO-d₆) : δ 12.49 (1H, br.s), 7.93 (2H, d, J=8.5Hz), 7.78 (2H, d, J=8.5Hz), 7.61-7.56 (2H, m), 7.55-7.42 (3H, m), 3.52 (2H, t, J=7.3Hz), 2.56 (2H, t J=7.3Hz).

### Example 8(8)

### 3-[4-(2-pyridylethynyl)phenylsulfonyl]propionic acid

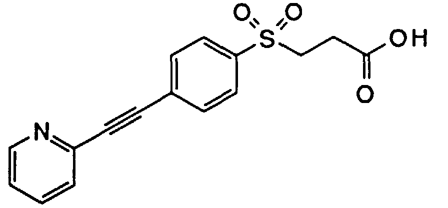

TLC : Rf 0.39 (chloroform : methanol : acetic acid = 100 : 10 : 1),
NMR (CDCl₃ + CD₃OD (3 drops)) : δ 8.63 (1H, m), 7.94 (2H, d, J=8.6Hz), 7.83-7.73 (3H, m), 7.60 (1H, m), 7.36 (1H, m), 3.47 (2H, m), 2.75 (2H, m).

### Example 8(9)

### 3-[4-(4-methoxyphenylethynyl)phenylsulfonyl]propionic acid

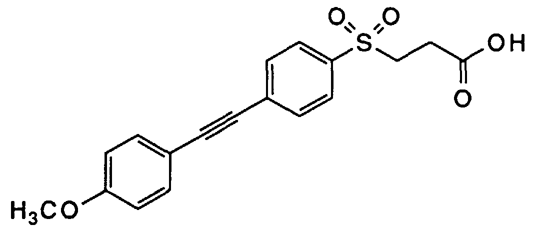

TLC : Rf 0.42 (chloroform : methanol : acetic acid = 100 : 10 : 1),
NMR (CDCl₃ + CD₃OD (3 drops)) : δ 7.87 (2H, d, J=8.6Hz), 7.66 (2H, d, J=8.6Hz), 7.50 (2H, d, J=9.0Hz), 6.91 (2H, d, J=9.0Hz), 3.85 (3H, s), 3.44 (2H, m), 2.73 (2H, m).

### Example 8(10)

### 2-benzyl-3-[4-(4-tolylethynyl)phenylsulfonyl]propionic acid

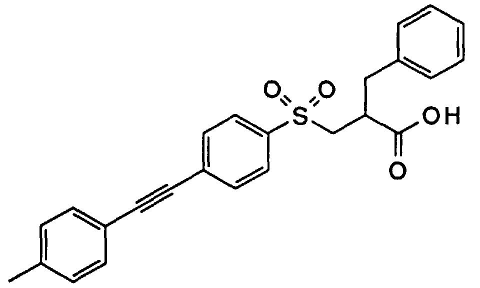

TLC : Rf 0.45 (chloroform : methanol : acetic acid = 100 : 10 : 1),
NMR (CDCl₃ + CD₃OD (3 drops)) : δ 7.77 (2H, d, J=8.6Hz), 7.62 (2H, d, J=8.6Hz), 7.45 (2H, d, J=8.1Hz), 7.30-7.15 (5H, m), 7.04 (2H, m), 3.67 (1H, m), 3.15-3.05 (3H, m), 2.82 (1H, m), 2.40 (3H, s).

### Example 8(11)

### 3-(4-methoxyphenylthio)propionic acid

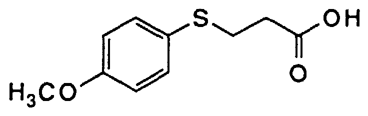

TLC : Rf 0.48 (chloroform : methanol = 10 : 1),
NMR (CDCl₃) : δ 7.39 (2H, d, J=8.8Hz), 6.85 (2H, d, J=8.8Hz), 3.80 (3H, s), 3.04 (2H, t, J=7.2Hz), 2.61 (2H, t, J=7.2Hz).

### Example 9

### N-t-butoxy-3-(4-methoxyphenylthio)propionamide

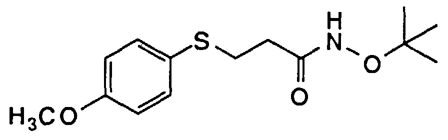

To a solution of the compound prepared in Example 8(11) (1.00 g) in DMF (20 ml), t-butoxyamine hydrochloride (652 mg), triethylamine (0.8 ml), 1-ethyl-3-(3-dimethylaminopropyl)-carbodiimide (EDC) hydrochloride (995 mg) and 1-hydroxybenzotriazole (HOBt) hydrate (795 mg) were added at 0°C. The mixture was stirred for 16 hours at room temperature. To the reaction mixture, ethyl acetate and water were added. The organic phase was washed with 0.1 N hydrochloric acid, a saturated aqueous solution of sodium bicarbonate, water and a saturated aqueous solution of sodium chloride, successively, dried over anhydrous magnesium sulfate and concentrated to give the title compound (1.35 g) having the following physical data.
TLC : Rf 0.76 (chloroform : methanol = 10 : 1).

### Example 9(1)

### N-benzyloxy-3-(4-methoxyphenylthio)propionamide

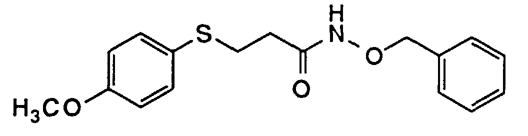

The title compound (423 mg) having the following physical data was obtained by the same procedure as Example 9, using the compound prepared in Example 8(11) (300 mg) and benzyloxyamine hydrochloride (271 mg).
TLC : Rf 0.65 (chloroform : methanol = 10 : 1),
NMR (DMSO-d₆) : δ 7.38 (5H, m), 7.33 (2H, d, J=8.6Hz), 6.91 (2H, d, J=8.6Hz), 4.77 (2H, s), 3.74 (3H, s), 3.01 (2H, t, J=7.4Hz), 2.21 (2H, t, J=7.4Hz).

### Example 10

### N-benzyloxy-3-(4-methoxyphenylsulfonyl)propionamide

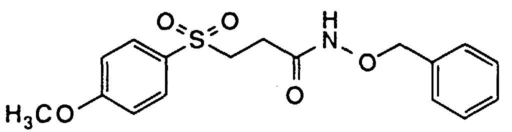

The title compound (152 mg) having the following physical data was obtained by the same procedure as Example 5, using the compound prepared in Example 9(1) (159 mg).
TLC : Rf 0.48 (chloroform : methanol = 20 : 1).

### Reference example 3

### N-t-butoxycarbonyl-N-t-butoxycarbonyloxy-3-(4-methoxyphenylthio)propionamide

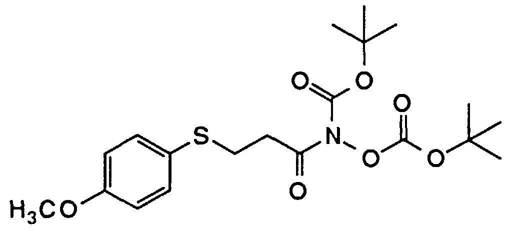

To a solution of the compound prepared in Example 8(11) (106 mg) in DMF (5 ml), N-t-butoxycarbonyl-N-t-butoxycarbonyloxyamine (120 mg), EDC hydrochloride (106 mg) and 4-(dimethylamino)pyridine (6 mg) were added at 0°C. The mixture was stirred for 16 hours at room temperature. To the reaction solution, water was added and it was extracted with ethyl acetate. The extract was washed with a saturated aqueous solution of ammonium chloride, a saturated aqueous solution of sodium bicarbonate, a saturated aqueous solution of sodium chloride, dried over anhydrous magnesium sulfate and concentrated to give the title compound (211 mg) having the following physical data.
TLC : Rf 0.48 (hexane : ethyl acetate = 4 : 1).
NMR (DMSO-d₆) : δ 7.35 (2H, d, J=8.6Hz), 6.93 (2H, d, J=8.6Hz), 3.74 (3H, s), 3.18 (2H, m), 3.05 (2H, m), 1.46 (9H, s), 1.43 (9H, s).

### Reference example 4

### N-t-butoxycarbonyl-N-t-butoxycarbonyloxy-3-(4-methoxyphenylsulfinyl)propionamide

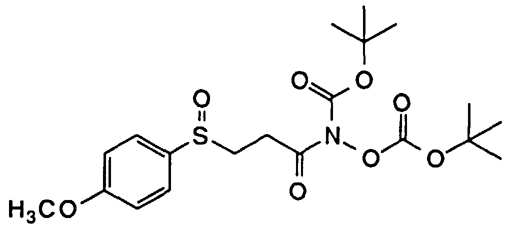

The title compound (quant) having the following physical data was obtained by the same procedure as Example 4, using the compound prepared in Reference example 3 (190 mg).
TLC : Rf 0.36 (chloroform : methanol = 20 : 1),
NMR (DMSO-d₆) : δ 7.58 (2H, d, J=8.6Hz), 7.15 (2H, d, J=8.6Hz), 3.83 (3H, s), 3.28-3.08 (2H, m), 3.05-2.80 (2H, m), 1.46 (9H, s), 1.44 (9H, s).

### Example 11

### N-hydroxy-3-(4-methoxyphenylthio)propionamide

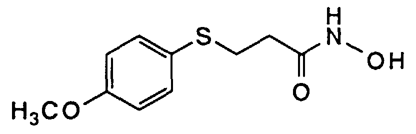

The mixture of the compound prepared in Example 9 (200 mg) and 30% hydrogen bromide in acetic acid (2 ml) was stirred for 1 hour at room temperature. The reaction mixture was concentrated. The residue was purified by column chromatography (chloroform : methanol = 10 : 1) to give the title compound (138 mg) having the following physical data.
TLC : Rf 0.33 (chloroform : methanol = 10 : 1),
NMR (DMSO-d₆) : δ 10.42 (1 H, br.s), 8.79 (1H, s), 7.33 (2H, d, J=8.6Hz), 6.92 (2H, d, J=8.6Hz), 3.75 (3H, s), 3.01 (2H, t, J=7.4Hz), 2.20 (2H, t, J=7.4Hz).

### Example 11(1)∼11(2)

The following compounds were obtained by the same procedure as Example 11 (deprotection under acidic condition ; for example, hydrogen bromide in acetic acid, trifluoroacetic acid are used.), or the same desired procedure (hydrogenolysis) as Example 11, using the compounds prepared in Reference example 4 or Example 10.

### Example 11(1)

### N-hydroxy-3-(4-methoxyphenylsulfinyl)propionamide

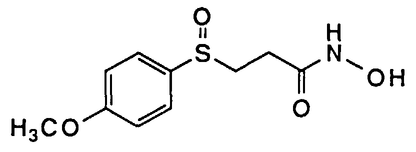

TLC : Rf 0.46 (chloroform : methanol = 4 : 1).
NMR (DMSO-d₆) : δ 10.50 (1H, br.s), 8.82 (1H, br.s), 7.58 (2H, d, J=8.6Hz), 7.15 (2H, d, J=8.6Hz), 3.82 (3H, s), 3.20-3.03 (1H, m), 3.00-2.83 (1H, m), 2.43-2.25 (1H, m), 2.10-1.93 (1H, m).

### Example 11(2)

### N-hydroxy-3-(4-methoxyphenylsulfonyl)propionamide

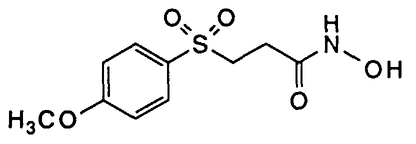

TLC : Rf 0.66 (chloroform : methanol = 4 : 1),
NMR(DMSO-d₆) : δ 10.50 (1H, br.s), 8.85 (1H, s), 7.82 (2H, d, J=8.6Hz), 7.17 (2H, d, J=8.6Hz), 3.87 (3H, s), 3.43 (2H, m), 2.27 (2H, m).

### Example 12

### 2-t-butoxycarbonylamino-3-(4-methoxyphenylthio)propionic acid benzyl ester

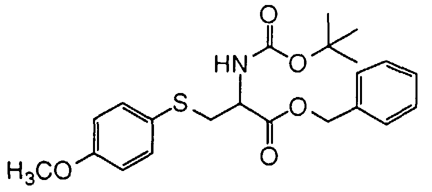

To a solution of 2-t-butoxycarbonylamino-3-hydroxypropionic acid benzyl ester (29.5 g) in dichloromethane (300 ml), triethylamine (21 ml) and mesyl chloride (8.6 ml) were added and the reaction solution was stirred for 30 minutes at 4°C. The reaction solution was added to 1N hydrochloric acid cooling with ice. The obtained yellow oil was dissolved into dichloromethane (300 ml), and diisopropylethylamine (17 ml) and 4-methoxybenzenethiol (12 ml) were added thereto at 4°C and the reaction solution was stirred for 3 hours at room temperature and concentrated. The residue was purified by column chromatography (n-hexane : ethyl acetate = 8 : 1) to give the title compound (24.8 g) having the following physical data.
TLC : Rf 0.65 (ethyl acetate : n-hexane = 1 : 2),
NMR (CD₃OD) : δ 7.42-7.20 (7H, m), 6.80 (2H, d, J=9.0Hz), 5.35 (1H, m), 5.02 (1H, d, J=12.5Hz), 4.85 (1H, d, J=12.5Hz), 4.55 (1H, m), 3.78 (3H, 5), 3.25 (2H, d, J=5.0Hz), 1.40 (9H, s).

### Example 13

### 2-t-butoxycarbonylamino-3-(4-methoxyphenylsulfonyl)propionic acid benzyl ester

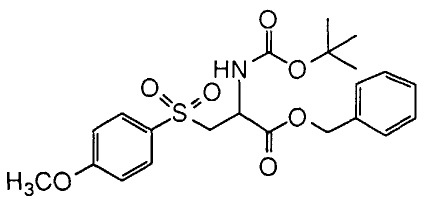

To a solution of the compound prepared in Example 12 (20.9 g) in dichloromethane (200 ml), 70% m-chloroperbenzoic acid (26.0 g) was added and the reaction solution was stirred for 72 hours at room temperature. The reaction solution was added to water and it was extracted with dichloromethane. The organic layer was dried over anhydrous magnesium sulfate, filterd and concentrated. The residue was washed with n-hexane and the precipitate was dissolved into ethyl acetate. The organic layer was washed with a saturated aqueous solution of sodium bicarbonate, a saturated aqueous solution of sodium chloride, dried over anhydrous magnesium sulfate, filtered and concentrated. The residue was washed with n-hexane to give the title compound (16.7 g) having the following physical data.
TLC : Rf 0.29 (ethyl acetate : n-hexane = 1 : 2),
NMR (CD₃OD) : δ 7.78 (2H, d, J=9.0Hz), 7.45-7.30 (5H, m), 6.98 (2H, d, J=9.0Hz), 5.50 (1H, d, J=7.5Hz), 5.18 (1H, d, J=11.0Hz), 5.06 (1H, d, J=11.0Hz), 4.60 (1H, m), 3.88 (3H, s), 3.72 (2H, d, J=5.0Hz), 1.35 (9H, s).

### Example 14

### 2-t-butoxycarbonylamino-3-(4-methoxyphenylsulfonyl)propionic acid

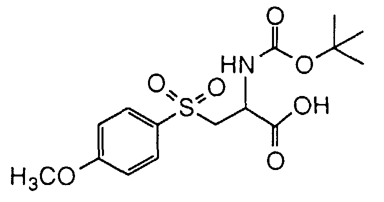

Under an atmosphere of nitrogen, to a solution of the compound prepared in Example 13 (1.35 g) in THF (15 ml), 10% palladium - carbon (100 g) was added and the reaction solution was stirred for 45 minutes at room temperature under an atmosphere of hydrogen. 10% palladium - carbon was removed from the reaction solution and then the solution was concentrated. The residue was washed with ether to give the title compound (1.02 g) having the following physical data.
TLC : Rf 0.57 (chloroform : methanol : acetic acid = 9 : 1 : 1),
NMR (DMSO-d₆) : δ 7.79 (2H, d, J=8.8Hz), 7.16 (2H, d, J=8.8Hz), 7.05 (1H, d, J=8.4Hz), 4.26 (1H, m), 3.87 (3H, s), 3.61 (2H, m), 1.32 (9H, s).

### Example 15

### 2-amino-3-(4-methoxyphenylsulfonyl)propionic acid benzyl ester hydrochloride

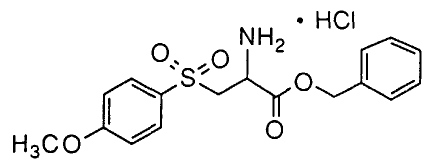

The title compound having the following physical data was obtained by the same procedure as Example 8, using the compound prepared in Example 13.
TLC : Rf 0.54 (chloroform : methanol = 18 : 1),
NMR (DMSO-d₆) : δ 9.00-8.80 (3H, br), 7.85 (2H, d, J=9.0Hz), 7.42-7.35 (5H, m), 7.15 (2H, d, J=9.0Hz), 5.18 (1H, d, J=12.5Hz), 4.98 (1H, d, J=12.5Hz), 4.45 (1H, t J=5.0Hz), 3.95 (2H, d, J=5.0Hz), 3.86 (3HJ, s).

### Example 16∼16(2)

The following compounds were obtained by the same procedure as Example 9, using the compound prepared in Example 15 and the corresponding carboxylic acids.

### Example 16

### 2-[N-[N-(t-butoxycarbonyl)phenylglycyl]amino]-3-(4-methoxyphenylsulfonyl)propionic acid benzyl ester

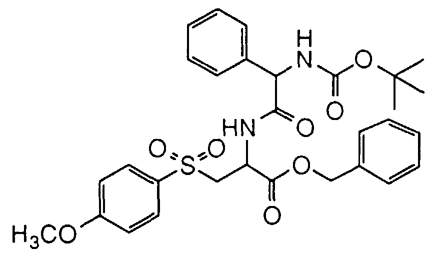

TLC : Rf 0.51 (ethyl acetate : n-hexane = 1 : 1),
NMR (DMSO-d₆) : δ 8.88 and 8.75 (total 1H, d and d, J=7.8 and 7.8Hz), 7.79 and 7.74 (total 2H, d and d, J=9.0 and 9.0Hz), 7.42-7.20 (10H, m), 7.14 and 7.10 (total 2H, d and d, J=9.0 and 9.0Hz), 5.20-4.95 (3H, m), 4.80-4.65 and 4.63-4.45 (total 1H, m and m), 3.88 and 3.87 (total 3H, s and s), 3.80-3.60 (2H, m), 1.38 (9H, s).

### Example 16(1)

### 2-acetylamino-3-(4-methoxyphenylsulfonyl)propionic acid benzyl ester

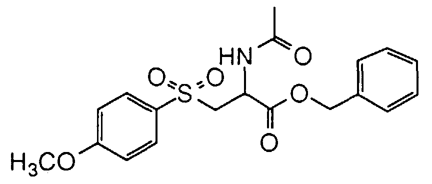

TLC : Rf 0.53 (chloroform : methanol = 18 : 1),
NMR (CDCl₃) : δ 7.76 (2H, d, J=9.2Hz), 7.45-7.30 (5H, m), 6.99 (2H, d, J=9.2Hz), 6.54 (1H, d, J=7.0Hz), 5.19 (1H, d, J=11.8Hz), 5.09 (1H, d, J=11.8Hz), 4.87 (1H, m), 3.88 (3H, s), 3.80-3.70 (2H, m), 1.93 (3H, s).

### Example 16(2)

### 2-[N-[N-(t-butoxycarbonyl)glycyl]amino]-3-(4-methoxyphenylsulfonyl)propionic acid benzyl ester

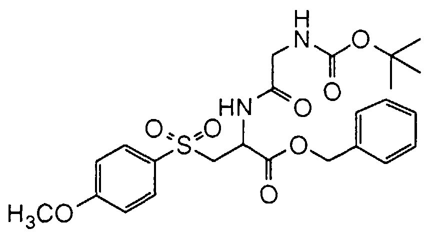

TLC : Rf 0.31 (ethyl acetate : n-hexane = 1 : 1),
NMR (DMSO-d₆) : δ 8.40 (1H, d, J=8.0Hz), 7.80 (2H, d, J=9.0Hz), 7.45-7.25 (5H, m), 7.18 (2H, d, J=9.0Hz), 6.95 (1H, m), 5.08 (2H, s), 4.70 (1H, m), 3.88 (3H, s), 3.80-3.60 (2H, m), 3.45-3.30 (2H, m, overlap with H₂O in DMSO), 1.40 (9H, s).

### Example 17∼17(2)

The following compounds were obtained by the same procedure as Example 14, using the compounds prepared in Example 16 ∼ 16(2).

### Example 17

### 2-[N-[N-(t-butoxycarbonyl)phenylglycyl]amino]-3-(4-methoxyphenylsulfonyl)propionic acid

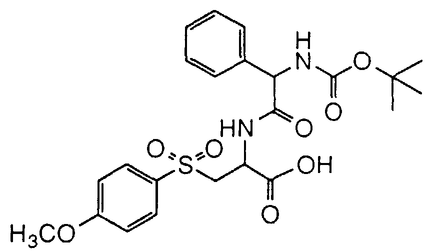

TLC : Rf 0.30 (chloroform : methanol : acetic acid = 90 : 10 : 1),
NMR (CD₃OD) : δ 7.81 and 7.72 (total 2H, each d, J=9.2Hz), 7.35 (5H, m), 7.10 and 7.50 (total 2H, each d, J=9.2Hz), 5.11 and 5.03 (total 1 H, each brs), 4.76 and 4.55 (total 1H, each dd, J=8.6Hz, 3.7Hz), 3.90 and 3.89 (total 3H, each s), 3.84-3.57 (2H, m), 1.44 (9H, s).

### Example 17(1)

### 2-acetylamino-3-(4-methoxyphenylsulfonyl)propionic acid

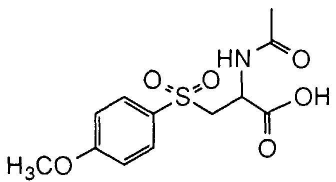

TLC : Rf 0.46 (ethyl acetate : acetic acid : water = 3 : 1 : 1),
NMR (CDCl₃ + CD₃OD) : δ 7.82 (2H, d, J=8.8Hz), 7.03(2H, d, J=8.8Hz), 4.73 (1H, m), 3.89 (3H, s), 3.82-3.70 (2H, m), 1.91 (3H, s).

### Example 17(2)

### 2-[N-[N-(t-butoxycarbonyl)glycyl]amino]-3-(4-methoxyphenylsulfonyl)propionic acid

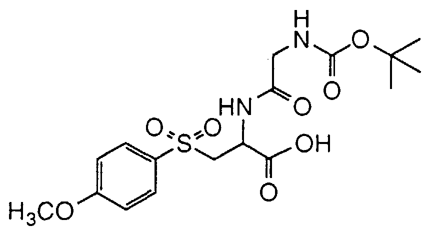

TLC : Rf 0.38 (chloroform : methanol : acetic acid = 80 : 20 : 1),
NMR (CDCl₃ + CD₃OD) : δ 7.82 (2H, d, J=9.0Hz), 7.03 (2H, d, J=9.0Hz), 4.75 (1H, m), 3.89 (3H, s), 3.75 (4H, m), 1.46 (9H, s).

### Example 18∼28(2)

The following compounds were obtained by the same procedure as a series of reactions of Example 8 → Example 9 (the corresponding carboxylic acids are used.) → Example 14, using the compound prepared in Example 16(2).

### Example 18

### 2-[N-[N-(t-butoxycarbonyl)phenylglycyl-glycyl]amino]-3-(4-methoxyphenylsulfonyl)propionic acid

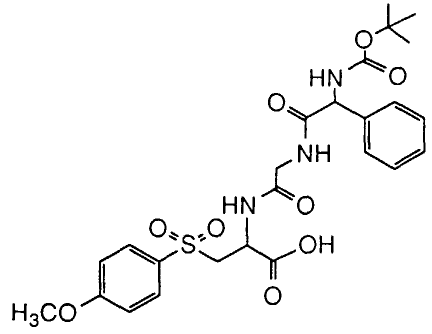

TLC : Rf 0.43 (chloroform : methanol : acetic acid = 80 : 20 : 1),
NMR (DMSO-d₆) : δ 8.29 (2H, m), 7.77 and 7.75 (total 2H, each d, J=9.0Hz), 7.41 (2H, m), 7.30 (4H, m), 7.12 and 7.10 (total 2H, each d, J=9.0Hz), 5.25 (1H, m), 4.53 (1H, m), 3.84 and 3.82 (total 3H, each s), 3.78-3.45 (4H, m), 1.38 (9H, s).

### Example 18(1)

### 2-[N-(N-acetylglycyl)amino]-3-(4-methoxyphenylsulfonyl)propionic acid

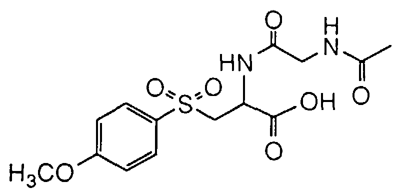

TLC : Rf 0.32 (ethyl acetate : acetic acid : water = 3 : 1 : 1),
NMR (DMSO-d₆+CD₃OD) : δ 7.80 (2H, d, J=8.8Hz), 7.15 (2H, d, J=8.8Hz), 4.60 (1H, dd, J=8.3 and 4.0Hz), 3.89 (3H, s), 3.85-3.50 (4H, m), 1.89 (3H, s).

### Example 18(2)

### 2-[N-[N-(benzylcarbonyl)glycyl]amino]-3-(4-methoxyphenylsulfonyl)propionic acid

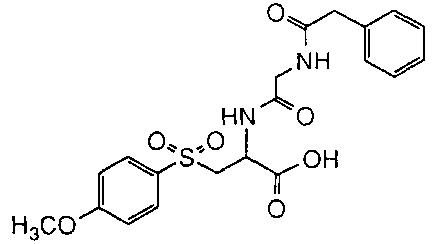

TLC : Rf 0.61 (ethyl acetate : acetic acid : water = 3 : 1 : 1),
NMR (DMSO-d₆+CD₃OD) : δ 7.79 (2H, d, J=8.8Hz), 7.40-7.20 (5H, m), 7.12 (2H, d J=8.8Hz), 4.59 (1H, m), 3.85 (3H, s), 3.82-3.40 (6H, m).

### Example 19∼19(6)

The following compounds were obtained by the same procedure as a series of reactions of Example 9 → Example 14, using the compounds prepared in Example 14, 17 ∼ 17(2), 18 ∼ 18(2).

### Example 19

### N-hydroxy-2-t-butoxycarbonylamino-3-(4-methoxyphenylsulfonyl)propionamide

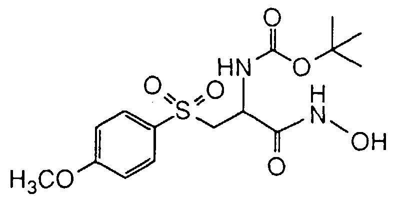

TLC : Rf 0.62 (chloroform : methanol : acetic acid = 9 : 1 : 1).

### Example 19(1)

### N-hydroxy-2-[N-[N-(t-butoxycarbonyl)phenylglycyl]amino]-3-(4-methoxyphenylsulfonyl)propionamide

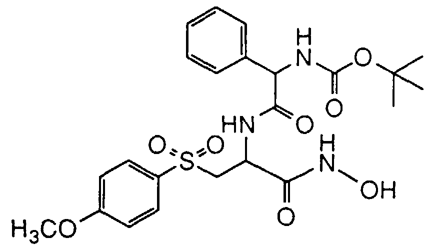

TLC : Rf 0.55 (chloroform : methanol : acetic acid = 90 : 10 : 1),
NMR (DMSO-d₆) : δ 10.65 (1H, br), 9.04 and 8.98 (total 1H, each br), 8.59 and 8.57 (total 1H, each d, J=8.2Hz), 7.77 and 7.66 (total 2H, each d, J=9.0Hz), 7.32 (6H, m), 7.13 and 7.08 (total 2H, each d, J=9.0Hz), 5.00 (1H, d, J=8.2Hz), 4.50 (1H, m), 3.87 (3H, s), 3.59 (2H, m), 1.38 (9H, s).

### Example 19(2)

### N-hydroxy-2-acetylamino-3-(4-methoxyphenyfsulfonyl)propionamide

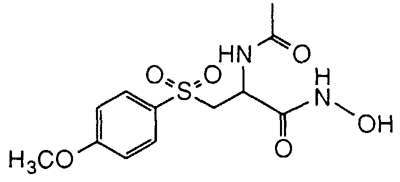

TLC : Rf 0.31 (chloroform : methanol : acetic acid = 80 : 10 : 1),
NMR (DMSO-d₆) : δ 11.00-8.60 (2H, br), 8.06 (1H, d, J=8.6Hz), 7.76 (2H, d, J=8.0Hz), 7.14 (2H, d, J=8.0Hz), 4.56 (1H, m), 3.86 (3H, s), 3.55 (2H, m), 1.63 (3H, s).

### Example 19(3)

### N-hydroxy-2-[N-[N-(t-butoxycarbonyl)glycyl]amino]-3-(4-methoxyphenylsulfonyl)propionamide

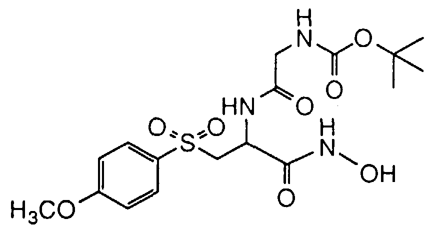

TLC : Rf 0.40 (chloroform : methanol : acetic acid = 90 : 10 : 1),
NMR (DMSO-d₆) : δ 10.62 (1H, brs), 8.99 (1H, brs), 8.15 (1H, d, J=8.4Hz), 7.75 (2H, d, J=8.8Hz), 7.14 (2H, d, J=8.8Hz), 6.87 (1H, m), 4.59 (1H, m), 3.87 (3H, s), 3.65-3.24 (4H, m), 1.39 (9H, s).

### Example 19(4)

### N-hydroxy-2-[N-[N-(t-butoxycarbonyl)phenylglycyl-glycyl]amino]-3-(4-methoxyphenylsulfonyl)propionamide

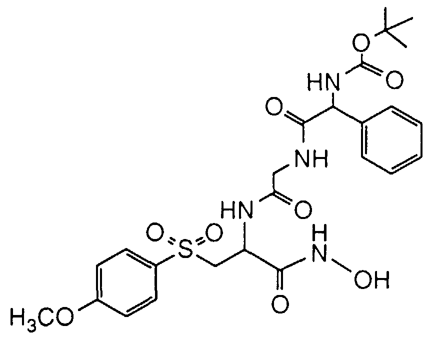

TLC : Rf 0.45 (chloroform : methanol : acetic acid = 90 : 10 : 1),
NMR (DMSO-d₆) : δ 11.00-10.40 (1H, br), 9.20-8.60 (1H, br), 8.23 (2H, m), 7.75 and 7.73 (total 2H, each d, J=8.6Hz), 7.44-7.29 (6H, m), 7.12 and 7.08 (total 2H, each d, J=8.6Hz), 5.24 (1H, m), 4.60 (1H, m), 3.83 and 3.80 (total 3H, each s), 3.66-3.33 (4H, m, overlap with H2O in DMSO), 1.38 (9H, s).

### Example 19(5)

### N-hydroxy-2-[N-(N-acetylglycyl)amino]-3-(4-methoxyphenylsulfonyl)propionamide

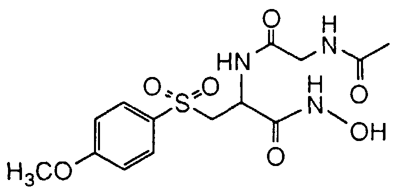

TLC : Rf 0.45 (ethyl acetate : acetic acid : water = 3 : 1 : 1),
NMR (DMSO-d₆ + CD₃OD) : δ 7.76 (2H, m), 7.14 (2H, m), 4.60 (1H, m), 3.87 (3H, s), 3.70-3.30 (4H, m), 1.87 (3H, s).

### Example 19(6)

### N-hydroxy-2-[N-(N-(benzylcarbonyl)glycyl]amino]-3-(4-methoxyphenylsulfonyl)propionamide

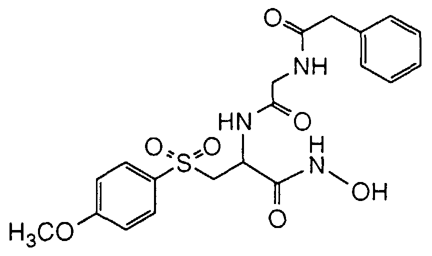

TLC : Rf 0.37 (chloroform : methanol : acetic acid = 90 : 10 : 1),
NMR (DMSO-d₆ + CD₃OD) : δ 7.78 (2H, m), 7.30 (7H, m), 4.60 (1H, m), 3.90-3.30 (9H, m).

### Example 20∼20(4)

The following compounds were obtained by the same procedure as Example 8, using the compounds prepared in Example 14, 19, 17, 19(1), 19(4).

### Example 20

### 2-amino-3-(4-methoxyphenylsulfonyl)propionic acid trifluroacetic acid salt

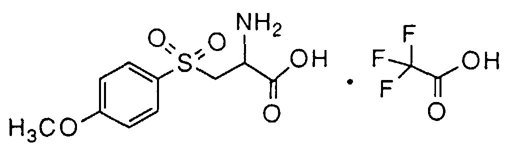

TLC : Rf 0.60 (chloroform : methanol : acetic acid = 5 : 4 : 1),
NMR (CD₃OD) : δ 7.94 (2H, d, J=9.2Hz), 7.18 (2H, d, J=9.2Hz), 4.43 (1H, dd, J=8.6Hz, 3.4Hz), 3.92 (3H, s), 3.89 (1H, dd, J=15.2Hz, 3.4Hz), 3.71 (1H, dd, J=15.2Hz, 8.6Hz).

### Example 20(1)

### N-hydroxy-2-amino-3-(4-methoxyphenylsulfonyl)propionamide hydrochloride

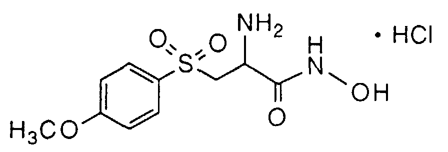

TLC : Rf 0.64 (chloroform : methanol : acetic acid = 5 : 4 : 1),
NMR (DMSO-d₆) : δ 11.38 (1H, s), 9.60-9.25 (1H, br), 8.90-8.50 (3H, br), 7.90 (2H, d, J=8.8Hz), 7.21 (2H, d, J=8.8Hz), 3.90 (4H, m), 3.75 (2H, m).

### Example 20(2)

### 2-[N-(phenylglycyl)amino]-3-(4-methoxyphenylsulfonyl)propionic acid trifluroacetic acid salt

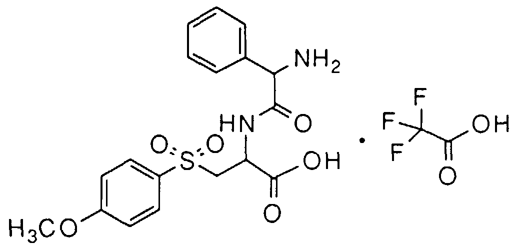

TLC : Rf 0.24 and 0.29 (ethyl acetate : acetic acid : water = 3 : 1 : 1),
NMR (CD₃OD) : δ 7.83 and 7.67 (total 2H, each d, J=8.8Hz), 7.48 (5H, m), 7.13 and 7.03 (total 2H, each d, J=8.8Hz), 4.88 and 4.61 (total 2H, each m, overlap with H₂O in CD₃OD), 3.91 and 3.89 (total 3H, each s), 3.80 and 3.73 (total 1H, each m), 3.62-3.50 (1H, m).

### Example 20(3)

### N-hydroxy-2-[N-(phenylglycyl)amino]-3-(4-methoxyphenylsulfonyl)propionamide trifluroacetic acid salt

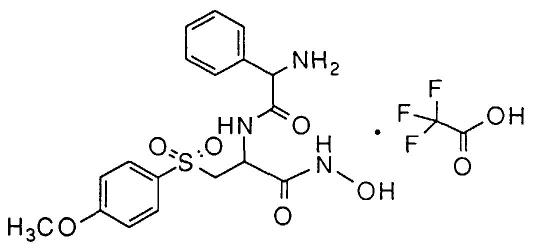

TLC : Rf 0.46 and 0.51 (ethyl acetate : acetic acid : water = 3 : 1 : 1),
NMR (DMSO-d₆) : δ 10.90 (1 H, br), 9.04 (2H, m), 8.58 (3H, br), 7.82 and 7.58 (total 2H, each d, J=8.8Hz), 7.45 (5H, m), 7.17 and 7.06 (total 2H, each d, J=8.8Hz), 4.98 and 4.81 (total 1H, each br), 4.50 (1H, m), 3.88 and 3.86 (total 3H, each s), 3.64 (1H, m), 3.40 (1H, m, overlap with H₂O in DMSO).

### Example 20(4)

### N-hydroxy-2-[N-(phenylglycyl-glycyl)amino]-3-(4-methoxyphenylsulfonyl)propionamide trifluroacetic acid salt

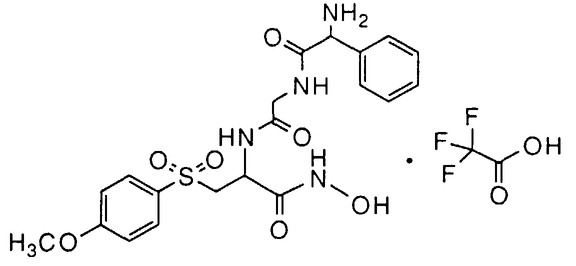

TLC : Rf 0.48 (ethyl acetate : acetic acid : water = 3 : 1 : 1),
NMR (DMSO-d₆) : δ 10.81 (1H, m), 9.00 (1H, m), 8.64 (4H, m), 8.35 (1H, m), 7.77 and 7.74 (total 2H, each d, J=8.8Hz), 7.53 (2H, m), 7.43 (3H, m), 7.15 and 7.07 (total 2H, each d, J=8.8Hz), 5.07 (1H, m), 4.61 (1H, m), 3.87 and 3.79 (total 3H, each s), 3.68-3.34 (4H, m, overlap with H₂O in DMSO).

### Reference example 5

### S-oxiranecarboxylic acid t-butyl ester

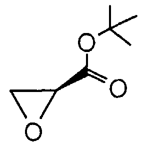

To a solution of S-oxiranecarboxylic acid potassium salt (1.26 g) in dichloromethane (45 ml), pyridinium p-tosylate (1.50 g) and N, N'-diisopropyl-O-t-butylisourea (1.50 g) were added at 0°C and the reaction solution was stirred for 4 hours at room temperature. The reaction solution was passed through a short silica gel column (n-hexane : ethyl acetate = 3 : 1) to give the title compound (932 mg) having the following physical data.
TLC : Rf 0.70 (ethyl acetate : n-hexane = 1 : 3),
NMR (CDCl₃) : δ 3.32 (1H, dd, J=2.8Hz, 3.7Hz), 2.94-2.85 (2H, m), 1.50 (9H, s).

### Example 21

### 2R-hydroxy-3-(4-bromophenylsulfonyl)propionic acid t-butyl ester

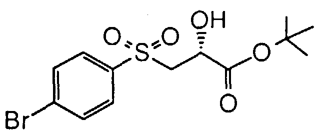

To a mixture solution of the compound prepared in Reference example 5 (565 mg) in water (8.0 ml) + benzene (8.0 ml), poly ethylene glycol 4000 (98 mg) and 4-bromophenylsulfinic acid sodium salt (4.3 g) were added and the reaction solution was refluxed for 7 hours. The reaction solution was added to water and it was extracted with ethyl acetate. The organic layer was washed with a saturated aqueous solution of sodium chloride, dried over anhydrous magnesium sulfate, filtered and concentrated. The residue was purified by silica gel column chromatography (toluene : ethyl acetate = 8 : 1) to give the title compound (490 mg) having the following physical data.
TLC : Rf 0.56 (ethyl acetate : n-hexane = 1 : 2),
NMR (CDCl₃) : δ 7.28 (2H, d, J=8.8Hz), 7.70 (2H, d, J=8.8Hz), 4.55-4.48 (1H, m), 3.63 (1H, dd, J=3.0, 14.7Hz), 3.44 (1H, dd, J=7.9, 14.7Hz), 3.08 (1H, d, J=4.1HZ), 1.51 (9H, s).

### Example 22

### 2R-hydroxy-3-[4-(4-tolylethynyl)phenylsulfonyl]propionic acid

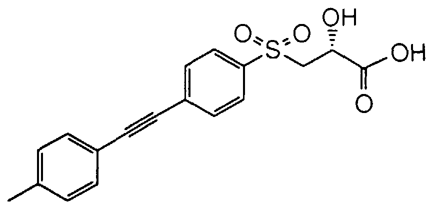

The title compound was obtained by the same procedure as a series of reactions of Example 7 → Example 8, using the compound prepared in Example 21.
TLC : Rf 0.23 (chloroform : methanol : acetic acid = 8 : 1 : 1),
NMR (DMSO-d₆) : δ 7.91 (2H, d, J=8.0Hz), 7.75 (2H, d, J=8.0Hz), 7.50 (2H, d, J=8.0Hz), 7.27 (2H, d, J=8.0Hz), 4.22-4.26 (1H, m), 3.46-3.64 (2H, m), 2.35 (3H, s).

### Example 23

### 2S-hydroxy-3-[4-(4-tolylethynyl)phenylsulfonyl]propionic acid

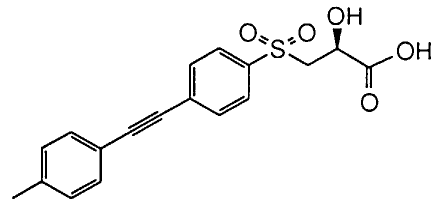

The title compound was obtained by the same procedure as Reference example 5 → Example 21 → Example 7 → Example 8, using the R-oxiranecarboxylic acid potassium salt instead of S-oxiranecarboxylic acid potassium salt in Reference example 5.
TLC : Rf 0.25 (chloroform : methanol : acetic acid = 8 : 1 : 1),
NMR (DMSO-d₆) : δ 13.21-12.23 (1H, br.), 7.92 (2H, d, J=8.5 Hz), 7.76 (2H, d, J=8.5 Hz), 7.50 (2H, d, J=8.2 Hz), 7.27 (2H, d, J=8.2 Hz), 4.41-4.35 (1H, m), 3.75-3.56 (2H, m), 2.36 (3H, s).

### Example 24

### 3-[4-(4-tolylethynyl)phenylsulfonyl]butyric acid

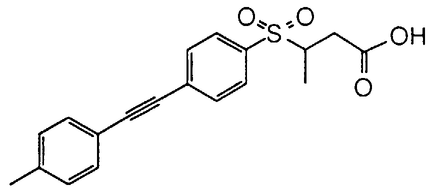

The title compound was obtained by the same procedure as a series of reactions of Example 1 → Example 5 → Example 7 → Example 8, using 2-butenoic acid t-butyl ester and 4-bromothiophenol instead of 2-propenoic acid t-butyl ester and 4-aminothiophenol, respectively, in Example 1
TLC : Rf 0.47 (chloroform : methanol : water = 4 : 1 : 0.1),
NMR (DMSO-d₆) : δ 7.92 (2H, d, J=8.6Hz), 7.81 (2H, d, J=7.8Hz), 7.52 (2H, d, J=8.6Hz), 7.29 (2H, d, J=7.8Hz), 3.71-3.60 (1H, m), 2.76 (1H, dd, J=16.3Hz, 4.7Hz), 2.43-2.30 (4H, m), 1.24 (3H, d, J=7.0Hz).

### Example 25

### 3-[4-(4-tolylcarbonylmethyl)phenylsulfonyl]propionic acid

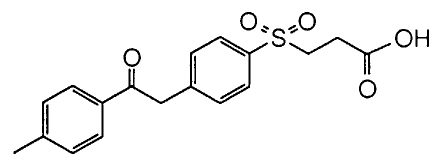

The title compound was obtained by the same procedure as a series of reactions of Example 7 → Example 8, using the compound prepared in Example 6.
TLC : Rf 0.46 (ethyl acetate : acetic acid = 99 : 1),
NMR (DMSO-d₆) : δ 7.96 (2H, d, J=8.2Hz), 7.84 (2H, d, J=8.2Hz), 7.54 (2H, d, J=8.2Hz), 7.36 (2H, d, J=8.2Hz), 4.54 (2H, s), 3.51 (2H, t, J=7.4Hz), 2.60-2.45 (2H), 2.39 (3H,s).

### Example 26

### 3-[4-(4-tolylvinyl)phenylsulfonyl]propionic acid

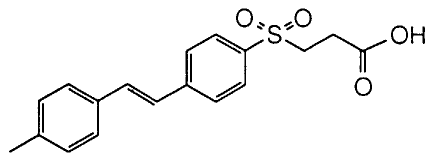

### (1) intermediate : the preparation of 3-[4-[2-hydroxy-2-(4-tolyl)ethyl]phenyl]sulfonylpropionic acid

To a solution of the compound prepared in Example 25 (347 mg) in ethanol (20 ml), sodium borohydride (111 mg) was added and the reaction solution was stirred for 1 hour at room temperature. The reaction mixture was concentrated, neutralized by adding 1N hydrochloric acid and extracted with ethyl acetate. The extract was washed with a saturated aqueous solution of sodium chloride, dried over anhydrous magnesium sulfate, concentrated and washed with ether to give the above intermediate (273 mg) having the following physical data.
TLC : Rf 0.33 (chloroform : methanol : acetic acid = 90 : 10 : 1),
NMR (CD₃OD) : δ 7.76 (2H, d, J=8.4Hz), 7.39 (2H, d, J=8.4Hz), 7.16 (2H, d, J=8.2Hz), 7.10 (2H, d, J=8.2Hz), 4.94-4.78 (1H), 3.45 (2H, t, J=7.0Hz), 3.20-2.98 (2H,m), 2.61 (2H, t, J=7.0Hz), 2.29 (3H, s).

### (2) title compound : the preparation of 3-[4-(4-tolylvinyl)phenylsulfonyl]propionic acid

To a solution of the above intermediate (273 mg) in toluene (10 ml), p-toluenesulfonic acid · 1 hydrate (30 mg) was added and the reaction solution was stirred for 2 hours at 50°C, 2 hours at 70°C, and further 4 hours at 90°C. The reaction mixture was filtered after the temperature of the reaction mixture was cooled to room temperature to give the title compound (221 mg) having the following physical data.
TLC : Rf 0.25 (chloroform : methanol = 85 : 15),
NMR(CDCl₃ + CD₃OD) : δ 7.87 (2H, d, J=8.4Hz), 7.67 (2H, d, J=8.4Hz), 7.45 (2H, d, J=8.0Hz), 7.25 (1H, d, J=1 6.2Hz), 7.20 (2H, d, J=8.2Hz), 7.08 (1H, d, J=16.2Hz), 3.44 (2H, t, J=7.2Hz), 2.73 (2H, t, J=7.2Hz), 2.38 (3H, s).

### Example 27∼27(2)

The title compound was obtained by the same procedure as a series of reactions of Example 7 → Example 8, ,using the compound prepared in Reference example 2.

### Example 27

### 2-methyl-3-[4-(1-heptynyl)phenylsulfonyl]propionic acid

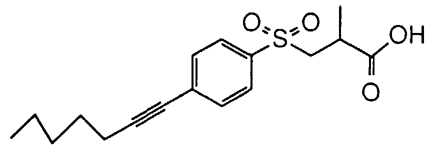

TLC : Rf 0.33 (chloroform : methanol = 9 : 1),
NMR (DMSO-d₆) : δ 12.53 (1H, br s), 7.82 (2H, d, J=8.4Hz), 7.61 (2H, d, J=8.4Hz), 3.61 (1H, dd, J=7.4Hz, J=14.6Hz), 3.39 (1H, dd, J=5.3Hz, J=14.6Hz), 2.70-2.56 (1H, m), 2.45 (2H, t, J=7.2Hz), 1.64-1.22 (6H, m), 1.14 (3H, d, J=7.4Hz), 0.87 (3H, t, J=6.8 Hz).

### Example 27(1)

### 2-methyl-3-[4-(2-benzofuranyl)phenylsulfonyl]propionic acid

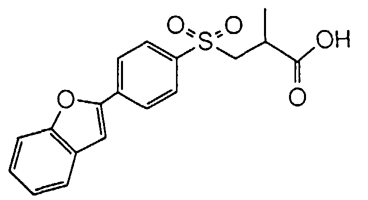

TLC : Rf 0.37 (chloroform : methanol = 4 : 1),
NMR (DMSO-d₆) : δ 12.57 (1H, br s), 8.17 (2H, d, J=8.2Hz), 7.99 (2H, d, J=8.2Hz), 7.74-7.65 (3H, m), 7.43-7.26 (2H, m), 3.67 (1H, dd, J=7.0Hz, J=14.7Hz), 3.45 (1H, dd, J=5.3Hz, J=14.7Hz), 2.80-2.63 (1H, m), 1.17 (3H, d, J=7.0Hz).

### Example 27(2)

### 2-methyl-3-[4-(4-hydroxy-but-1-ynyl)phenylsulfonyl]propionic acid

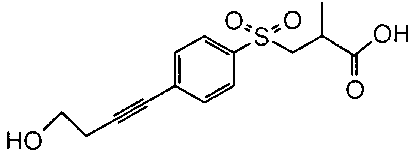

TLC : Rf 0.36 (chloroform : methanol : acetic acid = 9 : 1 : 0. 5),
NMR (DMSO-d₆) : δ 7.83 (2H, d, J=8.4Hz), 7.62 (2H, d, J=8.4Hz), 4.07 (1H, br s), 3.66 (1H, dd, J=7.2Hz, J=14.9Hz), 3.58 (2H, t, J=6.7Hz), 3.34-3.25 (2H, m), 2.58 (2H, t, J=6.7Hz), 1.12 (3H, d, J=7.2Hz).

### Example 28∼28(3)

The following compounds were obtained by the same procedure as a series of reactions of Example 9 → Example 11, using the compounds prepared in Example 27 ∼ 27(2), 8(5).

### Example 28

### N-hydroxy-2-methyl-3-[4-(1-heptynyl)phenylsulfonyl]propionamide

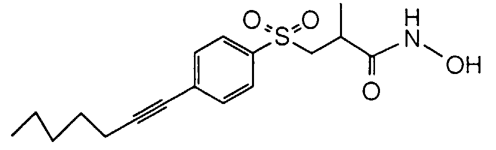

TLC : Rf 0.34 (chloroform : methanol = 9 : 1),
NMR (DMSO-d₆) : δ 10.54 (1H, br s), 7.82 (2H, d, J=8.6Hz), 7.60 (2H, d, J=8.6Hz), 3.55 (1H, dd, J=7.3Hz, J=14.3Hz), 3.28 (1H, dd, J=5.1 Hz, J=14.3Hz), 2.64-2.39 (3H, m), 1.63-1.21 (6H, m), 1.06 (3H, d, J=7.3Hz), 0.87 (3H, t, J=7.1Hz).

### Example 28(1)

### N-hydroxy-2-methyl-3-[4-(2-benzofuranyl)phenylsulfonyl]propionamide

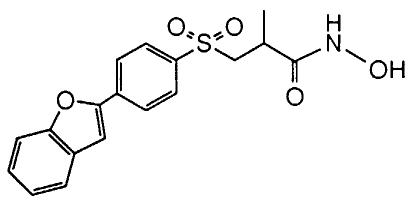

TLC : Rf 0.22 (chloroform : methanol = 9 : 1),
NMR (DMSO-d₆) : δ 10.57 (1H, br s), 8.80 (1H, br s), 8.16 (2H, d, J=8.6Hz), 7.98 (2H, d, J=8.6Hz), 7.74-7.65 (3H, m), 7.43-7.26 (2H, m), 3.61 (1H, dd, J=7.3Hz, J=14.3Hz), 3.32 (1H, dd, J=5.2Hz, J=14.3Hz), 2.59 (1H, m), 1.09 (3H, d, J=7.3Hz).

### Example 28(2)

### N-hydroxy-2-methyl-3-[4-(4-hydroxy-but-1-ynyl)phenylsulfonyl]propionamide

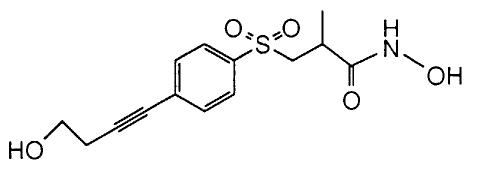

TLC : Rf 0.20 (chloroform : methanol : acetic acid = 9 : 1 : 0.5),
NMR (DMSO-d₆) : δ 10.54 (1H, br s), 8.79 (1H, s), 7.82 (2H, d, J=8.3Hz), 7.61 (2H, d, J=8.3Hz), 3.66-3.48 (3H, m), 3.38-3.20 (1H, m), 2.61-2.50 (3H, m), 1.05 (3H, d, J=7.0Hz).

### Example 28(3)

### N-hydroxy-2-methyl-3-[4-(4-tolylethynyl)phenylsulfonyl]propionamide

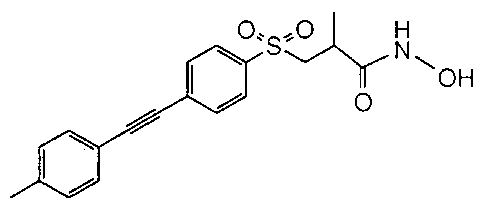

TLC : Rf 0.29 (chloroform : methanol = 9 : 1),
NMR (DMSO-d₆) : δ 10.56 (1H, br s), 7.89 (2H, d, J=8.8Hz), 7.76 (2H, d, J=8.8Hz), 7.48 (2H, d, J=8.0Hz), 7.25 (2H, d, J=8.0Hz), 3.59 (1H, dd, J=7.1Hz, J=14.4Hz), 3.32 (1H, dd, J=5.0Hz, J=14.4Hz), 2.67-2.54 (1H, m), 2.33 (3H, s), 1.07 (3H, d, J=7.1Hz).

### Example 29

### 3-[4-(phenylethynyl)phenylsulfinyl]propionic acid

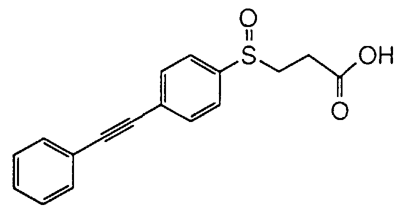

3-[4-(phenylethynyl)phenylsulfinyl]propionic acid 1-butyl ester (t-butyl ester of the title compound) was obtained by the same procedure as a series of reactions of Reference example 1 → Example 4 → Example 7, using the compound prepared in Example 1(1). The title compound having the following physical data was obtained by the same procedure as Example 8, using t-butyl ester.
TLC : Rf 0.37 (chloroform : methanol : water = 4 : 1 : 0.1),
NMR (DMSO-d₆) : δ 7.79-7.72 (4H, m), 7.63-7.58 (2H, m), 7.48-7.44 (3H, m), 3.37-3.23 (1H, m), 3.07-2.93 (1H, m), 2.69-2.50 (1H, m), 2.42-2.27 (1H, m).

### Example 30

### 3-[4-(phenylethynyl)phenylthio]propionic acid

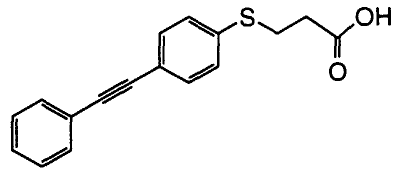

To a solution of the t-butyl ester prepared in the course of Example 29 (46 mg) in THF (2 ml), Lawesson's Reagent (55 mg) was added at 0°C and the reaction solution was stirred for 15 minutes at 0°C. The oil obtained by concentrating of the reaction solution, was purified by silica gel column chromatography (chloroform : methanol = 97 : 3) to give the title compound (11 mg) having the following physical data.
TLC : Rf 0.49 (chloroform : methanol : water = 4 : 1 : 0.1),
NMR (CDCl₃ + CD₃OD (2 drops)) : δ 7.55-7.29 (9H, m), 3.20 (2H, t J=7.4Hz), 2.65(2H, t, J=7.4Hz).

### Example 31

### 3-[4-(benzoylamino)phenylsulfonyl]-2-propenoic acid

The title compounds having the following physical data were obtained by the same procedure as a series of reactions of Example 1 → Example 2 → Example 4, followed by the separation procedure of E and Z by passing through a silica gel column, further followed by the same procedure as Example 8, using 2-propynoic acid t-butyl ester instead of 2-propenoic acid t-butyl ester in Example 1.

### (1) cis form

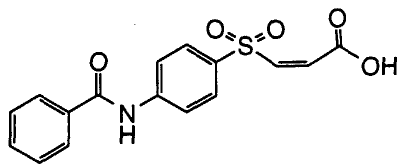

TLC : Rf 0.68 (chloroform : methanol : water = 6 : 4 : 0.5),
NMR (DMSO-d₆) : δ 10.72 (1H, s), 8.09 (2H, d, J=8.9Hz), 8.00 (2H, d, J=7.8Hz), 7.90 (2H, d, J=8.9Hz), 7.62-7.56 (3H, m), 6.93 (1H, d, J=12.7Hz), 6.83 (1H, d, J=12.7Hz).

### (2) trans form

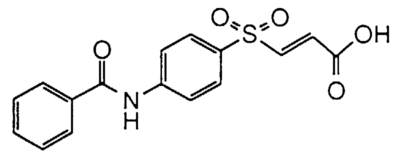

TLC : Rf 0.70 (chloroform : methanol : water = 6 : 4 : 0.5),
NMR (DMSO-d₆) : δ 10.73 (1H, s), 8.12 (2H, d, J=9.0Hz), 8.02-7.92 (4H, m), 7.63-7.57 (3H, m), 7.66 (1H, d, J=13.8Hz), 6.67 (1H, d, J=13.8Hz).

### Example 32

### 3-(4-bromophenylsulfonyl)propionic acid

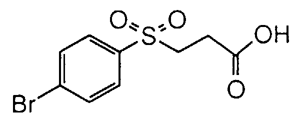

The title compound having the following physical data was obtained by the same procedure as Example 8, using the compound prepared in Example 6.
TLC : Rf 0.41 (chloroform : methanol : acetic acid = 100 : 10 : 1),
NMR (CDCl₃ + CD₃OD (3 drops)) : δ 7.79 (2H, d, J=9.0Hz), 7.74 (2H, d, J=9.0Hz), 3.44 (2H, m), 2.72 (2H, m).

### [Formulation example]

### Formulation example 1

The following components were admixed in conventional method and punched out to obtain 100 tablets each containing 50 mg of active ingredient.

| | |
|---|---|
| · 3-[4-(phenylcarbonylamino)phenylsulfonyl)propionic acid | 5.0 g |
| · Carboxymethyl Cellulose calcium (disintegrating agent) | 0.2 g |
| · Magnesium stearate (lubricating agent) | 0.1 g |
| · Microcrystalline cellulose | 4.7 g |

### Formulation example 2

The following components were admixed in conventional method. The solution was sterilized in conventional manner, placed 5 ml portions into ampoules and freeze-dried to obtain 100 ampoules each containing 20 mg of the active ingredient.

| | |
|---|---|
| · 3-[4-(phenylcarbonylamino)phenylsulfonyl]propionic acid | 2.0 g |
| · mannitol | 20 g |
| · distilled water | 1000 ml |

## Claims

1. Matrix metalloproteinase inhibitors containing aryl (sulfide, sulfoxide, sulfone) of the formula (I) 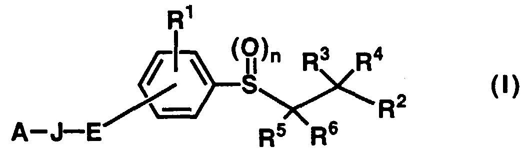 (wherein
R¹ is hydrogen, or C1-4 alkyl,
R² is -COOR⁷ or -CONHOR⁸,
R⁷ is hydrogen, C1-8 alkyl, phenyl, or
C1-4 alkyl substituted by phenyl, -OCOR²³ (in which R²³ is C1-4 alkyl.), or -CONR²⁴R²⁵ (in which R²⁴ and R²⁵, each independently, is hydrogen or C1-4 alkyl.),
R⁸ is hydrogen, C1-8 alkyl, phenyl, or C1-4 alkyl substituted by phenyl,
E is -CONR⁹-, -NR⁹CO-, -OCO-, -COO-, -CH₂-O-, -CO-CH₂-, -(CH₂)₂-, -CH=CH-or -C≡C- (in which R⁹ is hydrogen, C1-4 alkyl, phenyl, or C1-4 alkyl substituted by phenyl, and the left side of each group is attached to J group.),
J is bond or C1-8 alkylene,
A is
1) hydrogen,
2) C1-8 alkyl,
3) Ar group (Ar group is carbocyclic ring or heterocyclic ring optionally substituted by 1 ∼ 3 of
i) C1-15 alkyl,
ii) C1-15 alkoxy,
iii) halogen,
iv) nitro,
v) cyano,
vi) guanidino,
vii) amidino,
viii) hydroxy,
ix) benzyloxy,
x) NR¹²R¹³ (in which R¹² and R¹³, each independently, is hydrogen, C1-4 alkyl or -COOR¹⁴ (in which R¹⁴ is C1-4 alkyl or benzyloxy.).),
xi) -COOR¹⁵ (in which R¹⁵ is hydrogen, C1-4 alkyl, phenyl, or C1-4 alkyl substituted by phenyl.),
xii) trifluoromethyl,
xiii) carbocyclic ring,
xiv) heterocyclic ring or
xv) C1-4 alkyl substituted by hydroxy, C1-4 alkoxy, NR¹²R¹³ (in which R¹² and R¹³ are as hereinbefore defined.), -COOR¹⁵ (in which R¹⁵ is as hereinbefore defined.), carbocyclic ring or heterocyclic ring.)
or
4) C1-4 alkyl substituted by hydroxy or C1-4 alkoxy, or
A, J and E taken together, represent methyl, halogen, trifluoromethyl, nitro, cyano, formyl, phenyl, hydroxy, NR¹⁶R¹⁷ (in which R¹⁶ and R¹⁷, each independently, is hydrogen, C1-4 alkyl, -COOR¹⁸ (in which R18 is C1-4 alkyl or benzyl.).), or heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are as hereinbefore defined.) or CONR²⁴R²⁵ (in which R²⁴ and R²⁵ are as hereinbefore defined.).),
R³ and R⁴, each independently, is
(1) hydrogen,
(2) C1-8 alkyl (with the proviso that one of the carbon atoms in C1-8 alkyl may be replaced by a sulfur atom.),
(3) -COOR¹⁹ (in which R¹⁹ is hydrogen, C1-8 alkyl, phenyl, or C1-4 alkyl substituted by phenyl.),
(4) Ar₁ group (Ar₁ group is carbocyclic ring or heterocyclic ring optionally substituted by 1 ∼ 3 of C1-4 alkyl, C1-4 alkoxy, halogen, hydroxy or trifluoromethyl.),
(5) hydroxy,
(6) -NR²⁰R²¹ (in which R²⁰ and R²¹, each independently, is hydrogen, C1-4 alkyl, -COOR²² or -COR²² (in which R²² is C1-4 alkyl or benzyl.),
(7) 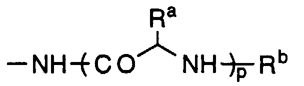 (in which R^{a} is hydrogen or phenyl, R^{b} is hydrogen, -COOR²² or -COR²² (in which R²² is as hereinbefore defined.), p is 1 or 2.), or
(8) C1-8 alkyl substituted by substituent selected from the following (a) ∼ (f) (with the proviso that one of the carbon atoms in C1-8 alkyl may be replaced by a sulfur atom.);
(a) -COOR¹⁹ (in which R¹⁹ is as hereinbefore defined.)
(b) C1-4 alkoxy,
(c) hydroxy,
(d) benzyloxy,
(e) -NR²⁰R²¹ (in which R²⁰ and R²¹ are as hereinbefore defined.), or
(f) Ar₁ group (in which Ar₁ is as hereinbefore defined.)
,or R³ and R⁴ taken together with the carbon to which they are attached, form C3-7 cycloalkyl,
R⁵ and R⁶ are hydrogen or methyl, or R³ and R⁵ taken together, form a bond and R⁴ and R⁶ are as hereinbefore defined, and
n is 0, 1 or 2;
With the proviso that :
when A, J and E taken together, form phenyl, and R² is CONHOH, then n is 1 or 2.)
, or non-toxic salts thereof, as active ingredient.

2. Aryl (sulfide, sulfoxide, sulfone) derivatives of the formula (I) 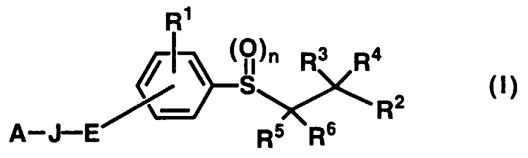 (wherein
R1 is hydrogen, or C1-4 alkyl,
R² is -COOR⁷ or -CONHOR⁸,
R⁷ is hydrogen, C1-8 alkyl, phenyl, or
C1-4 alkyl substituted by phenyl, -OCOR²³ (in which R²³ is C1-4 alkyl.), or -CONR²⁴R²⁵ (in which R²⁴ and R²⁵, each independently, is hydrogen or C1-4 alkyl.),
R⁸ is hydrogen, C1-8 alkyl, phenyl, or C1-4 alkyl substituted by phenyl,
E is -CONR⁹-, -NR⁹CO-, -OCO-, -COO-, -CH₂-O-, -CO-OH₂-, -(CH₂)₂-, -CH=CH-or -C≡C- (in which R⁹ is hydrogen, C1-4 alkyl, phenyl, or C1-4 alkyl substituted by phenyl, and the left side of each group is attached to J group.),
J is bond or C1-8 alkylene,
A is
1) hydrogen,
2) C1-8 alkyl,
3) Ar group (Ar group is carbocyclic ring or heterocyclic ring optionally substituted by 1 ∼ 3 of
i) C1-15 alkyl,
ii) C1-15 alkoxy,
iii) halogen,
iv) nitro,
v) cyano,
vi) guanidino,
vii) amidino,
viii) hydroxy,
ix) benzyloxy,
x) NR¹²R¹³ (in which R¹² and R¹³, each independently, is hydrogen, C1-4 alkyl or -COOR¹⁴ (in which R¹⁴ is C1-4 alkyl or benzyloxy.).),
xi) -COOR¹⁵ (in which R¹⁵ is hydrogen, C1-4 alkyl, phenyl, or C1-4 alkyl substituted by phenyl.),
xii) trifluoromethyl,
xiii) carbocyclic ring,
xiv) heterocyclic ring or
xv) C1-4 alkyl substituted by hydroxy, C1-4 alkoxy, NR¹²R¹³ (in which R¹² and R¹³ are as hereinbefore defined.), -COOR¹⁵ (in which R¹⁵ is as hereinbefore defined.), carbocyclic ring or heterocyclic ring.)
or
4) C1-4 alkyl substituted by hydroxy or C1-4 alkoxy, or
A, J and E taken together, represent methyl, halogen, trifluoromethyl, nitro, cyano, formyl, phenyl, hydroxy, NR¹⁶R¹⁷ (in which R¹⁶ and R¹⁷, each independently, is hydrogen, C1-4 alkyl, -COOR¹⁸ (in which R¹⁸ is C1-4 alkyl or benzyl.).), or heterocyclic ring (this heterocyclic ring may be optionally substituted by 1 ∼4 of C1-4 alkyl, C1-4 alkoxy, halogen, trifluoromethyl, hydroxy, carboxyl, C1-8 alkoxycarbonyl, nitro, NR²⁴R²⁵ (in which R²⁴ and R²⁵ are as hereinbefore defined.) or CONR²⁴R²⁵ (in which R²⁴ and R²⁵ are as hereinbefore defined.).),
R³ and R⁴, each independently, is
(1) hydrogen,
(2) C1-8 alkyl (with the proviso that one of the carbon atoms in C1-8 alkyl may be replaced by a sulfur atom.),
(3) -COOR¹⁹ (in which R¹⁹ is hydrogen, C1-8 alkyl, phenyl, or C1-4 alkyl substituted by phenyl.),
(4) Ar₁ group (Ar₁ group is carbocyclic ring or heterocyclic ring optionally substituted by 1 ∼ 3 of C1-4 alkyl, C1-4 alkoxy, halogen, hydroxy or trifluoromethyl.),
(5) hydroxy,
(6) -NR²⁰R²¹ (in which R²⁰ and R²¹, each independently, is hydrogen, C1-4 alkyl, -COOR²² or -COR²² (in which R²² is C1-4 alkyl or benzyl.),
(7) 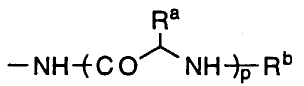 (in which R^{a} is hydrogen or phenyl, R^{b} is hydrogen, -COOR²² or -COR²² (in which R²² is as hereinbefore defined.), p is 1 or 2.), or
(8) C1-8 alkyl substituted by substituent selected from the following (a) ∼ (f) (with the proviso that one of the carbon atoms in C1-8 alkyl may be replaced by a sulfur atom.);
(a) -COOR¹⁹ (in which R¹⁹ is as hereinbefore defined.)
(b) C1-4 alkoxy,
(c) hydroxy,
(d) benzyloxy,
(e) -NR²⁰R²¹ (in which R²⁰ and R²¹ are as hereinbefore defined.), or
(f) Ar₁ group (in which Ar₁ is as hereinbefore defined.)
,or R³ and R⁴ taken together with the carbon to which they are attached, form C3-7 cycloalkyl,
R⁵ and R⁶ are hydrogen or methyl, or R³ and R⁵ taken together, form a bond and R⁴ and R⁶ are as hereinbefore defined, and
n is 0, 1 or 2;
With the proviso that :
(a) when A, J and E taken together, form phenyl, and R² is CONHOH, then n is 1 or 2.
(b) the following compounds (1) ∼ (58) are excluded:
(1) 3-(4-methylphenylsulfonyl)propionic acid isopropyl ester,
(2) 3-(4-methylphenylsulfonyl)propionic acid phenyl ester,
(3) 3-(4-methylphenylsulfonyl)propionic acid sodium salt,
(4) 3-(4-methylphenylsulfonyl)propionic acid methyl ester,
(5) 3-(4-methylphenylsulfonyl)propionic acid ethyl ester,
(6) 3-(4-methylphenylsulfonyl)propionic acid,
(7) 3-(4-ethylphenylsulfonyl)propionic acid,
(8) 3-(4-methoxyphenylsulfonyl)propionic acid phenyl ester,
(9) 3-(4-methoxyphenylsultonyl)propionic acid,
(10) 3-(4-nitrophenylsulfonyl)propionic acid methyl ester,
(11) 3-(4-nitrophenylsulfonyl)propionic acid isopropyl ester,
(12) 3-(4-nitrophenylsulfonyl)propionic acid,
(13) 3-(4-aminophenylsulfonyl)propionic acid ethyl ester,
(14) 3-(4-aminophenylsulfonyl)propionic acid,
(15) 3-(4-hydroxyphenylsulfonyl)propionic acid,
(16) 3-(4-hydroxyphenylsulfonyl)propionic acid phenyl ester,
(17) 3-(4-bromophenylsulfonyl)propionic acid methyl ester,
(18) 3-(4-bromophenylsulfonyl)propionic acid ethyl ester,
(19) 3-(4-bromophenylsulfonyl)propionic acid phenyl ester,
(20) 3-(4-chlorophenylsulfonyl)propionic acid methyl ester,
(21) 3-(4-chlorophenylsulfonyl)propionic acid ethyl ester,
(22) 3-(4-chlorophenylsulfonyl)propionic acid t-butyl ester,
(23) 3-(4-chlorophenylsulfonyl)propionic acid isopropyl ester,
(24) 3-(4-chlorophenylsulfonyl)propionic acid,
(25) 3-(4-chlorophenylsulfonyl)propionic acid phenyl ester,
(26) 3-(4-iodophenylsulfonyl)propionic acid ethyl ester,
(27) 3-(4-iodophenylsulfonyl)propionic acid methyl ester,
(28) 3-(4-acetylaminophenylsulfonyl)propionic acid methyl ester,
(29) 3-(4-acetylaminophenylsulfonyl)propionic acid ethyl ester,
(30) 3-(4-vinylphenylsulfonyl)propionic acid sodium salt
(31) 3-(4-carboxyphenylsulfonyl)propionic acid,
(32) 3-(4-cyanophenylsulfonyl)propionic acid ethyl ester,
(33) 3-(4-formylphenylsulfonyl)propionic acid ethyl ester,
(34) 3-(4-biphenylsulfonyl)propionic acid methyl ester,
(35) 2-amino-3-(2-methylphenylsulfonyl)propionic acid,
(36) 2-amino-3-(3-methylphenylsulfonyl)propionic acid,
(37) 2-amino-3-(4-methylphenylsulfonyl)propionic acid,
(38) 2-amino-3-(4-fluorophenylsulfonyl)propionic acid,
(39) 2-t-butoxycarbonylamino-3-(4-fluorophenylsulfonyl)propionic acid,
(40) 2-amino-3-(4-chlorophenylsulfonyl)propionic acid,
(41) 2-t-butoxycarbonylamino-3-(4-chlorophenylsulfonyl)propionic acid,
(42) 2-amino-3-(3-trifluoromethylphenylsulfonyl)propionic acid,
(43) 2-amino-3-(4-nitrophenylsulfonyl)propionic acid,
(44) 2-amino-3-(2-nitrophenylsulfonyl)propionic acid,
(45) 2-amino-3-(4-aminophenylsulfonyl)propionic acid,
(46) 2-amino-3-(2-aminophenylsulfonyl)propionic acid,
(47) 2,2-dimethyl-3-(4-hydroxyphenylthio)propionic acid,
(48) 4-(2-carboxy-2-methylpropylmercapto)phenyl **2-phenylbutyrate,**
(49) 4-(2-carboxy-2-methylpropylsulfinyl)phenyl **2-phenylbutyrate,**
(50) 4-(2-carboxy-2-methylpropylsulfonyl)phenyl **2-phenylbutyrate,**
(51) 4-(2-carboxy-2-methylpropylmercapto)phenyl 2-(4-**methoxyphenyl)isobutyrate,**
(52) 4-(2-carboxy-2-methylpropylmercapto)phenyl 2-(3,4-diethylphenyl)isobutyric acid,
(53) 4-(2-carboxy-2-methylpropylmercapto)phenyl 2-(1,2,3,4-tetrahydro-6-naphthyl)butyrate,
(54) 4-(2-carboxy-2-methylpropylmercapto)phenyl 2-(1-methyl-2-pyrrole)butyrate,
(55) 4-(2-carboxy-2-methylpropylsulfinyl)phenyl 2-(1-methyl-2-pyrrole)butyrate,
(56) 3-(4-bromophenylthio)propionic acid,
(57) N-t-butoxy-3-(4-bromophenylthio)propionamide,
(58) N-t-butoxy-3-(4-biphenylthio)propionamide.)
, or non-toxic salts thereof.

3. A compound according to claim 2, in which R² is -COOR⁷.

4. A compound according to claim 2, in which R² is -CONHOR⁸.

5. A compound according to claim 3 or claim 4, in which A is hydrogen, C1-8 alkyl, or C1-4 alkyl substituted by hydroxy, or A, J and E taken together, represent methyl, halogen, trifluoromethyl, nitro, cyano, formyl, hydroxy, or NR¹⁶R¹⁷.

6. A compound according to claim 3 or claim 4, in which A is carbocyclic ring optionally substituted by substituents, or A, J and E taken together, represent phenyl.

7. A compound according to claim 3 or claim 4, in which A is heterocyclic ring optionally substituted by substituents, or A, J and E taken together, represent heterocyclic ring.

8. A compound according to claim 5, which is
(1) 3-(4-aminophenylthio)propionic acid t-butyl ester,
(2) 3-(4-hydroxyphenylthio)propionic acid t-butyl ester,
(3) 2-methyl-3-(4-hydroxyphenylthio)propionic acid t-butyl ester,
(4) 2-benzyl-3-(4-bromophenylthio)propionic acid t-butyl ester,
(5) 3-(4-methoxyphenylthio)propionic acid t-butyl ester,
(6) 3-(4-bromophenylsulfonyl)propionic acid t-butyl ester,
(7) 2-benzyl-3-(4-bromophenylsulfonyl)propionic acid t-butyl ester,
(8) 3-[4-(1-heptynyl)phenylsulfonyl]propionic acid t-butyl ester,
(9) 3-[4-(1-heptynyl)phenylsulfonyl]propionic acid,
(10) 3-(4-methoxyphenylthio)propionic acid,
(11) N-t-butoxy-3-(4-methoxyphenylthio)propionamide,
(12) N-benzyloxy-3-(4-methoxyphenylthio)propionamide,
(13) N-benzyloxy-3-(4-methoxyphenylsulfonyl)propionamide,
(14) N-hydroxy-3-(4-methoxyphenylthio)propionamide,
(15) N-hydroxy-3-(4-methoxyphenylsulfinyl)propionamide,
(16) N-hydroxy-3-(4-methoxyphenylsulfonyl)propionamide,
(17) 2-t-butoxycarbonylamino-3-(4-methoxyphenylthio)propionic acid benzyl ester,
(18) 2-t-butoxycarbonylamino-3-(4-methoxyphenylsulfonyl)propionic acid benzyl ester,
(19) 2-t-butoxycarbonylamino-3-(4-methoxyphenylsulfonyl)propionic acid,
(20) 2-amino-3-(4-methoxyphenylsulfonyl)propionic acid benzyl ester hydrochloride,
(21) 2-[N-[N-(t-butoxycarbonyl)phenylglycyl]amino]-3-(4-methoxyphenylsulfonyl)propionic acid benzyl ester,
(22) 2-acetylamino-3-(4-methoxyphenylsulfonyl)propionic acid benzyl ester,
(23) 2-[N-[N-(t-butoxycarbony)glycyl]amino]-3-(4-methoxyphenylsulfonyl)propionic acid benzyl ester,
(24) 2-[N-[N-(t-butoxycarbonyl)phenylglycyl]amino]-3-(4-methoxyphenylsulfonyl)propionic acid,
(25) 2-acelylamino-3-(4-methoxyphenylsulfonyl)propionic acid,
(26) 2-[N-[N-(t-butoxycarbonyl)glycyl]amino]-3-(4-methoxyphenylsulfonyl)propionic acid,
(27) 2-[N-[N-(t-butoxycarbonyl)phenylglycyl-glycyl]amino]-3-(4-methoxyphenylsulfonyl)propionic acid,
(28) 2-[N-(N-acetylglycyl)amino]-3-(4-methoxyphenylsulfonyl)propionic acid,
(29) 2-[N-[N-(benzylcarbonyl)glycyl]amino]-3-(4-methoxyphenylsulfonyl)propionic acid,
(30) N-hydroxy-2-t-butoxycarbonylamino-3-(4-methoxyphenylsulfonyl)propionamide,
(31) N-hydroxy-2-[N-[N-(t-butoxycarbonyl)phenylglycyl]amino]-3-(4-methoxyphenylsulfonyl)propionamide,
(32) N-hydroxy-2-acetylamino-3-(4-methoxyphenylsulfonyl)propionamide,
(33) N-hydroxy-2-[N-[N-(t-butoxycarbonyl)glycyl]amino]-3-(4-methoxyphenylsulfonyl)propionamide,
(34) N-hydroxy-2-[N-[N-(t-butoxycarbonyl)phenylglycyl-glycyl]amino]-3-(4-methoxyphenylsulfonyl)propionamide,
(35) N-hydroxy-2-[N-(N-acetylglycyl)amino]-3-(4-methoxyphenylsulfonyl)propionamide,
(36) N-hydroxy-2-[N-[N-(benzylcarbonyl)glycyl]amino]-3-(4-methoxyphenylsulfonyl)propionamide,
(37) 2-amino-3-(4-methoxyphenylsulfonyl)propionic acid trifluoroacetic acid salt,
(38) N-hydroxy-2-amino-3-(4-methoxyphenylsulfonyl)propionamide hydrochloride,
(39) 2-[N-(phenylglycyl)amino]-3-(4-methoxyphenylsulfonyl)propionic acid trifluoroacetic acid salt,
(40) N-hydroxy-2-[N-(phenylglycyl)amino]-3-(4-methoxyphenylsulfonyl)propionamide trifluoroacetic acid salt,
(41) N-hydroxy-2-[N-(phenylglycyl-glycyl)amino]-3-(4-methoxyphenylsulfonyl)propionamide trifluoroacetic acid salt, or
(42) 3-(4-bromophenylsulfonyl)propionic acid.

9. A compound according to claim 6, which is
(1) 7(3) 3-[4-(2-pyridylethynyl)phenylsulfonyl]propionic acid t-butyl ester,
(2) 3-[4-(2-pyridylethynyl)phenylsulfonyl]propionic acid,
(3) 2-methyl-3-[4-(2-benzofuranyl)phenylsulfonyl]propionic acid, or
(4) N-hydroxy-2-methyl-3-[4-(2-benzofuranyl)phenylsulfonyl]propionamide.

10. A compound according to claim 7, which is
(1) 3-[4-(benzoylamino)phenylthio]propionic acid t-butyl ester,
(2) 3-(4-benzyloxyphenylthio)propionic acid t-butyl ester,
(3) 3-[4-(benzoylamino)phenylsulfinyl]propionic acid t-butyl ester,
(4) 3-[4-(benzoylamino)phenylsulfonyl]propionic acid t-butyl ester,
(5) 3-[4-(benzyloxy)phenylsulfonyl]propionic acid t-butyl ester,
(6) 2-methyl-3-[4-(4-tolylethynyl)phenylsulfonyl]propionic acid t-butyl ester,
(7) 3-[4-(phenylethynyl)phenylsulfonyl]propionic acid t-butyl ester,
(8) 3-[4-(4-methoxyphenylethynyl)phenylsulfonyl]propionic acid t-butyl ester,
(9) 3-[4-(4-tolylethynyl)phenylsulfonyl]propionic acid t-butyl ester,
(10) 3-[4-(benzoylamino)phenylthio]propionic acid,
(11) 3-[4-(benzoylamino)phenylsulfinyl]propionic acid,
(12) 3-[4-(benzoylamino)phenylsulfonyl]propionic acid,
(13) 3-[4-(benzyloxy)phenylsulfonyl]propionic acid,
(14) 2-methyl-3-[4-(4-tolylcarbonylmethyl)phenylsulfonyl]propionic acid,
(15) 2-methyl-3-[4-(4-tolylethynyl)phenylsulfonyl]propionic acid,
(16) 3-[4-(phenylethynyl)phenylsulfonyl]propionic acid,
(17) 3-[4-(4-methoxyphenylethynyl)phenylsulfonyl]propionic acid,
(18) 2-benzyl-3-[4-(4-tolylethynyl)phenylsulfonyl]propionic acid,
(19) 2R-hydroxy-3-(4-bromophenylsulfonyl)propionic acid t-butyl ester,
(20) 2R-hydroxy-3-[4-(4-tolylethynyl)phenylsulfonyl]propionic acid,
(21) 2S-hydroxy-3-[4-(4-tolylethynyl)phenylsulfonyl]propionic acid,
(22) 3-[4-(4-tolylethynyl)phenylsulfonyl]butyric acid,
(23) 3-[4-(4-tolylcarbonylmethyl)phenylsulfonyl]propionic acid,
(24) 3-[4-(4-tolylvinyl)phenylsulfonyl]propionic acid,
(25) 2-methyl-3-[4-(1-heptynyl)phenylsulfonyl]propionic acid,
(26) 2-methyl-3-[4-(4-hydroxy-but-1-ynyl)phenylsulfonyl]propionic acid,
(27) N-hydroxy-2-methyl-3-[4-(1-heptynyl)phenylsulfonyl)propionamide,
(28) N-hydroxy-2-methyl-3-[4-(4-hydroxy-but-1-ynyl)phenylsulfonyl]propionamide,
(29) N-hydroxy-2-methyl-3-[4-(4-tolylethynyl)phenylsulfonyl]propionamide,
(30) 3-[4-(phenylethynyl)phenylsulfinyl]propionic acid,
(31) 3-[4-(phenylethynyl)phenylthio]propionic acid,
(32) cis-3-[4-(benzoylamino)phenylsulfonyl]-2-propenoic acid, or
(33) trans-3-[4-(benzoylamino)phenylsulfonyl]-2-propenoic acid.
